# EUROPEAN PATENT APPLICATION

(11) **EP 4 585 596 A1**
(43) Date of publication of application: **16.07.2025**
(21) Application number: 23862478.7
(22) Date of filing: 07.09.2023
(51) Int. Cl.: C07D 487/04, C07D 471/04, C07D 409/12, C07D 498/04, C07D 519/00, C07D 471/14, C07D 513/04, C07D 221/14, A61K 31/519, A61K 31/473, A61P 35/00

(54) **NITROGEN-CONTAINING HETEROCYCLIC COMPOUND, PHARMACEUTICAL COMPOSITION THEREOF, AND USE THEREOF**

(30) Priority: 09.09.2022 CN 202211102124; 10.02.2023 CN 202310108466
(71) Applicant: Shanghai Yingli Pharmaceutical Co., Ltd, Shanghai 201203 (CN)
(72) Inventor: XU, Zusheng, Shanghai 201201 (CN); XIA, Lin, Shanghai 201203 (CN); LI, Xiangqian, Shanghai 201203 (CN); YANG, Haoran, Shanghai 201203 (CN)
(74) Representative: Acapo AS
(86) International application number: PCT/CN2023/117514
(87) International publication number: WO 2024/051784

(57) **Abstract**

Disclosed is a nitrogen-containing heterocyclic compound, pharmaceutical compositions thereof and use thereof. The present disclosure provides a nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof. The nitrogen-containing heterocyclic compound represented by formula I is expected to treat and/or prevent various PI3K -meditated diseases.

## Description

The present disclosure claims the priority to the Chinese patent application with the filing No. 202211102124.1 on September 9, 2022 and the Chinese patent application with the filing No. 202310108466.2 on February 10, 2023, the contents of which are incorporated herein by reference in entirety.

### Technical field

The present disclosure relates to a nitrogen-containing heterocyclic compound, pharmaceutical compositions thereof and use thereof

### Background

Cancer has become a frequent and common disease that poses a serious threat to human health and life. PI3K/AKt/mTOR signaling pathway is one of the many mechanisms that regulate the cell cycle and apoptosis, and dysregulation of one component of the pathway can lead to tumor development. Receptor tyrosine kinase (RTK) regulates the activation of the PI3K/AKt signaling pathway in response to activation of growth factors, including Insulin-like Growth Factors (IGF), Epidermal Growth Factor (EGF) and Epidermal Growth Factor (EGF). These growth factors, including Insulin-like Growth Factors (IGF), Epidermal Growth Factor (EGF), and Hepatocyte growth factor (HGF), activate RTK by phosphorylating tyrosine residues, while PI3K binds to phosphorylated tyrosine residues to activate the catalytic subunit of PI3K. p110α, the catalytic subunit of type IA PI3K, is activated and then activates PI3K by binding to p85α. After activation of the catalytic subunit of type IA PI3K, p110α activates PI3K by binding to p85α, which further phosphorylates phosphatidylinositol 4,5-bisphosphate (PIP2) to phosphatidylinositol 3,4,5-trisphosphate (PIP3). PIP3, as an important second messenger and mediator, recruits AKt from the cytoplasm to the membrane through its interactions with the PH domains of AKt. Mediates the membrane translocation of AKt, phosphatidylinositol-dependent kinase 1 (PDK1) and phosphatidylinositol-dependent kinase 2 (PDK2), respectively. Phosphorylation of Thr308 and Ser473 by PDK1 and phosphatidylinositol-dependent kinase 2 (PDK2), respectively, is required for AKt activation. Fully activated AKt regulates cell proliferation and apoptosis through its upstream and downstream regulation. Dysregulation of the PI3K signaling pathway is involved in almost all human cancers.PIK3CA (encoding the PI3K catalytic subunit α) is one of the most commonly mutated oncogenes in human tumors. Mutations in the oncogene PIK3CA have been found in about 2-5% of human solid tumors, with mutation rates of about 32%, 27%, 25%, 8%, and 4% in colon, glioblastoma, gastric, breast, and lung cancers, respectively, and 11% in esophageal squamous carcinoma and 6% in esophageal adenocarcinoma, among other gastrointestinal tumors.

### Summary

According to various embodiments, a nitrogen-containing heterocyclic compound, pharmaceutical compositions thereof and use thereof are provided.

The disclosure provides a nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof: 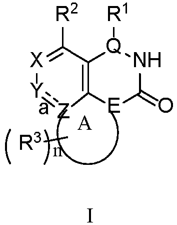 Wherein,
Z is N or C;
E is N, C or CH;
X is CH, CR^{X} or N;
Y is -C(O), CR^{Y} or N;
Q is CH or N;
a is a single bond or a double bond;
when a is a single bond, Y is -C(O)- or N;
when a is a double bond, Y is CR^{Y} or N;
R¹ is-L¹-R^{1A}; L¹ is a bond, R^{1A} is C₆₋₂₀ aryl , C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
R² is -L²-R^{2A}; L² is a bond,-NH-,-NH-C(O)-or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3}, 5- to 6- membered heteroaryl containing 1-4 heteroatoms independently selected from O, S and N substituted with one or more R^{2C4} or C₈₋₁₀ benzocycloalkenyl substituted with one or more R^{2C5}; R^{2C1}, R^{2C2}, R^{2C3}, R^{2C4} and R^{2C5} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
R³ is -L³-R^{3A}; L³ is a bond, R^{3A} is halogen, C₁₋₆ alkyl, =O or C₁₋₆ alkyl substituted with one or more R^{3C} , or, two optional R³ together with the atom they are attached form C₃₋₈ cycloalkane or C₆₋₂₀ aromatic ring;
R^{3C} is independently deuterium, halogen or-NR^{3C1}R^{3C2};
R^{3c1} and R^{3C2}are independently hydrogen or C₁₋₆ alkyl
R^{Y} and R^{X} are independently -L^{Y}-R^{YA}; L^{Y} is a bond,-NH-or -NH-C(O)-; R^{YA} is hydrogen, halogen, cyano, -N₃, hydroxyl,-NH₂, C₂₋₆ alkynyl, 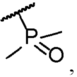 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N , 4- to 10-membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{Y44} ,-COR ^{YA5}, 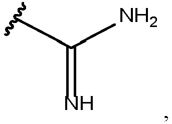 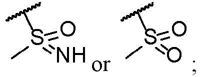
R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11} ; R^{YA11} is independently halogen or phenyl;
ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
n is 0, 1, 2, 3 or 4.

In a certain embodiment, with regard to the nitrogen-containing heterocyclic compound represented by formula I, a pharmaceutically acceptable salt thereof, a stereoisomer thereof, an isomer thereof or an isotopic compound thereof, some groups are as defined as follows, and the unmentioned group definitions are as described in any one of the embodiments of the present disclosure (this content is hereinafter referred to simply as "in a certain embodiment").

In a certain embodiment, the nitrogen-containing heterocyclic compound represented by formula I, a pharmaceutically acceptable salt thereof, a stereoisomer thereof, an isomer thereof or an isotopic compound thereof, the nitrogen-containing heterocyclic compound represented by formula I having the structure as represented by formula II: 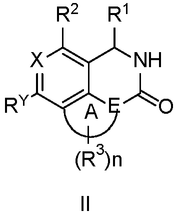 Wherein, E is N, CH or C;
X is N or CH;
R¹ is-L¹-R^{1A}; L¹ is a bond, R^{1A} is C₆₋₂₀ aryl, C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
R² is -L²-R^{2A}; L² is a bond,-NH-,-NH-C(O)- or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3}, 5- to 6- membered heteroaryl containing 1-4 heteroatoms independently selected from O, S and N substituted with one or more R^{2C4} or C₈₋₁₀ benzocycloalkenyl substituted with one or more R^{2C5}; R^{2C1}, R^{2C2}, R^{2C3}, R^{2C4} and R^{2C5} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
R³ is -L³-R^{3A}; L³ is a bond, R^{3A} is halogen, C₁₋₆ alkyl, =O or C₁₋₆ alkyl substituted with one or more R^{3C} , or, two optional R³ together with the atom they are attached form C₃₋₈ cycloalkane or C₆₋₂₀ aromatic ring;
R^{3C} is independently deuterium, halogen or -NR^{3C1}R^{3C2};
R^{3C1} and R^{3C2} are independently hydrogen or C₁₋₆ alkyl
R^{Y} is -L^{Y}-R^{YA}; L^{Y} is a bond,-NH-or -NH-C(O)-; R^{YA} is hydrogen, halogen, cyano, -N₃, hydroxyl, -NH₂, -C₂₋₆ alkynyl, 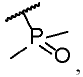 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{Y44} ,-COR ^{YA5}, 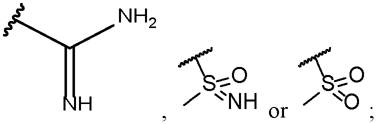
R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
n is 0, 1 or 2.

In a certain embodiment, the nitrogen-containing heterocyclic compound represented by formula II, a pharmaceutically acceptable salt thereof, a stereoisomer thereof, an isomer thereof or an isotopic compound thereof, wherein, the nitrogen-containing heterocyclic compound represented by formula II is defined as solution 1, solution 2 or solution 3:
solution 1:
   E is N or C;
   X is CH;
   R¹ is-L¹-R^{1A}; L¹ is a bond, R^{1A} is C₆₋₂₀ aryl, C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
   R² is -L²-R^{2A}; L² is a bond, -NH- or -NH-C(O)-, R^{2A} is 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{2C1}, C₆₋₂₀ aryl substituted with one or more R^{2C2} or C₈₋₁₀ benzoheterocycloalkenyl containing 1-3 heteroatoms independently selected from O, S and N substituted with one or more R^{2C3}; R^{2C1}, R^{2C2} and R^{2C3} are independently halogen, hydroxyl or C₁₋₆ alkyl substituted with one or more R^{2C11}; R^{2C11} is independently halogen;
   R³ is -L³-R^{3A}; L³ is a bond, R^{3A} is C₁₋₆ alkyl or C₁₋₆ alkyl substituted with one or more R^{3C};
   R^{3C} is independently deuterium, halogen or -NR^{3C1}R^{3C2};
   R^{3C1} and R^{3C2} are independently hydrogen or C₁₋₆ alkyl;
   R^{Y} is -L^{Y}-R^{YA}; L^{Y} is a bond, -NH- or -NH-C(O)-; R^{YA} is hydrogen, halogen, cyano, - N₃, hydroxyl, -NH₂, -C₂₋₆ alkynyl, 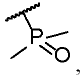 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3}, 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} ,-COR ^{YA5} or 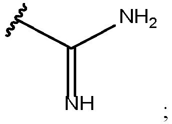
   R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
   ring A is C₆₋₁₀ aromatic ring, 4- to 10- membered cyclic olefin containing 1-3 heteroatoms independently selected from O, S and N or 5- to 12- membered heteroaromatic ring containing 1-3 heteroatoms independently selected from O, S and N;
   n is 0, 1 or 2.
solution 2:
   E is N, CH or C;
   X is N or CH;
   R¹ is -L¹-R^{1A}; L¹ is a bond, R^{1A} is C₆₋₂₀ aryl or C₆₋₂₀ aryl substituted with one or more R^{1C}; R^{1C} is independently halogen, C₁₋₆ alkyl or C₁₋₆ alkyl substituted with halogen;
   R² is -L²-R^{2A}; L² is a bond,-NH-,-NH-C(O)- or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3} or 5- to 6- membered heteroaryl containing 1-4 heteroatoms independently selected from O, S and N substituted with one or more R^{2C4}; R^{2C1}, R^{2C2}, R^{2C3} and R^{2C4} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
   R³ is -L³-R^{3A}; L³ is a bond, R^{3A} is halogen or =O, or, two optional R³ together with the atom they are attached form C₃₋₈ cycloalkane or C₆₋₂₀ aromatic ring;
   R^{Y} is -L^{Y}-R^{YA}; L^{Y} is a bond,-NH-or -NH-C(O)-; R^{YA} is hydrogen, halogen, cyano, -N₃, hydroxyl, -NH₂, 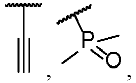 -B(OH)₂, -COOH, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} , -COR ^{YA5}, 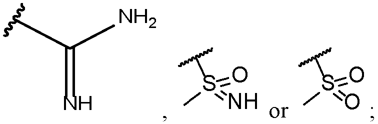
   R^{YA1}, R^{YA2}, R^{YA3}, R^{YA4} and R^{YA5} are independently halogen, hydroxyl, cyano, -NH₂, C₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
   ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
   n is 0, 1 or 2.
solution 3:
   E is N or C;
   X is CH;
   R¹ is -L¹-R^{1A}; L¹ is a bond, R^{1A} is C₆₋₂₀ aryl or C₆₋₂₀ aryl substituted with one or more R^{1C}; R^{1C} is independently halogen or C₁₋₆;
   R² is -L²-R^{2A}; L² is a bond, -NH- or -NH-C(O)-, R^{2A} is 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{2C1}, C₆₋₂₀ aryl substituted with one or more R^{2C2} or C₈₋₁₀ benzoheterocycloalkenyl containing 1-3 heteroatoms independently selected from O, S and N substituted with one or more R^{2C3}; R^{2C1}, R^{2C2} and R^{2C3} are independently halogen, hydroxyl or C₁₋₆ alkyl substituted with one or more R^{2C11}; R^{2C11} is independently halogen;
   n is 0;
   R^{Y} is -L^{Y}-R^{YA}; L^{Y} is a bond,-NH-or -NH-C(O)-; R^{YA} is hydrogen, halogen, cyano, -N₃, hydroxyl, 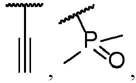 -B(OH)₂, -COOH, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{Y44} , -COR ^{YA5} or 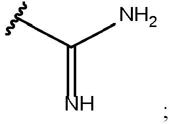
   R^{YA1}, R^{YA2}, R^{YA3}, R^{YA4} and R^{YA5} are independently halogen, hydroxyl, cyano, -NH₂, C₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
   ring A is C₆₋₁₀ aromatic ring, 4- to 10- membered cyclic olefin containing 1-3 heteroatoms independently selected from O, S and N or 5- to 12- membered heteroaromatic ring containing 1-3 heteroatoms independently selected from O, S and N.

In a certain embodiment, X is N or CH.

In a certain embodiment, when the definition of R¹, R^{1C}, R^{1D}, R^{2C1}, R^{2C2}, R^{2C3}, R^{2C4}, R^{2C5}, R^{2C11}, R^{3A}, R^{3C}, R^{YA}, R^{YA1}, R^{YA2}, R^{YA3}, R^{YA4}, R^{YA5} and R^{YA11} refers to halogen, the halogen is fluorine, chlorine, bromine or iodine.

In a certain embodiment, when the definition of R^{2C1}, R^{2C2}, R^{2C3}, R^{2C4}, R^{2C5}, R^{YA}, R^{YA1}, R^{YA2}, R^{YA3}, R^{YA4} and R^{YA5} refers to C₃₋₈ cycloalkyl, the C₃₋₈ cycloalkyl is cyclohexyl, - cyclopentyl, cyclobutyl or cyclopropyl, for example, cyclopropyl

In a certain embodiment, when the definition of R^{1C}, R^{1D}, R^{2C1}, R^{2C2}, R^{2C3}, R^{2C4}, R^{2C5}, R^{3A}, R^{3C1}, R^{3C2}, R^{YA} R^{YA1}, R^{YA2}, R^{YA3}, R^{YA4} and R^{YA5} refers to C₁₋₆ alkyl or -OC₁₋₆ alkyl, the C₁₋₆ alkyl is C₁₋₄ alkyl, for example, methyl, ethyl, n-propyl, isopropyl, n-butyl, sec-butyl, isobutyl or tert-butyl.

In a certain embodiment, when the definition of R^{1A}, R^{2A}, R^{YA1}, R^{YA2}, R^{YA3}, R^{YA4} andR^{YA5} refers to C₆₋₂₀ aryl, the C₆₋₂₀ aryl is phenyl or naphthyl.

In a certain embodiment, when R^{2A}and R^{YA} are independently "5- to 6- membered heteroaryl containing 1-4 heteroatoms independently selected from O, S and N", the"5- to 6-membered heteroaryl containing 1-4 heteroatoms independently selected from O, S and N" is"5- to 6- membered heteroaryl containing 1-4 heteroatoms independently selected from O and N", for example, 1H-Tetrazolyl 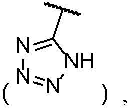 pyrazolyl 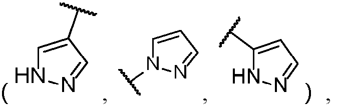 pyridinyl 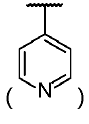 or oxazolyl

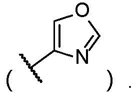

In a certain embodiment, when R^{2A} and R^{YA} are independently 8- to 10- membered bicyclic heteroaryl containing 1-3 heteroatoms independently selected from O, S and N, the "8-to 10- membered bicyclic heteroaryl containing 1-3 heteroatoms independently selected from O, S and N" is [1,2,4]-Triazolo[1,5-a]pyridinyl ( 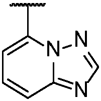 ), indazolyl ( 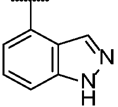 ), indolyl, isoquinolinyl, Benzothienyl or benzimidazolyl ( 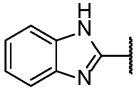 ).

In a certain embodiment, a is a double bond, Y is CR^{Y}, E is N, C or CH.

In a certain embodiment, X is N or CH.

In a certain embodiment, Z is C.

In a certain embodiment, E is N or C.

In a certain embodiment, when R^{1A} is "5- to 12-membered heteroaryl containing 1 to 4 heteroatoms independently selected from O, S and N" substituted with one or more R^{1D} , the R^{1A} is "5- to 6-membered heteroaryl containing 1 to 2 heteroatoms independently are N" substituted with one or more R^{1D}, for example, pyridinyl substituted with one or more R^{1D}.

In a certain embodiment, when R^{2A} is 8- to 10- membered bicyclic heteroaryl containing 1-3 heteroatoms independently selected from O, S and N, or "8- to 10- membered bicyclic heteroaryl containing 1-3 heteroatoms independently selected from O, S and N" substituted with one or more R^{2C1}, the "8- to 10- membered bicyclic heteroaryl containing 1-3 heteroatoms independently selected from O, S and N" is "9- to 10- membered bicyclic heteroaryl containing 1-2 heteroatoms being N", for example, benzothienyl, indolyl, quinolinyl, isoquinolinyl, benzimidazolyl or benzothiazolyl.

In a certain embodiment, when R^{2A} is C₈₋₁₀ benzoheterocycloalkenyl containing 1-3 heteroatoms independently selected from O, S and N, the "C₈₋₁₀ benzoheterocycloalkenyl containing 1-3 heteroatoms independently selected from O, S and N" is 8- to 10- membered benzoheterocycloalkenyl containing 1-2 N, preferably indolinyl, for example,

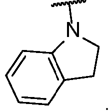

In a certain embodiment, when R^{2A} is "5- to 12-membered heteroaryl containing 1 to 4 heteroatoms independently selected from O, S and N" substituted with one or more R^{2C3}, the "5- to 12-membered heteroaryl containing 1 to 4 heteroatoms independently selected from O, S and N" is"5- to 6-membered heteroaryl containing 1 to 2 heteroatoms independently are N", for example, pyridinyl.

In a certain embodiment, when R^{2A} is C₈₋₁₀ benzocycloalkenyl, the C₈₋₁₀ benzocycloalkenyl is C₉₋₁₀ benzocycloalkenyl, for example, indanyl.

In a certain embodiment, when L^{Y} is -NH-C(O)-, the end C is connected with R^{YA}.

In a certain embodiment, when R^{YA} is "4- to 10- membered cyclic olefin containing 1-3 heteroatoms independently selected from O, S and N" or "4- to 10- membered cyclic olefin containing 1-3 heteroatoms independently selected from O, S and N" substituted with R^{YA2} , the "4- to 10- membered cyclic olefin containing 1-3 heteroatoms independently selected from O, S and N" is "4- to 6- membered cyclic olefin containing 1-3 heteroatoms independently selected from O and N", for example, Oxybutyl, azetidinyl, pyranyl or morpholinyl.

In a certain embodiment, when R^{YA} is "5- to 6-membered heteroaryl containing 1 to 4 heteroatoms independently selected from O, S and N" or "5- to 12-membered heteroaryl containing 1 to 4 heteroatoms independently selected from O, S and N" substituted with one or more R^{YA3}, the "5- to 6-membered heteroaryl containing 1 to 4 heteroatoms independently selected from O, S and N" is "5- to 6-membered heteroaryl containing 1 to 4 heteroatoms independently selected from O and N", for example, pyridinyl, pyrimidinyl, pyrazolyl, tetrazolyl, triazolyl, pyrrolyl, imidazolyl or oxazolyl.

In a certain embodiment, when R^{YA} is "8- to 10- membered bicyclic heteroaryl containing 1-3 heteroatoms independently selected from O, S and N", or "8- to 10- membered bicyclic heteroaryl containing 1-3 heteroatoms independently selected from O, S and N" substituted with one or more R^{YA4}, the "8- to 10- membered bicyclic heteroaryl containing 1-3 heteroatoms independently selected from O, S and N" is "9- to 10- membered bicyclic heteroaryl containing 1-3 heteroatoms N", for example, indazolyl, imidazo[1,2-a]pyridinyl, 3H-imidazo[4,5-b]pyridinyl, imidazo[1,2-a]pyridinyl, 5H-pyrrolo[3,2-D]pyrimidinyl, 7-azaindolyl or [1,2,4]triazolo[1,5-a]pyridinyl.

In a certain embodiment, when R^{YA1}, R^{YA2}, R^{YA3}, R^{YA4} and R^{YA5}are independently C₁₋₆ alkyl substituted with one or more R^{YA11}, R^{YA11} is halogen, the C₁₋₆ alkyl substituted with one or more R^{YA11} is C₁₋₂ alkyl substituted with one or more halogen, for example, Trifluoromethyl or difluoromethyl.

In a certain embodiment, R¹ is 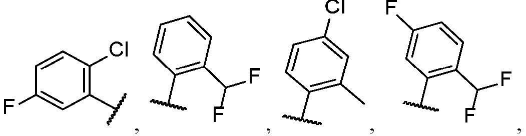 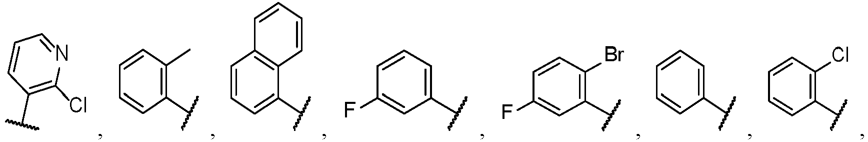 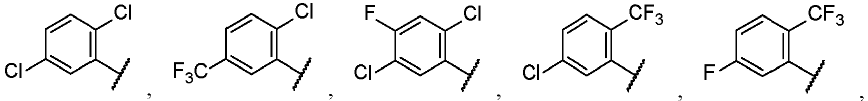 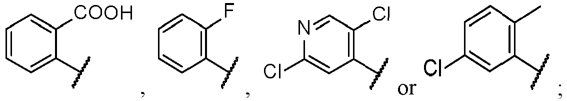 for example,

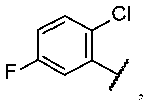

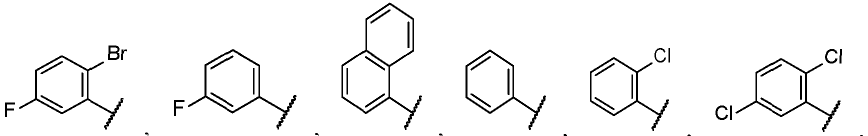

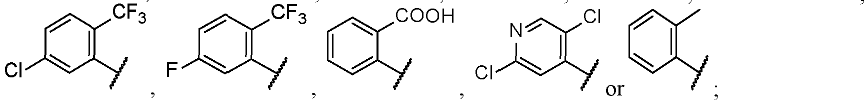

In a certain embodiment, R² is 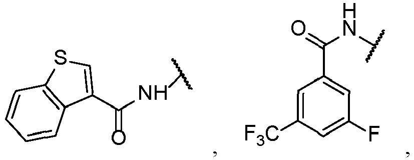 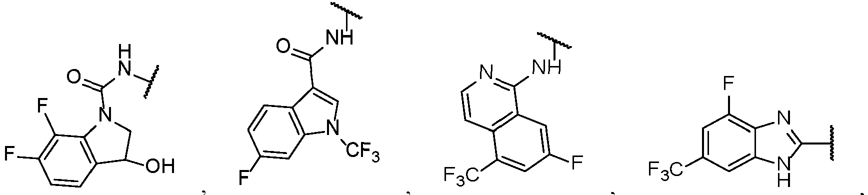 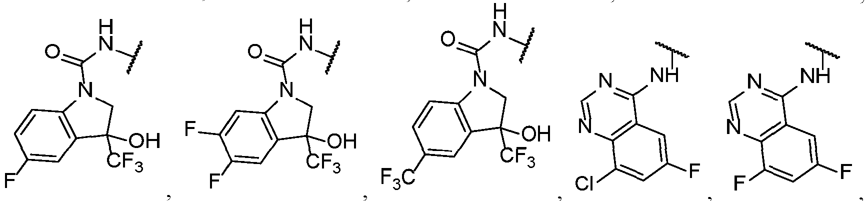 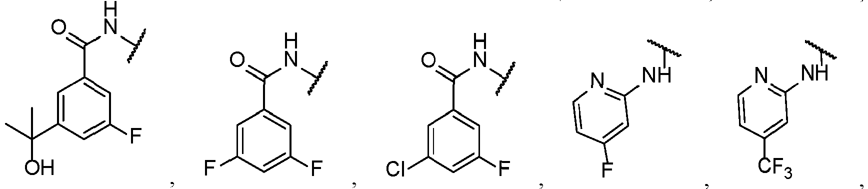 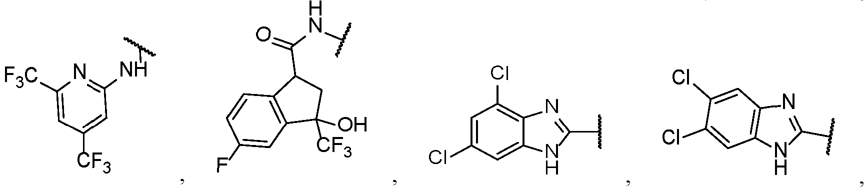 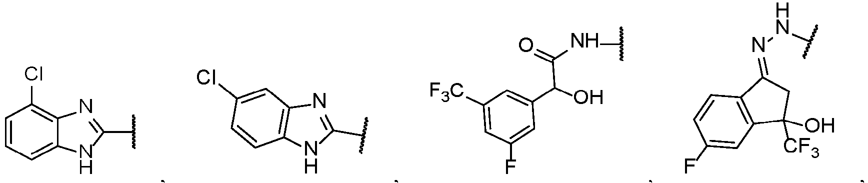 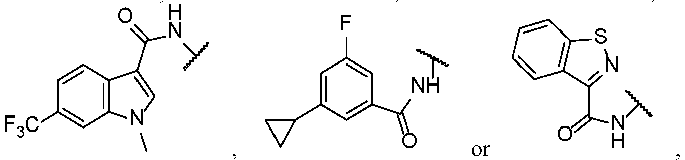 for example, 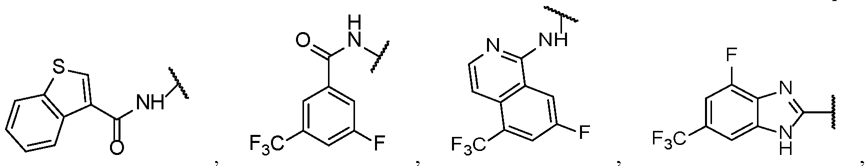 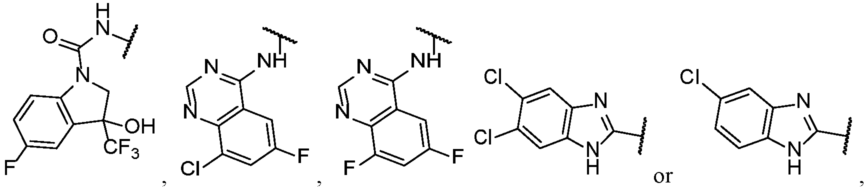 for another example,

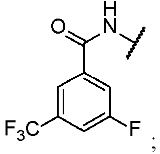

In a certain embodiment, R^{Y} is hydrogen, -CN, -N₃, -F, Br, -OH, -NH₂, -B(OH)₂, - COOH, -CONH₂, -COOMe, -CH₃, -OCH₃,

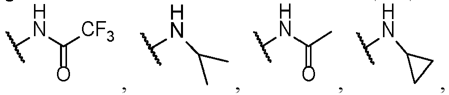

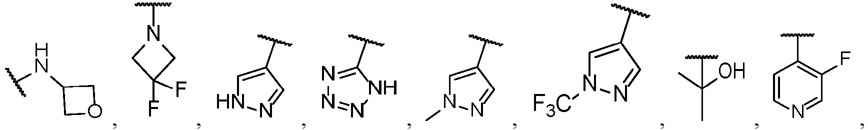

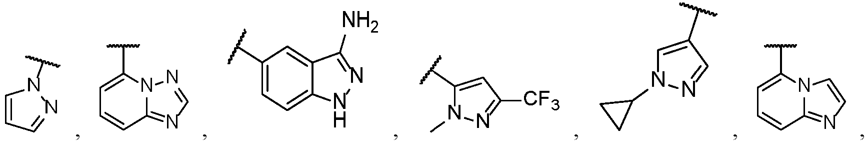

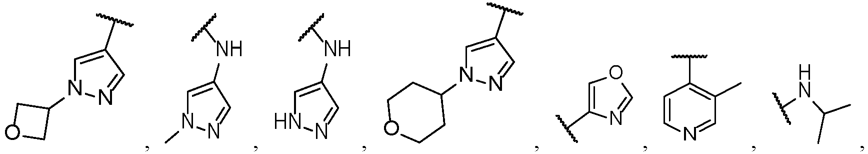

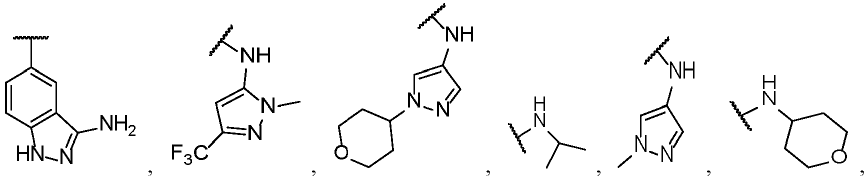

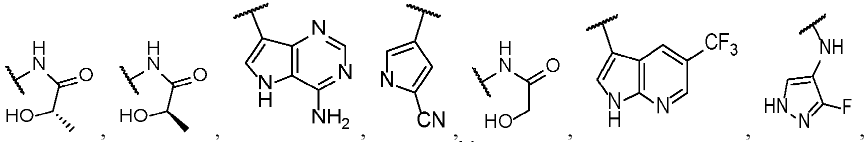

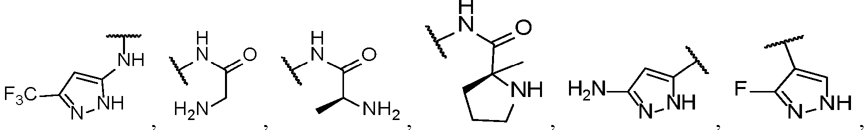

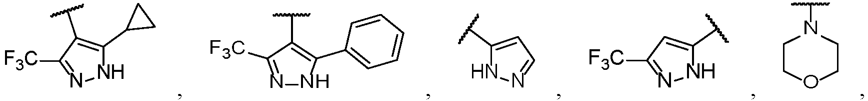

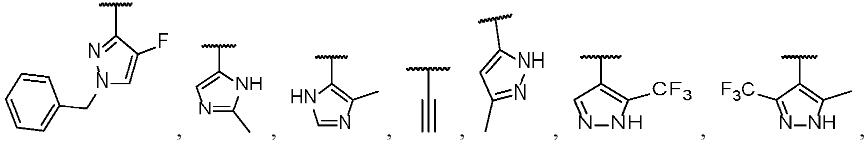

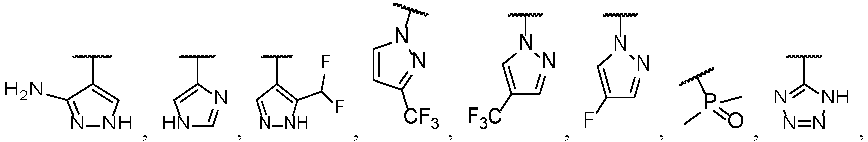

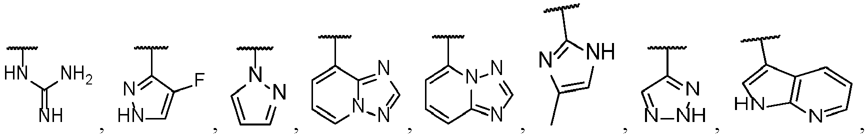

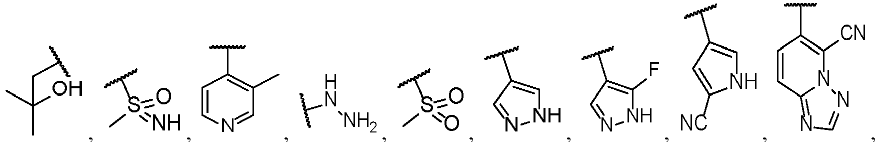

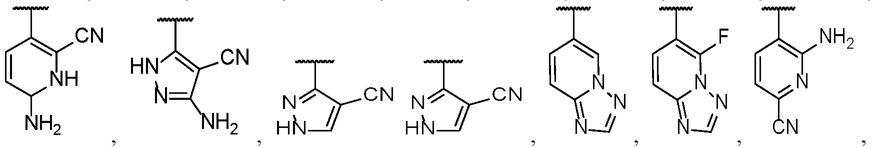

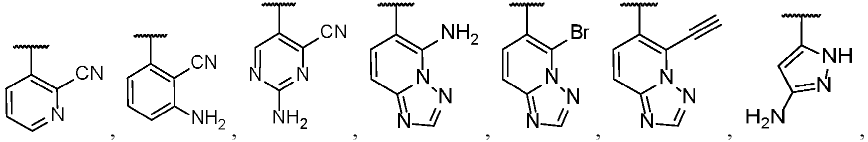

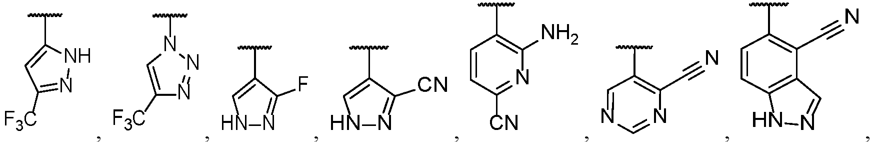

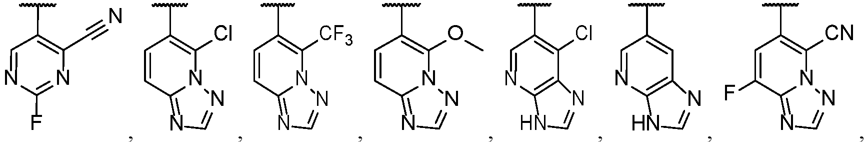

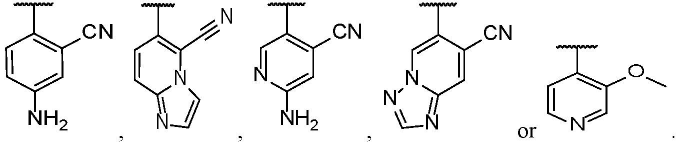

In a certain embodiment, R³ is F, -Me, -Et, -CD₃, 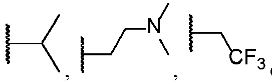 or =O, two optional R³ together with the atom they are attached form Cyclopropane or benzene ring.

In a certain embodiment, n is 0, 1 or 2.

In a certain embodiment, Q is CH.

In a certain embodiment, X is CH, Y is CR^{Y}.

In a certain embodiment, 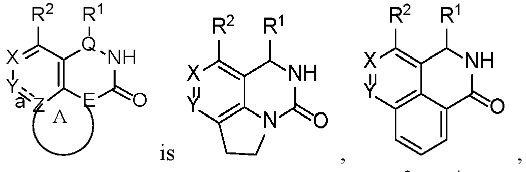 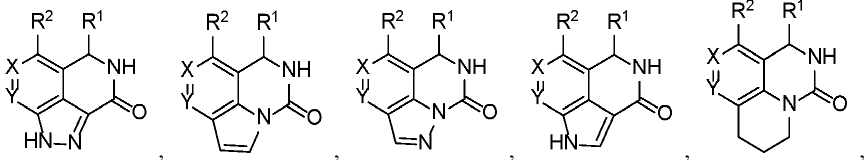 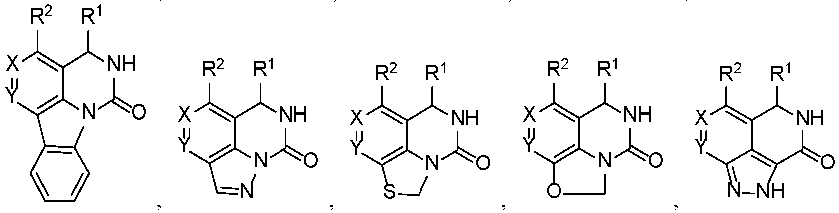 or 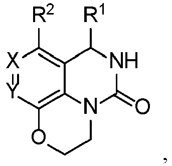 for example, 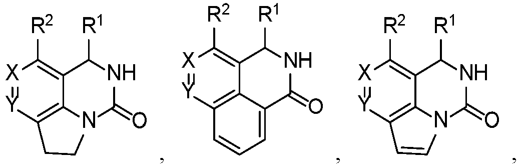 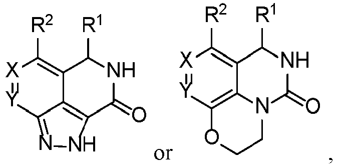 the groups in the formula are defined as previously described.

In a certain embodiment, 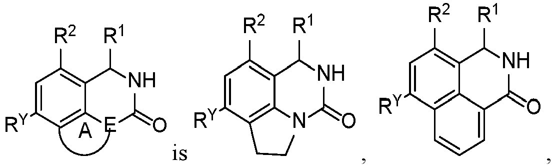 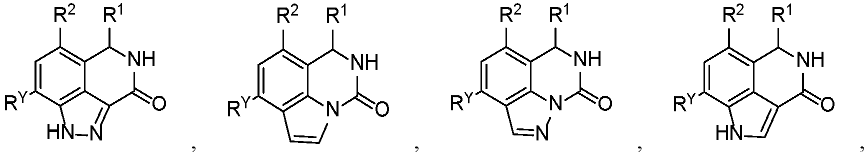 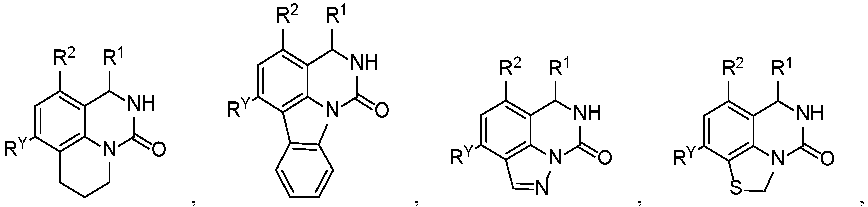 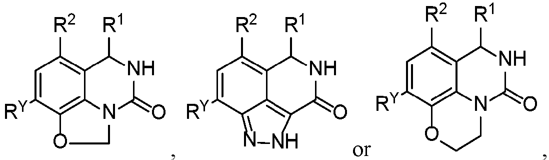 for example, 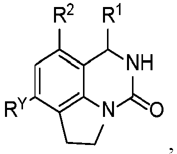 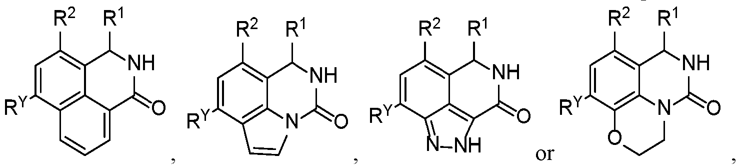 the groups in the formula are defined as previously described.

In a certain embodiment, 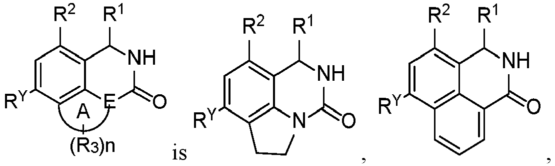 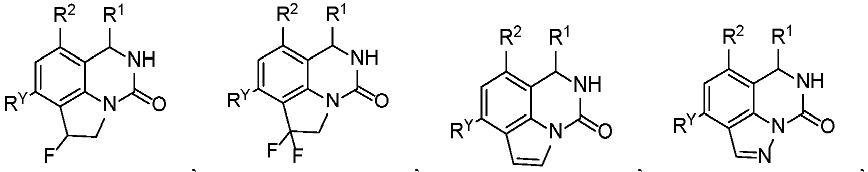 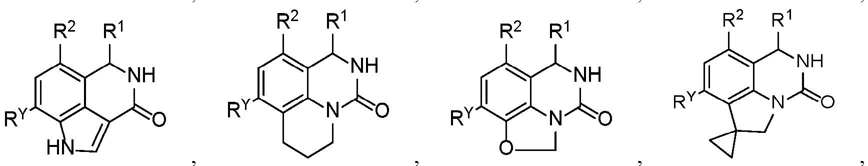 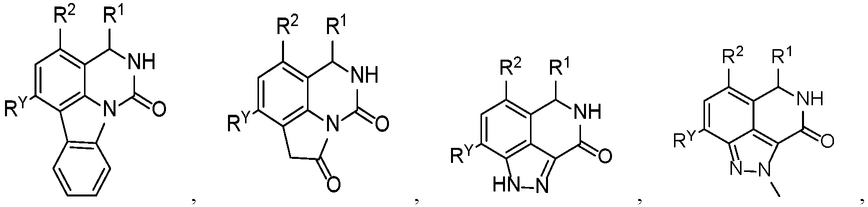 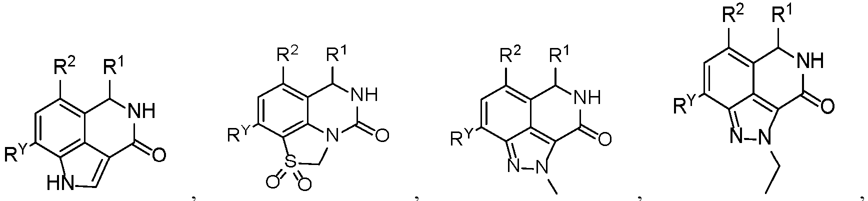 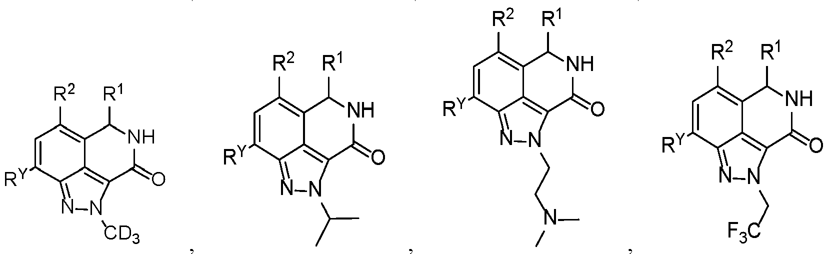 or 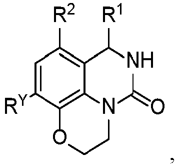 the groups in the formula are defined as previously described.

In a certain embodiment, 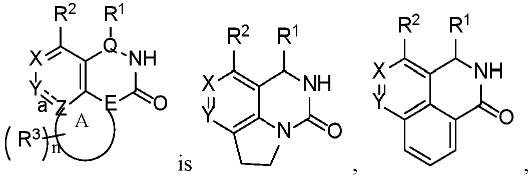 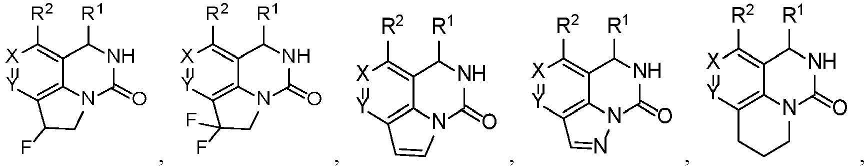 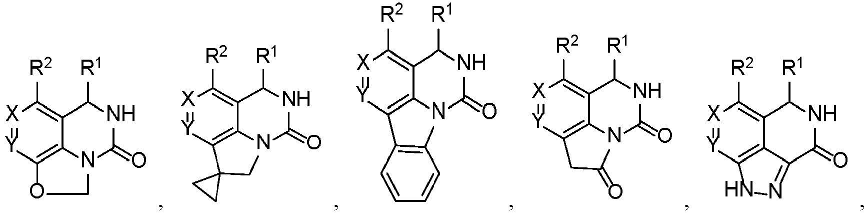 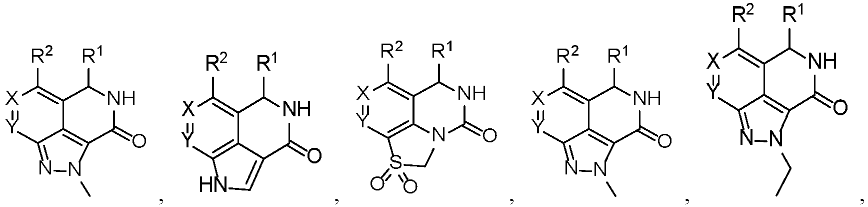 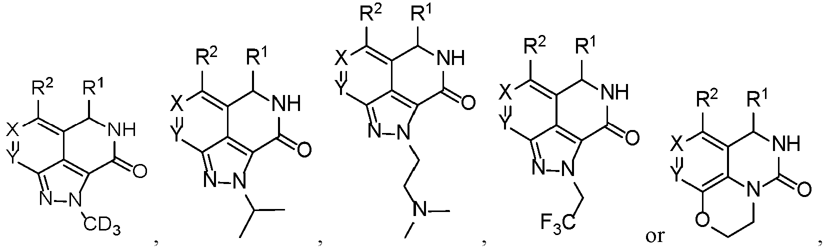 the groups in the formula are defined as previously described.

In a certain embodiment, the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof, wherein, the nitrogen-containing heterocyclic compound represented by formula I is any structure of the following compounds:

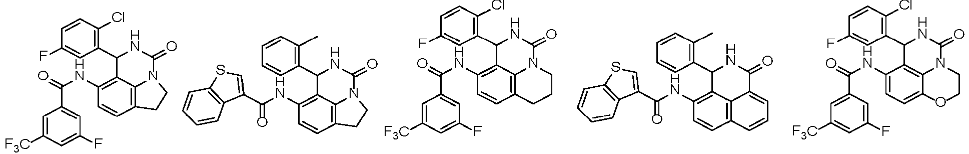

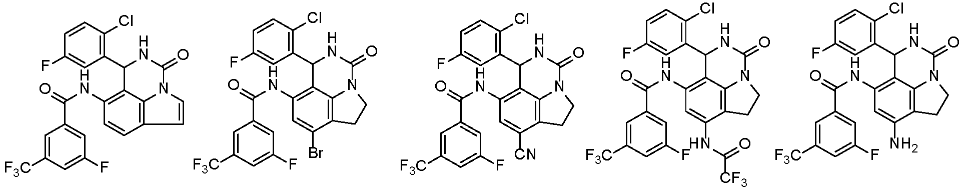

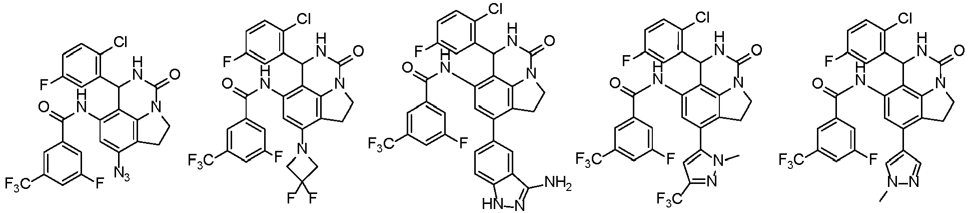

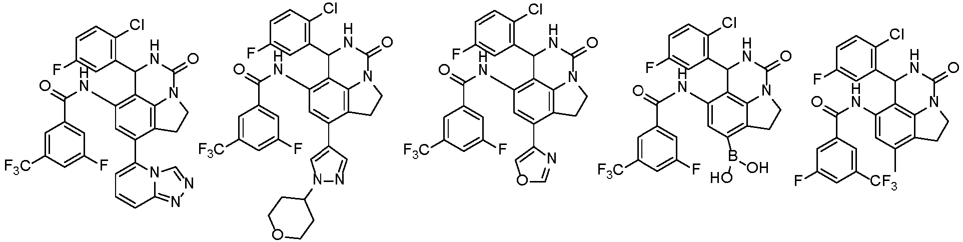

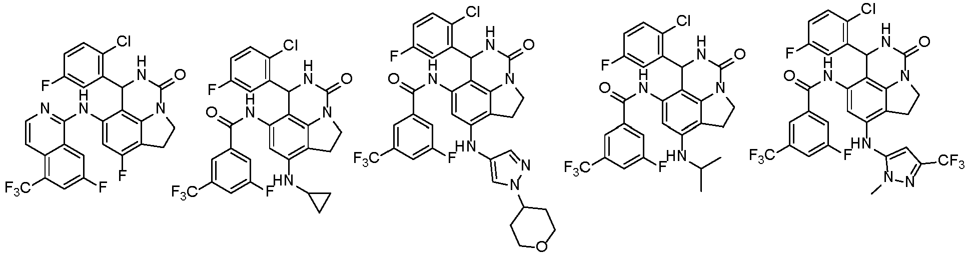

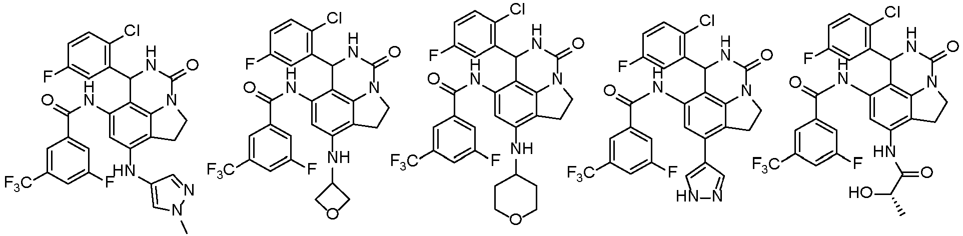

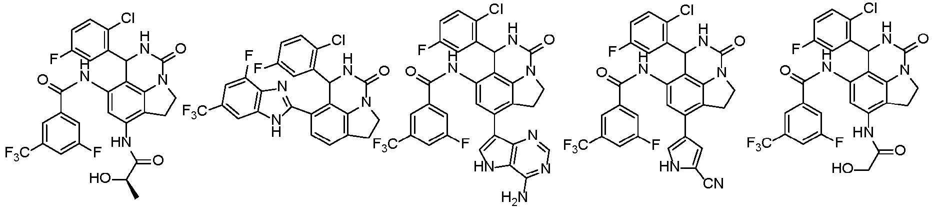

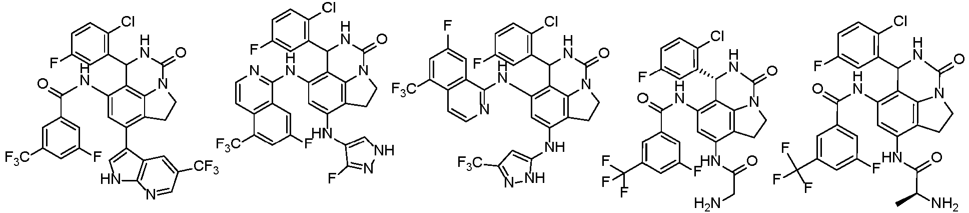

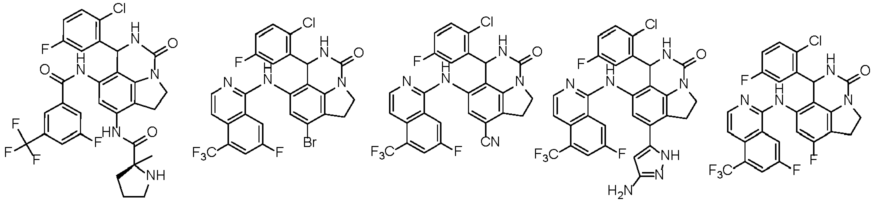

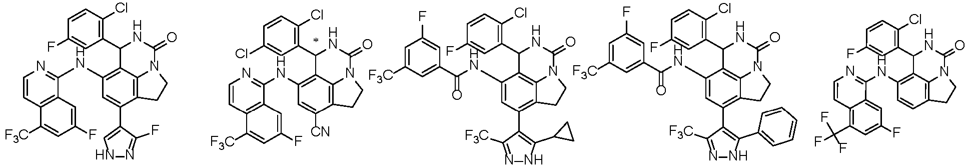

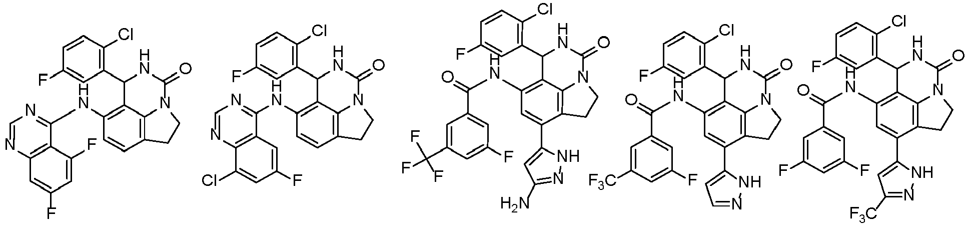

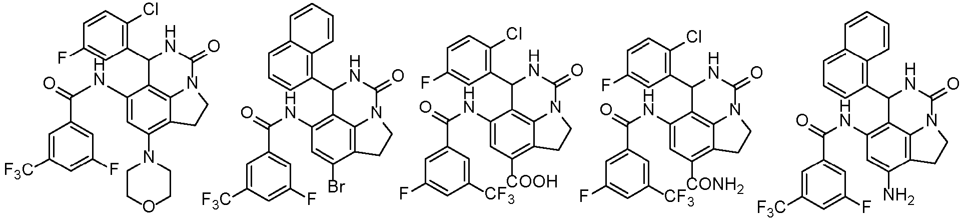

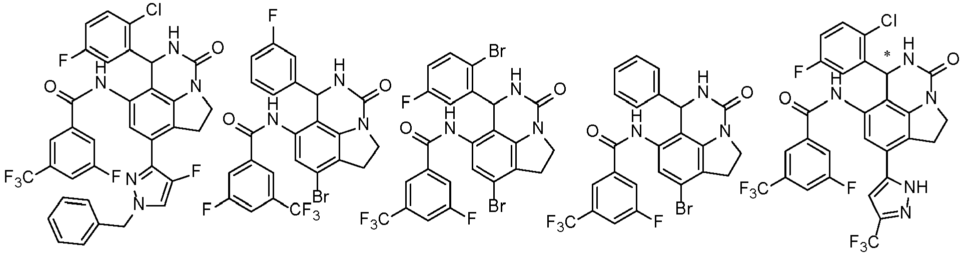

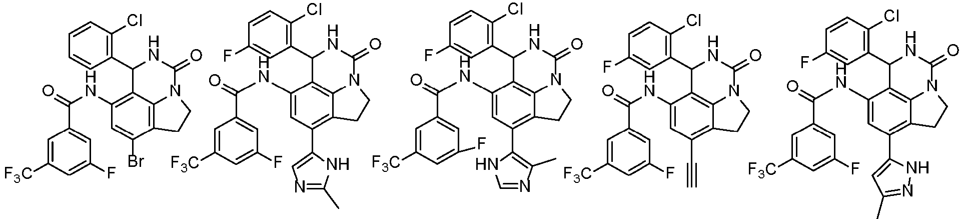

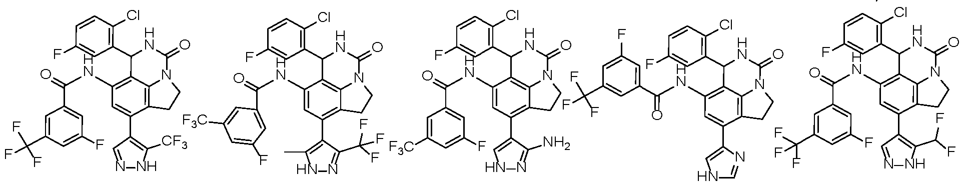

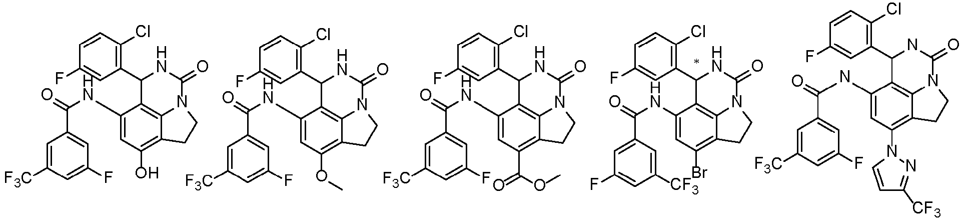

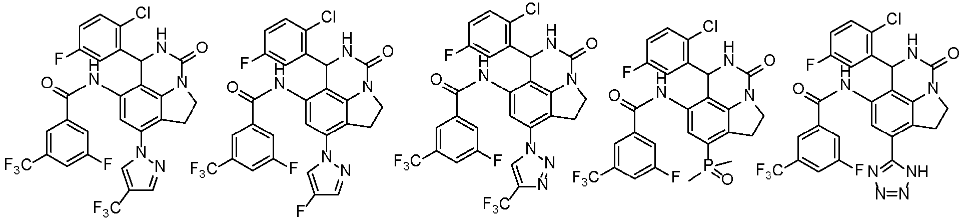

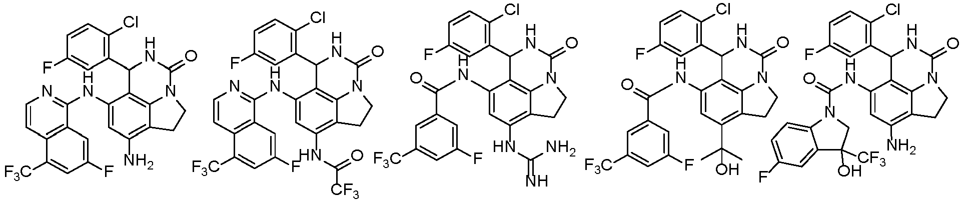

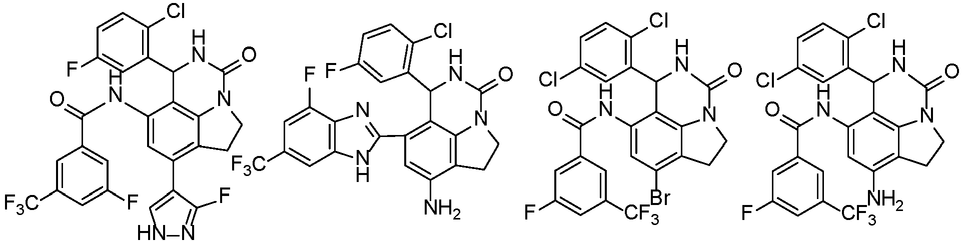

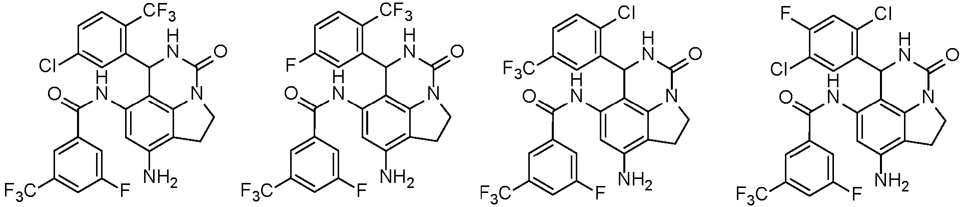

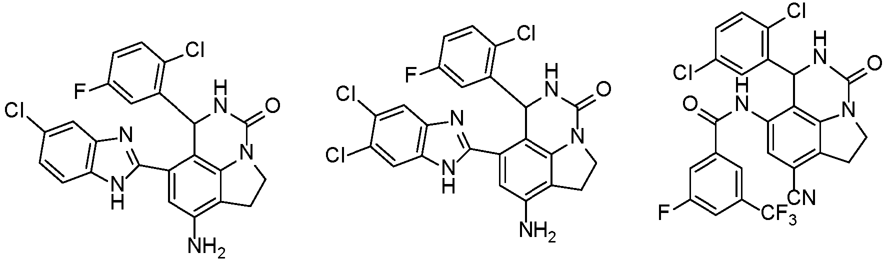

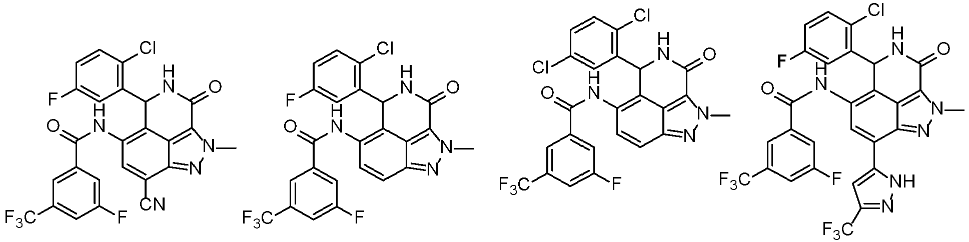

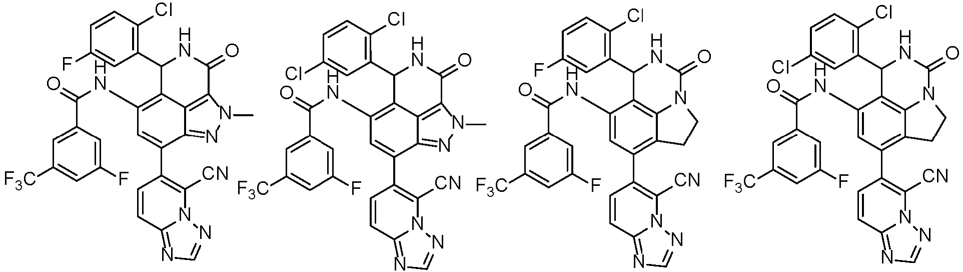

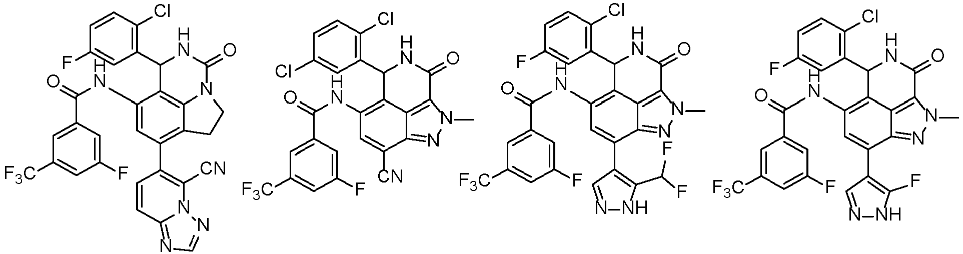

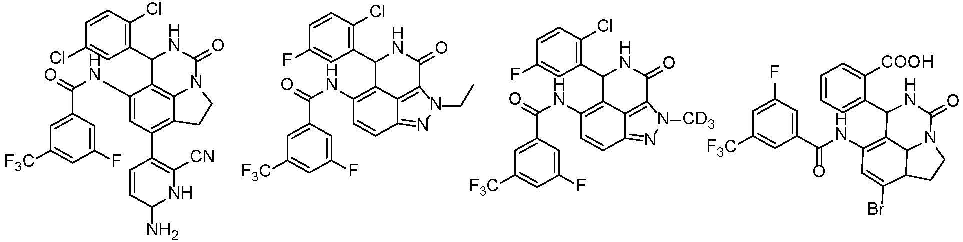

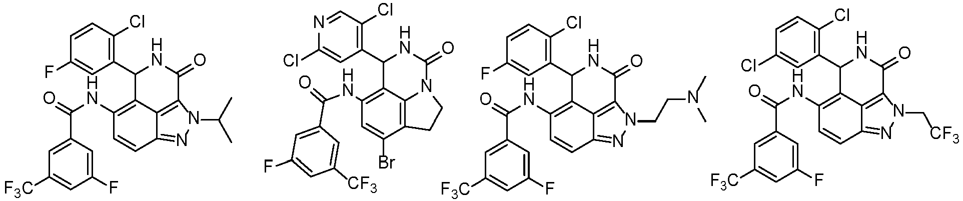

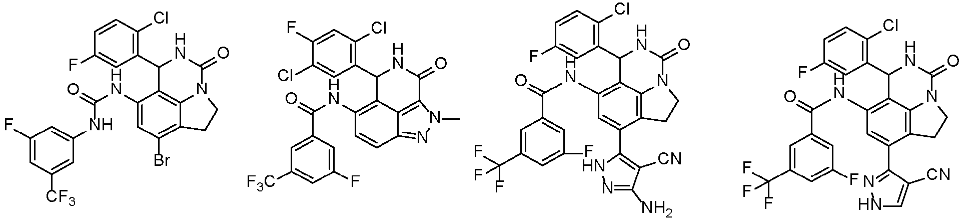

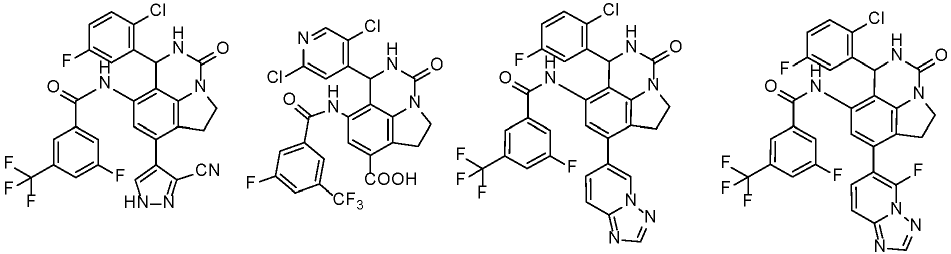

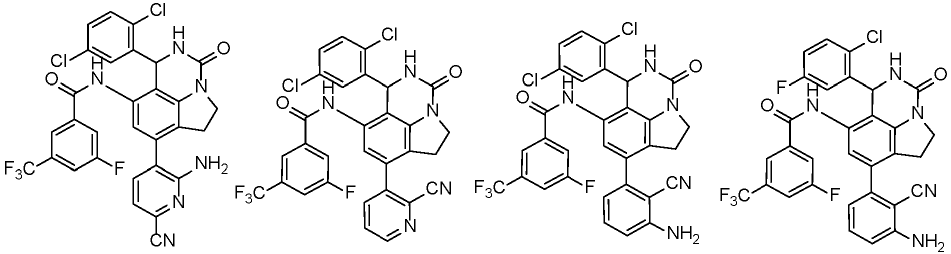

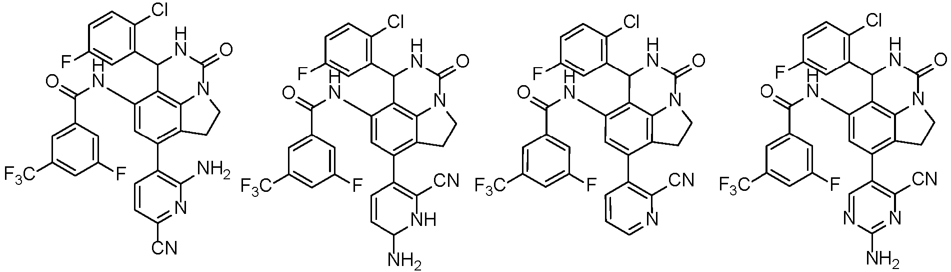

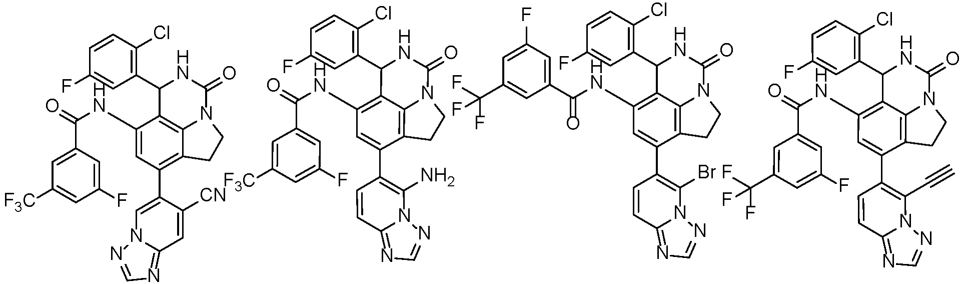

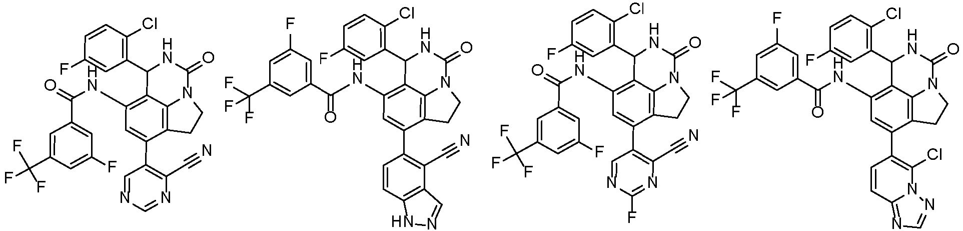

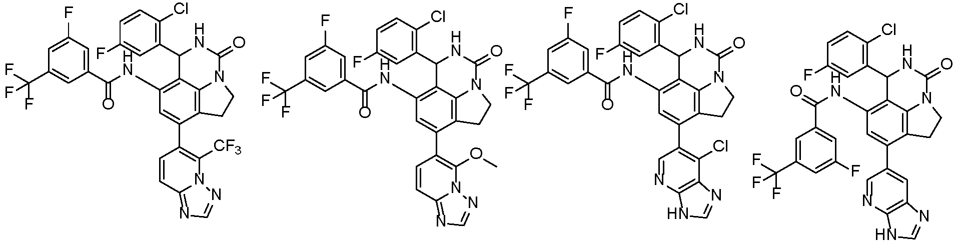

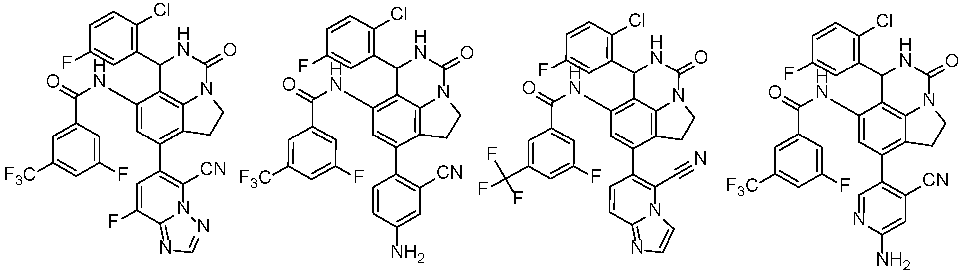

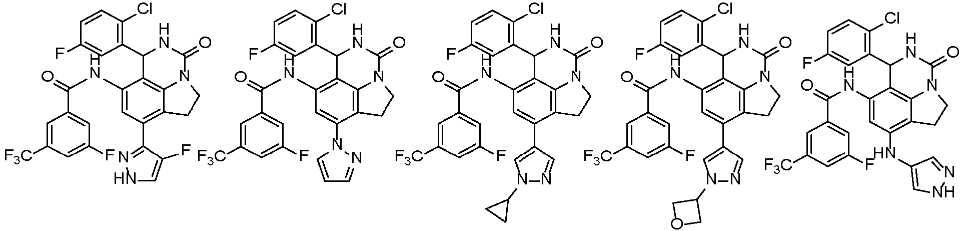

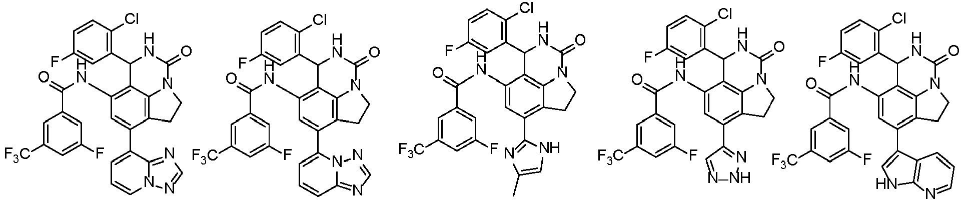

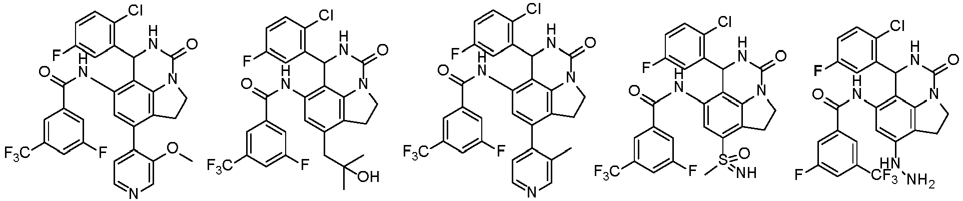

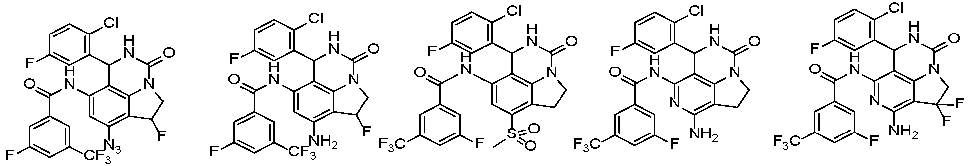

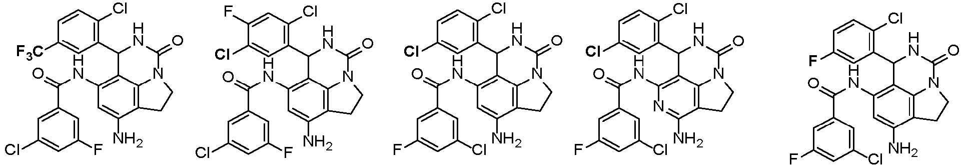

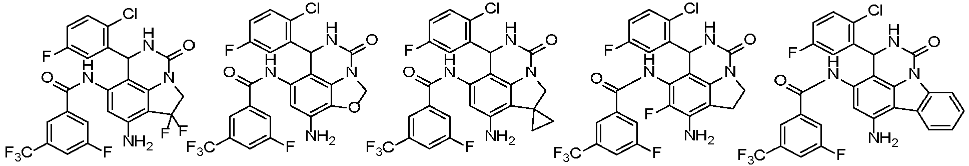

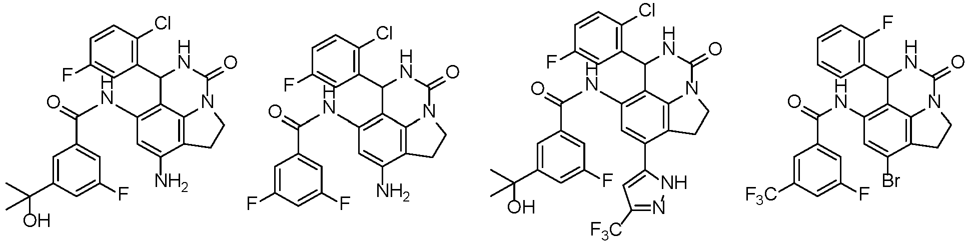

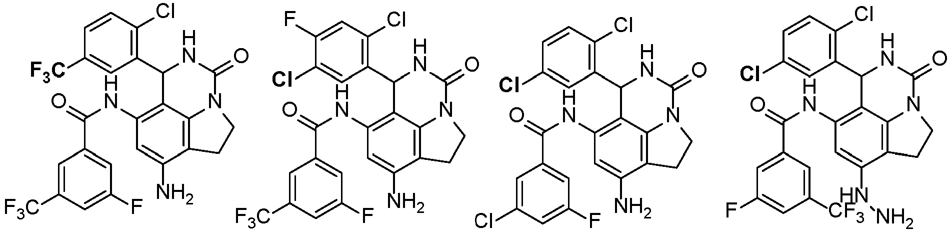

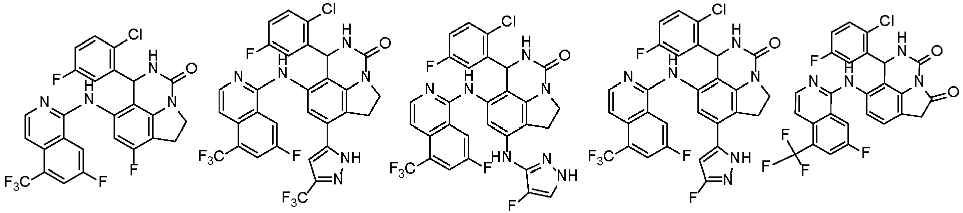

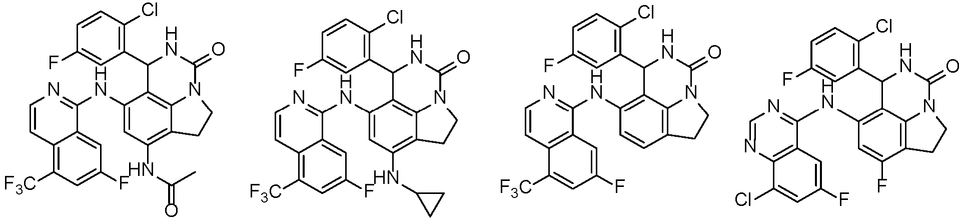

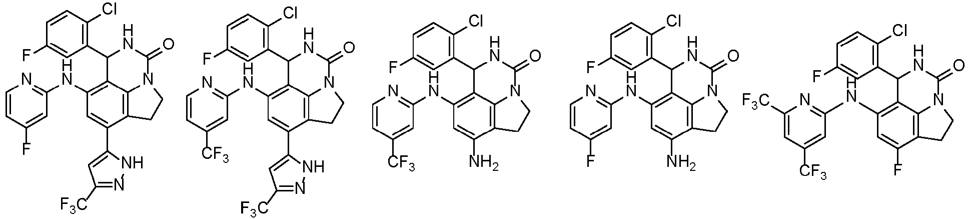

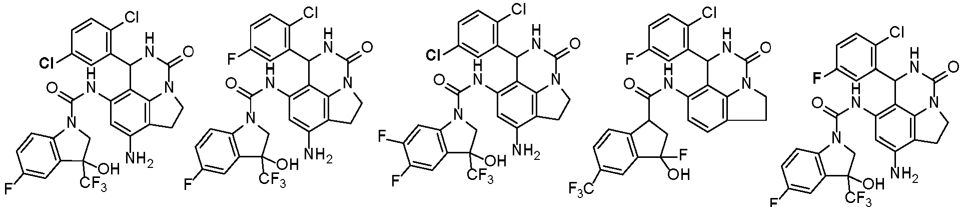

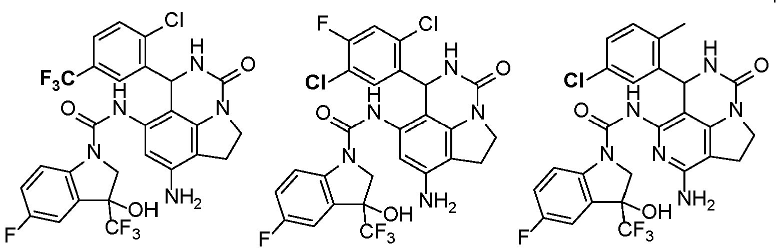

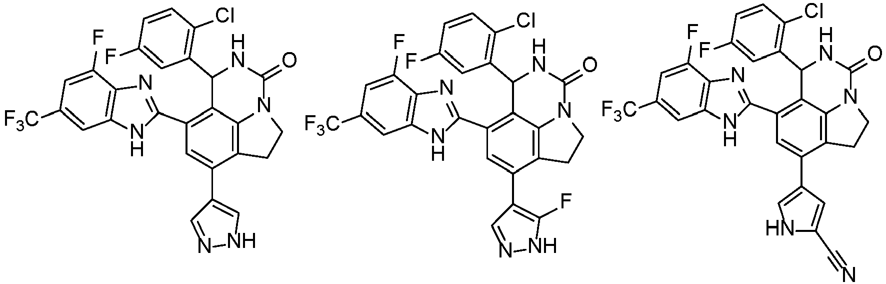

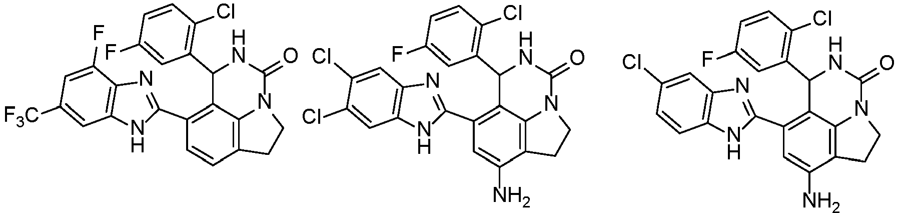

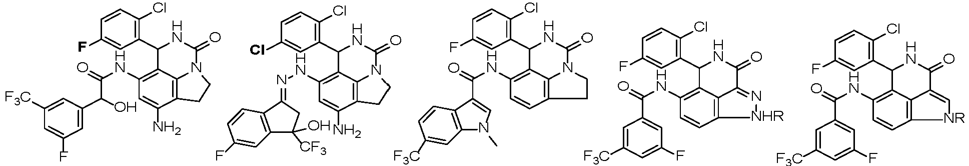

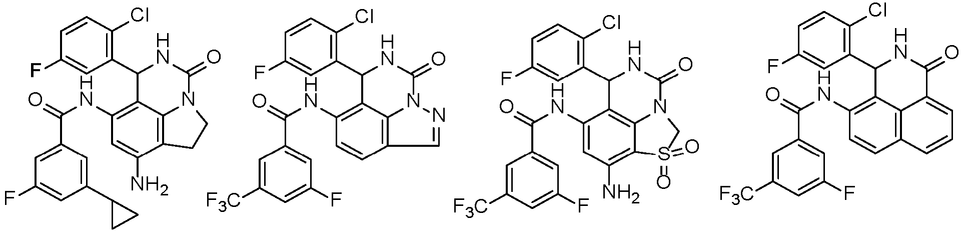

In a certain embodiment, the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof, wherein, the nitrogen-containing heterocyclic compound represented by formula I is any of the following compounds:
compound 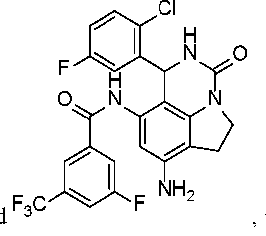 which has a retention time of 5.153 min under the following conditions: equipments: SFC-150 (Waters); chromatographic column: AS 20*250mm, 10um (Daicel); column temperature: 35 °C; mobile phase: CO₂/MeOH[0.2%NH₃(7M in MeOH) ] = 70/30; flow rate: 100 g/min; back pressure: 100 bar; wavelength: 214 nm;
compound 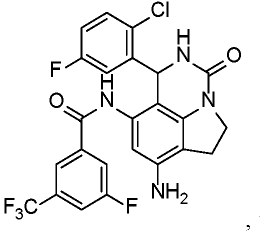 which has a retention time of 5.581min under the following conditions: equipments: SFC-150 (Waters); chromatographic column:AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar; wavelength: 214 nm;
compound 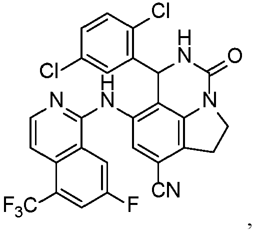 which has a retention time of 4.512 min under the following conditions: equipments: SFC-150 (Waters); chromatographic column: AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar ; wavelength: 214 nm;
compound 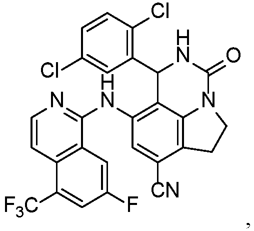 which has a retention time of 5.408min under the following conditions: equipments: SFC-150 (Waters); chromatographic column: AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar; wavelength: 214 nm;
compound 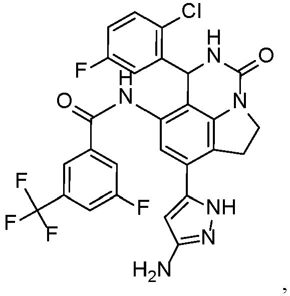 which has a retention time of 4.561min under the following conditions: equipments: SFC-150 (Waters); chromatographic column: AS 20*250mm, 10um (Daicel); column temperature:35 °C; mobile phase: CO₂/MeOH[0.2%NH₃(7M in MeOH) ] = 60/40; flow rate: 100 g/min; back pressure: 100 bar; wavelength: 214 nm;
compound 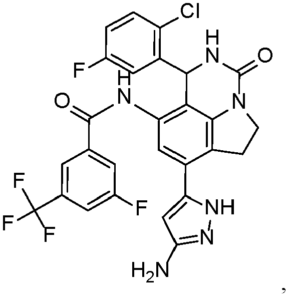 which has a retention time of 5.012min under the following conditions: equipments: SFC-150 (Waters); chromatographic column:AS 20*250mm,10um (Daicel); column temperature:35 °C; mobile phase: CO₂/MeOH[0.2%NH₃(7M in MeOH) ] = 60/40; flow rate: 100 g/min ; back pressure: 100 bar; wavelength: 214 nm;
compound 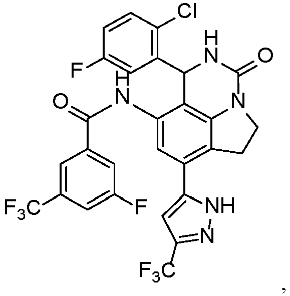 which has a retention time of 4.561min under the following conditions: equipments: SFC-150 (Waters); chromatographic column: AS 20*250mm,10um (Daicel); column temperature:35 °C; mobile phase: CO₂/MeOH[0.2%NH₃(7M in MeOH) ] = 70/30; flow rate: 100 g/min ; back pressure: 100 bar; wavelength: 214 nm;
compound 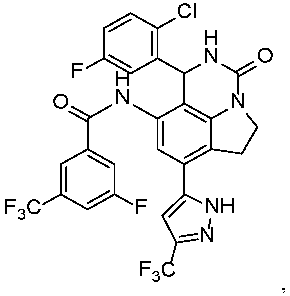 which has a retention time of 5.561min under the following conditions: equipments: SFC-150 (Waters); chromatographic column:AS 20*250mm,10um (Daicel); column temperature:35 °C; mobile phase: CO₂/MeOH[0.2%NH₃(7M in MeOH) ] = 70/30; flow rate: 100 g/min ; back pressure: 100 bar; wavelength: 214 nm;
compound 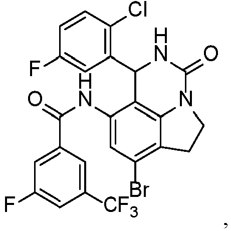 which has a retention time of 0.707min under the following conditions: equipments: SFC-150 (Waters); chromatographic column:OJ-H 4.6*100mm 5um; column temperature: 40 °C; mobile phase:CO₂/MeOH[0.2%NH₃(7M in MeOH) ]; flow rate: 3.0mL/min ; back pressure: 2000psi; wavelength: 214 nm;
compound 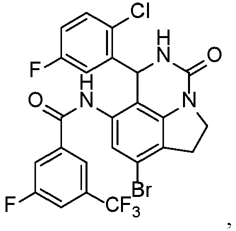 which has a retention time of 1.449min under the following conditions: equipments: SFC-150 (Waters); chromatographic column:OJ-H 4.6*100mm 5um; column temperature: 40 °C; mobile phase: CO₂/MeOH[0.2%NH₃(7M in MeOH) ]; flow rate: 3.0mL/min ; back pressure: 2000psi; wavelength: 214 nm;
compound 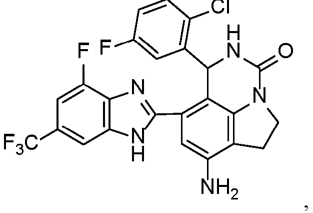 which has a retention time of 3.245min under the following conditions: equipments: SFC-150 (Waters); chromatographic column: OD 20*250mm, 10um (Daicel); column temperature: 35°C; mobile phase: CO₂/MeOH[0.2%NH₃(7M in MeOH) ] = 65/35; flow rate: 100.0mL/min; back pressure: 100bar; wavelength: 214 nm;
compound 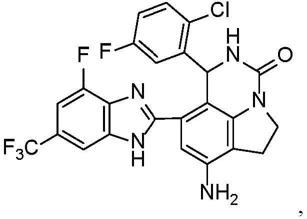 which has a retention time of 3.456min under the following conditions: equipments: SFC-150 (Waters); chromatographic column: OD 20*250mm,10um (Daicel); column temperature:35 °C; mobile phase: CO₂/MeOH[0.2%NH₃(7M in MeOH) ] = 65/35; flow rate: 100.0mL/min; back pressure: 100bar; wavelength: 214 nm;
Compound 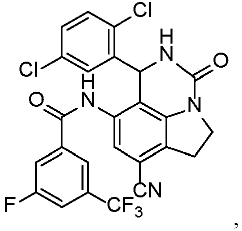 which has a retention time of 0.56 min under the following conditions: equipments: SFC-150 (Waters); chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 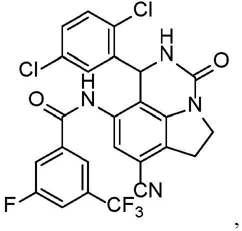 which has a retention time of 1.21min under the following conditions: equipments: SFC-150 (Waters); chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 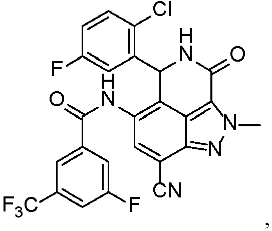 which has a retention time of 0.38min under the following conditions: equipments: SFC-150 (Waters); chromatographic column: AS 20*250mm, 10um (Daicel); column temperature: 35 °C; mobile phase: CO₂/MeOH[0.2%NH₃(7M in MeOH) ] = 45/55; flow rate: 120 g/min; back pressure:100bar; wavelength: 214 nm;
compound 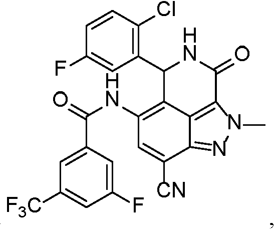 which has a retention time of 0.8min under the following conditions: equipments: SFC-150 (Waters); chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 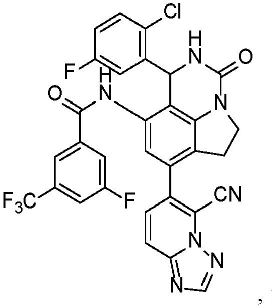 which has a retention time of 1.74min under the following conditions: equipments: SFC-150 (Waters); chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 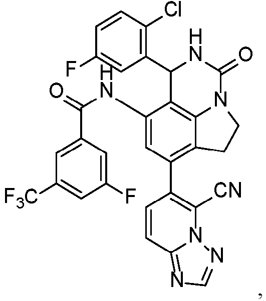 which has a retention time of 3.45min under the following conditions: equipments: SFC-150 (Waters); chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 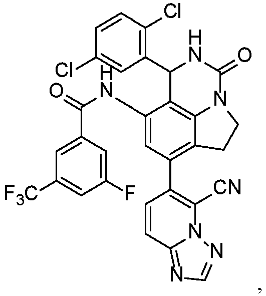 which has a retention time of 1.08min under the following conditions: equipments: SFC-150 (Waters); chromatographic column: AS 20*250mm,10um (Daicel); column temperature: 35°C; mobile phase: CO₂/MeOH[0.2%NH₃(7M in MeOH) ] = 45/55; flow rate: 120 g/min; back pressure:100bar; wavelength: 214 nm;
compound 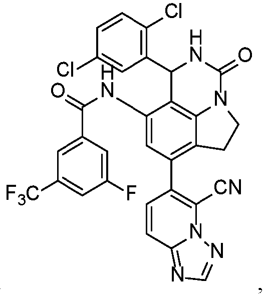 which has a retention time of 2.32min under the following conditions: equipments:SFC-150(Waters); chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 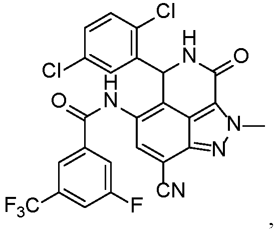 which has a retention time of 0.38min under the following conditions: equipments:SFC-150 (Waters); chromatographic column: AS 20*250mm,10um(Daicel); column temperature:35°C; mobile phase: CO₂/MeOH[0.2%NH₃(7M in MeOH) ] = 45/55; flow rate: 120 g/min; back pressure: 100 bar; wavelength: 214 nm;
compound 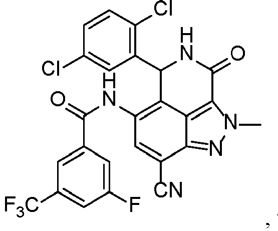 which has a retention time of 0.84min under the following conditions: equipments: SFC-150 (Waters); chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 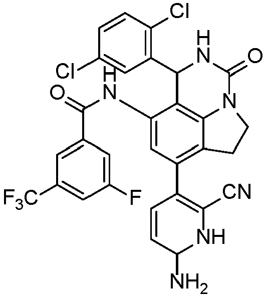 , which has a retention time of 2.32min under the following conditions: equipments: SFC-150 (Waters); chromatographic column: AS 20*250mm,10um (Daicel); column temperature:35°C; mobile phase: CO₂/MeOH[0.2%NH₃(7M in MeOH)] = 45/55; flow rate: 120 g/min; back pressure:100bar; wavelength: 214 nm;
compound 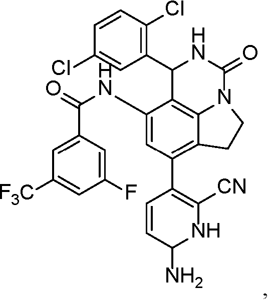 which has a retention time of 3.38min under the following conditions: equipments:SFC-150(Waters); chromatographic column: AS 20*250mm,10um (Daicel); column temperature: 35°C; mobile phase: CO₂/MeOH[0.2%NH₃(7M in MeOH) ] = 45/55; flow rate: 120 g/min; back pressure:100bar; wavelength: 214 nm;
compound 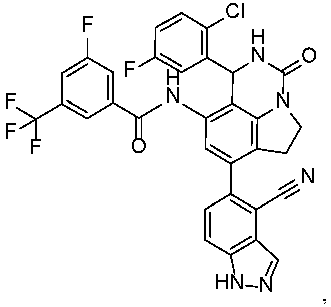 which has a retention time of 2.58min under the following conditions: equipments:SFC-150(Waters); chromatographic column: AS 20*250mm,10um (Daicel); column temperature: 35°C; mobile phase: CO₂/MeOH[0.2%NH₃(7M in MeOH) ] = 45/55; flow rate: 100 g/min; back pressure:100bar; wavelength: 214 nm;
compound 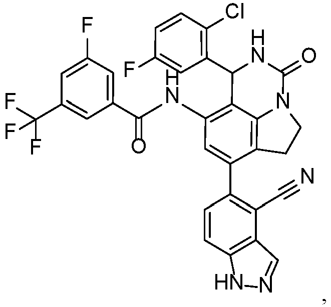 which has a retention time of 2.91min under the following conditions: equipments:SFC-150(Waters); chromatographic column: AS 20*250mm,10um (Daicel); column temperature: 35°C; mobile phase: CO₂/MeOH[0.2%NH₃(7M in MeOH) ] = 45/55; flow rate: 100 g/min; back pressure:100bar; wavelength: 214 nm.

In a certain embodiment, the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof, wherein, the nitrogen-containing heterocyclic compound represented by formula I is any of the following compounds:

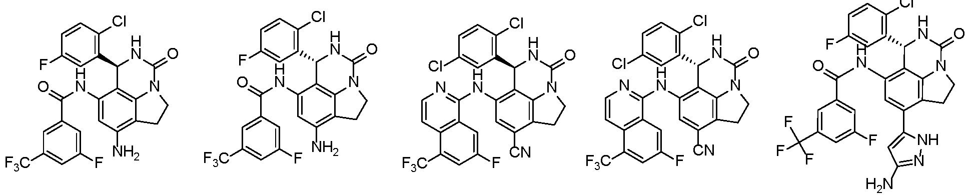

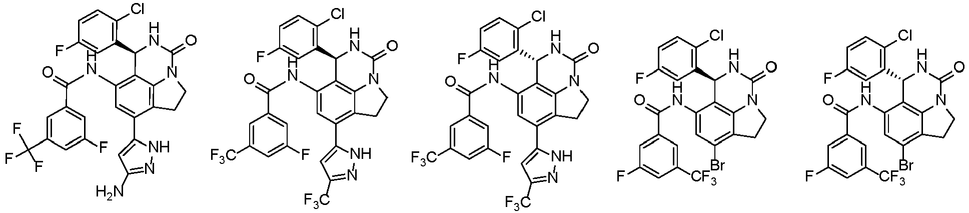

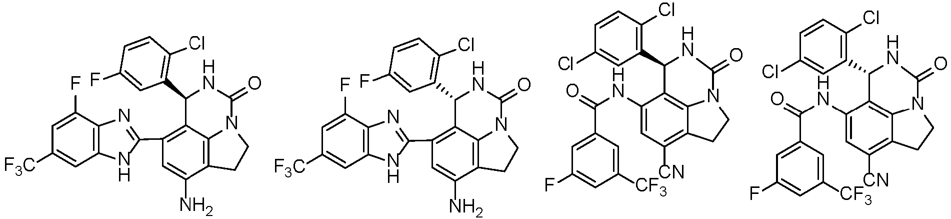

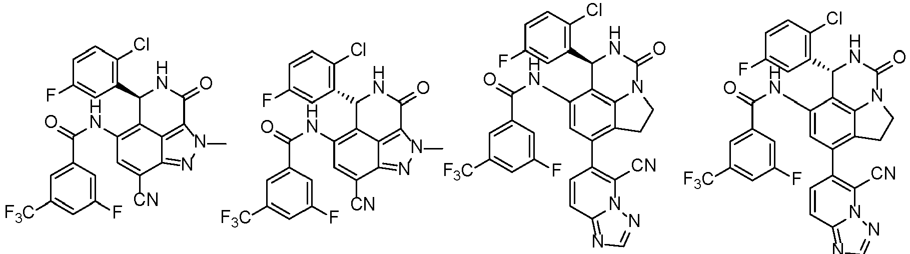

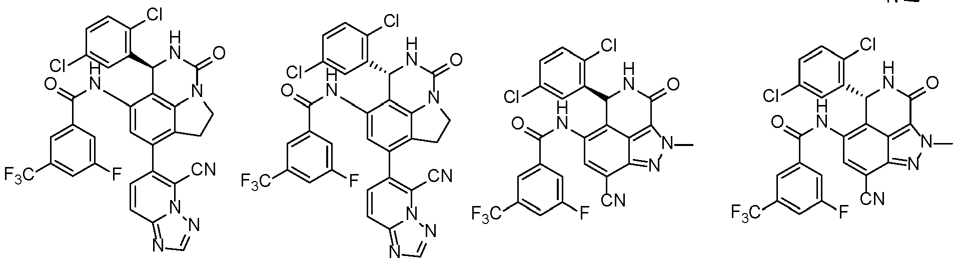

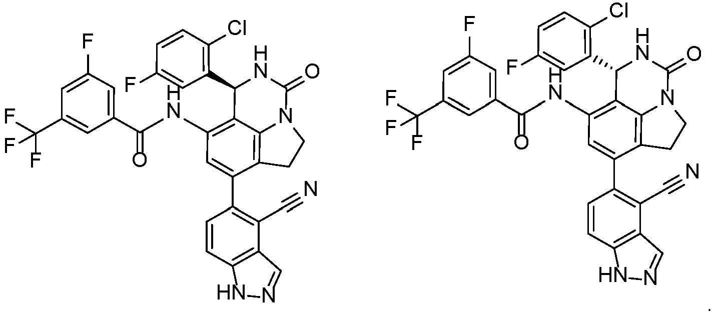

The present disclosure also provides a pharmaceutical composition, comprising substance A and a pharmaceutical adjuvant; the substance A is the above-mentioned nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof.

The present disclosure also provides a method for inhibiting PI3K, the method comprising administrating to a patient an therapeutically effective amount of substance A, wherein the substance A is the above-mentioned nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof.

The present disclosure also provides a method for treating or preventing an PI3K-mediated disease, the method comprising administrating to a patient an therapeutically effective amount of substance A;

the substance A is the above-mentioned nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof.

the PI3K-mediated disease is, for example, cancer; the cancer is, for example, one or more of colon cancer, pancreatic cancer, breast cancer, prostate cancer, lung cancer, brain cancer, ovarian cancer, cervical cancer, testicular cancer, renal carcinoma, head or neck cancer, bone cancer, skin cancer, rectal cancer, liver cancer, colon cancer, esophageal cancer, gastric cancer, pancreatic cancer, thyroid cancer, bladder cancer, lymphoma, leukemia and melanoma.

The term "more" refers to 2, 3, 4 or 5.

The term "pharmaceutically acceptable salt" refers to a salt prepared from compounds of the present disclosure with relatively non-toxic, pharmaceutically acceptable acids or bases. When compounds of the present disclosure contain relatively acidic functional groups, base addition salts can be obtained by contacting the neutral form of such compounds with a sufficient amount of pharmaceutically acceptable bases, either in pure solution or a suitable inert solvent. The pharmaceutically acceptable base addition salts include but are not limited to: lithium salt, sodium salt, potassium salt, calcium salt, aluminum salt, magnesium salt, zinc salt, bismuth salt, ammonium salt and diethanolamine salt. When compounds of the present disclosure contain relatively basic functional groups, acid addition salts can be obtained by contacting the neutral form of such compounds with a sufficient amount of pharmaceutically acceptable acids, either in pure solution or a suitable inert solvent. The pharmaceutically acceptable acids include inorganic acids, and the inorganic acids include but are not limited to: hydrochloric acid, hydrobromic acid, hydroiodic acid, nitric acid, carbonic acid, phosphoric acid, phosphorous acid and sulfuric acid. The pharmaceutically acceptable acids include organic acids, and the organic acids include but are not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, hydrogen tartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)) and amino acid (such as glutamic acid and arginine). When compounds of the present disclosure contain relatively acidic functional groups and relatively basic functional groups, such compounds can be converted into base addition salts or acid addition salts. For details, reference can be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to substance formed by combining compounds of the present disclosure with stoichiometric or non-stoichiometric solvents. The solvent molecules in the solvate may be present in a regular arrangement or a non-ordered arrangement. The solvents include but are not limited to: water, methanol and ethanol.

With regard to term "solvate of a pharmaceutically acceptable salt", the "pharmaceutically acceptable salt" and "solvate" as described above refer to: 1. substance prepared by compounds of the present disclosure and relatively non-toxic, pharmaceutically acceptable acids or bases; and 2. substance formed by combining compounds of the present disclosure with stoichiometric or non-stoichiometric solvents. The "solvate of a pharmaceutically acceptable salt" includes, but is not limited to, the hydrochloric acid monohydrate of compounds of the present disclosure.

The term "stereoisomer" refers to an isomer in which the atoms or atomic groups in a molecule have the same interconnection order but different spatial arrangements, such as cis-trans isomers, optical isomers or atropisomers. These stereoisomers can be separated, purified and enriched by means of asymmetric synthesis methods or chiral separation methods (including but not limited to thin layer chromatography, rotation chromatography, column chromatography, gas chromatography and high-pressure liquid chromatography) or can also be obtained by means of chiral resolution via forming bonds (chemical bonding, etc.) or forming salts (physical bonding) with other chiral compounds, etc.

The term "tautomer" refers to a functional group isomer resulting from the rapid movement of an atom in two positions in a molecule. For example, acetone and 1-propene-2-ol can be converted into each other by the rapid movement of hydrogen atoms on oxygen and α-carbon.

The term "crystal form" refers to substance in which ions or molecules are arranged strictly and periodically in a three-dimensional space according to a certain way and are repeated regularly and periodically at a certain interval; and since the periodic arrangements are different, there can be multiple crystal forms, which is also known as polymorphism.

The term "isotopic compound" refers to a compound in which one or more atoms are substituted with one or more atoms having a specific atomic mass or mass number. Examples of isotopes that can be incorporated into compounds of the present disclosure include, but are not limited to, isotopes of hydrogen, carbon, nitrogen, oxygen, fluorine, sulfur and chlorine (e.g., 2H, 3H, 13C, 14C, 15N, 180, 170, 18F, 35S and 36Cl). The isotopic compounds of the present disclosure can generally be prepared by substituting non-isotopically-labeled reagents with isotopically-labeled reagents according to the methods described herein.

The term "halogen" refers to fluorine, chlorine, bromine or iodine.

The term"C₃₋₈ cycloalkyl" refers cyclopropyl, cyclobutyl, cyclopentyl, cyclohexyl or cycloheptyl.

The term "C₁₋₆ alkyl" preferably is C₁₋₄ alkyl, furthermore, methyl, ethyl, n-propyl, isopropyl, n-butyl, sec-butyl, isobutyl or tert-butyl.

The term "C₁₋₆ alkyl substituted with one or more halogen" refers to"C₁₋₂alkyl substituted with one or more halogen", for example, Trifluoromethyl or difluoromethyl.

The term "alkyl" refers to straight or branched alkyl having specified number of carbon atoms. Examples of alkyl include methyl, ethyl, n-propyl, isopropyl, n-butyl, tert-butyl, isobutyl, sec-butyl, n-pentyl, n-hexyl, n-heptyl, n-octyl and similar alkyl groups.

The terms "cycloalkyl" and "carbocyclic ring" refer to a saturated cyclic group consisting only of carbon atoms having a specified number of carbon atoms (e.g., C₃-C₆), which is a monocyclic, bridged or spiro ring. The cycloalkyl includes, but is not limited to, cyclopropyl, cyclobutyl, cyclopentyl, and cyclohexyl.

The term "aryl" refers to an aromatic group consisting of carbon atoms, each ring having aromaticity. For example, phenyl or naphthyl.

The term "heteroaryl" refers to a cyclic group having a specified number of ring Atoms (e.g., 5-12 members), a specified number of heteroatoms (e.g., 1, 2, or 3) and specified heteroatom species (one or more of N, O and S), which is monocyclic or polycyclic, and has at least one aromatic ring (according to the Hückel's rule). Heteroaryls are linked to other fragments of the molecule through aromatic or non-aromatic rings. Heteroaryls include, but are not limited to, furyl, pyrrolyl, thienyl, pyrazolyl, imidazolyl, oxazolyl, thiazolyl, pyridinyl, pyrimidinyl, and indolyl.

The term "hydroxyl" refers to a -OH group.

The term "cyano" refers to a -CN group.

The term "oxo" refers to a=O group.

The above preferred conditions may be combined arbitrarily to obtain preferred embodiments of the present disclosure without departing from the general knowledge in the art.

The reagents and starting materials used in the present disclosure are commercially available.

The positive effect of the present disclosure is that the present disclosure provides a nitrogen-containing heterocyclic compound, a pharmaceutical composition thereof, and an application thereof, wherein the nitrogen-containing heterocyclic compound has a better inhibitory effect on a wide range of PI3K-mediated disease cells, and is expected to treat and/or prevent a wide range of diseases mediated by PI3K.

### Detailed description of the embodiments

The present disclosure is further illustrated by the following examples, which are not intended to limit the present disclosure. Experimental procedures without specified conditions in the following examples were performed in accordance with conventional procedures and conditions, or in accordance with instructions.

In the present disclosure, room temperature refers to ambient temperature, or 10-35°C. Overnight refers to 8-15 hours. Reflux refers to the reflux temperature of a solvent at atmospheric pressure.

Brine is referred to saturated sodium chloride solution.

The following is a list of abbreviations used in the examples:
DMF N, N-dimethylformamide
Pd(PPh3)4 tetrakis(triphenylphosphine)palladium
Pd2(dba)3 tris(dibenzylideneacetone)dipalladium
Pd(dppf)Cl2 1,1'-Bis(diphenylphosphino)ferrocene]dichloropalladium(II)
EDTA Ethylene Diamine Tetraacetic Acid
HATU 2-(7-Azabenzotriazol-1-yl)-N,N,N',N'-tetramethyluronium hexafluorophosphate
DIEA diisopropylethylamine
S-Phos 2-Dicyclohexylphosphineo-2',6'-dimethoxybiphenyl
LDA Lithium diisopropylamide
PPA Polyphosphoric acid
Eaton's reagent Phosphorus pentoxide methanesulfonic acid

Example 1 Synthetic route of compound YL001

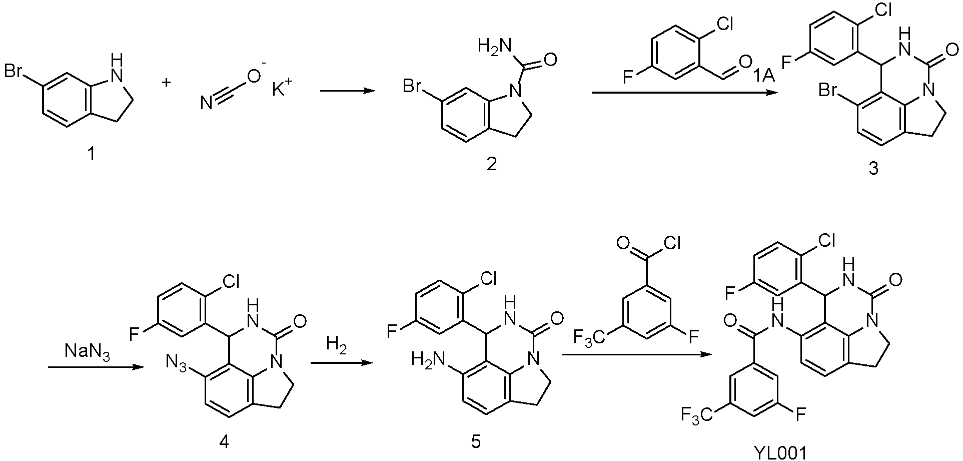

### Synthesis of compound 2

Potassium cyanate (410 mg, 5.05 mmol) was added to a solution of 6-bromodihydroindole (compound 1, 500 mg, 2.52 mmol) in acetic acid (3 mL) in a reaction vial, then and the reaction continued stirring in an ice-water bath for 1 h. Upon completion, the reaction mixture was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 55 % to 65 %) to give compound 2 as a white solid (460 mg, 1.908 mmol, 99.01 %) at 75.6 % yield. LC-MS (ESI): m/z 241.0/243.0 (M+H) ⁺. ¹H NMR (400 MHz, DMSO-d6) δ 7.99 (d, J = 1.9 Hz, 1H), 7.10 - 7.05 (m, 1H), 6.98 (dd, J = 7.9, 1.9 Hz, 1H), 6.42 (s, 2H), 3.88 (dd, J = 9.3, 8.3 Hz, 2H), 3.05 (td, J = 8.7, 8.3, 1.2 Hz, 2H).

### Synthesis of compound 3

Compound 2 (420 mg, 1.7 mmol), 2-chloro-5-fluorobenzaldehyde (compound 1A, 668 mg, 4.2 mmol) and polyphosphoric acid (5 mL) were combined in a reaction vial. The reaction mixture was stirred at 100°C-105°C for 34 min, then was poured into ice-cold saturated aqueous sodium bicarbonate. The aqueous phase was extracted with ethyl acetate, and the organic phases were combined, washed with brine, concentrated at reduced pressure, and the residue was purified by automatic column (Biotage) (mobile phase: petroleum ether/ethyl acetate 50/50 to 28/72) to give a light yellow solid target compound 3 (0.4 g). LC-MS (ESI): m/z 381.1 (M+H) ⁺.

### Synthesis of compound 4

Compound 3 (172 mg, 0.45 mmol), sodium azide (62 mg, 0.95 mmol), cuprous iodide (9 mg, 0.05 mmol), sodium vitamin C acid (5 mg, 0.03 mmol), trans-(1R,2R)-N,N-dimethyl 1,2-cyclohexanediamine (11 mg, 0.08 mmol), ethanol (3 mL) and water (1.3 mL) were combined in a reaction flask. The reaction mixture was stirred at 90°C overnight, then cooled to room temperature, added ethyl acetate and water to dilute. The organic phase was separated and the aqueous phase was extracted with ethyl acetate. The organic phases were combined, washed with brine, and concentrate to 10 mL at reduced pressure, and the resulting crude product was used directly in the next step. LC-MS (ESI): m/z 344.1 (M+H) ⁺.

### Synthesis of compound 5

Compound 4 (crude, 9 mL ethyl acetate solution) and platinum dioxide (102 mg, 0.45 mmol) were combined in a reaction vial. After degassed and purged with hydrogen for three times, the mixture was stirred at room temperature overnight. Filtered, the filtrate was concentrated at reduced pressure and the resulting crude product was purified by automatic column (Biotage) (mobile phase: petroleum ether/ethyl acetate 35/65 to 20/80) to give target compound 5 as a light yellow solid (25 mg). LC-MS (ESI): m/z 318.1 (M+H) ⁺.

### Synthesis of compound YL001

Compound 5 (15 mg, 0.05 mmol), pyridine (8 mg, 0.1 mmol), 3-fluoro-5-(trifluoromethyl)benzoyl chloride (12 mg, 0.05 mmol) and acetonitrile (1 mL) were combined in a reaction vial. The mixture was stirred at room temperature under nitrogen protection for 1 h. When completed, the reaction was quenched by adding brine. The aqueous phase was extracted with ethyl acetate. The organic phases were combined, washed with brine and concentrated at reduced pressure to obtain the crude product. The resulting crude product was purified by automatic column (Biotage) (mobile phase: petroleum ether/ethyl acetate 30/70 to 20/80) to give the light yellow solid target compound YL001 (3.5 mg, 11%).

Spectrogram data of compound YL001: LC-MS (ESI): m/z 508.1 (M+H) ⁺. ¹H NMR (400 MHz, DMSO-d6) δ 10.11 (s, 1H), 7.93 (d, J = 8.5 Hz, 1H), 7.90 - 7.81 (m, 2H), 7.43 (d, J = 2.6 Hz, 1H), 7.32 (dd, J = 8.9, 5.2 Hz, 1H), 7.20 (d, J = 7.8 Hz, 1H), 7.09 (ddd, J = 8.8, 8.0, 3.1 Hz, 1H), 6.83 (dd, J = 9.3, 3.1 Hz, 1H), 6.72 (d, J = 7.8 Hz, 1H), 6.13 - 6.06 (m, 1H), 3.99 (dtd, J = 23.4, 10.3, 7.3 Hz, 2H), 3.23 - 3.17 (m, 2H).

### Example 2 Synthetic route of compound YL002

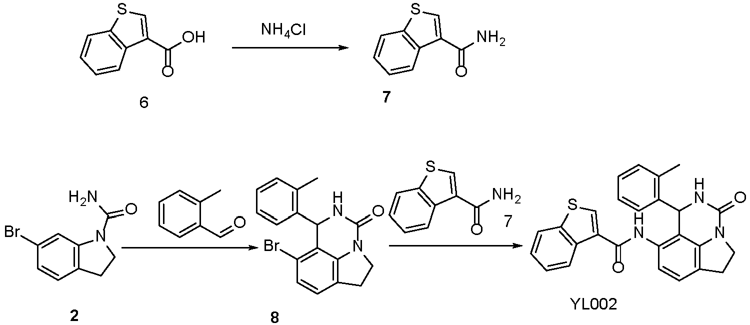

### Synthesis of compound 7

1-benzothiophene-3-carboxylic acid (compound 6, 2 g, 11.2 mmol), ammonium chloride (774 mg, 14.6 mmol), 2-(7-azobenzotriazole)-*N,N,N',N'*-tetramethyluronium hexafluorophosphate (6.4 g, 16.8 mmol), N,N-diisopropylethylamine (6 mL, 33.3 mmol) and anhydrous *N,N*-dimethylformamide (8 mL) were combined in a reaction flask. The mixture was stirred at room temperature under nitrogen protection for 1 hour, then was diluted by adding ethyl acetate and water. The organic phase was separated and the aqueous phase was extracted with ethyl acetate. The organic phase was combined, washed with brine, concentrated at reduced pressure, and the resulting crude product was purified by automatic column (Biotage) (mobile phase: petroleum ether/ethyl acetate 40/60 to 20/80) to give target compound 7 (1.26 g) as a light yellow solid. LC-MS (ESI): m/z 178.1 (M+H) ⁺.

### Synthesis of compound 8

Compound 2 (410 mg, 1.7 mmol), 2-methylbenzaldehyde (492 mg, 4.1 mmol) and polyphosphoric acid (6 mL) were combined in a reaction flask. The mixture was stirred at 100°C-105°C for half an hour, then cooled to room temperature. Diluted by adding ethyl acetate and water. The organic phase was separated and the aqueous phase was extracted with ethyl acetate. The organic phases were combined, washed with brine, concentrated at reduced pressure, and the resulting crude product was purified by automatic column (Biotage) (mobile phase: petroleum ether/ethyl acetate 50/50 to 30/70) to give target compound 8 (180 mg) as light yellow solid. LC-MS (ESI): m/z 343.1 (M+H)⁺.

### Synthesis of YL002

Compound 8 (100 mg, 0.29 mmol), compound 7 (106 mg, 0.60 mmol), cuprous iodide (30 mg, 0.16 mmol), N,N-dimethylethylenediamine (26 mg, 0.30 mmol), potassium phosphate (190 mg, 0.9 mmol), potassium iodide (26 mg, 0.16 mmol) and N,N-dimethylformamide (1 mL) were combined in a reaction vial. The mixture was microwaved at 150°C for 9 hours under nitrogen protection. When completed, the reaction was quenched by adding brine. The aqueous phase was extracted with ethyl acetate. The organic phases were combined, washed with brine and concentrated at reduced pressure to obtain the crude product. The resulting crude product was purified by automatic column (Biotage) (mobile phase: petroleum ether/ethyl acetate 10/90 to 0/100) to give the light yellow solid compound YL002 (36.6 mg, 17%). Of which, 5.6 mg was used for activity testing, the rest was used to do other reactions.

Spectrogram data of compound YL002: LC-MS (ESI): m/z 440.1 (M+H) ⁺; ¹H NMR (400 MHz, DMSO-d6) δ 9.77 (s, 1H), 8.24 - 8.16 (m, 1H), 8.14 - 7.95 (m, 2H), 7.45 - 7.36 (m, 2H), 7.32 (d, J = 2.8 Hz, 1H), 7.17 (d, J = 7.8 Hz, 1H), 7.08 - 6.96 (m, 3H), 6.91 (dd, J = 7.4, 1.7 Hz, 1H), 6.77 (d, J = 7.8 Hz, 1H), 6.07 (d, J = 2.7 Hz, 1H), 4.06 - 3.89 (m, 2H), 3.22 (dd, J = 10.0, 7.3 Hz, 2H), 2.17 (s, 3H).

### Example 3 Synthetic route of compound YL003

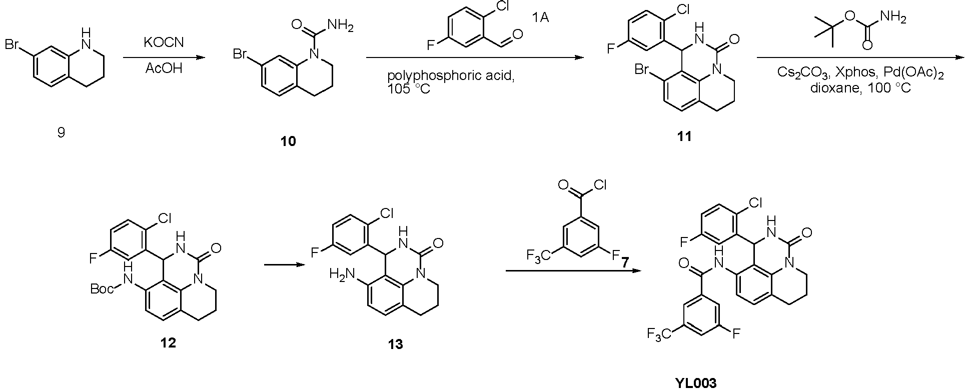

### Synthesis of compound 10

7-Bromo-1,2,3,4-tetrahydroquinoline (compound 9, 2 g, 53.277 mmol) was dissolved in acetic acid (15 mL). After degassed and purged with N₂, the mixture was added potassium cyanate (1.53 g, 18.86 mmol) at 0 °C and reacted at 5 °C for 1 h. The reaction mixture was concentrated to dryness and adjusted to pH > 7 with saturated sodium bicarbonate solution. The organic phase was separated and the aqueous phase was extracted with dichloromethane. The organic phases were combined, washed with water, brine, dried with anhydrous Na₂SO₄, filtered and the filtrate was evaporated to obtain crude compound 10 (2.4 g, 99.76%). ESI:(m/z) = 257.0 [M+H]⁺; ¹H NMR (400 MHz, Chloroform-d) δ 7.59 (d, J = 2.0 Hz, 1H), 7.16 (dd, J = 8.2, 2.0 Hz, 1H), 7.00 (d, J = 8.1 Hz, 1H), 5.19 (s, 2H), 3.70 (t, J = 6.3 Hz, 2H), 2.69 (t, J = 6.6 Hz, 2H), 1.92 (p, J = 6.5 Hz, 2H).

### Synthesis of compound 11

2-chloro-5-fluorobenzaldehyde (compound 1A, 0.31 g, 1.96 mmol) was added to a solution of compound 10 (0.5 g, 1.96 mmol) in polyphosphoric acid (50 g) and the resulting mixture was reacted at 105°C for 20 min. The reaction mixture was poured into an ice-cold solution of sodium hydroxide (2 mol/L), then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound 11 (180 mg, 23.21%). ESI:(m/z) = 396.9 [M+H]⁺ ; ¹H NMR (400 MHz, Chloroform-d) δ 7.39 (dd, J = 8.8, 5.0 Hz, 1H), 7.15 (d, J = 8.2 Hz, 1H), 7.02 (d, J = 8.2 Hz, 1H), 6.94 (ddd, J = 8.8, 7.7, 3.0 Hz, 1H), 6.31 (dd, J = 9.0, 3.0 Hz, 1H), 6.06 (dd, J = 3.3, 1.2 Hz, 1H), 5.70 (d, J = 3.2 Hz, 1H), 3.93 (ddd, J = 12.9, 9.2, 3.8 Hz, 1H), 3.72 (dddd, J = 12.9, 6.5, 3.7, 1.2 Hz, 1H), 2.91 - 2.72 (m, 2H), 2.12 - 1.91 (m, 2H).

### Synthesis of compound 12

Compound 11 (140 mg, 0.354 mmol) was dissolved in 1,4-dioxane (5 mL), to which were added cesium carbonate (346 mg, 1.062 mmol), tert-butyl carbamate (62 mg, 0.531 mmol), XPHOS (34 mg, 0.071 mmol) and palladium acetate (8 mg, 0.035 mmol). The resulting mixture was stirred at 100°C for 1 hr, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated to dryness and the residue was purified by column chromatography (mobile phase: dichloromethane/methanol 10/1 to 10/1) to give compound 12 (100 mg, 65.44%). ESI:(m/z) = 432.1 [M+H]⁺.

### Synthesis of compound 13

Compound 12 (100 mg, 0.776 mmol) in dichloromethane (5 mL) was added trifluoroacetic acid (1 mL), then the resulting mixture was stirred at room temperature for 1 h. The reaction mixture was added dropwise to an ice-cold saturated sodium bicarbonate solution to adjust pH > 7, then diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 13 (100 mg). ESI:(m/z) = 332.0 [M+H]⁺.

### Synthesis of compound YL003

Compound 13 (30 mg, 0.09 mmol) was dissolved in acetonitrile (2 mL), and the mixture was added pyridine (0.015 mL, 0.181 mmol). After degassed and purged with N₂ and cooled to 0°C, the above mixture was added 3-fluoro-5-(trifluoromethyl)benzoyl chloride (25 mg, 0.109 mmol) and stirred at room temperature for 1 h, then diluted with dichloromethane and water. The organic phases were separated and the aqueous phase was extracted with dichloromethane. The organic phases were combined, washed with water, brine, dried over anhydrous Na₂SO₄, filtered, and the filtrate was evaporated and the residue was purified by prep-HPLC to give compound YL003 (13 mg, 27.5%). ESI:(m/z) = 522.0 [M+H]⁺; ¹H NMR (400 MHz, DMSO-d6) δ 10.12 (s, 1H), 7.97 (s, 1H), 7.91 (dd, J = 19.3, 9.0 Hz, 2H), 7.76 (d, J = 3.4 Hz, 1H), 7.34 (dd, J = 8.8, 5.2 Hz, 1H), 7.18 (d, J = 8.0 Hz, 1H), 7.11 (ddd, J = 8.9, 8.0, 3.1 Hz, 1H), 6.81 (d, J = 8.0 Hz, 1H), 6.70 (dd, J = 9.3, 3.1 Hz, 1H), 6.05 (dd, J = 3.3, 1.4 Hz, 1H), 3.82 (ddd, J = 12.5, 8.2, 4.2 Hz, 1H), 3.76 - 3.67 (m, 1H), 2.92 - 2.76 (m, 2H), 1.94 (q, J = 5.4 Hz, 2H).

### Example 4 Synthetic route of compound YL004

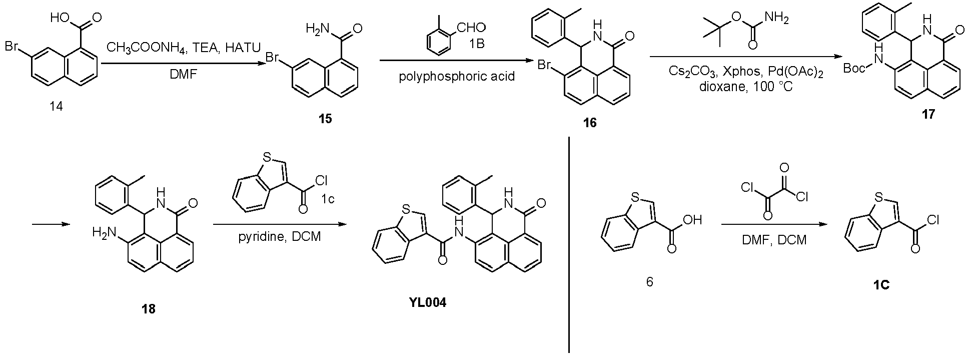

### Synthesis of compound 15

7-Bromo-1-naphthoic acid (compound 14, 1 g, 3.983 mmol) was dissolved in DMF (15 mL), to which were added TEA (1.661 mL, 11.948 mmol) and HATU (2.27 g, 5.974 mmol) and the reaction mixture was stirred at room temperature for 0.5 h, then was added ammonium acetate (610 mg, 7.966 mmol) and the reaction mixture was stirred at room temperature for 0.5 hr, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 1/1) to give compound 15 (980 mg, 98.39%).

### Synthesis of compound 16

Compound 15 (0.8 g, 3.199 mmol) was dissolved in polyphosphoric acid (80 g), to which was added 2-methylbenzaldehyde (0.31 g, 2.559 mmol) and the mixture was stirred at 105 °C for 2 h. The reaction mixture was poured into an ice-cold solution of sodium hydroxide (2N) and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound 16 (120 mg, 10.65%). ESI:(m/z) = 352.0 [M+H]⁺.

### Synthesis of compound 17

Compound 16 (115 mg, 0.326 mmol) was dissolved in 1,4-dioxane (5 mL), to which was added cesium carbonate (320 mg, 0.979 mmol), tert-butyl carbamate (57 mg, 0.49 mmol), XPHOS (31 mg, 0.065 mmol) and palladium acetate (7.3 mg, 0.033 mmol) and the resulting mixture was stirred at 100°C for 1 h, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 10/1 to 10/1) to give compound 17 (120 mg, 94.61%). ESI:(m/z) = 389.2 [M+H]⁺; ¹H NMR (400 MHz, Chloroform-d) δ 8.39 (dd, J = 7.2, 1.2 Hz, 1H), 8.02 (dd, J = 8.3, 1.3 Hz, 1H), 7.87 (s, 2H), 7.58 (dd, J = 8.2, 7.2 Hz, 1H), 7.22 (dd, J = 6.4, 1.5 Hz, 2H), 7.12 - 7.03 (m, 1H), 6.88 (d, J = 7.8 Hz, 1H), 6.37 - 6.21 (m, 2H), 5.93 (s, 1H), 2.45 (s, 3H), 1.43 (s, 9H).

### Synthesis of compound 18

Compound 17 (120 mg, 0.776 mmol) was dissolved in dichloromethane (5 mL), to which was added trifluoroacetic acid (1 mL) and the reaction mixture was stirred at room temperature for 1 hour. The reaction mixture was added dropwise to an ice-cold saturated sodium bicarbonate solution to adjust pH > 7, and diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 18 (100 mg). ESI:(m/z) = 289.1 [M+H]⁺.

### Synthesis of compound 1C

1-Benzothiophene-3-carboxylic acid (200 mg, 0.776 mmol) was dissolved in dichloromethane (5 mL). After degassed and purged with N₂ and cooled to 0°C, the resulting solution was added oxalyl chloride (0.285 ML, 3.367 mmol) and 2 drops of DMF and stirred at room temperature for 1 h. The reaction mixture was evaporated to dryness to obtain the crude compound 1C (200 mg).

### Synthesis of compound YL004

Compound 18 (40 mg, 0.139 mmol) was dissolved in dichloromethane (5 mL), to which was added pyridine (0.056 mL, 0.694 mmol). After degassed and purged with N₂ and cooled to 0°C, the resulting solution was added compound 1C (41 mg, 0.208 mmol) and stirred at room temperature for 0.5 h, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and prepared by HPLC to give compound YL004 (13 mg, 27.5%).

Data for compound YL004: ESI:(m/z) = 449.0 [M+H]⁺ ; ¹H NMR (400 MHz, DMSO-d6) δ 10.04 (s, 1H), 8.75 (d, J = 3.0 Hz, 1H), 8.25 (ddd, J = 18.1, 7.8, 1.3 Hz, 3H), 8.18 - 8.14 (m, 1H), 8.10 - 8.03 (m, 2H), 7.73 (dd, J = 8.3, 7.2 Hz, 1H), 7.54 (d, J = 8.7 Hz, 1H), 7.48 - 7.37 (m, 2H), 7.00 (td, J = 7.3, 1.4 Hz, 1H), 6.92 - 6.81 (m, 2H), 6.58 (d, J = 3.0 Hz, 1H), 6.51 (dd, J = 7.9, 1.4 Hz, 1H), 2.16 (s, 3H).

### Example 5 Synthetic route of compound YL005

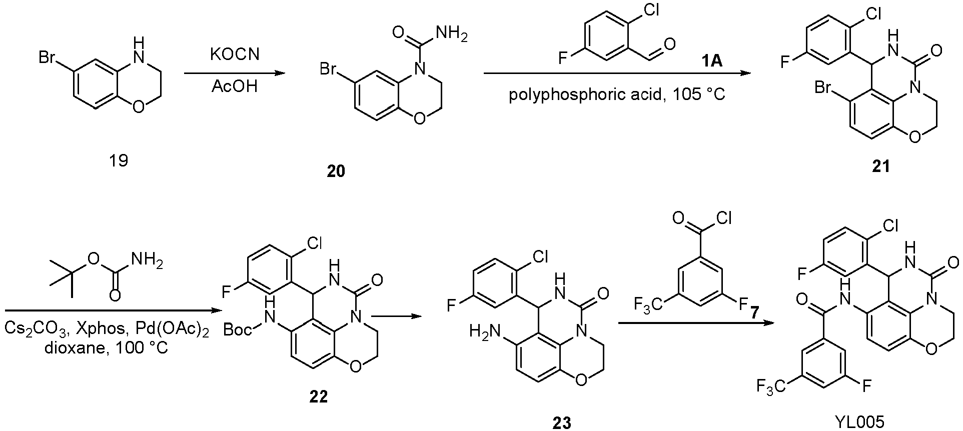

### Synthesis of compound 20

6-Bromo-3,4-dihydro-2H-benzo[1,4]oxazine hydrochloride (compound 19, 2 g, 9.43 mmol) was dissolved in acetic acid (15 mL). After degassed and purged with N₂, the suspension was added potassium cyanate (1.52 g, 18.686 mmol) at 0°C and stirred at 5°C for 1 h. The reaction mixture was concentrated to dryness and adjusted pH > 7 with saturated sodium bicarbonate solution. The organic phase was separated and the aqueous phase was extracted with dichloromethane. The organic phases were combined, washed with water, brine, dried over anhydrous Na₂SO₄, filtered and the filtrate was evaporated to give crude compound 20 (2.4 g, 99.76%). ESI: (m/z) = 257.0 [M+H]⁺; ¹H NMR (400 MHz, Chloroform-d) δ 7.54 (d, J = 2.4 Hz, 1H), 7.15 (dd, J = 8.8, 2.3 Hz, 1H), 6.81 (d, J = 8.7 Hz, 1H), 5.25 (s, 2H), 4.31 - 4.17 (m, 2H), 3.90 - 3.79 (m, 2H).

### Synthesis of compound 21

Compound 20 (1 g, 3.89 mmol) was dissolved in polyphosphoric acid (100 g), to which was added 2-chloro-5-fluorobenzaldehyde (0.62 g, 3.89 mmol) and reacted at 105°C for 20 min. The reaction mixture was poured into an ice-cold sodium hydroxide solution (2N) and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound 21 (600 mg, 38.71%). ESI:(m/z) = 398.9 [M+H]⁺; ¹H NMR (400 MHz, Chloroform-d ) δ 7.40 (dd, J = 8.8, 5.0 Hz, 1H), 7.14 (d, J = 8.7 Hz, 1H), 6.96 (ddd, J = 8.8, 7.6, 3.0 Hz, 1H), 6.87 (d, J = 8.7 Hz, 1H), 6.36 (dd, J = 8.9, 3.0 Hz, 1H), 6.07 (dd, J = 3.2 , 1.1 Hz, 1H), 5.75 (d, J = 3.3 Hz, 1H), 4.34 (ddd, J = 11.9, 6.2, 2.8 Hz, 1H), 4.26 - 4.16 (m, 2H), 3.71 (ddd, J = 13.1, 6.8, 2.9 Hz, 1H).

### Synthesis of compound 22

Compound 21 (300 mg, 0.754 mmol) was dissolved in 1,4-dioxane (5 mL), to which were added cesium carbonate (737.46 mg, 2.263 mmol), tert-butyl carbamate (132.6 mg, 1.132 mmol), XPHOS (72 mg, 0.151 mmol) and palladium acetate (17 mg, 0.075 mmol). The reaction mixture was stirred at 100°C for 1 h, then diluted with dichloromethane and water. The organic phases were separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 10/1 to 10/1) to give compound 22 (100 mg, 65.44%). ESI: (m/z) = 434.1 [M+H]⁺ ; ¹H NMR (400 MHz, Chloroform- d) δ 7.40 (dd, J = 8.8, 5.0 Hz, 1H), 7.02 - 6.90 (m, 3H), 6.50 (dd, J = 8.9, 3.0 Hz, 1H), 6.07 (dd, J = 3.2, 1.2 Hz, 1H), 5.58 (d, J = 5.1 Hz, 2H), 4.32 (ddd , J = 11.1, 5.9, 2.9 Hz, 1H), 4.23 (dt, J = 7.5, 3.8 Hz, 1H), 4.20 - 4.12 (m, 1H), 3.75 (ddd, J = 13.4, 6.6, 2.9 Hz, 1H), 1.39 (s, 9H).

### Synthesis of compound 23

Trifluoroacetic acid (1 mL) was added to a solution of compound 22 (100 mg, 0.776 mmol) in dichloromethane (5 mL) and the reaction mixture was stirred at room temperature for 1 h. The reaction mixture was added dropwise to an ice-cold saturated sodium bicarbonate solution to adjust pH > 7, and diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 23 (100 mg). ESI:(m/z) = 334.1 [M+H]⁺.

### Synthesis of compound YL005

Compound 23 (70 mg, 0.21 mmol) was dissolved in dichloromethane (5 mL), to which was added pyridine (0.034 mL, 0.419 mmol). After degassed and purged with N₂ and cooled to 0°C, the mixture was added 3-fluoro-5-(trifluoromethyl)benzoyl chloride (57 mg, 0.252 mmol) and stirred at room temperature for 1 h, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and prepared by HPLC to give compound YL005 (44 mg, 40.05%). ESI:(m/z) = 522.0 [M+H]⁺ ; ¹H NMR (400 MHz, DMSO-d6) δ 10.03 (s, 1H), 7.93 (d, J = 5.7 Hz, 2H), 7.87 (dt, J = 11.4, 2.5 Hz, 2H), 7.33 (dd, J = 8.9, 5.1 Hz, 1H), 7.12 (ddd, J = 8.8, 7.9, 3.1 Hz, 1H), 6.94 (d, J = 8.6 Hz, 1H), 6.83 (dd, J = 9.2, 3.1 Hz, 1H), 6.77 (d, J = 8.6 Hz, 1H), 6.07 (dd, J = 3.1, 1.3 Hz, 1H), 4.37 (ddd, J = 11.1, 6.3, 2.9 Hz, 1H), 4.25 (ddd, J = 11.2, 6.1, 3.0 Hz, 1H), 3.95 (ddd, J = 13.3, 6.4, 3.1 Hz, 1H), 3.79 (ddd, J = 13.3, 6.2, 2.9 Hz, 1H).

### Example 6 Synthetic route of compound YL006

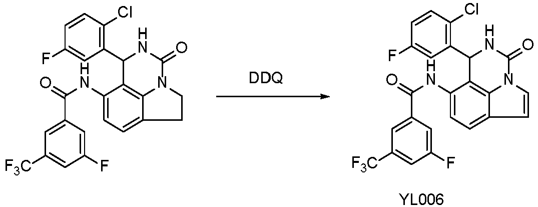

YL001 (15 mg, 0.05 mmol), DDQ (8 mg, 0.1 mmol) and anhydrous 1,4-dioxane (1 mL) were combined in a reaction vial. The mixture was stirred at room temperature for 16 h. The reaction was monitored by LCMS and quenched by the addition of brine upon completion. The aqueous phase was extracted with ethyl acetate. The organic phases were combined, washed with brine and concentrated at reduced pressure to obtain the crude product. The resulting crude product was separated by SFC to give (5 mg, 33%) compound YL006. LC-MS (ESI): m/z 506.1 (M+H)⁺.

### Example 7 Synthetic route of compound YL007

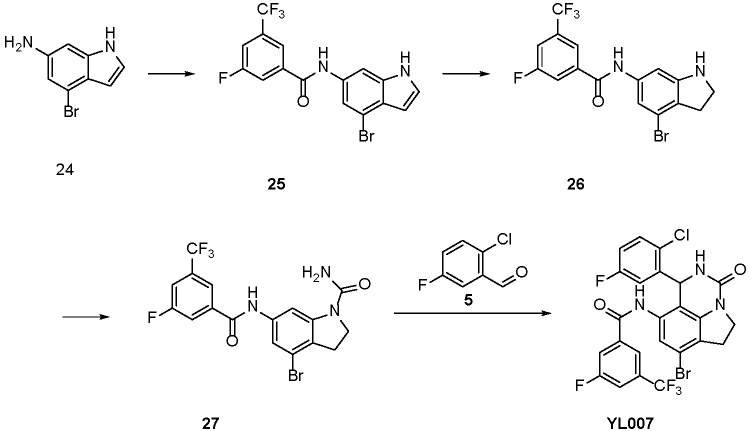

### Synthesis of compound 25

To a 100 mL of three-necked flask were sequentially added 4-bromo-1H-indol-6-amine (compound 24, 1000 mg, 4.738 mmol), triethylamine (1.317 mL, 9.476 mmol) and dichloromethane (40 mL). Cooled to 0°C, the above mixture was added 3-fluoro-5-(trifluoromethyl)benzoyl chloride (1180.73 mg, 5.212 mmol) and stirred at 0°C for 30 min, then stirred at room temperature for 1 h. The reaction mixture was concentrated and purified by column chromatography (eluent: petroleum ether: ethyl acetate = 5:1-3:1) to give compound 25 (1809 mg, 4.509 mmol, 95.18%).

### Synthesis of compound 26

Compound 25 (802 mg, 1.999 mmol), acetic acid (30 mL) and sodium cyanoborohydride (376.89 mg, 5.998 mmol) were added sequentially in a 100 mL three-necked flask. The reaction mixture was stirred at room temperature for 18 h, then concentrated and added with ethyl acetate and saturated aqueous sodium bicarbonate, and the organic phase was concentrated and purified by column chromatography (eluent: petroleum ether: ethyl acetate = 10:1-3:1) to give compound 26 (350 mg, 0.868 mmol, 43.42%).

### Synthesis of compound 27

To a 100 mL three-necked flask was added compound 26 (320 mg, 0.794 mmol) and acetic acid (10 mL) sequentially. Then cooled to 0°C, the mixture was added potassium cyanate (128.77 mg, 1.587 mmol). The reaction mixture was stirred at 0°C for half an hour. The reaction mixture was concentrated and the residue was added potassium cyanate (128.77 mg, 1.587 mmol). Filtered, and the filter cake was washed with water and dried to give compound 27 (280 mg, 0.628 mmol, 79.06%).

### Synthesis of compound YL007

Polyphosphoric acid (20 mL) in a 100 mL of three-necked flask was heated to 105°C, then was added compound 27 (150 mg, 0.336 mmol) and 2-chloro-5-fluorobenzaldehyde (53.28 mg, 0.336 mmol) sequentially. The reaction continued at this temperature for 30 min. to which were added dichloromethane and saturated aqueous sodium bicarbonate, the organic phase was concentrated and purified by column chromatography (eluent: dichloromethane: methanol = 20:1-10:1) to give compound YL007 (47 mg, 0.080 mmol, 23.83%). LC-MS (ESI): m/z 587.9 (M+H)⁺; ¹H NMR (400 MHz, DMSO-d₆) δ 10.16 (s, 1H), 7.95 (dt, J = 8.6, 1.9 Hz, 1H), 7.89 - 7.77 (m, 2H), 7.54 (d, J = 2.6 Hz, 1H), 7.32 (dd, J = 8.8, 5.2 Hz, 1H), 7.09 (ddd, J = 8.8, 8.0, 3.1 Hz, 1H), 6.95 (s, 1H), 6.90 (dd, J = 9.2, 3.1 Hz, 1H), 6.14 - 5.97 (m, 1H), 4.16 - 3.87 (m, 2H), 3.17 (ddd, J = 9.6, 6.9, 4.6 Hz, 2H).

### Example 8 Synthetic route of compound YL008

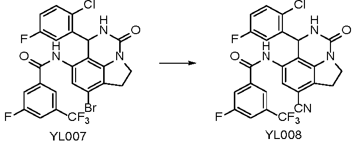

YL007 (50 mg, 0.085 mmol), zinc cyanide (15.01 mg, 0.128 mmol), zinc powder (0.56 mg, 0.009 mmol), Pd(PPh₃)₄ (9.85 mg, 0.009 mmol) and DMF (1 mL) were added sequentially to a microwave tube (10 mL). The resulting mixture was microwaved at 120 °C under nitrogen protection for 1.5h. Upon completion, the reaction mixture was directly subjected to reversed-phase column chromatographed (eluent: 10 nM NH₄HCO₃: MeCN=40:60) to yield compound YL008 (4 mg, 0.008 mmol, 8.81%). LC-MS (ESI): m/z 533.1 (M+H)⁺; ¹H NMR (400 MHz, DMSO-d₆) δ 10.26 (s, 1H), 7.96 (d, J = 8.4 Hz, 1H), 7.84 (d, J = 15.3 Hz, 2H), 7.63 (d, J = 2.5 Hz, 1H), 7.33 (dd, J = 8.9, 5.1 Hz, 1H), 7.18 - 7.04 (m, 2H), 6.93 (dd, J = 9.1, 3.1 Hz, 1H), 6.15 (d, J = 2.7 Hz, 1H), 4.07 (td, J = 8.9, 6.8 Hz, 2H), 3.35 (s, 2H).

### Example 9 Synthetic route of compound YL009

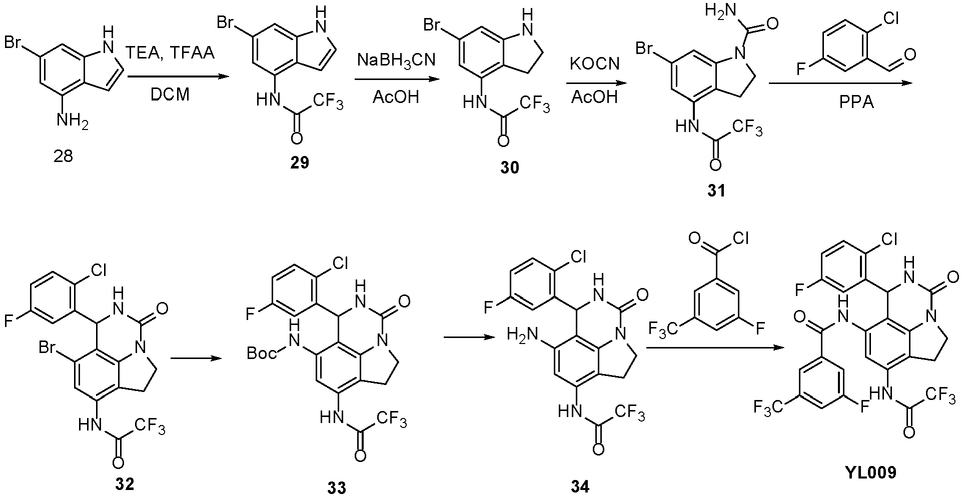

### Synthesis of compound 29

Triethylamine (8.891 mL, 63.963 mmol) was added to a solution of 6-Bromo-4-aminoindole (compound 28, 4.5 g, 21.321 mmol) in dichloromethane (50 mL). After degassed and purged with N₂, the above mixture was added trifluoroacetic anhydride (8.96 g, 42.642 mmol) at 0°C, then warmed to room temperature and stirred for 3 h. The reaction mixture was diluted with ethyl acetate and water. The organic phases were separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 29 (5.8 g, 88.55%). ESI:(m/z) = 306.9 [M+H]⁺.

### Synthesis of compound 30

Compound 29 (3.85 g, 12.538 mmol) was dissolved in acetic acid (40 mL), to which was added sodium cyanoborohydride (2.36 g, 37.614 mmol) and the resulting mixture was stirred at room temperature for 2 h, then was added supplemental sodium cyanoborohydride (2.36 g, 37.614 mmol) and stirred at room temperature for 2h. The reaction mixture was concentrated to dryness and poured into ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 30 (1.47 g, 37.89%). ESI:(m/z) = 309.8 [M+H]⁺.

### Synthesis of compound 31

A suspension of compound 30 (1.47 g, 4.756 mmol) in acetic acid (15 mL) was cooled to 5°C and added potassium cyanate (770 mg, 9.512 mmol), then the reaction mixture was stirred at 5°C for 1 h. The reaction mixture was concentrated to dryness and adjusted to basic by adding saturated aqueous sodium bicarbonate and water, filtered to obtain the crude compound 31 (1.58 g, 94.6%). ESI:(m/z) = 351.9 [M+H]⁺.

### Synthesis of compound 32

Compound 31 (1.5 g, 4.26 mmol) was dissolved in polyphosphoric acid (150 g), to which was added 2-chloro-5-fluorobenzaldehyde (1.22 g, 7.668 mmol) and the resulting mixture was stirred at 105°C for 0.5 h. The reaction mixture was dropped into an ice-cold saturated sodium bicarbonate and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 32 (800 mg, 30%). ESI:(m/z) = 491.9 [M+H]⁺.

### Synthesis of compound 33

Cesium carbonate (1527.8 mg, 2.263 mmol), tert-butyl carbamate (366.21 mg, 3.126 mmol), XPHOS (149 mg, 0.313 mmol) and palladium acetate (35 mg, 0.156 mmol) were added to a solution of compound 32 (770 mg, 1.563 mmol) in 1,4-dioxane (10 mL), and the resulting mixture was stirred at 100 °C overnight, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 10/1 to 20/1) to give compound 33 (170 mg, 51.95%). ESI:(m/z) = 434.1 [M+H]⁺.

### Synthesis of compound 34

Trifluoroacetic acid (1 mL) was added to a solution of compound 33 (170 mg, 0.321 mmol) in dichloromethane (3 mL) and the reaction mixture was stirred at room temperature for 1 h. The reaction mixture was added dropwise to an ice-cold saturated sodium bicarbonate solution to adjust pH > 7, and diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give crude compound 34 (140 mg). ESI:(m/z) = 429.0 [M+H]⁺.

### Synthesis of compound YL009

Pyridine (0.056 mL, 0.694 mmol) was added to a solution of compound 34 (140 mg, 0.350 mmol) in dichloromethane (10 mL). After degassed and purged with N₂ and cooled to 0°C, the above mixture was added 3-fluoro-5-(trifluoromethyl)benzoyl chloride (118.9 mg, 0.525 mmol) and stirred at room temperature for 5 h, then diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound YL009 (15 mg). ESI:(m/z) = 619.0 [M+H]⁺; ¹H NMR (400 MHz, DMSO-d6) δ 11.24 (s, 1H), 10.19 (s, 1H), 7.95 (d, J = 8.6 Hz, 1H), 7.85 (d, J = 12.7 Hz, 2H), 7.52 (d, J = 2.6 Hz, 1H), 7.33 (dd, J = 8.8, 5.2 Hz, 1H), 7.10 (ddd, J = 8.8, 7.9, 3.1 Hz, 1H), 6.86 (dd, J = 9.2, 3.1 Hz, 1H), 6.79 (s, 1H), 6.09 (dd, J = 2.6, 1.0 Hz, 1H), 4.17 - 3.83 (m, 2H), 3.21 - 2.99 (m, 2H).

### Synthetic route of compound YL010-P1 and YL010-P2

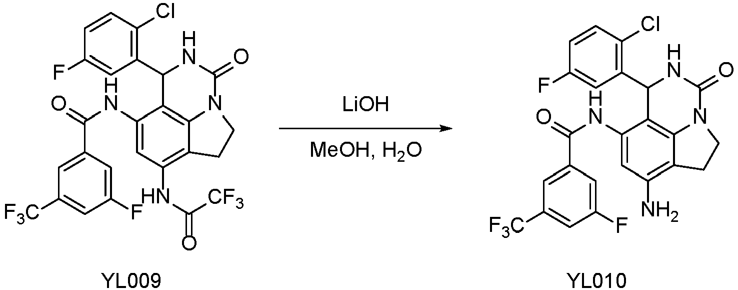

### Synthesis of compound YL010

Water (1 mL) and LiOH (28.5 mg, 0.679 mmol) were added to a solution of compound YL009 (70 mg, 0.113 mmol) in methanol (3 mL), and the reaction mixture was stirred at 60°C for 3 h. The reaction mixture was concentrated to dryness and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol = 1/0 to 20/1) to give compound YL010 (11 mg). ESI:(m/z) = 523.2 [M+H]⁺; ¹HNMR (400 MHz, DMSO-d6) δ 9.92 (s, 1H), 7.93 - 7.88 (m, 1H), 7.87 - 7.78 (m, 2H), 7.28 (dd, J = 8.8, 5.2 Hz, 1H), 7.24 (d, J = 2.7 Hz, 1H), 7.05 (ddd, J = 8.8, 7.9, 3.1 Hz, 1H), 6.77 (dd, J = 9.3, 3.1 Hz, 1H), 6.03 (s, 1H), 5.92 (dd, J = 2.7, 1.3 Hz, 1H), 5.29 (s, 2H), 4.02 - 3.87 (m, 2H), 2.96 (dd, J = 9.5, 7.4 Hz, 2H).

Compound YL010 was purified by SFC chiral resolution to obtain compounds YL010-P1 and YL010-P2.
Chiral resolution conditions: instrument: SFC-150 (Waters); column: AS 20*250mm, 10um (Daicel)
Column temperature: 35 °C; mobile phase: CO₂/MeOH [0.2% NH₃ (7M in MeOH)] = 70/30
Flow rate: 100 g/min; back pressure: 100 bar; wavelength: 214 nm;
Sample solution: 198 mg in 40 mL of methanol; injection volume: 2.0 mL

The compounds YL010-P1 (retention time 5.153 min) and YL010-P2 (retention time 5.581 min) were obtained by SFC chiral resolution.

YL010-P1: LC-MS (ESI): m/z 522.86(M+H)⁺; ¹H NMR (400 MHz, DMSO-d₆) δ 9.93 (s, 1H), 7.85 (s,3H), 7.36 - 7.24 (m, 2H), 7.17 - 7.03 (m, 1H), 6.78 (d, J = 9.3 Hz, 1H), 6.04 (s, 1H), 5.97 - 5.80 (m, 1H), 5.30 (s, 2H),3.98 (m,2H),2.96 (d, J = 2.1 Hz, 2H).

YL010-P2 : LC-MS (ESI): m/z 522.86(M+H) ⁺; ¹H NMR (400 MHz, DMSO-d₆) δ 9.93 (s, 1H), 7.85 (s, 3H), 7.26 (m, 2H), 7.11 (t, 1H), 6.78 (d, 1H), 6.04 (s, 1H), 5.93 (s, 1H), 5.30 (s, 2H), 3.95 (dt, J = 13.1, 8.4 Hz, 2H), 2.96 (t, 2H).

### Synthetic route of compound YL011

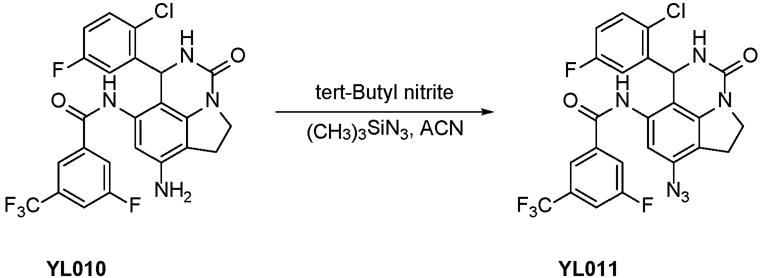

### Synthesis of compound YL011

A solution of compound YL010 (30 mg, 0.057 mmol) in acetonitrile (3 mL) was degassed and purged with N₂, then cooled to 0°C, was added tert-butyl nitrite (8.88 mg, 0.086 mmol) and azidotrimethylsilane (7.93 mg, 0.069 mmol), and the resulting mixture was stirred at room temperature for 1 h. The reaction mixture was concentrated to dryness and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol = 1/0 to 20/1) to give compound YL011 (6.4 mg). ESI:(m/z) = 549.1 [M+H]⁺; ¹H NMR (400 MHz, DMSO-*d6)* δ 10.16 (s, 1H), 7.95 (dt, J = 8.6, 2.0 Hz, 1H), 7.85 (d, J = 12.5 Hz, 2H), 7.51 (d, J = 2.6 Hz, 1H), 7.32 (dd, J = 8.8, 5.2 Hz, 1H), 7.09 (ddd, J = 8.9, 8.0, 3.1 Hz, 1H), 6.86 (dd, J = 9.2, 3.1 Hz, 1H), 6.62 (s, 1H), 6.08 (dd, J = 2.6, 1.0 Hz, 1H), 4.07 - 3.92 (m, 2H), 3.16 (td, J = 8.3, 2.3 Hz, 2H).

### Synthetic route of compound YL012

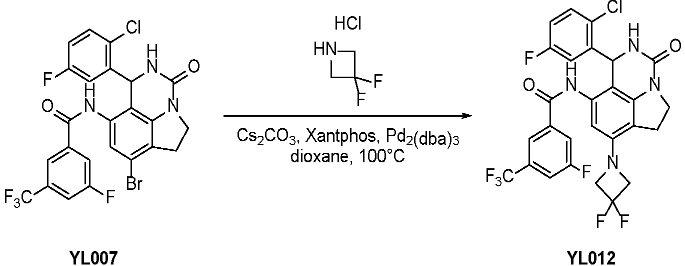

### Synthesis of compound YL012

Compound YL007 (100 mg, 0.170 mmol) was dissolved in 1,4-dioxane (5 mL). Cesium carbonate (110.78 mg, 0.34 mmol), 3,3-difluorotrimethylenimine hydrochloride (15.86 mg, 0.187 mmol), Xantphos (9.84 mg, 0.017 mmol) and Pd₂(dba)₃ (7.78 mg, 0.009 mmol) were added to the above mixture. After degassed and purged with N₂, the reaction mixture was stirred at 100°C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol = 1/0 to 20/1) to give compound YL012 (58 mg). ESI:(m/z) = 599.0 [M+H]⁺; ¹H NMR (400 MHz, DMSO-d6) δ 10.06 (s, 1H), 7.92 (dd, J = 8.4, 2.4 Hz, 1H), 7.89 - 7.81 (m, 2H), 7.40 (d, J = 2.7 Hz, 1H), 7.30 (dd, J = 8.8, 5.2 Hz, 1H), 7.07 (ddd, J = 8.9, 7.9, 3.1 Hz, 1H), 6.80 (dd, J = 9.3, 3.1 Hz, 1H), 5.99 (dd, J = 2.6, 1.2 Hz, 1H), 5.93 (s, 1H), 4.47 - 4.27 (m, 4H), 4.02 - 3.87 (m, 2H), 3.19 (t, J = 8.5 Hz, 2H).

### Synthetic route of compound YL013

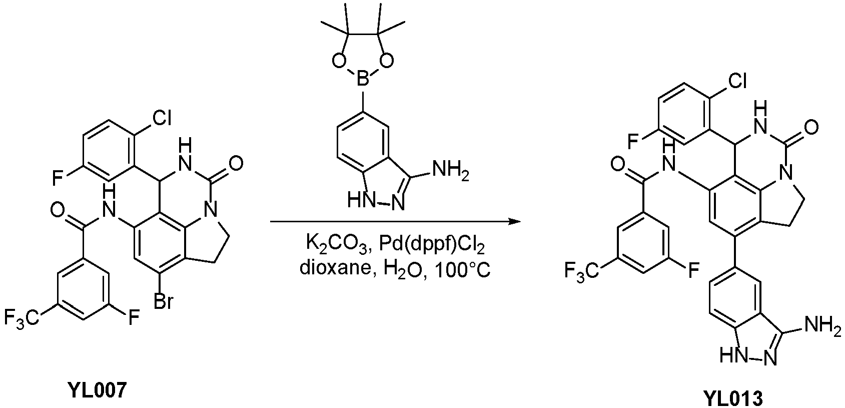

### Synthesis of compound YL013

Compound YL007 (100 mg, 0.170 mmol) was dissolved in 1,4-dioxane (5 mL) and water (1 mL), to which were added potassium carbonate (47.11 mg, 0.341 mmol), 3-amino-1H-indazole-5-boronic acid pinacol ester (50 mg, 0.187 mmol) and Pd(dppf)Cl₂ (12.5 mg. 0.017 mmol). After degassed and purged with N₂, the reaction mixture was stirred at 100°C for 1 hr, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol = 1/0 to 20/1) to give compound YL013 (45 mg). ESI:(m/z) = 639.2 [M+H]⁺; ¹H NMR (400 MHz, DMSO-d6) δ 11.48 (s, 1H), 10.21 (s, 1H), 7.92 (q, J = 10.9, 9.2 Hz, 4H), 7.53 (d, J = 2.7 Hz, 1H), 7.44 (dd, J = 8.7, 1.7 Hz, 1H), 7.38 - 7.27 (m, 2H), 7.11 (ddd, J = 8.9, 7.9, 3.1 Hz, 1H), 6.95 - 6.85 (m, 2H), 6.15 (dd, J = 2.8, 1.1 Hz, 1H), 5.44 (s, 2H), 4.12 - 3.93 (m, 2H), 3.48 (dt, J = 17.2, 8.7 Hz, 1H), 2.28-3.30 (m, 1H).

### Synthetic route of compound YL014

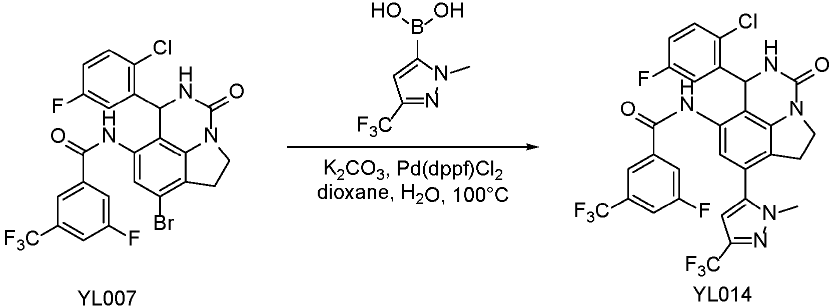

### Synthesis of compound YL014

Compound YL007 (100 mg, 0.170 mmol) was dissolved in 1,4-dioxane (2 mL) and water (0.4 mL), to which were added potassium carbonate (47.11 mg, 0.341 mmol), 1-methyl-3-trifluoromethylpyrazole-5-boronic acid (82.42 mg, 0.425 mmol) and Pd(dppf)Cl₂ (12.5 mg, 0.017 mmol). After degassed and purged with N₂, the reaction mixture was stirred at 100°C 1 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol = 1/0 to 20/1) to give compound YL014 (65 mg). ESI:(m/z) = 656.0 [M+H]⁺; ¹H NMR (400 MHz, DMSO-d6) δ 10.22 (s, 1H), 7.95 (d, J = 8.5 Hz, 1H), 7.92 - 7.84 (m, 2H), 7.58 (d, J = 2.6 Hz, 1H), 7.35 (dd, J = 8.8, 5.2 Hz, 1H), 7.11 (ddd, J = 8.8, 7.9, 3.1 Hz, 1H), 6.99 (s, 1H), 6.94 (dd, J = 9.2, 3.0 Hz, 1H), 6.91 (s, 1H), 6.18 - 6.13 (m, 1H), 4.08 - 3.95 (m, 2H), 3.88 (s, 3H), 3.22 (t, J = 8.5 Hz, 2H).

### Synthetic route of compound YL015

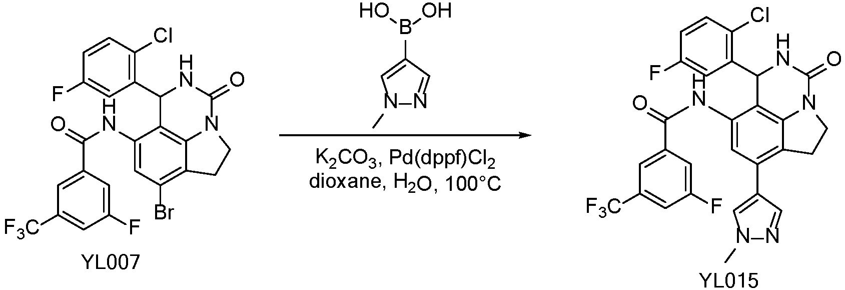

### Synthesis of compound YL015

Compound YL007 (100 mg, 0.170 mmol) was dissolved in 1,4-dioxane (2 mL) and water (0.4 mL), to which were added potassium carbonate (47.11 mg, 0.341 mmol), 1-methyl-1H-pyrazole-4-boronic acid (50 mg, 0.187 mmol) and Pd(dppf)Cl₂ (12.5 mg. 0.017 mmol). After degassed and purged with N₂, the reaction mixture was stirred at 100°C for 1 hr, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol = 1/0 to 20/1) to give compound YL015 (60 mg). ESI:(m/z) = 588.1 [M+H]⁺; ¹H NMR (400 MHz, DMSO-d6) δ 10.16 (s, 1H), 8.11 (s, 1H), 7.99 - 7.78 (m, 4H), 7.47 (d, J = 2.6 Hz, 1H), 7.32 (dd, J = 8.8, 5.1 Hz, 1H), 7.09 (td, J = 8.4, 3.0 Hz, 1H), 6.97 (s, 1H), 6.88 (dd, J = 9.2, 3.1 Hz, 1H), 6.12 - 6.01 (m, 1H), 4.04 (ddd, J = 20.3, 10.4, 4.5 Hz, 2H), 3.89 (s, 3H), 3.37-3.44 (m, 2H).

### Synthetic route of compound YL016

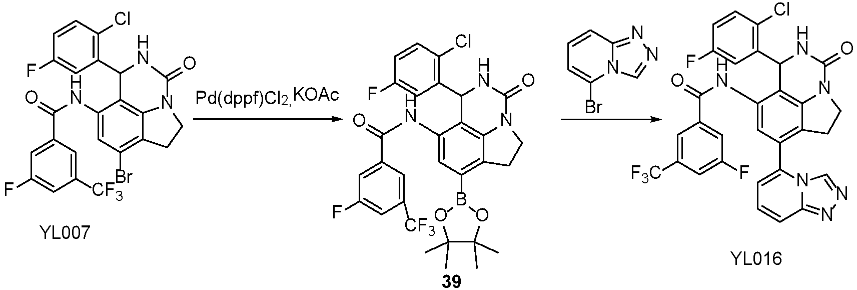

### Synthesis of compound 39

Compound YL007 (0.5 g, 0.852 mmol), potassium acetate (250.85 mg, 2.556 mmol), biboronic acid pinacol ester (540.89 mg, 2.130 mmol), and dichloro[1,1'-bis(diphenylphosphino)ferrocene]palladium (62.34 mg, 0.085 mmol) were placed in a reaction flask and dissolved in dioxane (7 mL). Protected by nitrogen, the resulting mixture was warmed to 100°C, and stirred for two hours, then was added potassium acetate (83.6 mg, 0.852 mmol), biboronic acid pinacol ester (216.0 mg, 0.852 mmol) and dichloro[1,1'-bis(diphenylphosphino)ferrocene]palladium (62.34 mg, 0.085 mmol), and continued the reaction for two hours, then concentrated. The residue was purified by Prep-TLC (unfolding agent DCM: MeOH=10:1) to give a yellowish brown solid target compound 39 (400 mg, 74.06%). LC-MS (ESI): m/z 634.1(M+H) ⁺.

### Synthesis of compound YL016

Compound 39 (100 mg, 0.158 mmol), potassium carbonate (39.7 mg, 0.287 mmol), 5-bromo[1,2,4]triazolo[4,3-a]pyridine (28.4 mg, 0.143 mmol), dichloro[1,1'-bis(diphenylphosphino)ferrocene]palladium (10.51 mg, 0.014 mmol) were combined in a reaction vial, then dissolved in dioxane (2 mL) and water (0.4 mL). Protected by nitrogen, the resulting mixture was warmed to 100°C and stirred for two hours, then removed solvent, and the residue was purified by column chromatography (mobile phase: dichloromethane/methanol 1/0 to 20/1) to give the target compound YL016 (25 mg, 27.89%). LC-MS (ESI): m/z 625.1(M+H)⁺; ¹H NMR (400 MHz, DMSO-d6) δ 10.26 (s, 1H), 9.15 (s, 1H), 8.07 - 7.77 (m, 4H), 7.62 (d, J = 2.6 Hz, 1H), 7.51 (dd, J = 9.3, 6.7 Hz, 1H), 7.36 (dd, J = 8.8, 5.1 Hz, 1H), 7.19 - 6.95 (m, 4H), 6.24 (d, J = 2.6 Hz, 1H), 4.13 - 3.92 (m, 2H), 3.19 - 3.05 (m, 2H).

### Synthetic route of compound YL017

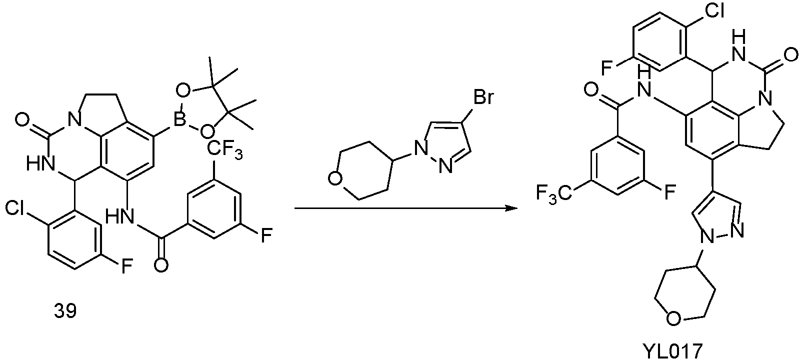

Compound 39 (100 mg, 0.158 mmol), potassium carbonate (39.7 mg, 0.287 mmol), 4-bromo-1-(3,4,5,6-tetrahydropyran-4-yl)pyrazole (33.15 mg, 0.143 mmol), dichloro[1,1'-bis(diphenylphosphino)ferrocene]palladium (10.51 mg, 0.014 mmol ) were combined in a reaction vial, dissolved in dioxane (2 mL) and water (0.4 mL). Protected by nitrogen, the resulting mixture was warmed to 100°C and stirred for two hours, then was removed the solvent, and purified by column chromatography (mobile phase: dichloromethane/methanol 1/0 to 20/1) to give the target compound YL019 (15 mg, 15.89%). LC-MS (ESI): m/z 658.16(M+H) ⁺, ¹HNMR (400 MHz, DMSO-d6) δ 10.18 (s, 1H), 8.21 (s, 1H), 8.00 - 7.82 (m, 4H), 7.48 (d, J = 2.7 Hz, 1H), 7.33 (dd, J = 8.8, 5.1 Hz, 1H), 7.15 - 7.06 (m, 1H), 7.00 (s, 1H), 6.87 (dd, J = 9.2, 3.1 Hz, 1H), 6.08 (dd, J = 2.7, 1.1 Hz, 1H), 4.51 - 4.38 (m, 1H), 4.15 - 3.94 (m, 4H), 3.57 - 3.33 (m, 4H), 2.00 (td, J = 10.2, 9.0, 4.0 Hz, 4H).

### Synthetic route of compound YL018

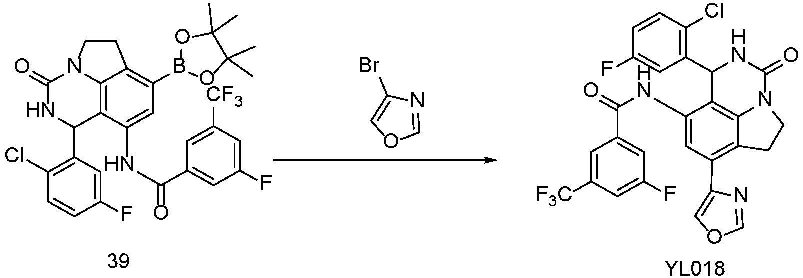

### Synthesis of compound YL018

Compound 39 (100 mg, 0.158 mmol), potassium carbonate (39.7 mg, 0.287 mmol), 4-bromo-1,3-oxazole (21.22 mg, 0.143 mmol), and dichloro[1,1'-bis(diphenylphosphino)ferrocene]palladium (10.51 mg, 0.014 mmol) were placed in a reaction vial, then dissolved in dioxane (2 mL) and water (0.4 mL). Protected by nitrogen, the resulting mixture was warmed to 100°C and stirred for two hours, then removed the solvent, and the residue was purified by column chromatography (mobile phase: dichloromethane/methanol 1/0 to 20/1)to give the target compound YL018 (33 mg, 40.02%). LC-MS (ESI): m/z 575.08(M+H) ⁺. ¹H NMR (400 MHz, DMSO-d6) δ 10.26 (s, 1H), 8.28 (d, J = 0.8 Hz, 1H), 7.90 (s, 1H), 7.89 (m, 2H),7.56 (d, J = 2.6 Hz, 1H), 7.46 (d, J = 0.8 Hz, 1H), 7.39 (s, 1H), 7.35 (m, 1H), 7.11 (m, 1H), 6.96 - 6.89 (m, 1H), 6.21 - 6.13 (m, 1H), 4.07 (d, J = 10.7 Hz, 2H), 3.56 (d, J = 8.5 Hz, 2H).

### Synthetic route of compound YL019

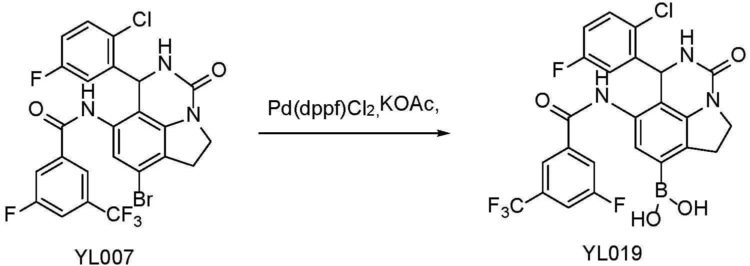

YL007 (100 mg, 0.170 mmol), potassium acetate (50.17 mg, 0.511 mmol), biboronic acid pinacol ester (108.18 mg, 0.426 mmol), and dichloro[1,1'-bis(diphenylphosphino)ferrocene]palladium (12.47 mg, 0.017 mmol) were placed in a reaction flask, then dissolved in dioxane ( 1 mL). Protected by nitrogen, the resulting mixture was warmed to 100°C, and stirred for two hours, then was added potassium acetate (16.72 mg, 0.170 mmol), biboronic acid pinacol ester (43.2 mg, 0.170 mmol) and dichloro[1,1'-bis(diphenylphosphino)ferrocene]palladium (12.47 mg, 0.017 mmol) and the reaction continued stirring for two hours, subjected to reversed phase purification ( mobile phase: water/acetonitrile 1/0 to 2/1) to give compound YL019 (13 mg, 13.83%). LC-MS (ESI): m/z 552.08(M+H) ⁺.

### Synthetic route of compound YL020

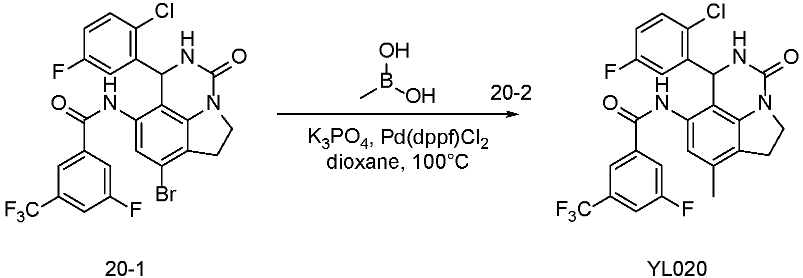

### Synthesis of compound YL020

Potassium phosphate (54, 26 mg, 0.256 mmol), 20-2 (20 mg, 0.341 mmol) and Pd(dppf)Cl₂ (6.24 mg, 0.009 mmol) were added to a suspension of 20-1 (50 mg, 0.085 mmol) in 1,4-dioxane (2 mL). After degassed and purged with N₂, the reaction mixture was stirred at 100°C for 1 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol = 1/0 to 20/1) to give compound YL020 (17 mg). ESI:(m/z) = 522.1 [M+H]⁺; ¹H NMR (400 MHz, DMSO-d6) δ 10.07 (s, 1H), 7.93 (d, J = 8.5 Hz, 1H), 7.89 - 7.82 (m, 2H), 7.40 (d, J = 2.5 Hz, 1H), 7.31 (ddd, J = 8.9, 5.1, 1.6 Hz, 1H), 7.08 (tt, J = 8.8, 2.4 Hz, 1H), 6.82 (dt, J = 9.3, 2.3 Hz, 1H), 6.57 (s, 1H), 6.06 (s, 1H), 4.00 (ddd, J = 19.8, 10.0, 5.2 Hz, 2H), 3.13 (t, J = 8.8 Hz, 2H), 2.21 (s, 3H).

### Synthetic route of compound YL021

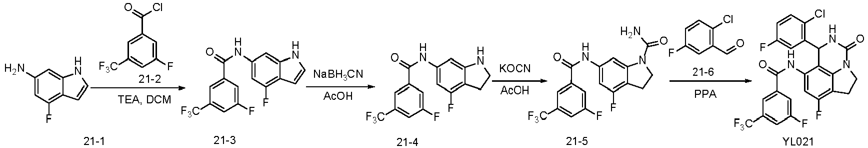

### Synthesis of compound 21-3

Triethylamine (0.56 mL, 3.996 mmol) was added to a solution of 21-1 (300 mg, 1.998 mmol) in dichloromethane (8 mL). After degassed and purged with N₂, the above mixture was added 3-fluoro-5-(trifluoromethyl)benzoyl chloride (500 mg, 2.198 mmol) at 0°C. The reaction mixture was warmed to room temperature and stirred for 1 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 21-3 (600 mg, 93.19 %). ESI:(m/z) = 341.0 [M+H]⁺; ¹H NMR (400 MHz, DMSO-d6) δ 11.48 - 11.39 (m, 1H), 10.51 (s, 1H), 8.18 (td, J = 1.6, 0.8 Hz, 1H), 8.13 (dt, J = 9.3, 2.0 Hz, 1H), 7.96 (dt, J = 8.6, 2.0 Hz, 1H), 7.89 (t, J = 1.2 Hz, 1H), 7.37 (dd, J = 3.1, 2.4 Hz, 1H), 7.21 (dd, J = 12.5, 1.5 Hz, 1H), 6.46 (ddd, J = 3.0, 2.0, 0.9 Hz, 1H).

### Synthesis of compound 21-4

Sodium cyanoborohydride (166 mg, 2.645 mmol) was added to a suspension of compound 21-3 (300 mg, 0.882 mmol) in acetic acid (5 mL), and the reaction mixture was stirred at room temperature overnight. The reaction mixture was concentrated to dryness and poured into ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phase: petroleum ether/ethyl acetate 1/0 to 3/1) to give compound 21-4 (160 mg, 37.89%). ESI:(m/z) = 343.2 [M+H]⁺; ¹H NMR (400 MHz, DMSO-d6) δ 10.31 (s, 1H), 8.13 - 8.10 (m, 1H), 8.08 (dt, J = 9.3, 2.0 Hz, 1H), 7.95 (dt, J = 8.7, 2.0 Hz, 1H), 6.88 - 6.79 (m, 2H), 6.00 - 5.95 (m, 1H), 3.50 (td, J = 8.6, 1.8 Hz, 2H), 2.93 (t, J = 8.5 Hz, 2H).

### Synthesis of compound 21-5

Potassium cyanate (76 mg, 0.935 mmol) was added to a suspension of 21-4 (160 mg, 0.467 mmol) in acetic acid (5 mL) at 5°C, and the reaction mixture was stirred at 5°C for 0.5 h. The reaction mixture was concentrated to dryness, and the residue was added saturated aqueous sodium bicarbonate to adjust to basic, and added water, filtered to obtain the crude compound 21-5 (200 mg). ESI:(m/z) = 386.1 [M+H]⁺.

### Synthesis of compound YL021

2-chloro-5-fluorobenzaldehyde (148 mg, 7.668 mmol) was added to a suspension of 21-5 (180 mg, 0.845 mmol) in polyphosphoric acid (20 g) and the reaction mixture was stirred at 105°C for 2 h. The reaction mixture was added dropwise to an ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) to give compound YL021 (64 mg). ESI:(m/z) = 526.0 [M+H]⁺; ¹H NMR (400 MHz, DMSO-d6) δ 10.17 (s, 1H), 7.95 (dt, J = 8.6, 1.9 Hz, 1H), 7.84 (d, J = 14.9 Hz, 2H), 7.53 (d, J = 2.6 Hz, 1H), 7.32 (dd, J = 8.8, 5.2 Hz, 1H), 7.13 - 7.04 (m, 1H), 6.87 (dd, J = 9.2, 3.1 Hz, 1H), 6.63 (d, J = 9.9 Hz, 1H), 6.08 (d, J = 2.5 Hz, 1H), 4.04 (pd, J = 10.4, 7.8 Hz, 2H), 3.25 (t, J = 8.6 Hz, 2H).

### Synthetic route of compound YL022

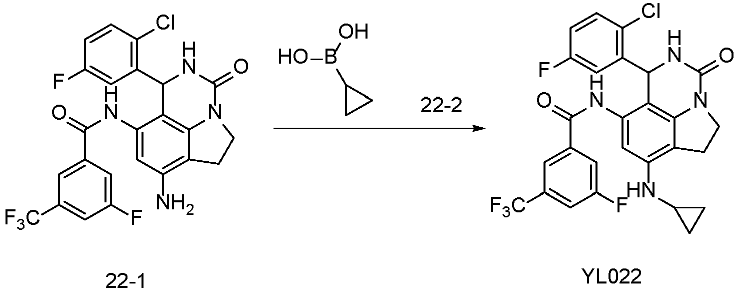

### Synthesis of compound YL022

Sodium carbonate (20.27 mg, 0.191 mmol), 22-2 (16 mg, 0.191 mmol), 2,2'-bipyridine (15 mg, 0.096 mmol) and anhydrous copper acetate (17 mg, 0.096 mmol) were added to a suspension of 22-1 (50 mg, 0.096 mmol) in 1,4-dioxane (2 mL) and. The reaction mixture was stirred at 70°C for 2 h. The reaction mixture was concentrated to dryness and the residue was purified by reversed-phase column to give compound YL022 (18 mg). ESI:(m/z) = 563.2 [M+H]⁺; ¹H NMR (400 MHz, DMSO-d6) δ 10.04 (s, 1H), 7.91 (d, J = 8.5 Hz, 1H), 7.89 - 7.82 (m, 2H), 7.33 - 7.22 (m, 2H), 7.07 (ddd, J = 8.8, 7.9, 3.1 Hz, 1H), 6.77 (dd, J = 9.3, 3.1 Hz, 1H), 6.27 (s, 1H), 5.99 (s, 1H), 5.93 (dd, J = 2.7, 1.2 Hz, 1H), 4.05 - 3.84 (m, 2H), 2.97 (t, J = 8.5 Hz, 2H), 2.38 - 2.27 (m, 1H), 0.66 (dd, J = 6.6, 2.3 Hz, 2H), 0.43 (t, J = 3.1 Hz, 2H).

### Example 23 Synthetic route of compound YL023

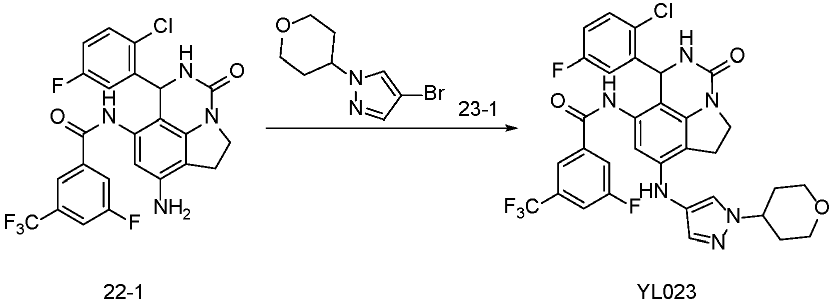

### Synthesis of compound YL023

Cesium carbonate (63 mg, 0.192 mmol), 23-1 (33 mg, 0.144 mmol), 2-(di-tert-butylphosphino)-3,6-dimethoxy-2'-4'-6'tri-1-propyl-1,1'-bis-phenyl (5 mg. 0.01 mmol) and 2-(di-tert-butylphosphino)-3,6-dimethoxy-2',4',6'-triisopropyl-1,1'-biphenyl(2-amino-1,1'-biphenyl-2-yl) palladium(II) methanesulfonate (8 mg, 0.01 mmol) were added to a suspension of 22-1 (50 mg, 0.096 mmol) in 1,4-dioxane (2 mL), and the resulting mixture was stirred at 100°C overnight. The reaction mixture was concentrated to dryness and the residue was purified by prep-HPLC to give compound YL023 (34 mg). ESI:(m/z) = 673.2 [M+H]⁺; ¹H NMR (400 MHz, DMSO-d6) δ 9.95 (s, 1H), 7.90 (d, J = 8.4 Hz, 1H), 7.79 (d, J = 5.9 Hz, 2H), 7.69 (s, 1H), 7.37 (s, 1H), 7.33 - 7.25 (m, 3H), 7.06 (td, J = 8.4, 3.1 Hz, 1H), 6.78 (dd, J = 9.3, 3.1 Hz, 1H), 6.14 (s, 1H), 5.97 - 5.91 (m, 1H), 4.32 (dd, J = 10.1, 5.1 Hz, 1H), 3.96 (ddt, J = 21.9, 11.7, 3.4 Hz, 4H), 3.43 (td, J = 11.5, 4.4 Hz, 3H), 3.02 (t, J = 10.4 Hz, 2H), 1.97 - 1.86 (m, 4H).

### Example 24 Synthetic route of compound YL024

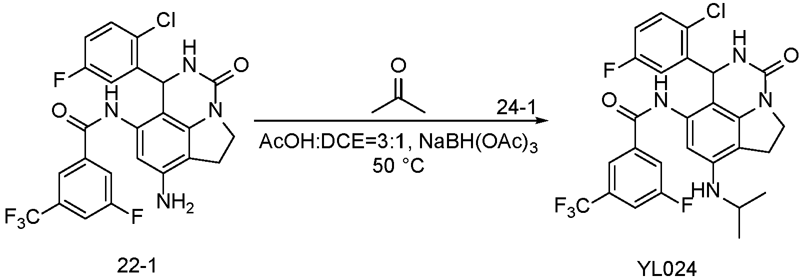

### Synthesis of compound YL024

Sodium borohydride triacetate (102 mg, 0.48 mmol) and 24-1 (2 mL) were added to a suspension of 22-1 (50 mg, 0.096 mmol) in acetic acid (3 mL) and dichloroethane (1 mL), and the resulting mixture was stirred at 70°C overnight. The reaction mixture was concentrated to dryness and the residue was purified by column chromatography (DCM: MeOH = 20: 1) to give compound YL024 (26 mg). ESI:(m/z) = 565.2 [M+H]⁺; ¹H NMR (400 MHz, DMSO-d6) δ 9.96 (s, 1H), 7.91 (d, J = 8.5 Hz, 1H), 7.85 (d, J = 13.4 Hz, 2H), 7.34 - 7.21 (m, 2H), 7.06 (td, J = 8.4, 3.1 Hz, 1H), 6.78 (dd, J = 9.3, 3.1 Hz, 1H), 5.99 (s, 1H), 5.95 - 5.90 (m, 1H), 5.11 (d, J = 8.2 Hz, 1H), 4.04 - 3.86 (m, 2H), 3.62 - 3.47 (m, 1H), 2.98 (t, J = 8.6 Hz, 2H), 1.15 (t, J = 6.0 Hz, 6H).

### Example 25 Synthetic route of compound YL025

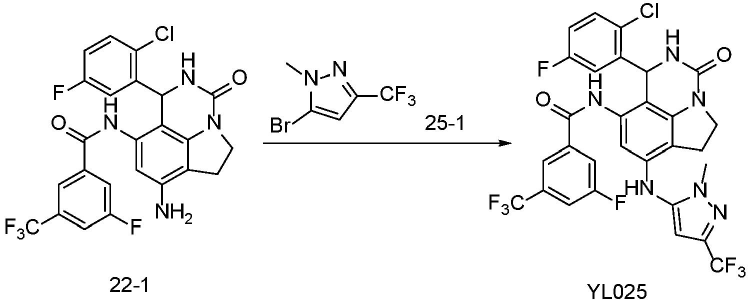

### Synthesis of compound YL025

Cesium carbonate (37.5 mg, 0.115 mmol), 25-1 (33 mg, 0.143 mmol), 2-(di-tert-butylphosphino)-3,6-dimethoxy-2'-4'-6'tri-1-propyl-1,1'-bis-phenyl (5 mg. 0.01 mmol) and 2-(di-tert-butylphosphino)-3,6-dimethoxy-2',4',6'-triisopropyl-1,1'-biphenyl(2-amino-1,1'-biphenyl-2-yl) palladium(II) methanesulfonate (8 mg, 0.01 mmol) were added to a suspension of 22-1 (50 mg, 0.096 mmol) in 1,4-dioxane (2 mL). The resulting mixture was stirred at 100°C overnight. The reaction mixture was concentrated to dryness and the residue was purified by reversed-phase column to give compound YL025 (9 mg). ESI:(m/z) = 671.1 [M+H]⁺; ¹HNMR (400 MHz, DMSO-d6) δ 9.99 (s, 1H), 8.03 (s, 1H), 7.91 (d, J = 8.4 Hz, 1H), 7.79 (s, 2H), 7.38 (d, J = 2.6 Hz, 1H), 7.30 (dd, J = 8.9, 5.1 Hz, 1H), 7.07 (td, J = 8.4, 3.1 Hz, 1H), 6.82 (dd, J = 9.2, 3.1 Hz, 1H), 6.43 (s, 1H), 6.06 (s, 1H), 6.02 - 5.97 (m, 1H), 4.08 - 3.93 (m, 2H), 3.73 (s, 3H), 3.03 (t, J = 10.3 Hz, 2H).

### Example 26 Synthetic route of compound YL026

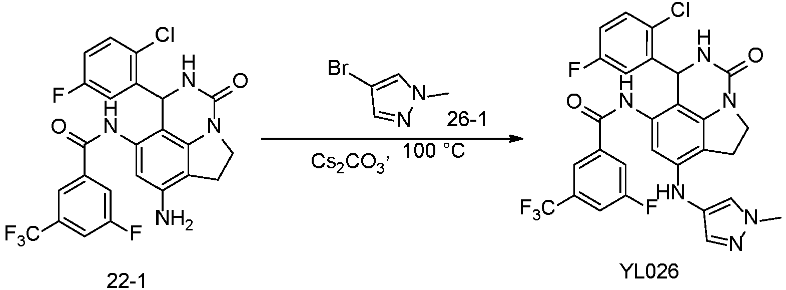

### Synthesis of compound YL026

Cesium carbonate (37.5 mg, 0.115 mmol), 26-1 (14 mg, 0.086 mmol), 2-(di-tert-butylphosphino)-3,6-dimethoxy-2'-4'-6'tri-1-propyl-1,1'-bis-phenyl (3 mg. 0.006 mmol) and 2-(di-tert-butylphosphino)-3,6-dimethoxy-2',4',6'-triisopropyl-1,1'-biphenyl(2-amino-1,1'-biphenyl-2-yl) palladium(II) (5 mg, 0.006 mmol) methanesulfonate were added to a suspension of 22-1 (30 mg, 0.057 mmol) in 1,4-dioxane (2 mL). The resulting mixture was stirred at 100 °C overnight. The reaction mixture was concentrated to dryness and the residue was purified by prep-HPLC to give compound YL026 (11 mg). ESI:(m/z) = 603.2 [M+H]⁺ ; ¹H NMR (400 MHz, DMSO-d6) δ 9.94 (s, 1H), 7.90 (d, J = 8.4 Hz, 1H), 7.80 (d, J = 7.2 Hz, 2H), 7.59 (s, 1H), 7.35 - 7.21 (m, 4H), 7.11 - 7.01 (m, 1H), 6.78 (dd, J = 9.3, 3.1 Hz, 1H), 6.12 (s, 1H), 5.95 (dd, J = 2.6, 1.2 Hz, 1H), 3.98 (td, J = 9.9, 7.1 Hz, 2H), 3.77 (s, 3H), 3.03 (d, J = 11.2 Hz, 2H).

### Example 27 Synthetic route of compound YL027

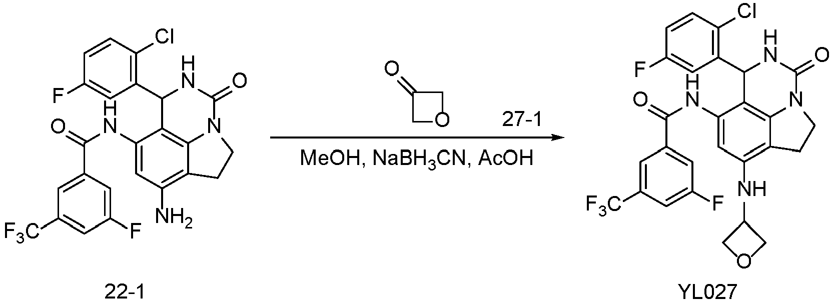

### Synthesis of compound YL027

22-1 (30 mg, 0.057 mmol) was dissolved in methanol (2 mL), acetic acid (2 drops) was added, 27-1 (11 mg, 0.143 mmol) was stirred at room temperature for 1 h. Sodium cyanoborohydride (8 mg, 0.115 mmol) was added and the reaction mixture was stirred at room temperature overnight. The reaction mixture was concentrated to dryness and the residue was purified by prep-HPLC to give compound YL027 (11 mg). ESI:(m/z) = 579.1 [M+H]⁺; ¹H NMR (400 MHz, DMSO-d6) δ 9.97 (s, 1H), 7.90 (d, J = 8.5 Hz, 1H), 7.83 (d, J = 12.2 Hz, 2H), 7.32 - 7.23 (m, 2H), 7.06 (ddd, J = 8.8, 7.9, 3.1 Hz, 1H), 6.77 (dd, J = 9.3, 3.1 Hz, 1H), 6.17 (d, J = 5.4 Hz, 1H), 5.92 (dd, J = 2.7, 1.2 Hz, 1H), 5.75 (s, 1H), 4.80 (d, J = 8.4 Hz, 2H), 4.57 - 4.43 (m, 3H), 4.05 - 3.88 (m, 2H), 3.04 (t, J = 8.6 Hz, 2H).

### Example 28 Synthetic route of compound YL028

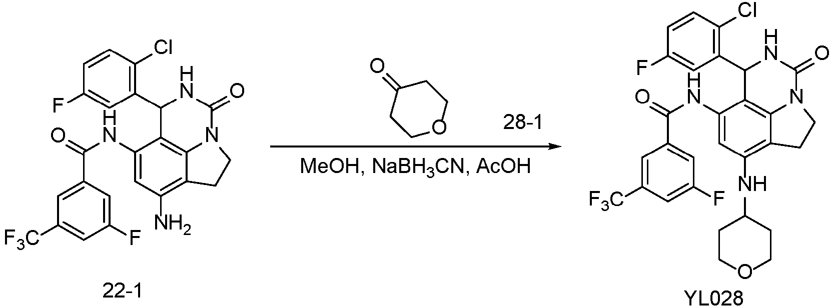

### Synthesis of compound YL028

22-1 (50 mg, 0.096 mmol) was dissolved in methanol (2 mL), to which were added acetic acid (2 drops) and 28-1 (24 mg, 0.239 mmol). The resulting mixture was stirred at room temperature for 1 h, then was added sodium cyanoborohydride (12 mg, 0.191 mmol) and the reaction mixture was stirred at room temperature overnight. The reaction mixture was concentrated to dryness and compound YL028 (13 mg) was obtained by preparation. ESI:(m/z) = 607.15 [M+H]⁺.

### Example 29 Synthetic route of compound YL029

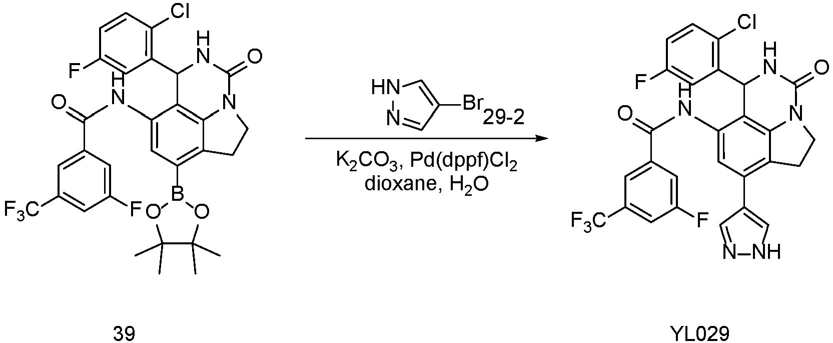

### Synthesis of compound YL029

Compound 39 (60 mg, 0.095 mmol) was dissolved in dioxane (2 mL) and water (0.4 mL), to which were added potassium carbonate (26.17 mg, 0.189 mmol), 29-2 (14 mg, 0.095 mmol), Pd(dppf)Cl₂ (7 mg, 0.009 mmol), and the resulting mixture was stirred at 100 °C for 1.5 h. The reaction mixture was concentrated to dryness and the residue was puirified by prep-HPLC to give compound YL029 (10 mg). ESI:(m/z) = 574.8 [M+H]⁺; ¹HNMR (400 MHz, DMSO-d6) δ 13.10 (s, 1H), 10.17 (s, 1H), 8.07 (s, 1H), 7.99 - 7.83 (m, 4H), 7.47 (d, J = 2.6 Hz, 1H), 7.32 (dd, J = 8.8, 5.2 Hz, 1H), 7.09 (ddd, J = 8.7, 7.8, 3.1 Hz, 1H), 7.01 (s, 1H), 6.87 (dd, J = 9.2, 3.1 Hz, 1H), 6.07 (dd, J = 2.8, 1.1 Hz, 1H), 4.17 - 3.94 (m, 2H), 3.51 - 3.39 (m, 2H).

### Example 30 Synthetic route of compound YL030

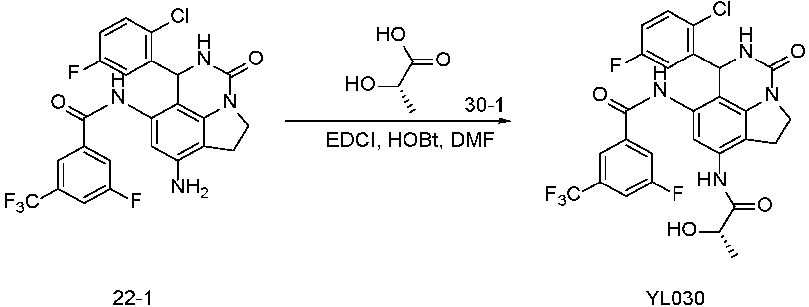

### Synthesis of compound YL030

22-1 (40 mg, 0.077 mmol) was dissolved in DMF (2 mL), to which were added 30-1 (6.7mg, 0.077 mmol), EDCI (30 mg, 0.153 mmol), HOBt (21 mg, 0.153 mmol) and the reaction mixture was stirred at room temperature overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by prep-HPLC to give compound YL030 (10 mg). ESI:(m/z) = 595.0 [M+H]⁺; ¹H NMR (400 MHz, DMSO-d6) δ 10.18 (s, 1H), 9.32 (s, 1H), 7.95 (d, J = 8.6 Hz, 1H), 7.89 - 7.80 (m, 2H), 7.47 (d, J = 2.7 Hz, 1H), 7.32 (dd, J = 8.8, 5.2 Hz, 1H), 7.15 - 7.05 (m, 2H), 6.82 (dd, J = 9.2, 3.1 Hz, 1H), 6.04 (dd, J = 2.5, 1.1 Hz, 1H), 5.82 (d, J = 5.2 Hz, 1H), 4.24 - 4.11 (m, 1H), 4.06 - 3.89 (m, 2H), 3.15 (td, J = 7.9, 7.2, 3.0 Hz, 2H), 1.30 (d, J = 6.7 Hz, 3H).

### Example 31 Synthetic route of compound YL031

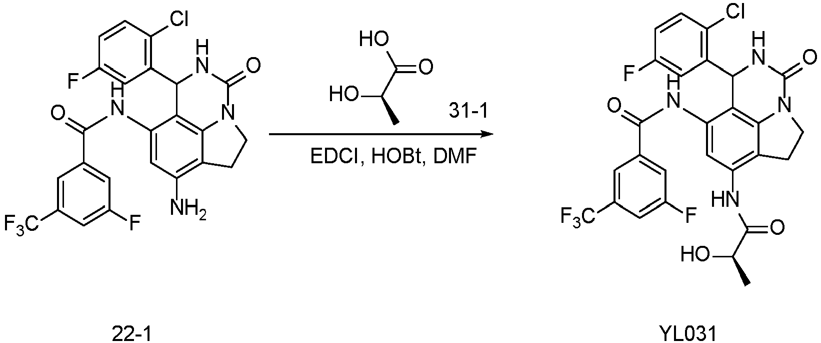

### Synthesis of compound YL031

22-1 (40 mg, 0.077 mmol) was dissolved in DMF (2 mL), to which were added 31-1 (6.7 mg, 0.077 mmol), EDCI (30 mg, 0.153 mmol) and HOBt (21 mg, 0.153 mmol) and the reaction mixture was stirred at room temperature overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by prep-HPLC to give compound YL031 (8 mg). ESI:(m/z) = 595.0 [M+H]⁺; ¹H NMR (400 MHz, DMSO-d6) δ 10.18 (s, 1H), 9.30 (s, 1H), 8.00 - 7.92 (m, 1H), 7.89 - 7.79 (m, 2H), 7.47 (d, J = 2.6 Hz, 1H), 7.32 (dd, J = 8.8, 5.1 Hz, 1H), 7.15 (s, 1H), 7.09 (td, J = 8.4, 3.1 Hz, 1H), 6.83 (dd, J = 9.1, 3.1 Hz, 1H), 6.04 (dd, J = 2.6, 1.1 Hz, 1H), 5.84 (d, J = 5.1 Hz, 1H), 4.17 (qd, J = 6.7, 5.0 Hz, 1H), 4.09 - 3.92 (m, 2H), 3.25 - 3.07 (m, 2H), 1.31 (d, J = 6.8 Hz, 3H).

### Example 32 Synthetic route of compound YL032

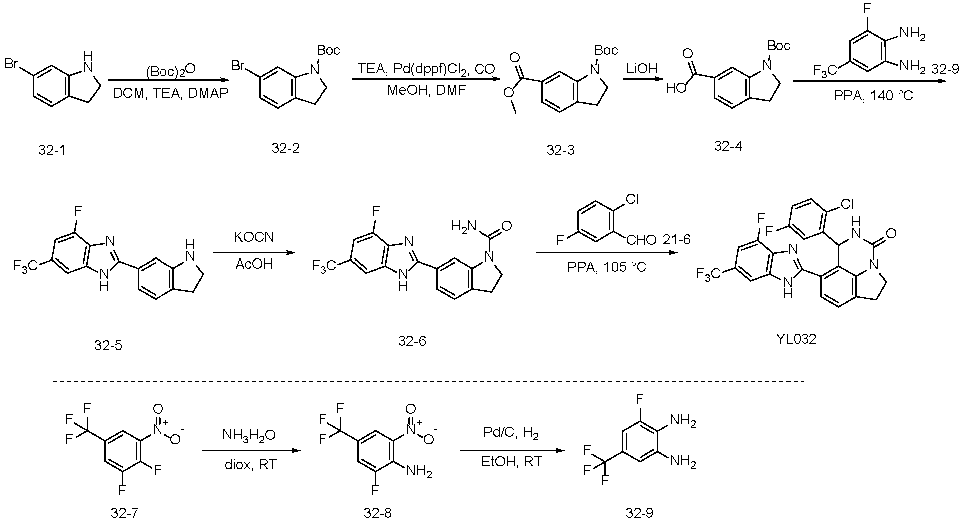

### Synthesis of compound 32-8

Ammonia (6.61 g, 52.842 mmol, 28%) was added to a suspension of 32-7 (0.6 g, 2.642 mmol) in dioxane (5 mL) and the reaction mixture was stirred at room temperature for 2 h. TLC showed that the reaction was complete and new spots were formed, and the reaction mixture was concentrated to dryness to obtain the crude compound 32-8 (0.59 g, 99.64%).

### Synthesis of compound 32-9

5% wet palladium carbon (102 mg, 0.527 mmol) was added to a solution of 32-8 (0.59 g, 2.633 mmol) in ethanol (10 mL). After degassed and purged with H₂, the reaction mixture was stirred at room temperature overnight under atmosphere of H₂. The reaction mixture was filtered and concentrated to dryness to give the crude compound 32-9 (0.5 g, 97.83%). ESI: (m/z) = 195.1 [M+H]⁺; ¹H NMR (400 MHz, Chloroform-d) δ 6.84 (dd, J = 10.2, 1.8 Hz, 1H), 6.75 (s, 1H), 3.79 - 3.33 (m, 4H).

### Synthesis of compound 32-2

Triethylamine (7.4 mL, 53.01 mmol), di-tert-butyl dicarbonate (7.71 g, 35.34 mmol) and DMAP (0.22 g, 1.767 mmol) were added to a suspension of 32-1 (3.5 g, 17.67 mmol) in dichloromethane (50 mL) and the resulting mixture was stirred at room temperature overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue purified by column chromatography (mobile phase: petroleum ether/ethyl acetate 1/0 to 10/1) to give compound 32-2 (3.8 g, 72.12%). ¹H NMR (400 MHz, DMSO-d6) δ 7.82 (s, 1H), 7.13 (d, J = 7.9 Hz, 1H), 7.07 (dd, J = 7.9, 1.9 Hz, 1H), 3.91 (t, J = 8.7 Hz, 2H), 3.01 (t, J = 8.7 Hz, 2H), 1.50 (s, 9H).

### Synthesis of compound 32-3

Triethylamine (2.2 mL, 15.93 mmol) and Pd(dppf)Cl₂ (0.47 g, 0.637 mmol) were added to a suspension of 32-2 (1.9 g, 0.882 mmol) in methanol (10 mL) and DMF (10 mL). After degassed and purged with CO, the reaction mixture was stirred at 100 °C under atmosphere of CO overnight. The reaction mixture was concentrated to dryness and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: petroleum ether/ethyl acetate 1/0 to 5/1) to give compound 32-3 (950 mg, 53.67%). ESI:(m/z) = 222.1 [M-tBu]⁺.

### Synthesis of compound 32-4

32-3 (840 mg, 3.03 mmol) was dissolved in methanol (3 mL), to which were added tetrahydrofuran (3 mL), water (3 mL), and lithium hydroxide (763 mg, 18.17 mmol), and the resulting mixture was stirred at 60 °C for 3 h. The reaction mixture was concentrated to dryness and adjusted to neutral by adding 0.5 M diluted hydrochloric acid dropwise in an ice-cold water bath, the aqueous phase was extracted with ethyl acetate and the organic phases were concentrated to dryness to give the crude compound 32-4 (750 mg, 94.04%). ESI:(m/z) = 208.1 [M-tBu]⁺.

### Synthesis of compound 32-5

32-9 (295 mg, 1.52 mmol) was added to a suspension of 32-4 (400 mg, 1.52 mmol) in polyphosphoric acid (8 g) and the resulting mixture was stirred at 140 °C for 1 h. The reaction mixture was adjusted to pH>7 by dropwise addition of ice-cold sodium hydroxide solution (2N) and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 2/1) to give compound 32-5 (170 mg, 34.83%). ESI:(m/z) = 322.6 [M+H]⁺.

### Synthesis of compound 32-6

Potassium cyanate (80 mg, 0.996 mmol) was added to a suspension of 32-5 (160 mg, 0.498 mmol) in acetic acid (3 mL) at 5 °C, and the reaction mixture was stirred at 5 °C for 0.5 hr. The reaction mixture was concentrated to dryness and adjusted to pH>7 by dropwise addition of ice-cold saturated sodium bicarbonate, and diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 32-6 (200 mg). ESI:(m/z) = 365.2 [M+H]⁺.

### Synthesis of compound YL032

21-6 (110 mg, 0.686 mmol) was added to a suspension of 32-6 (100 mg, 0.274 mmol) in polyphosphoric acid (7g) and and the resulting mixture was stirred at 105 °C for 2 h. The reaction mixture was adjusted to pH > 7 by dropwise addition of ice-cold saturated sodium hydroxide solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound YL032 (30 mg, 21.65%). ESI:(m/z) = 505.0 [M+H]⁺; ¹H NMR (400 MHz, DMSO-*d6*) δ 13.52 (s, 1H), 7.65 - 7.53 (m, 3H), 7.47 (d, J = 7.7 Hz, 1H), 7.44 - 7.33 (m, 2H), 7.08 (d, J = 3.6 Hz, 1H), 7.03 (td, J = 8.4, 3.0 Hz, 1H), 6.52 (dd, J = 9.5, 3.0 Hz, 1H), 4.00 (td, J = 9.0, 8.4, 4.7 Hz, 2H), 3.32 (s, 2H).

### Example 33 Synthetic route of compound YL033

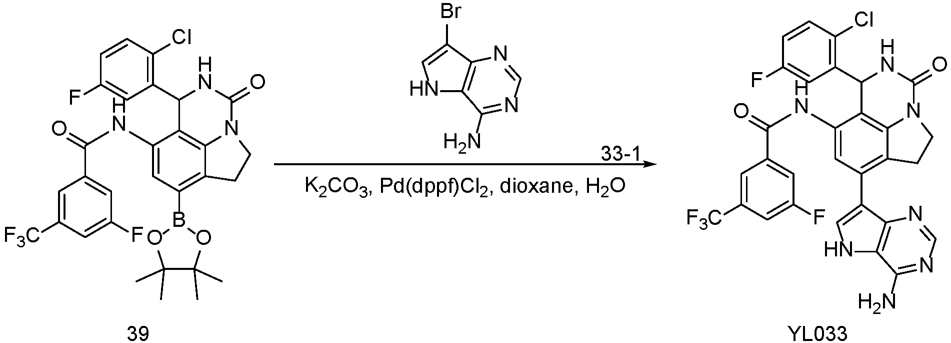

### Synthesis of compound YL033

Potassium carbonate (45.8 mg, 0.331 mmol), 33-1 (23.53 mg, 0.11 mmol) and Pd(dppf)Cl₂ (8.1 mg, 0.011 mmol) were added to a suspension of intermediate 39 (70 mg, 0.11 mmol) in dioxane (2 mL) and water (0.4 mL), and the reaction mixture was stirred at 100 °C for 2 hours. The reaction mixture was concentrated to dryness and the residue was purified by prep-HPLC to afford compound YL033 (13 mg, 18.4%). ESI:(m/z) = 640.0 [M+H]⁺ ; ¹H NMR (400 MHz, DMSO-*d6*) δ 11.35 (s, 1H), 10.29 (s, 1H), 8.16 (s, 1H), 8.03 - 7.84 (m, 5H), 7.46 (d, J = 2.6 Hz, 1H), 7.33 (dd, J = 8.8, 5.2 Hz, 1H), 7.11 (td, J = 8.3, 3.1 Hz, 1H), 6.93 - 6.81 (m, 3H), 6.06 (d, J = 2.6 Hz, 1H), 4.20 - 3.94 (m, 4H).

### Example 34 Synthetic route of compound YL034

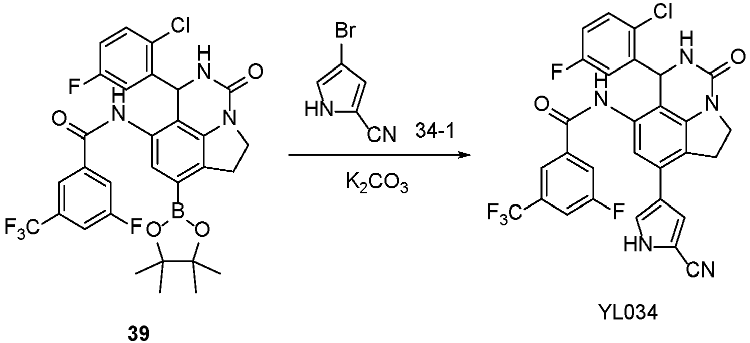

### Synthesis of compound YL034

Potassium carbonate (45.8 mg, 0.331 mmol), 34-1 (19 mg, 0.11 mmol) and Pd(dppf)Cl₂ (8.1 mg, 0.011 mmol) were added to a suspension of intermediate 39 (70 mg, 0.11 mmol) in dioxane (2 mL) and water (0.4 mL), and the resulting mixture was stirred at 100 °C for 2 h, and the reaction mixture was stirred at 100 °C for 2 h, then concentrated to dryness, and the residue was purified by prep-HPLC to afford compound YL034 (8 mg). ESI:(m/z) = 598.0 [M+H]⁺; ¹H NMR (400 MHz, DMSO-*d6*) δ 12.60 (s, 1H), 10.19 (s, 1H), 8.00 - 7.83 (m, 3H), 7.56 (d, J = 1.7 Hz, 1H), 7.50 (d, J = 2.7 Hz, 1H), 7.37 - 7.27 (m, 2H), 7.10 (td, J = 8.3, 3.0 Hz, 1H), 6.98 (s, 1H), 6.87 (dd, J = 9.2, 3.1 Hz, 1H), 6.07 (d, J = 2.6 Hz, 1H), 4.03 (dtd, J = 30.3, 10.5, 6.5 Hz, 2H), 3.45 (d, J = 9.6 Hz, 2H).

### Example 35 Synthetic route of compound YL035

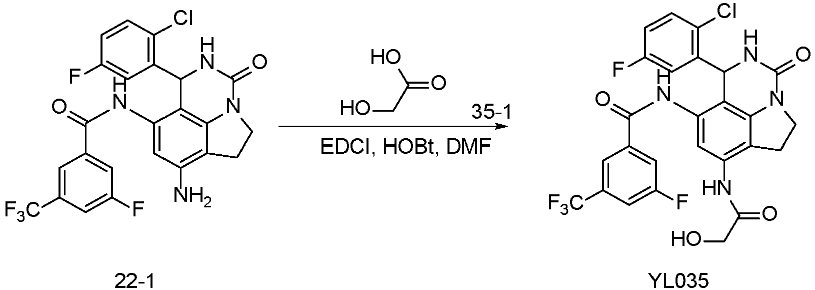

### Synthesis of compound YL035

35-1 (26.17 mg, 0.189 mmol), EDCI (30 mg, 0.153 mmol) and HOBt (21 mg, 0.153 mmol) were added to a suspension of 22-1 (40 mg, 0.077 mmol) in DMF (2 mL), and the reaction mixture was stirred at room temperature overnight and diluted with ethyl acetate and water. The organic phase was separated and the aqueous phase was extracted with ethyl acetate. The organic phases were combined, washed with water, brine, dried over anhydrous Na₂SO₄, filtered and the filtrate was evaporated to obtain compound YL035 (3.5 mg). ESI:(m/z) = 580.5 [M+H]⁺; ¹H NMR (400 MHz, DMSO-*d6*) δ 10.19 (s, 1H), 9.32 (s, 1H), 7.94 (d, J = 8.3 Hz, 1H), 7.84 (d, J = 8.6 Hz, 2H), 7.46 (d, J = 2.7 Hz, 1H), 7.32 (dd, J = 8.9, 5.2 Hz, 1H), 7.15 (s, 1H), 7.13 - 7.06 (m, 1H), 6.82 (dd, J = 9.2, 3.1 Hz, 1H), 6.03 (d, J = 2.5 Hz, 1H), 4.07 - 3.90 (m, 4H), 3.22 - 3.09 (m, 2H).

### Example 36 Synthetic route of compound YL036

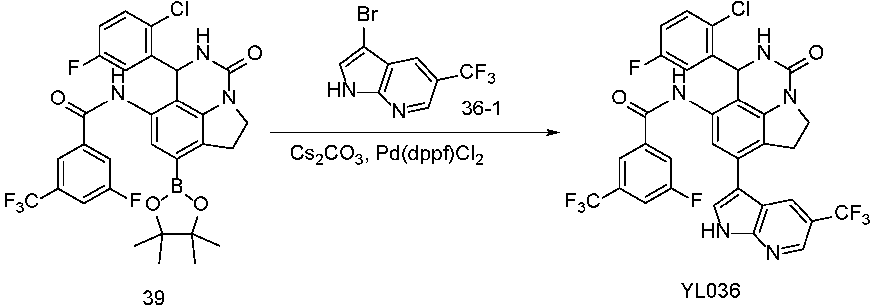

### Synthesis of compound YL036

Water (0.4 mL), potassium carbonate (45.8 mg, 0.331 mmol), 36-1 (29.3 mg, 0.11 mmol) and Pd(dppf)Cl₂ (8.1 mg, 0.011 mmol) were added to a suspension of intermediate 39 (70 mg, 0.11 mmol) in dioxane (2 mL), and the reaction mixture was stirred at 100 °C for 2 hours. The reaction mixture was concentrated to dryness and the residue was purified by prep-HPLC to afford compound YL036 (4.5 mg). ESI:(m/z) = 692.0 [M+H]⁺ ; ¹H NMR (400 MHz, DMSO-*d6*) δ 12.62 (s, 1H), 10.20 (s, 1H), 8.65 (d, J = 2.0 Hz, 1H), 8.43 (d, J = 2.1 Hz, 1H), 8.04 (s, 1H), 7.96 (d, J = 8.5 Hz, 1H), 7.88 (d, J = 13.6 Hz, 2H), 7.52 (d, J = 2.6 Hz, 1H), 7.35 (dd, J = 8.8, 5.2 Hz, 1H), 7.16 - 7.04 (m, 2H), 6.94 (dd, J = 9.2, 3.1 Hz, 1H), 6.21 - 6.12 (m, 1H), 4.04 (dt, J = 9.8, 6.8 Hz, 2H), 3.17 (s, 2H).

### Example 37 Synthetic route of compound YL037

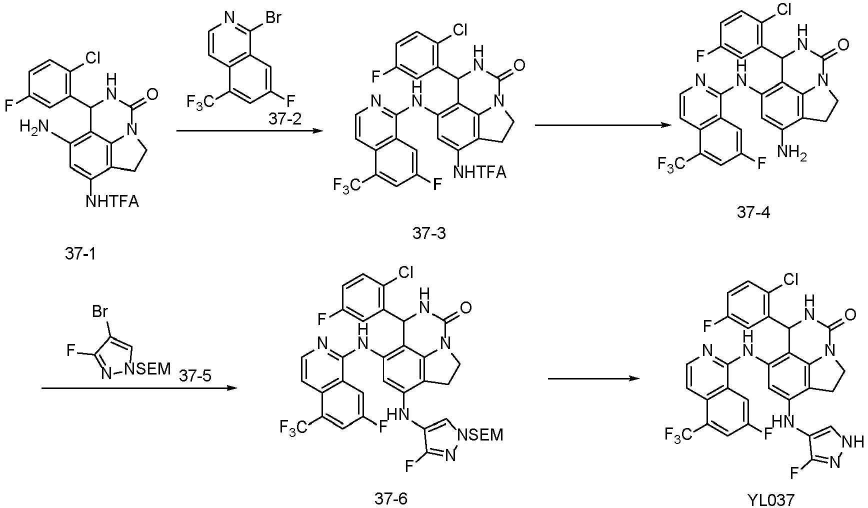

### Synthesis of compound 37-3

Cesium carbonate (1.03 g, 3.172 mmol), 37-2 (0.98 g, 3.331 mmol), Xantphos (0.18 g, 0.317 mmol) and Pd₂(dba)₃ (0.15 g, 0.159 mmol) were added to a suspsension of 37-1 (0.68 g, 1.586 mmol) in dioxane (15 mL). After degassed and purged with N₂, the reaction mixture was stirred at 110 °C for 2 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 37-3 (0.65 g, 63.73%). ESI: (m/z) = 642.0 [M+H]⁺; ¹H NMR (400 MHz, DMSO-d6) δ 11.23 (s, 1H), 9.00 (s, 1H), 8.25 - 8.19 (m, 1H), 8.14 (dd, J = 8.8, 2.4 Hz, 1H), 7.87 (d, J = 6.1 Hz, 1H), 7.41 (d, J = 2.5 Hz, 1H), 7.12 - 7.06 (m, 2H), 6.94 (td, J = 8.4, 3.1 Hz, 1H), 6.75 (s, 1H), 6.63 (dd, J = 9.3, 3.1 Hz, 1H), 5.92 (d, J = 1.9 Hz, 1H), 4.01 (dq, J = 22.9, 10.3 Hz, 2H), 3.21 - 3.09 (m, 2H).

### Synthesis of compound 37-4

Lithium hydroxide (0.25 g, 6.076 mmol) was added to a suspension of 37-3 (0.65 g, 1.013 mmol) in methanol (6 mL) and water (2 mL), and the resulting mixture was stirred at 70 °C for 3 h. The reaction mixture was concentrated to dryness and diluted with dichloromethane and water. The organic phase was separated, and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 37-4 (480 mg, 86.83%). ESI:(m/z) = 546.0 [M+H]⁺.

### Synthesis of compound 37-6

Cesium carbonate (83.56 mg, 9.512 mmol), 37-5 (56.8 mg, 0.192 mmol), t-BuBrettphos (12.44 mg, 0.026 mmol) and t-BuBrettphos Pd G3 (1.57 mg, 0.002 mmol) were added to a suspension of37-4 (70 mg, 0.128 mmol) in 1,4-dioxane (2 mL). The reaction mixture was stirred at 100°C overnight. The reaction mixture was concentrated to dryness and the residue was purified by column chromatograpy (mobile phase: dichloromethane/methanol 1/0 to 20/1) to give compound 37-6 (40 mg, 41.16%). ESI:(m/z) = 760.0 [M+H]⁺.

### Synthesis of compound YL037

Trifluoroacetic acid (1 mL) was added to a suspension of 37-6 (40 mg, 0.053 mmol) in dichloromethane (3 mL), and the reaction mixture was stirred at room temperature for 2 h. The reaction mixture was concentrated to dryness and added dropwise to ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) and prepared to give compound YL037 (3 mg, 10%). ESI:(m/z) = 630.0 [M+H]⁺ ; ¹H NMR (400 MHz, DMSO-*d6*) δ 12.32 (s, 1H), 8.77 (s, 1H), 8.10 (td, J = 8.8, 2.6 Hz, 2H), 7.85 (d, J = 6.2 Hz, 1H), 7.66 (d, J = 2.0 Hz, 1H), 7.17 (d, J = 2.5 Hz, 1H), 7.08 - 7.00 (m, 3H), 6.92 (td, J = 8.4, 3.1 Hz, 1H), 6.57 (dd, J = 9.4, 3.1 Hz, 1H), 5.84 (s, 1H), 5.76 - 5.70 (m, 1H), 4.07 - 3.90 (m, 2H), 3.04 (dt, J = 13.3, 5.1 Hz, 2H).

### Example 38 Synthetic route of compound YL038

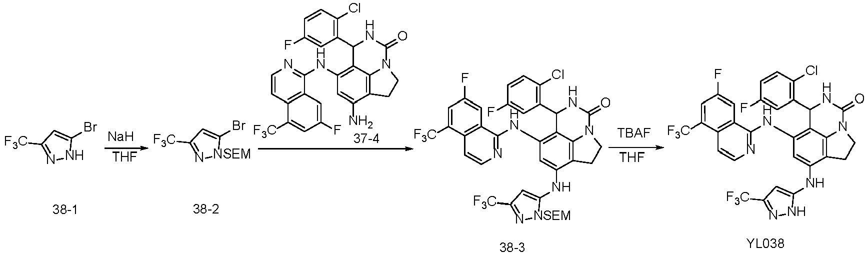

### Synthesis of compound 38-2

A solution of 38-1 (150 mg, 0.698 mmol) in tetrahydrofuran (5 mL) was degassed and purged with N₂, cooled to 0 °C, was added NaH (42 mg, 1.047 mmol, 60%) and the reaction mixture was warmed to room temperature and stirred for 0.5 h, then was added SEM-Cl (174.5 mg, 1.047 mmol) and the reaction mixture was stirred at room temperature for 2 h. It was quenched with saturated aqueous ammonium chloride solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 10/1) to give compound 38-2 (220 mg, 91.32%). ¹H NMR (400 MHz, DMSO-d6) δ 7.22 (d, J = 56.7 Hz, 1H). 5.54 (d, J = 3.6 Hz, 2H), 3.57 (q, J = 7.6 Hz, 2H), 0.90 - 0.75 (m, 2H), -0.07 (d, J = 3.0 Hz, 9H).

### Synthesis of compound 38-3

Cesium carbonate (120 mg, 0.366 mmol), 38-2 (95 mg, 0.275 mmol), t-BuBrettphos (17.8 mg, 0.037 mmol) and t-BuBrettphos Pd G3 (31.32 mg, 0.037 mmol) were added to a solution of 37-4 (100 mg, 0.183 mmol) in 1,4-dioxane (2 mL), and the reaction mixture was stirred at 100 °C overnight. The reaction mixture was concentrated to dryness and the residue was purified by column chromatograpy (mobile phase: dichloromethane/methanol 1/0 to 20/1) to give compound 38-3 (50 mg, 33.3%). ESI:(m/z) = 810.2 [M+H]⁺.

### Synthesis of compound YL038

TBAF (1 mL) was added to a suspension of 38-3 (50 mg, 0.062 mmol) in tetrahydrofuran (5 mL), and the reaction mixture was stirred at 80 °C for 4 hours, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound YL038 (12 mg, 28.5%). ESI:(m/z) = 680.1 [M+H]⁺; ¹H NMR (400 MHz, DMSO-*d6*) δ 13.19 (s, 1H), 8.83 (s, 1H), 8.23 (s, 1H), 8.11 (d, J = 9.1 Hz, 2H), 7.88 (d, J = 6.2 Hz, 1H), 7.25 (s, 1H), 7.06 (dd, J = 9.0, 5.3 Hz, 2H), 6.93 (td, J = 8.3, 3.1 Hz, 1H), 6.60 (dd, J = 9.3, 3.1 Hz, 1H), 6.29 (d, J = 16.2 Hz, 2H), 5.83 (d, J = 1.8 Hz, 1H), 4.00 (q, J = 9.4, 8.7 Hz, 2H), 3.08 - 3.00 (m, 2H).

### Example 39 Synthetic route of compound YL039

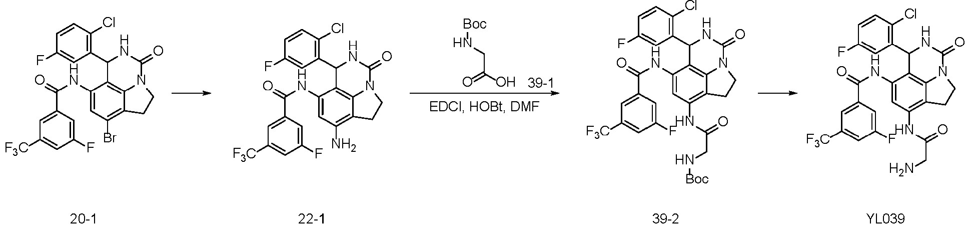

### Synthesis of compound 22-1

Potassium carbonate (710 mg, 5.113 mmol), L-proline (80 mg, 0.682 mmol) and CuI (60 mg, 0.341 mmol) were added to a suspension of 20-1 (1 g, 1.704 mmol) in DMSO (7 mL). After degassed and purged with N₂, the above mixture was added ammonia (2.13 g, 17.04 mmol) at 100 °C overnight. The reaction mixture was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 22-1 (340 mg, 38.2%). ESI:(m/z) = 523.1 [M+H]⁺.

### Synthesis of compound 39-2

39-1 (25.13 mg, 0.143 mmol), EDCI (37 mg, 0.191 mmol) and HOBt (26 mg, 0.191 mmol) was added to a suspension of 22-1 (50 mg, 0.096 mmol) in DMF (2 mL). The reaction mixture was stirred at room temperature overnight and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 39-2 (50 mg, 76.89%). ESI:(m/z) = 680.2 [M+H]⁺.

### Synthesis of compound YL039

Trifluoroacetic acid (1 mL) was added to a solution of 39-2 (50 mg, 0.074 mmol) in dichloromethane (3 mL), and the reaction mixture was stirred at room temperature for 1 h, then was added saturated sodium bicarbonate solution dropwise to adjust it to neutral, and diluted with dichloromethane and water. The organic phase was separated, and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound YL039 (15 mg, 35.2%). ESI:(m/z) = 580.1 [M+H]⁺; ¹H NMR (400 MHz, DMSO-*d6*) δ 10.18 (s, 1H), 7.94 (d, J = 8.5 Hz, 1H), 7.84 (d, J = 7.7 Hz, 2H), 7.45 (d, J = 2.7 Hz, 1H), 7.35 - 7.27 (m, 2H), 7.09 (td, J = 8.4, 3.1 Hz, 1H), 6.82 (dd, J = 9.2, 3.1 Hz, 1H), 6.03 (d, J = 2.5 Hz, 1H), 4.09 - 3.93 (m, 2H), 3.40 (s, 2H), 3.17 (td, J = 7.9, 7.4, 4.4 Hz, 2H).

### Example 40 Synthetic route of compound YL040

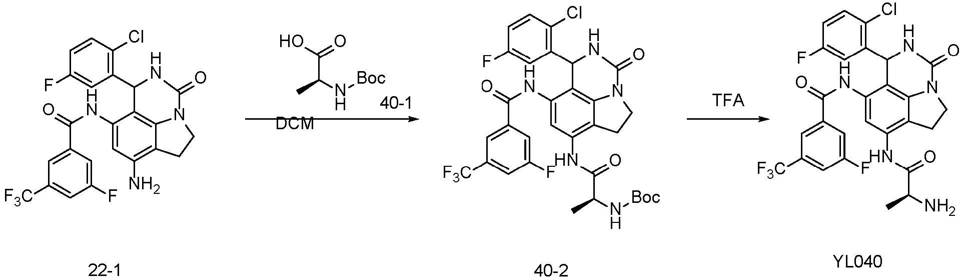

### Synthesis of compound 40-2

40-1 (90.5 mg, 0.478 mmol), TEA (0.04 mL, 0.287 mmol) and condensation agent HATU (91.3 mg, 0.287 mmol, 50% in EA) were added to a solution of 22-1 (50 mg, 0.096 mmol) in dichloromethane (1 mL), and the reaction mixture was stirred at room temperature for 2 h. The reaction mixture was diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatograpy (mobile phase: dichloromethane/methanol 1/0 to 20/1) to give compound 40-2 (50 mg, 75.34%). ESI:(m/z) = 694.1 [M+H]⁺.

### Synthesis of compound YL040

Trifluoroacetic acid (0.7 mL) was added to a solution of 40-2 (50 mg, 0.074 mmol) in dichloromethane (2.1 mL), and the resulting mixture was stirred at room temperature for 1 h. The reaction mixture was adjusted to neutral by adding saturated sodium bicarbonate solution dropwise, then was diluted with dichloromethane and water. The organic phase was separated, and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound YL040 (15 mg, 35.1%). ESI:(m/z) = 594.1 [M+H]⁺; ¹H NMR (400 MHz, DMSO-*d6*) δ 10.18 (d, J = 2.3 Hz, 1H), 7.98 - 7.90 (m, 1H), 7.88 - 7.80 (m, 2H), 7.45 (d, J = 2.6 Hz, 1H), 7.35 - 7.23 (m, 2H), 7.09 (td, J = 8.3, 3.1 Hz, 1H), 6.82 (dd, J = 9.2, 3.1 Hz, 1H), 6.04 (d, J = 2.6 Hz, 1H), 4.01 (dtd, J = 23.6, 10.4, 7.2 Hz, 2H), 3.51 (dd, J = 6.9, 4.1 Hz, 1H), 3.22 - 3.10 (m, 2H), 1.25 (s, 3H).

### Example 41 Synthetic route of compound YL041

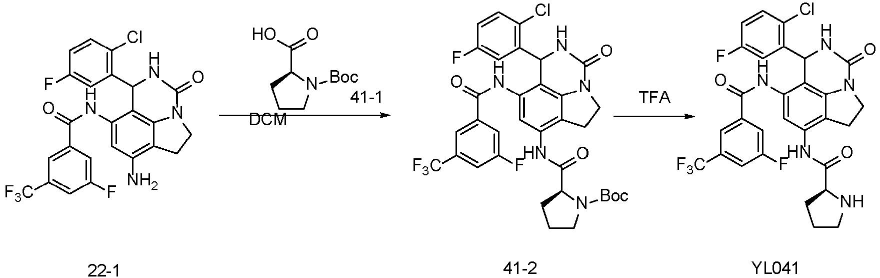

### Synthesis of compound 41-2

41-1 (103 mg, 0.478 mmol), TEA (0.04 mL, 0.287 mmol) and HATU (91.3 mg, 0.287 mmol, 50% in EA) were added to a suspension of 22-1 (50 mg, 0.096 mmol) in dichloromethane (1 mL). The reaction mixture was stirred at room temperature for 2 h, then was diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) to give compound 41-2 (50 mg, 72.6%). ESI:(m/z) = 720.0 [M+H]⁺.

### Synthesis of compound YL041

Trifluoroacetic acid (0.7 mL) was added to a suspension of 41-2 (50 mg, 0.074 mmol) in dichloromethane (2.1 mL). The reaction mixture was stirred at room temperature for 1 h, then was adjusted to neutral by adding saturated sodium bicarbonate solution dropwise, diluted with dichloromethane and water. The organic phase was separated, and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 10/1) to give compound YL041 (21 mg, 48.8%). ESI:(m/z) = 620.1 [M+H]⁺; ¹H NMR (400 MHz, DMSO-*d6*) δ 10.18 (s, 1H), 9.93 (d, J = 6.2 Hz, 1H), 7.94 (d, J = 8.3 Hz, 1H), 7.88 - 7.80 (m, 2H), 7.45 (d, J = 2.6 Hz, 1H), 7.37 - 7.28 (m, 2H), 7.13 - 7.05 (m, 1H), 6.82 (dd, J = 9.2, 3.1 Hz, 1H), 6.04 (d, J = 3.5 Hz, 1H), 4.11 - 3.92 (m, 2H), 3.80 - 3.69 (m, 1H), 3.20 - 3.11 (m, 2H), 2.91 (ddd, J = 24.7, 9.4, 6.5 Hz, 2H), 2.12 - 1.94 (m, 2H), 1.67 (q, J = 6.8 Hz, 2H).

### Example 42 Synthetic route of compound YL042

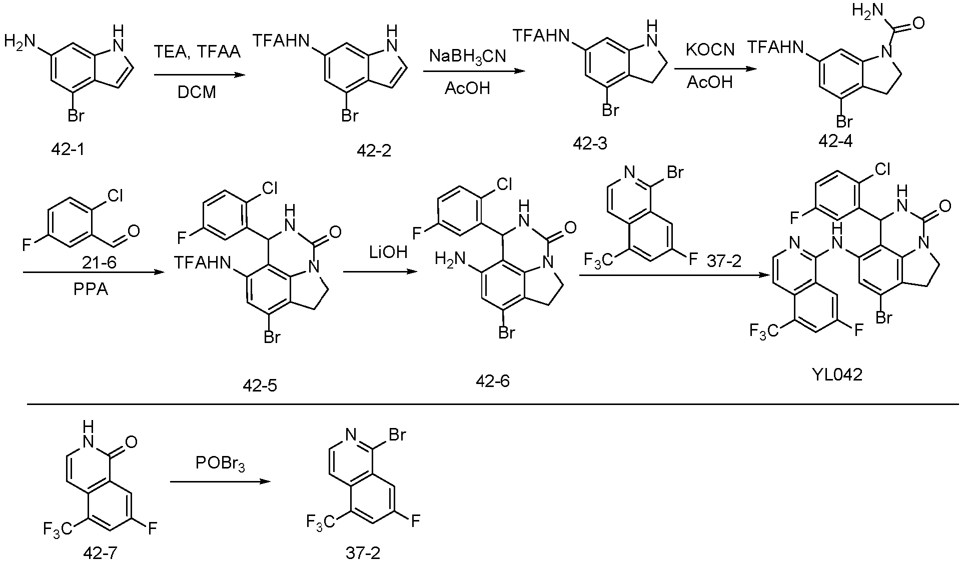

### Synthesis of compound 42-2

Triethylamine (5.9 mL, 42.64 mmol) was added to a suspension of 42-1 (3 g, 14.21 mmol) in dichloromethane (40 mL). After degassed and purged with N₂, the above mixture was stirred trifluoroacetic anhydride (5.97 g, 28.43 mmol) at 0 °C, warmed to room temperature, and stirred for 3 h, then was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 42-2 (3.8 g, 87.16%). ESI: (m/z) = 307.1 [M+H]⁺ ; ¹H NMR (400 MHz, DMSO-d6) δ 11.58 (s, 1H), 11.25 (s, 1H), 7.99 - 7.85 (m, 1H), 7.54 (d, J = 1.7 Hz, 1H), 7.51 - 7.48 (m, 1H), 6.38 (t, J = 2.6 Hz, 1H).

### Synthesis of compound 42-3

Sodium cyanoborohydride (1.17 g, 18.56 mmol) was added to a suspension of 42-2 (3.85 g, 12.538 mmol) in acetic acid (30 mL) and and the resulting mixture was stirred at room temperature overnight. The reaction mixture was concentrated to dryness and poured into ice-cold saturated aqueous sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 42-3 (0.7 g, 36.6%). ESI: (m/z) = 308.8 [M+H]+ ; ¹H NMR (400 MHz, DMSO-d6) δ 11.02 (s, 1H), 7.01 (d, J = 1.7 Hz, 1H), 6.82 (d, J = 1.7 Hz, 1H), 6.15 (d, J = 1.9 Hz, 1H), 3.49 (td, J = 8.7, 1.7 Hz, 2H), 2.89 (t, J = 8.6 Hz, 2H).

### Synthesis of compound 42-4

Potassium cyanate (730 mg, 9.06 mmol) was added to a suspension of 42-3 (1.4 g, 4.529 mmol) in acetic acid (25 mL) at 5 °C, and the reaction mixture was stirred at 5 °C for 0.5 h. The reaction mixture was concentrated to dryness and adjusted to basic by adding saturated aqueous sodium bicarbonate and was added water, filtered to obtain the crude compound 42-4 (1.9 g, crude). ESI:(m/z) = 352.8 [M+H]⁺.

### Synthesis of compound 42-5

21-6 (1.53 g, 9.66 mmol) was added to a suspension of 42-4 (1.7 g, 4.83 mmol) in polyphosphoric acid (10 g) and the resulting mixture was stirred at 105 °C for 0.5 h. The reaction mixture was added dropwise into ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 42-5 (1.2 g, 50%). ESI:(m/z) = 492.0 [M+H]⁺.

### Synthesis of compound 42-6

Lithium hydroxide (610 mg, 14.64 mmol) was added to a suspension of 42-5 (1.2 g, 2.441 mmol) in methanol (21 mL) and water (7 mL), and the resulting mixture was stired at 70 °C for 4 hours. The reaction mixture was concentrated to dryness and diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 42-6 (1 g, crude). ESI:(m/z) = 396.0 [M+H]⁺.

### Synthesis of compound 37-2

42-7 (2.8 g, 1.563 mmol) was dissolved in tribromooxyphosphine (17.36 g, 60.57 mmol) at 60 °C and the reaction mixture was stirred at 110 °C for 10 h. The reaction mixture was poured into ice-cold water, adjusted to pH=9 by adding 10N sodium hydroxide solution, and the aqueous phase was extracted with ethyl acetate. The organic phases were combined, washed with water, brine, dried over anhydrous Na₂SO₄, filtered and the filtrate was evaporated to obtain crude compound 37-2 (3.3 g, 92.7%).

### Synthesis of compound YL042

37-2 (446 mg, 1.516 mmol), cesium carbonate (494 mg, 1.516 mmol), Xantphos (87.8 mg, 0.152 mmol) and Pd₂(dba)₃ (69.4 mg, 0.076 mmol) were added to a suspension of 42-6 (300 mg, 0.758 mmol) in dioxane (7 mL), and the resulting mixture was stirred at 100 °C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound YL042 (300 mg, 65%). ESI: (m/z) = 611.0 [M+H]⁺.

### Example 43 Synthetic route of compound YL043

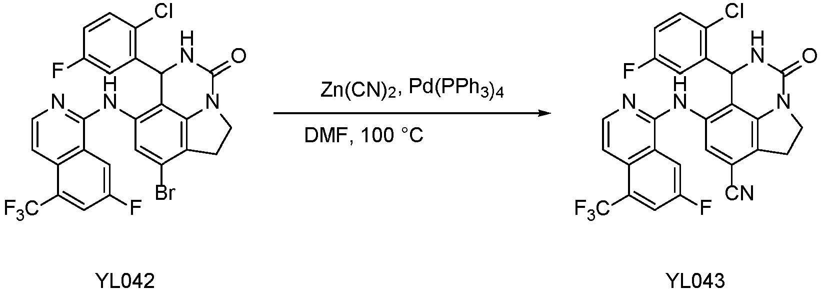

### Synthesis of compound YL043

zinc cyanide (7.7 mg, 0.066 mmol) and Pd(PPh₃)₄ (5.7 mg, 0.005 mmol) were added to a suspension of YL042 (20 mg, 0.033 mmol) in DMF (1 mL). The resulting mixture was stirred at 100 °C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) and reversed-phase column to give compound YL043 (7 mg). ESI:(m/z) = 556.0 [M+H]⁺ ; ¹H NMR (400 MHz, DMSO-d6) δ 9.08 (s, 1H), 8.18 (t, J = 9.8 Hz, 2H), 7.88 (d, J = 6.1 Hz, 1H), 7.52 (d, J = 2.3 Hz, 1H), 7.16 - 7.05 (m, 3H), 6.93 (dt, J = 8.8, 4.4 Hz, 1H), 6.72 - 6.65 (m, 1H), 6.01 (d, J = 2.3 Hz, 1H), 4.12 - 4.05 (m, 2H), 3.39 (d, J = 9.3 Hz, 2H).

### Example 44 Synthetic route of compound YL044

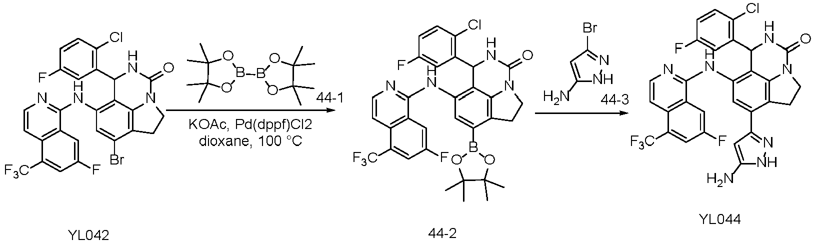

### Synthesis of compound 44-2

Potassium acetate (130 mg, 1.15 mmol), 44-1 (292 mg, 1.15 mmol), Pd(dppf)Cl₂ (48 mg, 0.066 mmol) were added to a suspension of YL042 (200 mg, 0.329 mmol) in dioxane (5 mL). The reaction mixture was stirred at 100 °C for 2 h, then was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 44-2 (200 mg, 92.8%). ESI:(m/z) = 657.0 [M+H]⁺.

### Synthesis of compound YL044

Potassium carbonate (42 mg, 0.305 mmol), 44-3 (49.4 mg, 0.305 mmol) and Pd(dppf)Cl₂ (11.2 mg, 0.015 mmol) were added to a suspension of 44-2 (100 mg, 0.152 mmol) in 1,4-dioxane (2.5 mL) and water (0.5 mL). The resulting mixture was stirred at 100 °C for 2 h and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) to give compound YL044 (22 mg, 32.2%). ESI:(m/z) = 612.0 [M+H]⁺ ; ¹H NMR (400 MHz, DMSO-d6) δ 11.67 (s, 1H), 8.98 (s, 1H), 8.25 (d, J = 10.0 Hz, 1H), 8.13 (dd, J = 8.8, 2.4 Hz, 1H), 7.86 (d, J = 6.2 Hz, 1H), 7.34 (s, 1H), 7.11 - 7.03 (m, 3H), 6.94 (td, J = 8.4, 3.1 Hz, 1H), 6.66 (dd, J = 9.2, 3.1 Hz, 1H), 5.93 (s, 1H), 5.65 (s, 1H), 4.94 (s, 2H), 4.01 (dq, J = 18.3, 10.4, 8.5 Hz, 2H), 3.42 (d, J = 7.3 Hz, 2H).

### Example 45 Synthetic route of compound YL045

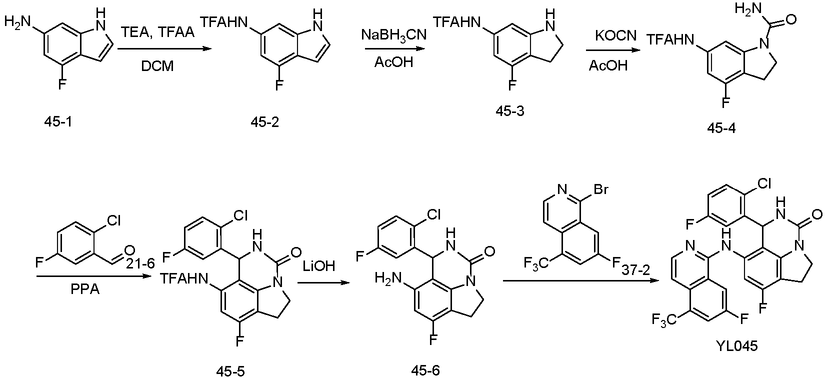

### Synthesis of compound 45-2

Triethylamine (2.8 mL, 19.98 mmol) was added to a suspension of 45-1 (1 g, 6.66 mmol) in dichloromethane (15 mL). After degassed and purged wtih N₂, the above mixture was added trifluoroacetic anhydride (2.8 g, 13.32 mmol) at 0 °C. The reaction mixture was warmed to room temperature and stirred for 3 h. The reaction mixture was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 45-2 (1.5 g, 91.46 %). ESI:(m/z) = 247.0 [M+H]⁺; ¹H NMR (400 MHz, DMSO-d6) δ 11.52 (s, 1H), 11.25 (s, 1H), 7.78 (t, J = 1.2 Hz, 1H), 7.48 - 7.35 (m, 1H), 7.12 (dd, J = 12.1, 1.6 Hz, 1H), 6.48 (t, J = 2.6 Hz, 1H).

### Synthesis of compound 45-3

Sodium cyanoborohydride (1.07 g, 17.062 mmol) was added to a suspension of 45-2 (1.4 g, 5.687 mmol) in acetic acid (10 mL) and stirred at room temperature overnight. The reaction mixture was concentrated to dryness and poured into ice-cold saturated aqueous sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 3/1) to give compound 45-3 (420 mg, 29.76%). ESI:(m/z) = 249.1 [M+H]⁺.

### Synthesis of compound 45-4

Potassium cyanate (275 mg, 3.385 mmol) was added to a suspension of 45-3 (0.42 g, 1.69 mmol) in acetic acid (5 mL) at 5 °C. The reaction mixture was stirred at 5 °C for 0.5 h. The reaction mixture was concentrated to dryness and adjusted to basic by adding saturated aqueous sodium bicarbonate, and water, filtered to obtain crude compound 45-4 (0.45 g, crude). ESI:(m/z) = 292.0 [M+H]⁺.

### Synthesis of compound 45-5

21-6 (0.52 g, 3.30 mmol) was added to a suspension of 45-4 (0.48 g, 1.65 mmol) in polyphosphoric acid (5 g) and the resulting mixture was stirred at 105 °C for 2 h. The reaction mixture was dropped into ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 45-5 (0.28 g, 39.4%). ESI:(m/z) = 432.0 [M+H]⁺.

### Synthesis of compound 45-6

Lithium hydroxide (163 mg, 3.89 mmol) was added to a suspension of 45-5 (0.28 g, 0.65 mmol) in methanol (6 mL) and water (2 mL). The reaction mixture was stirred at 70 °C for 4 h, then was concentrated to dryness and diluted with dichloromethane and water. The organic phase was separated, and the aqueous phase was extracted with dichloromethane. The organic phases were combined, washed with water, brine, dried over anhydrous Na₂SO₄, filtered and the filtrate was evaporated to give the crude compound 45-6 (200 mg, crude). ESI:(m/z) = 335.9 [M+H]⁺ .

### Synthesis of compound YL045

37-2 (175 mg, 0.596 mmol), cesium carbonate (194 mg, 0.596 mmol), Xantphos (34.8 mg, 0.06 mmol) and Pd₂(dba)₃ (27.3 mg, 0.03 mmol) were added to a suspension of 45-6 (100 mg, 0.298 mmol) in dioxane (2 mL). The resulting mixture was stirred at 100 °C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) and pulsed with ethyl acetate to give compound YL045 (80 mg, 48.9%). ESI:(m/z) = 549.0 [M+H]⁺; ¹H NMR (400 MHz, DMSO-*d6*) δ 8.97 (s, 1H), 8.23 - 8.10 (m, 2H), 7.89 (d, J = 6.2 Hz, 1H), 7.42 (d, J = 2.5 Hz, 1H), 7.14 - 7.03 (m, 2H), 6.92 (td, J = 8.4, 3.1 Hz, 1H), 6.63 (dd, J = 9.3, 3.1 Hz, 1H), 6.58 (d, J = 10.1 Hz, 1H), 5.92 (d, J = 1.9 Hz, 1H), 4.12 - 3.95 (m, 2H), 3.25 (t, J = 8.6 Hz, 2H).

### Example 46 Synthetic route of compound YL046

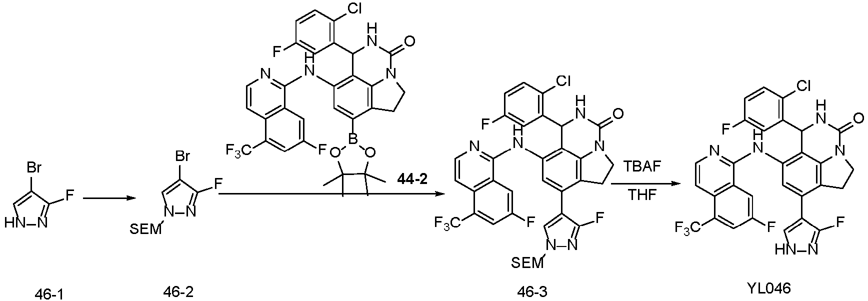

### Synthesis of compound 46-2

NaH (69 mg, 1.73 mmol, 60%) was added to a suspension of 46-1 (190 mg, 1.152 mmol) in tetrahydrofuran (5 mL) at 0 °C. After degassed and purged with N₂. The reaction mixture was warmed to room temperature and stirred for half an hour, then was added 2-(trimethylsilyl) ethoxymethyl chloride (288 mg, 1.73 mmol) and stirred at room temperature for 2 hours. The reaction was quenched with saturated ammonium chloride aqueous solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: petroleum ether/ethyl acetate 1/0 to 10/1) to give compound 46-2 (300 mg, 88.2%).

### Synthesis of compound 46-3

Potassium carbonate (63 mg, 0.457 mmol), 44-2 (54 mg, 0.183 mmol) and Pd(dppf)Cl₂ (11.2 mg, 0.015 mmol) were added to a suspension of 46-2 (100 mg, 0.152 mmol) in dioxane (2.5 mL) and water (0.5 mL). The resulting mixture was stirred at 100 °C for 1 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 46-3 (90 mg, 79.6%). ESI:(m/z) = 745.4 [M+H]⁺.

### Synthesis of compound YL046

TBAF (1.2 mL) was added to a suspension of 46-3 (90 mg, 0.152 mmol) in tetrahydrofuran (5 mL). The resulting mixture was stirred at 70 °C for 4 h, then diluted with ethyl acetate and water. The organic phase was separated, and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) to obtain compound YL046 (27 mg, 36.36%). ESI:(m/z) = 615.1 [M+H]⁺ ; ¹H NMR (400 MHz, DMSO-*d6*) δ 9.01 (s, 1H), 8.25 - 8.19 (m, 1H), 8.13 (dd, J = 8.8, 2.4 Hz, 1H), 8.04 (d, J = 2.1 Hz, 1H), 7.86 (d, J = 6.1 Hz, 1H), 7.37 (d, J = 2.5 Hz, 1H), 7.09 (dd, J = 8.8, 5.2 Hz, 2H), 6.95 (td, J = 8.4, 3.1 Hz, 1H), 6.88 (s, 1H), 6.66 (dd, J = 9.2, 3.1 Hz, 1H), 5.92 (t, J = 1.7 Hz, 1H), 4.03 (ddt, J = 18.4, 10.4, 4.7 Hz, 2H), 3.28-3.3 (m, 2H).

### Example 47 Synthetic route of compound YL047-P1 and YL047-P2

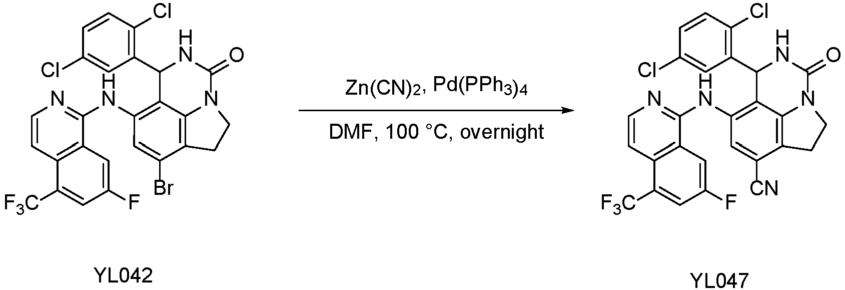

### Synthesis of compounds YL047-P1 and YL047-P2

zinc cyanide (31 mg, 0.263 mmol) and Pd(PPh₃)₄ (23 mg, 0.02 mmol) were added to a suspension of YL42 (180 mg, 0.131 mmol) in DMF (2 mL). The resulting mixture was stirred at 100 °C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) and SFC resolution (see Example YL010 for resolution conditions) to give compounds YL047-P1 (24 mg) and YL047-P2 (28 mg).
YL047-P1 (retention time 4.512 min), ESI:(m/z) = 556.0 [M+H]⁺;
¹H NMR (400 MHz, DMSO-d6) δ 9.07 (s, 1H), 8.18 (t, J = 9.3 Hz, 2H), 7.88 (d, J = 6.1 Hz, 1H), 7.52 (d, J = 2.3 Hz, 1H), 7.13 (s, 2H), 7.08 (dd, J = 8.9, 5.1 Hz, 1H), 6.92 (td, J = 8.4, 3.0 Hz, 1H), 6.68 (dd, J = 9.2, 3.1 Hz, 1H), 6.01 (d, J = 2.2 Hz, 1H), 4.07 (q, J = 8.6 Hz, 2H), 3.39 (s, 2H).
YL047-P2 (retention time 5.408 min), ESI:(m/z) = 556.0 [M+H]⁺;
¹H NMR (400 MHz, DMSO-d6) δ 9.07 (s, 1H), 8.18 (t, J = 9.8 Hz, 2H), 7.88 (d, J = 6.1 Hz, 1H), 7.52 (d, J = 2.3 Hz, 1H), 7.12 (d, J = 6.0 Hz, 2H), 7.08 (dd, J = 8.8, 5.2 Hz, 1H), 6.92 (td, J = 8.4, 3.0 Hz, 1H), 6.68 (dd, J = 9.2, 3.1 Hz, 1H), 6.01 (d, J = 2.3 Hz, 1H), 4.07 (q, J = 8.4 Hz, 2H), 3.42 - 3.37 (m, 2H).

### Example 48 Synthetic route of compound YL048

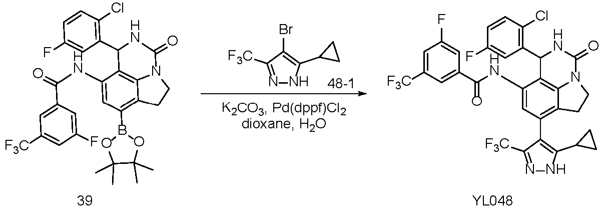

### Synthesis of compound YL048

Potassium carbonate (98.3 mg, 0.711 mmol), 48-1 (72.5 mg, 0.284 mmol) and Pd(dppf)Cl₂ (17.4 mg, 0.024 mmol) were added to a suspension of intermediate 39 (150 mg, 0.237 mmol) in dioxane (2 mL) and water (0.4 mL). The resulting mixture was stirred at 100 °C for 1 h then was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phase: dichloromethane/methanol 1/0 to 20/1) and prepared to give compound YL048 (12 mg, 7.4 %). ESI:(m/z) = 682.1 [M+H]⁺ ; ¹H NMR (400 MHz, DMSO-*d6*) δ 13.31 (s, 1H), 10.19 (d, J = 20.4 Hz, 1H), 7.94 (d, J = 8.4 Hz, 1H), 7.86 (d, J = 7.6 Hz, 2H), 7.51 (d, J = 2.6 Hz, 1H), 7.43 - 7.29 (m, 1H), 7.19 - 7.05 (m, 1H), 6.85 (dd, J = 18.3, 9.1 Hz, 1H), 6.58 (d, J = 3.8 Hz, 1H), 6.11 (d, J = 28.5 Hz, 1H), 4.08 - 3.90 (m, 2H), 3.17 - 2.85 (m, 2H), 1.73 (s, 1H), 1.05 - 0.73 (m,4H).

### Example 49 Synthetic route of compound YL049

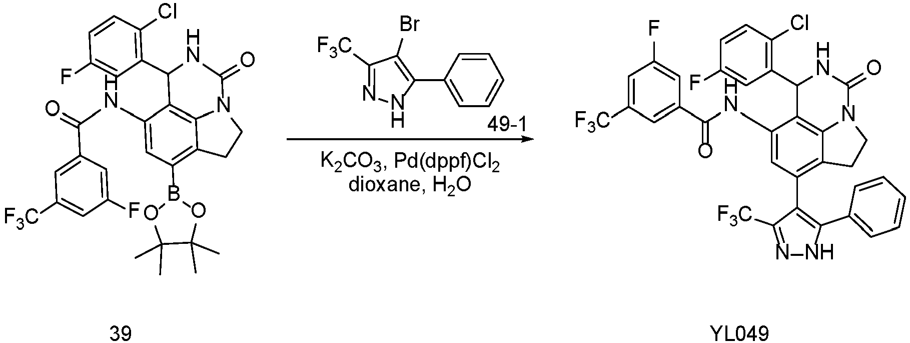

### Synthesis of compound YL049

Potassium carbonate (98.3 mg, 0.711 mmol), 4-bromo-5-phenyl-3-trifluoromethylpyrazole (83 mg, 0.284 mmol) and Pd(dppf)Cl₂ (17.4 mg, 0.024 mmol) were added to a suspension of compound 39 (150 mg, 0.237 mmol) in dioxane (2 mL) and water (0.4 mL). The resulting mixture was stirred at 100 °C for 1 h, then was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound YL049 (13 mg, 7.5 %). ESI:(m/z) = 718.1 [M+H]⁺; ¹H NMR (400 MHz, DMSO-d6) δ 14.17 (s, 1H), 10.21 (d, J = 11.8 Hz, 1H), 7.93 (d, J = 8.4 Hz, 1H), 7.87 - 7.77 (m, 2H), 7.48 (s, 1H), 7.44 - 7.34 (m, 6H), 7.12 (td, J = 8.4, 3.0 Hz, 1H), 6.78 (d, J = 9.3 Hz, 1H), 6.64 (d, J = 4.8 Hz, 1H), 6.10 (s, 1H), 3.87 (ddd, J = 31.4, 15.1, 6.7 Hz, 2H), 2.81 (dt, J = 16.3, 7.6 Hz, 1H), 2.62 (d, J = 37.8 Hz, 1H).

### Example 50 Synthetic route of compound YL050

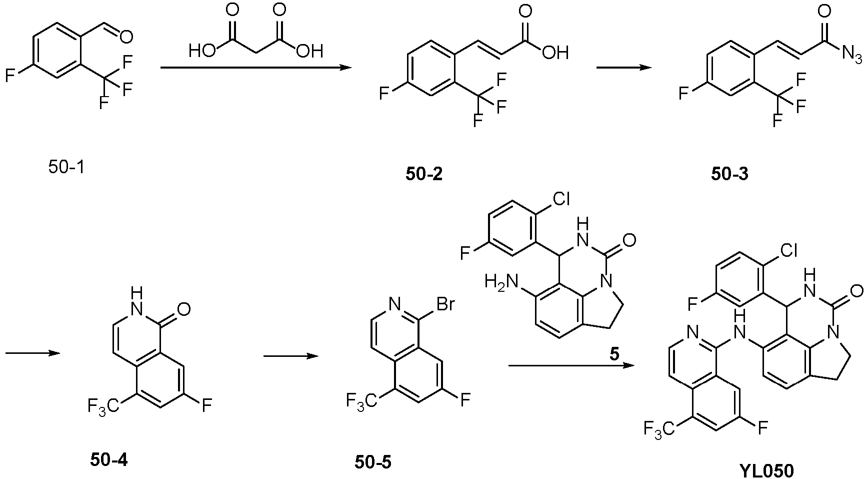

### Synthesis of compound 50-2

Malonic acid (2.98 g, 28.63 mmol) and piperidine (222 mg, 2.60 mmol) were added to a solution of 4-fluoro-2-(trifluoromethyl) benzaldehyde (compound 50-1, 5.00 g, 26.03 mmol) in pyridine. The reaction mixture was refluxed for 4.5 hours and cooled, poured into ice-water (100 mL), the mixture was stirred at 0 °C for 5 minutes and then was added hydrochloric acid (6N,50 mL), continued stirring for 15 min, filtered, and the filter cake was washed with water and dried to obtain a yellow solid 50-2 (3.23 g, 53%). LC-MS (ESI): no ionization. ¹H NMR (400 MHz, DMSO-d6): δ 12.76 (s, 1H), 8.13 (dd, J = 8.8, 5.4 Hz, 1H), 7.81 - 7.69 (m, 2H), 7.62 (td, J = 8.5, 2.8 Hz, 1H), 6.63 (d, J = 15.7 Hz, 1H).

### Synthesis of compound 50-3

To a solution of compound 50-2 (733 mg, 3.13 mmol) in anhydrous toluene (12 mL) was added diphenyl azide phosphate (0.68 mL, 3.13 mmol) and triethylamine (0.61 mL, 4.38 mmol) dropwise and the reaction mixture was stirred at room temperature for 5 h under nitrogen atmosphere. The solvent was removed and the residue was purified by column chromatography (mobile phase: ethyl acetate/petroleum ether 3 to 20%) to give compound 50-3 (580 mg, 71%). LC-MS (ESI): m/z 258.2 (M-N₃+CH₃CN) ⁺. ¹H NMR (400 MHz, DMSO-d6) δ 8.23 (dd, J= 8.9, 5.4 Hz, 1H), 7.86 (dd, J = 15.6, 2.2 Hz, 1H), 7.78 (dd, J = 9.1, 2.7 Hz, 1H), 7.67 (d, J = 2.7 Hz, 1H), 6.84 (d, J = 15.6 Hz. 1H).

### Synthesis of compound 50-4

A mixture of compound 50-3 (7.00 g, 27.01 mmol) and diphenylmethane (21 mL) was stirred at 270 °C for 3 hours. The reaction mixture was purified by column chromatography (mobile phase: ethyl acetate/(petroleum ether/dichloromethane 1:1) 0% to 30%) to give compound 50-4 (1.81 g, 29%). LC-MS (ESI): m/z 232.0 (M+H) ⁺. ¹H NMR (400 MHz, DMSO-d6) δ 11.78 (s, 1H), 8.19 (dd, J = 8.8, 2.8 Hz, 1H), 8.11 (dd, J = 8.8, 2.8 Hz, 1H), 7.43 - 7.34 (m, 1H), 6.63 - 6.55 (m, 1H).

### Synthesis of Compound 50-5

Compound 50-4 (116 mg, 0.50 mmol) was added to phosphine tribromide (2 g, 6.97 mmol) at 60 °C under nitrogen atmosphere and the reaction mixture was stirred at 110 °C for 4 hours. After cooling, the reaction mixture was slowly added ice-cold water (15 mL), extracted with ethyl acetate (15 mL×2), and the organic phases were combined and washed with brine (20 mL), dried over anhydrous sodium sulfate, and evaporated, and the residue was purified by column chromatography (mobile phase: ethyl acetate/petroleum ether 5% to 20%) to give white solid 50-5 (100 mg, 68%). LC-MS (ESI): m/z 295.9 (M+H) ⁺. ¹H NMR (400 MHz, DMSO-d6) δ 8.49 (d, J = 6.0 Hz, 1H), 8.44 (dd, J = 8.8, 2.6 Hz, 1H), 8.31 (dd, J = 9.2, 2.6 Hz, 1H), 8.01 - 7.95 (m, 1H).

### Synthesis of compound YL050

Compound 50-5 (37 mg, 0.13 mmol), compound 5 (40 mg, 0.13 mmol), Cesium carbonate (82 mg, 0.25 mmol), XantPhos (15 mg, 0.025 mmol), Pd₂(dba)₃ (12 mg, 0.013 mmol) were added to a reaction flask. After degassed and purged with nitrogen, the above mixture was added 1. 4-dioxane (1 mL), then after degassed and purged with nitrogen for three times and heated to 110°C and stirred for 16 hours. The solvent was evaporated and the residue was purified by column chromatography (mobile phase: methanol/dichloromethane 0% to 5%) to obtain the crude product. The crude product was purified by reversed-phase column (mobile phase: A: 10 mM NH₄HCO₃ aqueous solution; B: acetonitrile; B/A 10% to 60%) to give compound YL050 27 mg, 40%). LC-MS (ESI): m/z 531.2 (M+H) ⁺. ¹H NMR (400 MHz, DMSO-*d₆*) δ 8.92 (s, 1H), 8.23 (dd, J = 10.4, 2.5 Hz, 1H), 8.12 (dd, J = 8.8, 2.4 Hz, 1H), 7.84 (d, J = 6.1 Hz, 1H), 7.31 (d, J = 2.5 Hz, 1H), 7.19 (d, J = 7.8 Hz, 1H), 7.11 - 7.02 (m, 1H), 6.93 (ddd, J = 8.9, 8.0, 3.1 Hz, 1H), 6.71 (d, J = 7.7 Hz, 1H), 6.63 (dd, J = 9.3, 3.1 Hz, 1H), 5.99 - 5.91 (m, 1H), 4.09 - 3.90 (m, 1H), 3.26 - 3.17 (m, 2H).

### Example 51 Synthetic route of compound YL051

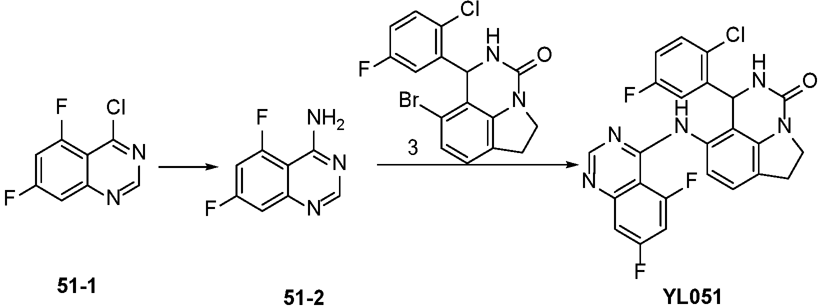

### Synthesis of compound 51-2

A solution of 4-chloro-5,7-difluoroquinazoline (compound 51-1, 200 mg, 1.00 mmol) and ammonia in methanol (7N, 10.0 mL) in a sealed tube was stirred at 30°C for 16 h. The reaction mixture was concentrated to dryness to give compound 51-2 (180 mg, 100%). LC-MS (ESI): m/z 181.9 (M+H) ⁺. ¹H NMR (400 MHz, DMSO-d6): δ 8.39 (s, 1H), 7.98 (s, 1H), 7.41 (ddd, J = 12.0, 9.4, 2.6 Hz, 2H), 7.29 (ddd, J = 10.1, 2.6, 1.4 Hz, 1H).

### Synthesis of compound YL051

Compound 51-2 (30 mg, 0.079 mmol), Compound 3 (16 mg, 0.086 mmol), Cesium carbonate (77 mg, 0.24 mmol), XantPhos (9.1 mg, 0.016 mmol), Pd₂(dba)₃ (7.2 mg, 0.008 mmol) were combined in a reaction flask. After degassed and purged with nitrogen, the above mixture was added 1,4-dioxane (1 mL), then after degassed and purged with nitrogen for three times and heated to 110°C and stirred for 16 hours. The solvent was evaporated and the residue was purified by column chromatography (mobile phase: methanol/dichloromethane 0% to 4%) to obtain the crude product. The crude product was purified by reversed-phase column (mobile phase: A: 10 mM NH₄HCO₃ aqueous solution; B: acetonitrile; B/A 10% to 60%) to give compound YL051 (32 mg, 84%). LC-MS (ESI): m/z 482.1 (M+H) ⁺. ¹H NMR (400 MHz, DMSO-*d₆*): δ 8.98 (s, 1H), 8.10 (s, 1H), 7.56 - 7.38 (m, 2H), 7.32 (dd, J = 9.3, 2.5 Hz, 1H), 7.20 (d, J = 7.8 Hz, 1H), 6.98 (dd, J = 8.8, 5.2 Hz, 1H), 6.91 (td, J = 8.4, 3.0 Hz, 1H), 6.84 (dd, J = 9.3, 3.0 Hz, 1H), 6.73 (d, J = 7.8 Hz, 1H), 6.06 (s, 1H), 4.09 - 3.90 (m, 2H), 3.27 - 3.16 (m, 2H).

### Example 52 Synthetic route of compound YL052

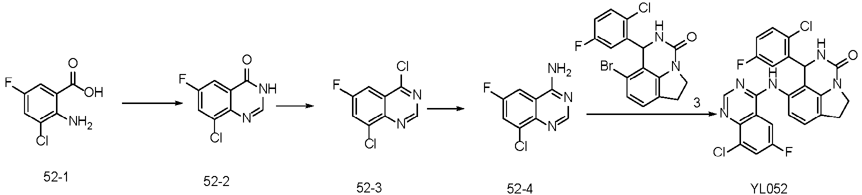

### Synthesis of compound 52-2

2-Amino-3-chloro-5-fluorobenzoic acid (2.50 g, 13.19 mmol), formamidine acetate (5.49 g, 52.75 mmol) and anhydrous ethanol (20 mL) were combined in a casserole and stirred at 110 °C for 16 hours. Cooled and filtered, the solid was washed with ethanol and dried to give a light gray solid 52-2 (2.30 g, 89%). LC-MS (ESI): m/z 198.8 (M+H)⁺. ¹HNMR (400 MHz, DMSO-*d6*): δ 12.63 (s, 1H), 8.21 (s, 1H), 8.06 (dd, J = 8.6, 3.0 Hz, 1H), 7.80 (dd, J = 8.3, 2.9 Hz, 1H).

### Synthesis of compound 52-3

A mixture of compound 52-2 (1.0 g, 1.24 mmol) and sulfoxide chloride (7.3 mL) in a reaction flask was stirred at reflux at 85 °C under nitrogen atmosphere for 10 h. The reaction mixture was concentrated to dryness, diluted with dichloromethane and concentrated to dryness again, and the residue was purified by column chromatography (mobile phase: dichloromethane/petroleum ether 10% to 80%) to give compound 52-3 (1.06 g, 97%). LC-MS (ESI): m/z 216.8 (M+H) ⁺. ¹H NMR (400 MHz, CDCl₃): δ 9.15 (s, 1H), 7.89 (ddd, J = 10.5, 8.1, 2.7 Hz, 2H).

### Synthesis of compound 52-4

A solution of 52-3 (268 mg, 1.24 mmol) and ammonia in methanol (7N, 15.0 mL) was added to a sealed tube and stirred at 30°C for 40 hours. The reaction mixture was concentrated to dryness to give compound 52-4 (328 mg, 100%). LC-MS (ESI): m/z 197.9 (M+H) ⁺. ¹H NMR (400 MHz, DMSO-d6): δ 8.46 (s, 1H), 8.11 (dd, J = 9.5, 2.8 Hz, 1H), 8.07 - 7.99 (m, 3H).

### Synthesis of compound YL052

Compound 52-4 (30 mg, 0.079 mmol), compound 3 (21 mg, 0.10 mmol), cesium carbonate (77 mg, 0.24 mmol), XantPhos (9.10 mg, 0.016 mmol), Pd₂(dba)₃ (7.2 mg, 0.008 mmol) were added to a reaction flask. After degassed and purged with nitrogen, the above mixture was added 1,4-dioxane (1 mL), then after degassed and purged with nitrogen for three times and heated to 110°C and stirred for 16 hours. The solvent was evaporated and the residue was purified by column chromatography (mobile phase: methanol/dichloromethane 0% to 4%) to obtain the crude product. The crude product was purified by reversed-phase column (mobile phase: A: 10 mM NH₄HCO₃ aqueous solution; B: acetonitrile; B/A 10% to 50%) to give compound YL052 (10 mg, 26%). LC-MS (ESI): m/z 498.0 (M+H) ⁺. ¹H NMR (400 MHz, DMSO-d₆): δ 9.60 (s, 1H), 8.32 (s, 1H), 8.08 (dd, J = 8.6, 2.7 Hz, 1H), 7.99 (dd, J = 9.5, 2.8 Hz, 1H), 7.43 (d, J = 2.5 Hz, 1H), 7.23 (d, J = 7.8 Hz, 1H), 7.09 (dd, J = 8.8, 5.2 Hz, 1H), 6.95 (td, J = 8.3, 3.0 Hz, 1H), 6.78 (dd, J = 9.3, 3.0 Hz, 1H), 6.74 (d, J = 7.7 Hz, 1H), 5.99 (s, 1H), 4.09 - 3.93 (m, 2H), 3.28 - 3.18 (m, 2H).

### Example 53 Synthetic route of compound YL053-P1 and YL053-P2

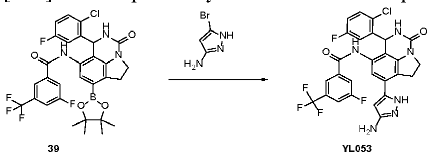

### Synthesis of compound YL053

To a reaction vial were added compound 39 (270 mg, 0.43 mmol), 3-bromo-1H-pyrazol-5-amine (140 mg, 0.86 mmol), 1,1'-bis(diphenylphosphino)ferrocene]dichloropalladium (32 mg, 0.044 mmol), potassium carbonate (119 mg, 0.86 mmol), 1,4-dioxane ( 11 mL) and water (2 mL). The mixture was stirred under nitrogen protection at 110°C for 4 hours. Upon completion, half of the reaction mixture was concentrated at reduced pressure to obtain the crude product. The resulting crude product was purified by automated chromatography (Biotage) (mobile phase: dichloromethane/methanol 92/8 to 90/10) to give the white solid target compound YL053 (19 mg, 15%). LC-MS (ESI): m/z 589.1 (M+H)⁺.¹H NMR (400 MHz, DMSO-d6) δ 11.70 (s, 1H), 10.17 (s, 1H), 7.97 - 7.84 (m, 3H), 7.45 (d, J = 2.7 Hz, 1H), 7.33 (dd, J = 8.8, 5.2 Hz, 1H), 7.13 - 7.03 (m, 2H), 6.87 (dd, J = 9.2, 3.1 Hz, 1H), 6.08 (dd, J = 2.6, 1.1 Hz, 1H), 5.66 (s, 1H), 4.95 (s, 2H), 4.02 (ddd, J = 22.1, 10.3, 5.8 Hz, 2H), 3.45 - 3.39 (m, 2H).

62 mg of YL053 was separated by SFC (instrument: SFC-150 (Waters), column: AS 20*250 mm, 10um (Daicel), column temperature: 35 °C, mobile phase: CO₂/MeOH [0.2% NH₃(7M in MeOH)] = 60/40, flow rate: 100 mL/min, back pressure: 100 bar, wavelength: 214 nm, cycle time: 6 min, sample solution: 69 mg dissolved in 20 mL of methanol, injection volume: 4 mL, and obtained white solid target compounds YL053-P1 (30.3 mg, 12%) and YL053-P2 (25.7 mg, 10%).

YL053-P1: Rention time: 4.561min; LC-MS (ESI): m/z 589.1 (M+H)⁺. ¹H NMR (400 MHz, DMSO-*d6*) δ 11.75 (s, 1H), 10.19 (s, 1H), 7.99 - 7.83 (m, 3H), 7.48 (d, J = 2.6 Hz, 1H), 7.33 (dd, J = 8.8, 5.1 Hz, 1H), 7.15 - 7.03 (m, 2H), 6.87 (dd, J = 9.2, 3.1 Hz, 1H), 6.09 (dd, J = 2.5, 1.1 Hz, 1H), 5.65 (s, 1H), 4.97 (s, 2H), 4.01 (dq, J = 18.1, 10.4, 8.5 Hz, 2H), 3.42 (s, 2H).

YL053-P2: Rention time: 5.012 min; LC-MS (ESI): m/z 589.1 (M+H) ⁺. ¹H NMR (400 MHz, DMSO-*d6*) δ 11.73 (s, 1H), 10.19 (s, 1H), 8.01 - 7.81 (m, 3H), 7.48 (s, 1H), 7.33 (dd, J = 8.9, 5.2 Hz, 1H), 7.15 - 7.01 (m, 2H), 6.87 (d, J = 9.2 Hz, 1H), 6.08 (dd, J = 2.6, 1.1 Hz, 1H), 5.64 (s, 1H), 5.08 (s, 2H), 4.09 - 3.95 (m, 2H), 3.41 (s, 2H).

### Example 54 Synthetic route of compound YL054

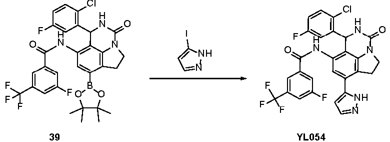

### Synthesis of compound YL054

To a reaction flask was added compound 39 (55 mg, 0.087 mmol), 5-iodopyrazole (33 mg, 0.17 mmol), 1,1'-bis(diphenylphosphino)ferrocene] palladium dichloride (7 mg, 0.009 mmol), potassium carbonate (23 mg, 0.17 mmol), 1,4-dioxane (2.5 mL) and water (0.5 mL). The mixture was stirred under nitrogen protection at 110°C for 3 hours. Upon completion, the reaction mixture was concentrated at reduced pressure to give the residue. The residue was purified by automated chromatography (Biotage) (mobile phase: petroleum ether/ethyl acetate 7/93 to 3/97) to obtain the crude product. The resulting crude product was prepared and purified (Welch Xtimate C18, 21.2*250 mm, 10um, water (10 mM ammonium bicarbonate)/acetonitrile, flow rate 30 mL/min, column temperature 25 °C, wavelength 254 nm) to give a white solid target compound YL054 (8.2 mg, 16%). LC-MS (ESI): m/z 574.1 (M+H) ⁺; ¹H NMR (400 MHz, DMSO-d6) δ 13.08 (s, 1H), 10.21 (s, 1H), 7.90 (td, J = 23.5, 22.9, 11.3 Hz, 4H), 7.49 (s, 1H), 7.34 (dd, J = 8.8, 5.1 Hz, 1H), 7.20 (s, 1H), 7.10 (td, J = 8.4, 3.1 Hz, 1H), 6.88 (dd, J = 9.2, 3.1 Hz, 1H), 6.67 (s, 1H), 6.13 - 6.08 (m, 1H), 4.09 - 3.98 (m, 2H), 3.47 (d, J = 8.8 Hz, 2H).

### Example 55 Synthetic route of compound YL055

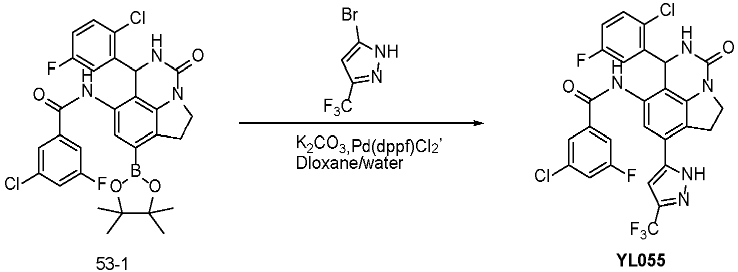

Synthetic method refers to the synthesis of compound 39, compound 53-1 can be synthesized by replacing 3-chloro-5-fluorobenzoyl chloride with 5-trifluoromethylbenzoyl chloride.

### Synthesis of compound YL055

compound 53-1 (60 mg, 0.109 mmol), 5-bromo-3-trifluoromethyl pyrazole (23.38 mg, 0.109 mmol), potassium carbonate (30.06 mg, 0.218 mmol) and dichloro[1,1'-bis(diphenylphosphino)ferrocene] palladium (7.96 mg, 0.011 mmol) were placed in a reaction flask and the mixture was added dioxane (1 mL) and water (0.20 mL), heated to 100 °C and stirred for two hours. The solvent was concentrated to dryness, the residue was purified by column chromatography (methanol: dichloromethane 1:20) to give compound YL055 (17 mg, 0.025 mmol, 23.13%). LC-MS (ESI): m/z 607.06(M+H)⁺.

### Example 56: Synthetic route of compound YL056

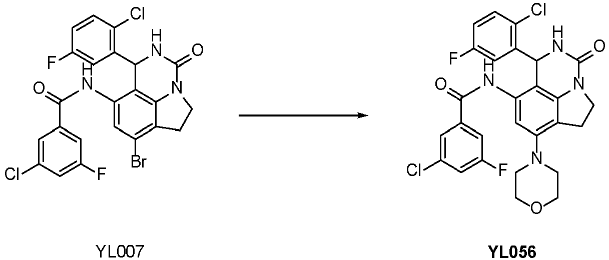

### Synthesis of compound YL056

Compound YL007 (150 mg, 0.256 mmol), Morpholine (0.022 mL, 0.256 mmol), LHMDS (65.41 mg, 0.391 mmol), cyjohnphos (2.98 mg, 0.009 mmol) and Pd₂(dba)₃ (6.88 mg, 0.009 mmol) were combined in a reaction vial, and the mixture was added DMA (1 mL), then microwaved at 120 °C for thirty minutes, extract with ethyl acetate and purified by column chromatography (methanol: dichloromethane 1:20) to give YL056 (3 mg, 0.005 mmol, 5.64%). LC-MS (ESI): m/z 593.95(M+H) ⁺. ¹H NMR (400 MHz, DMSO-*d₆*) δ 10.12 (s, 1H), 8.03 - 7.79 (m, 3H), 7.43 (d, J = 2.7 Hz, 1H), 7.32 (dd, J = 8.8, 5.1 Hz, 1H), 7.15 - 7.07 (m, 1H), 6.82 (dd, J = 9.2, 3.1 Hz, 1H), 6.55 (s, 1H), 6.12 - 5.94 (m, 1H), 3.98 (ddd, J = 13.8, 9.6, 7.8 Hz, 2H), 3.65 (d, J = 6.2 Hz, 2H), 3.12 (td, J = 8.2, 2.3 Hz, 2H), 3.03 (s, 3H), 2.84 (s, 3H).

### Example 57: Synthetic route of compound YL057

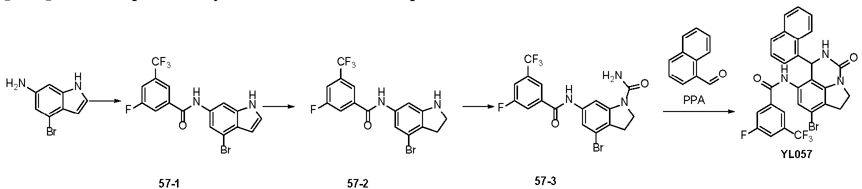

### Synthesis of compound 57-1

To a three-necked flask (100 mL) was added 4-bromo-1H-indol-6-amine (1000 mg, 4.738 mmol), triethylamine (1.317 mL, 9.476 mmol), and dichloromethane (40 mL) serially. Cooled to 0 °C, the reaction mixture was added 3-fluoro-5-(trifluoromethyl) benzoyl chloride (1180.73 mg, 5.212 mmol). The reaction mixture was stirred at 0 °C for 30 min and then warmed to room temperature for 1 h. The reaction mixture was concentrated by column chromatography (eluent: petroleum ether: ethyl acetate = 5:1-3:1) to give compound 57-1 (1809 mg. 4.509 mmol, 95.18%).

### Synthesis of compound 57-2

Compound 57-1 (802 mg, 1.999 mmol), acetic acid (30 mL), sodium cyanoborohydride (376.89 mg, 5.998 mmol) were added sequentially to a three-necked flask (100 mL). The reaction mixture was concentrated and added with ethyl acetate and saturated aqueous sodium bicarbonate, and the organic phase was concentrated by column chromatography (eluent: petroleum ether: ethyl acetate = 10:1-3:1) to give compound 57-2 (350 mg, 0.868 mmol, 43.42%).

### Synthesis of compound 57-3

To a three-necked flask (100 mL) was added compound 57-2 (320 mg, 0.794 mmol) and acetic acid (10 mL) sequentially. Cooled to 0 °C, the reaction was added potassium cyanate (128.77 mg, 1.587 mmol) and stirred at 0 °C for half an hour. The reaction mixture was concentrated and was added water and saturated aqueous sodium bicarbonate, filtered, and the filter cake was washed with water and dried to give 57-3 (280 mg, 0.628 mmol, 79.06%).

### Synthesis of compound YL057

Polyphosphoric acid (20 mL) in a double necked flask was warmed to 105 °C and was added compound 57-3 (4-bromo-2,3-dihydroindol-6-yl-5-fluoro-3-trifluoromethylbenzamide, 200 mg, 0.448 mmol), followed by naphthalene-1-carboxaldehyde (140.01 mg, 0.896 mmol) while stirring steadily, and the resulting mixture was stirred for 30 min. The reaction was quenched with sodium bicarbonate, extracted with dichloromethane, and purified by column chromatography (1:20 methanol/dichloromethane) to give YL057. LC-MS (ESI): m/z 585.37(M+H)⁺. ¹H NMR (400 MHz, DMSO-*d₆*) δ 9.94 (s, 1H), 8.09 (d, J = 8.6 Hz, 1H), 7.79 (dd, J = 19.9, 8.1 Hz, 3H), 7.60 (d, J = 2.7 Hz, 1H), 7.41 (d, J = 13.0 Hz, 2H), 7.31 (t, J = 7.8 Hz, 2H), 7.26 - 7.13 (m, 2H), 6.92 (s, 1H), 6.58 (d, J = 2.6 Hz, 1H), 4.16 - 4.00 (m, 2H), 3.28 - 3.14 (m, 2H).

### Example 58: Synthetic route of compound YL058

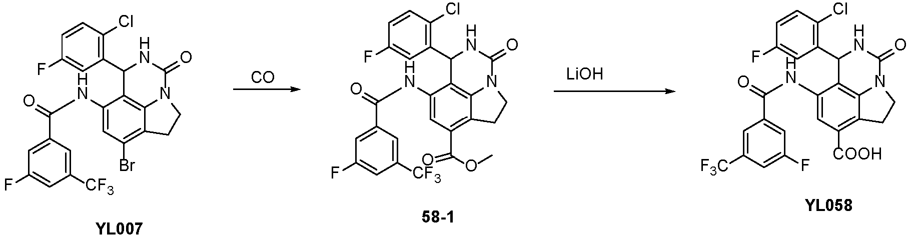

### Synthesis of compound 58-1

Compound YL007 (150 mg, 0.256 mmol), triethylamine (0.071 mL, 0.511 mmol), Pd(dppf)Cl₂ (18.71 mg, 0.026 mmol), *N*,*N*-dimethylformamide (3 mL), and methanol (3 mL) were added sequentially to a 50 mL three-necked flask. The reaction mixture was stirred under the protection of carbon monoxide at 60 °C for 18 h. LCMS showed only a small amount of product, to which were added dichloromethane and water and the organic phase was concentrated and purified by column chromatography (eluent: dichloromethane: methanol = 40:1-20:1) to give Intermediate 58-1 (9.2 mg, 0.016 mmol, 6.36%). LC-MS (ESI): m/z 566.1 (M+H)⁺; ¹H NMR (400 MHz, DMSO-d6) δ 10.24 (s, 1H), 7.96 (dd, *J*=8.3, 2.5 Hz, 1H), 7.91-7.82 (m, 2H), 7.57 (d, *J*=2.5 Hz, 1H), 7.39-7.25 (m, 2H), 7.10 (td, *J*=8.4, 3.1 Hz, 1H), 6.89 (dd, *J =* 9.2, 3.1 Hz, 1H), 6.17 (d, *J* = 2.4 Hz, 1H), 4.02 (dtd, *J =* 18.7, 10.4, 8.2 Hz, 2H), 3.85 (s, 3H),3.48 (t, *J =* 8.6 Hz, 2H).

### Synthesis of compound YL058

Compound 58-1 in a reaction was added water (0.2 mL), methanol (0.40 mL) and tetrahydrofuran (0.80 mL), then lithium hydroxide (19.28 mg, 0.459 mmol) and the reaction mixture was stirred at room temperature for two hours. Extracted with dichloromethane and purified on a silica column (1:20 methanol/dichloromethane) to afford the product YL058 (17 mg, 0.025 mmol, 23.13%). LC-MS (ESI): m/z 552.85(M+H)⁺. ¹H NMR (400 MHz, DMSO-*d₆*) δ 13.05 (s, 1H), 10.21 (s, 1H), 7.95 (d, J = 8.5 Hz, 1H), 7.88 (d, J = 4.6 Hz, 2H), 7.53 (d, J = 2.6 Hz, 1H), 7.33 (dd, J = 8.9, 5.2 Hz, 1H), 7.28 (s, 1H), 7.10 (dd, J = 3.1, 0.9 Hz, 1H), 6.88 (dd, J = 9.2, 3.1 Hz, 1H), 6.19 - 6.11 (m, 1H), 4.09 - 3.93 (m, 2H), 3.47 (t, J = 8.6 Hz, 2H).

### Example 59: Synthetic route of compound YL059

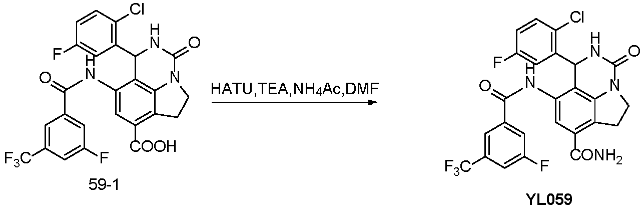

### Synthesis of compound YL059

Compound 59-1 (50 mg, 0.091 mmol), ammonium acetate (13.97 mg, 0.181 mmol) and HATU (51.68 mg, 0.136 mmol) in a reaction flask was added solvent DMF (1 mL) and trimethylamine (0.038 mL, 0.272 mmol) serially. The resulting mixture was stirred for one hour at room temperature. Extracted with ethyl acetate and purified by column chromatography (methanol: dichloromethane 1:20) to afford the product YL059 (26 mg, 0.045 mmol, 49.49%). LC-MS (ESI): m/z 551.87(M+H)⁺. ¹H NMR (400 MHz, DMSO-*d₆*) δ 10.23 (s, 1H), 8.02 - 7.84 (m, 3H), 7.76 (s, 1H), 7.53 (d, J = 2.6 Hz, 1H), 7.40 (s, 1H), 7.34 (dd, J = 8.9, 5.2 Hz, 1H), 7.15 - 7.07 (m, 2H), 6.87 (dd, J = 9.2, 3.1 Hz, 1H), 6.11 (dd, J = 2.5, 1.1 Hz, 1H), 4.11 - 3.88 (m, 2H), 3.57 - 3.35 (m, 2H).

### Example 60: Synthetic route of compound YL060

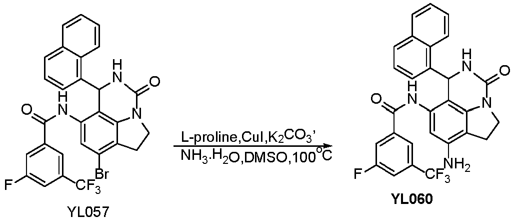

### Synthesis of compound YL060

YL057 (58 mg, 0.099 mmol), L-proline (4.57 mg, 0.040 mmol), cuprous iodide (3.78 mg, 0.020 mmol) and potassium carbonate (41.15 mg, 0.298 mmol) were added to a reaction vial. After the addition of 1.5 mL of DMSO as solvent, the mixture was degassed and purged with nitrogen and was added ammonia (8.70 mg, 0.248 mmol). The reaction mixture was stirred at 100 °C for 6 h. Extracted with ethyl acetate and purification on column (methanol: dichloromethane = 1:20) to obtain the product YL060 (14 mg, 0.026 mmol, 25.75%). LC-MS (ESI): m/z 521.49(M+H)⁺. ¹H NMR (400 MHz, DMSO-*d₆*) δ 9.67 (s, 1H), 8.06 (d, J = 8.6 Hz, 1H), 7.87 - 7.67 (m, 3H), 7.42 (s, 2H), 7.37 - 7.08 (m, 5H), 6.38 (d, J = 2.6 Hz, 1H), 6.01 (s, 1H), 5.22 (s, 2H), 4.11 - 3.91 (m, 2H), 3.00 (t, J = 8.5 Hz, 2H).

### Example 61: Synthetic route of compound YL061

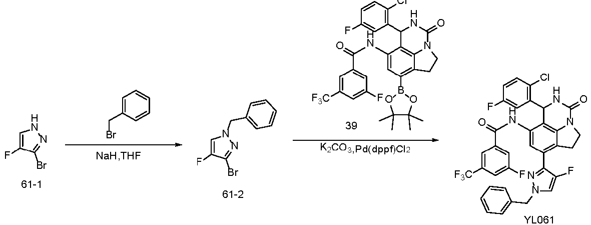

### Synthesis of compound 61-2

Sodium hydride (21.82 mg, 0.909 mmol) was added to a solution of 3-Bromo-4-fluoropyrazole (100 mg, 0.606 mmol) in THF(5mL) in a reaction flask in an ice water bath. Warmed to room temperature and stirred for thirty minutes, the above mixture was added (bromomethyl)benzene (0.108 mL, 0.909 mmol), stirred at room temperature for two hours, monitored, quenched with saturated aqueous ammonium chloride solution. Extracted with ethyl acetate and purification by column (ethyl acetate: petroleum ether = 1:3) to obtain the product 61-2 (110 mg, 0.431 mmol, 71.14%), no signal for liquid mass.

### Synthesis of compound YL061

Compound 61-2 (28 mg, 0.110 mmol), compound 39 (76.53 mg, 0.121 mmol), K₂CO₃ (30.34 mg, 0.220 mmol), Pd(dppf)Cl₂ (8.03 mg, 0.011 mmol) were combined in a reaction flask, then was added 1,4-dioxance (3.0 mL) and water (0.6 mL). After degassed and purged with nitrogen and heated to 100°C. The reaction was monitored for two hours, the solvent was concentrated to dryness, and the sample was purified by mixing on a column silica (ethyl acetate/petroleum ether: dichloromethane = 1:1:1) to give a yellow solid YL061 (50 mg, 0.073 mmol, 66.79%). LC-MS (ESI): m/z 682.14(M+H)⁺. ¹H NMR (400 MHz, DMSO-d6) δ 10.18 (s, 1H), 8.27 (d, J = 2.3 Hz, 1H), 7.94 (d, J = 9.1 Hz, 1H), 7.86 (d, J = 10.8 Hz, 2H), 7.49 (d, J = 2.9 Hz, 1H), 7.39 - 7.29 (m, 6H), 7.11 (dd, J = 8.4, 3.1 Hz, 1H), 6.91 - 6.82 (m, 2H), 6.09 (d, J = 2.4 Hz, 1H), 5.27 (s, 2H), 4.12 - 3.96 (m, 2H), 3.28 (s, 2H).

### Example 62: Synthetic route of compound YL062

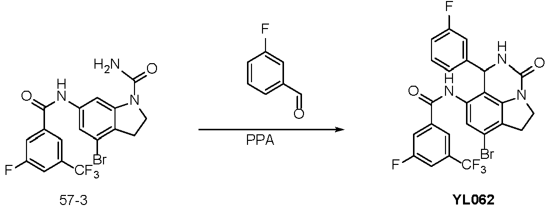

### Synthesis of compound YL062

Polyphosphoric acid (15 mL) in a double mouth flask was stirred steadily at 105 °C, then was added compound 57-3 (100 mg, 0.224 mmol), followed by 3-fluorobenzaldehyde (55.63 mg, 0.448 mmol) and stirred for 30 min. Upon completion, the reaction mixture was poured into ice-cold saturated aqueous sodium bicarbonate solution, was added dichloromethane and stirred until the organic phase was free of mucilage, extracted with dichloromethane and purified by column chromatography (methanol: dichloromethane) to give YL062 (28.4 mg, 0.049 mmol, 21.80%). LC-MS (ESI): m/z 552.03(M+H) ⁺. ¹H NMR (400 MHz, DMSO-d6) δ 10.24 (s, 1H), 8.05 - 7.93 (m, 1H), 7.85 (s, 2H), 7.65 (d, J = 3.1 Hz, 1H), 7.34 - 7.21 (m, 1H), 7.14 - 6.99 (m, 2H), 6.97 - 6.90 (m, 1H), 6.85 (dt, J = 9.7, 2.2 Hz, 1H), 5.82 (d, J = 2.9 Hz, 1H), 4.00 (td, J = 8.9, 5.4 Hz, 2H), 3.22 - 3.10 (m, 2H).

### Example 63: Synthetic route of compound YL063

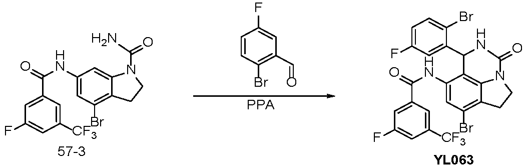

### Synthesis of compound YL063

Polyphosphoric acid (20 mL) was added to a double mouth flask, warmed to 105 °C, stirred steadily and compound 57-3 (100 mg, 0.224 mmol) was added, followed by 2-bromo-5-fluorobenzaldehyde (91.00 mg, 0.448 mmol) and stirred for 30 min. Upon completion, the reaction mixture was poured into ice-cold saturated aqueous sodium bicarbonate solution, dichloromethane was added and stirred until the organic phase was free of viscosity, extracted with dichloromethane and purified by column chromatography (methanol: dichloromethane) to give YL063 (48.2 mg, 0.073 mmol, 32.37%). LC-MS (ESI): m/z 631.94(M+H)⁺. ¹H NMR (400 MHz, DMSO-d6) δ 10.15 (s, 1H), 7.95 (d, J = 8.4 Hz, 1H), 7.83 (d, J = 11.2 Hz, 2H), 7.58 - 7.41 (m, 2H), 7.08 - 6.97 (m, 1H), 6.94 - 6.84 (m, 2H), 6.03 (dd, J = 2.4, 1.0 Hz, 1H), 4.03 (td, J = 9.5, 8.0 Hz, 2H), 3.17 (ddd, J = 9.5, 6.8, 4.2 Hz, 2H).

### Example 64: Synthetic route of compound YL064

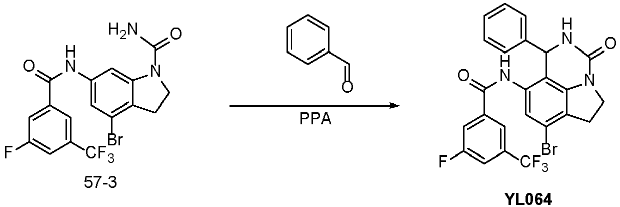

### Synthesis of compound YL064

Polyphosphoric acid (15 mL) was added to a double mouth flask, warmed to 105 °C, stirred steadily, and compound 57-3 (100 mg, 0.224 mmol) was added, followed by benzaldehyde (0.045 mL, 0.448 mmol) and stirred for 30 min. Upon completion, the reaction mixture was poured into ice-cold saturated aqueous sodium bicarbonate solution, dichloromethane was added and stirred until the organic phase was free of viscosity, extracted with dichloromethane, concentrated the residue was purified by column chromatography (methanol: dichloromethane=1:20) to give YL064 (38.5 mg, 0.068 mmol, 30.54%), LC-MS (ESI): m/z 534.04 (M+H) ⁺. ¹H NMR (400 MHz, DMSO-d6) δ 10.22 (s, 1H), 7.97 (d, J = 8.4 Hz, 1H), 7.85 (s, 2H), 7.62 (d, J = 3.0 Hz, 1H), 7.32 - 7.17 (m, 3H), 7.09 (q, J = 3.2 Hz, 3H), 5.80 (d, J = 3.0 Hz, 1H), 4.00 (d, J =3.4 Hz, 2H), 3.16 (s, 2H).

### Example 65: Synthetic route of compound YL065-P1 and YL065-P2

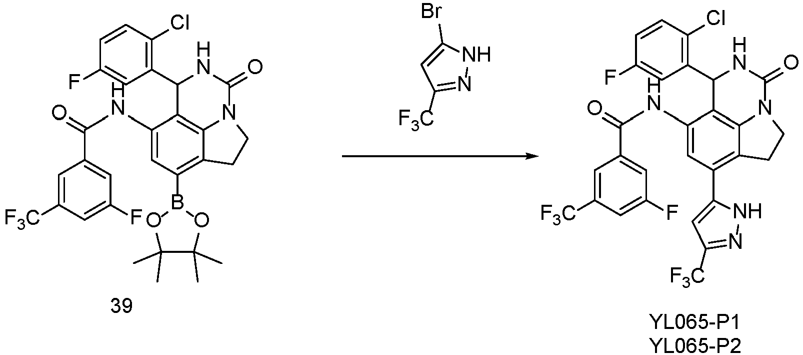

### Synthesis of compounds YL065-P1 and YL065-P2

Compound 39 (1 g, 1.578 mmol), 5-bromo-3-trifluoromethylpyrazole (0.31 g, 1.434 mmol), potassium carbonate (0.40 g, 2.869 mmol) and Pd(dppf)Cl₂ (0.10 g, 0.143 mmol) in a reaction vial were added dioxane (10 mL) and water (2.00 mL). After degassed and purged with nitrogen, the reaction mixture was stirred at 105 °C for 5 h, then extracted with ethyl acetate and water, the organicn phases were concentrated to dryness, and the residue was purified by column chromatograpy (mobile phase: dichloromethane/methanol 1/0 to 20/1) to give YL065 (560 mg, 0.872 mmol, 60.82%). Compounds YL065-P1 and YL065-P2 were obtained by SFC chiral resolution (method is same as in Example YL010).

YL065-P1: retention time: 4.561 min; LC-MS (ESI): m/z 642.09(M+H) ⁺; ¹H NMR (400 MHz, DMSO-d₆) δ 14.03 (d, J = 2.1 Hz, 1H), 10.30 (s, 1H), 8.10 - 7.84 (m, 3H), 7.61 (d, J = 2.7 Hz, 1H), 7.35 (dd, J = 8.8, 5.1 Hz, 1H), 7.18 (s, 1H), 7.13 (dd, J = 8.2, 3.1 Hz, 1H), 7.03 (d, J = 1.9 Hz, 1H), 6.93 (dd, J = 9.1, 3.1 Hz, 1H), 6.13 (d, J = 2.6 Hz, 1H), 4.07 (dd, J = 32.9, 6.5 Hz,2H), 3.53 (d, J = 6.9 Hz, 1H), 3.38 (d, J = 4.7 Hz, 1H).

YL065-P2: retention time: 5.561 min; LC-MS (ESI): m/z 642.09(M+H) ⁺; ¹H NMR (400 MHz, DMSO-d₆) δ 14.03 (s, 1H), 10.30 (s, 1H), 8.08 - 7.80 (m, 3H), 7.61 (d, J = 2.7 Hz, 1H), 7.35 (dd, J = 8.9, 5.2 Hz, 1H), 7.18 (s, 1H), 7.12 (d, J = 3.1 Hz, 1H), 7.03 (s, 1H), 6.93 (dd, J = 9.2, 3.1 Hz, 1H), 6.13 (d, J = 2.5 Hz, 1H), 4.06 (ddd, J = 23.1, 10.6, 6.5 Hz, 2H), 3.61 - 3.45 (m, 1H), 3.36 (d, J = 6.1 Hz,1H).

### Example 66: Synthetic route of compound YL066

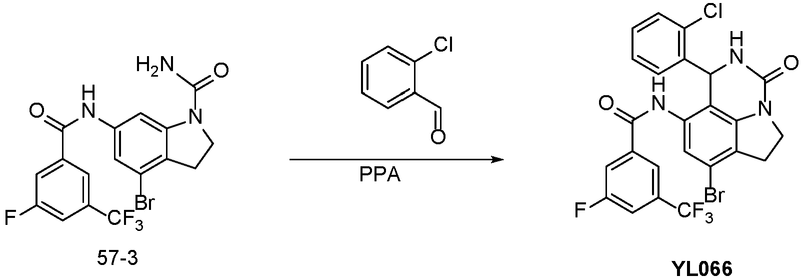

### Synthesis of compound YL066

Polyphosphoric acid (15 mL) in a double mouthed flask was warmed to 105 °C and stirred steadily, then was added compound 57-3 (100 mg, 0.224 mmol) followed by 2-chlorobenzaldehyde (63.01 mg, 0.448 mmol) and stirred for 30 min. Monitored to be completed, the reaction was quenched with sodium bicarbonate, extracted with dichloromethane and purified on a silica column (MeOH: DCM=1:20) to give compound YL066 (69.2 mg, 0.116 mmol, 51.57%). LC-MS (ESI): m/z 568.00(M+H) ⁺. ¹H NMR (400 MHz, DMSO-*d₆*) δ 10.19 (s, 1H), 7.95 (dd, J = 8.4, 2.3 Hz, 1H), 7.90 - 7.81 (m, 2H), 7.58 (d, J = 2.7 Hz, 1H), 7.30 - 7.12 (m, 4H), 6.95 (s, 1H), 6.13 (d, J = 2.6 Hz, 1H), 4.03 (td, J = 9.0, 6.2 Hz, 2H), 3.18 (dd, J = 10.2, 7.2 Hz, 2H).

### Example 67: Synthetic route of compound YL067

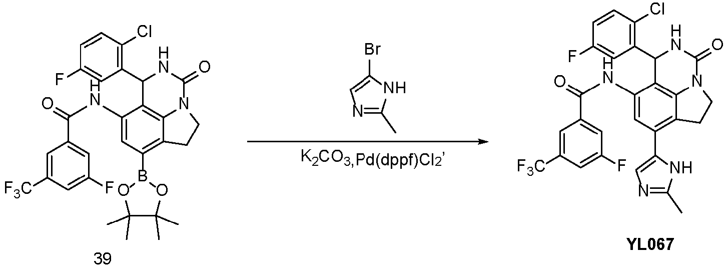

### Synthesis of compound YL067

Compound 39 (50 mg, 0.079 mmol), 5-bromo-2-methylimidazole (12.70 mg, 0.079 mmol), potassium carbonate (21.80 mg, 0.158 mmol) and Pd(dppf)Cl₂ (5.77 mg, 0.008 mmol) in a reaction vial were added 3 mL of mixed solvent (1,4 Dioxane: water = 5:1). After degassed and purged with nitrogen for three times, the reaction mixture was stirred at 100 °C overnight. Monitored by LC-MS, the solvent was concentrated to dryness, and purified on a silica column (MeOH: DCM = 1: 10) to give compound YL067 (8 mg, 0.013 mmol, 16.39%). LC-MS (ESI): m/z 588.11(M+H)⁺. ¹H NMR (400 MHz, DMSO-*d₆*) δ 11.98 (s, 1H), 10.17 (s, 1H), 8.01 - 7.85 (m, 3H), 7.45 (s, 1H), 7.32 (td, J = 8.6, 8.1, 4.6 Hz, 3H), 7.09 (td, J = 8.3, 3.0 Hz, 1H), 6.86 (dd, J = 9.4, 2.9 Hz, 1H), 6.08 (d, J = 2.6 Hz, 1H), 4.04 (ddd, J = 14.9, 10.2, 7.2 Hz, 2H), 3.39 (s, 2H), 2.31 (s, 3H).

### Example 68: Synthetic route of compound YL068

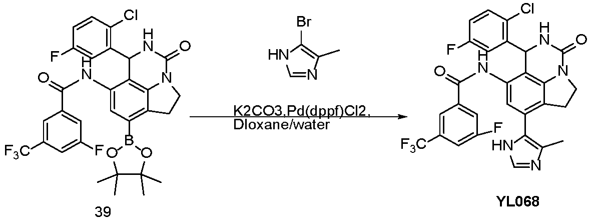

### Synthesis of compound YL068

Compound 39 (50 mg, 0.079 mmol), 5-bromo-4-methylimidazole (12.70 mg, 0.079 mmol), potassium carbonate (21.80 mg, 0.158 mmol) and Pd(dppf)Cl₂ (5.77 mg, 0.008 mmol) in a reaction vial were added 3 mL of mixed solvent (1,4-dioxane: water = 3:1). After degassed and purged with nitrogen for three times, the reaction mixture was stirred at 100 °C overnight. Monitored by LC-MS, the solvent was concentrated to dryness, and purified on a silica column (MeOH: DCM = 1:10) to obtain the product YL068 (8 mg, 0.013 mmol, 16.39%). LC-MS (ESI): m/z 588.11(M+H) ⁺. ¹H NMR (400 MHz, DMSO-*d₆*) δ 12.06 (s, 1H), 10.15 (s, 1H), 8.00 - 7.85 (m, 3H), 7.62 (s, 1H), 7.46 (d, J = 2.7 Hz, 1H), 7.33 (dd, J = 8.9, 5.1 Hz, 1H), 7.10 (td, J = 8.3, 3.1 Hz, 1H), 6.88 (dd, J = 9.1, 3.1 Hz, 1H), 6.80 (s, 1H), 6.11 (d, J = 2.6 Hz, 1H), 4.14 - 3.83 (m, 2H), 3.41 (d, J = 10.0 Hz,2H), 2.33 (s, 3H).

### Example 69: Synthetic route of compound YL069

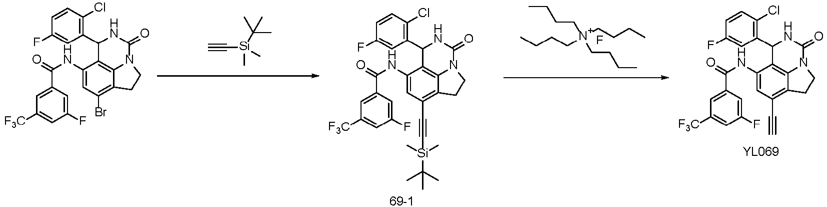

### Synthesis of compound 69-1

To a reaction flask were added compound YL007 (250 mg, 0.426 mmol, purity: 83.9 %), tert-butyldimethylsilyl acetylene (478.24 mg, 3.41 mmol), cuprous iodide (162.30 mg, 0.852 mmol), potassium iodide (70.73 mg, 0. 426 mmol), diisopropylamine(646.75 mg, 6.39 mmol), tetrakis(triphenylphosphine)palladium (246.19 mg, 0.213 mmol) and DMF (70 mL), and the mixture was heated to 100 °C and stirred under nitrogen protection overnight. The next day, cooled to room temperature, the reaction mixture was added saturated ethylenediaminetetraacetic acid solution and stirred at room temperature for 1 h, then diluted with ethyl acetate and water. The organic phase was separated and the aqueous phase was extracted with ethyl acetate. The organic phase was combined, washed with brine, dried over anhydrous Na₂SO₄, filtered, concentrated at reduced pressure and purified by automated chromatography (Biotage) (mobile phase: petroleum ether/ethyl acetate 100/45 to 100/50) to give a white solid as compound 69-1 (200 mg, purity: 95 %, yield: 72.6 %). LC-MS (ESI): m/z 646.3 (M+H) ⁺. ¹H NMR (400 MHz, DMSO-*d₆*) δ 10.11 (s, 1H), 7.94 (d, J = 8.4 Hz, 1H), 7.89 - 7.81 (m, 2H), 7.51 (d, J = 2.6 Hz, 1H), 7.32 (dd, J = 8.9, 5.1 Hz, 1H), 7.08 (ddd, J = 8.8, 7.9, 3.1 Hz, 1H), 6.88 (dd, J = 9.2, 3.1 Hz, 1H), 6.82 (s, 1H), 6.11 (dd, J = 2.5, 1.0 Hz, 1H), 4.06 - 3.97 (m, 2H), 3.24 - 3.17 (m, 2H), 0.98 (s, 9H), 0.17 (s, 6H).

### Synthesis of compound YL069

69-1 (130 mg, 0.426 mmol), THF (20 mL) and tetrabutylammonium fluoride in tetrahydrofuran (1 M, 0.65 mL) in a reaction vial and the mixture was stirred at room temperature for 0.5 h, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: petroleum ether/ethyl acetate 100/45 to 100/50) to give a white solid as compound YL069 (70 mg, purity: 97 %, yield: 65.4 %). LC-MS (ESI): m/z 532.1 (M+H) ⁺; ¹H NMR (400 MHz, DMSO-*d₆*) δ 10.13 (s, 1H), 7.94 (dt, J = 8.5, 1.9 Hz, 1H), 7.89 - 7.80 (m, 2H), 7.50 (d, J = 2.5 Hz, 1H), 7.32 (dd, J = 8.8, 5.2 Hz, 1H), 7.09 (ddd, J = 8.8, 8.0, 3.1 Hz, 1H), 6.88 (dd, J = 9.2, 3.1 Hz, 1H), 6.82 (s, 1H), 6.10 (dd, J = 2.6, 1.0 Hz, 1H), 4.43 (s, 1H), 4.07 - 3.97 (m, 2H), 3.23 (ddd, J = 11.1, 7.4, 3.1 Hz, 2H).

### Example 70: Synthetic route of compound YL070

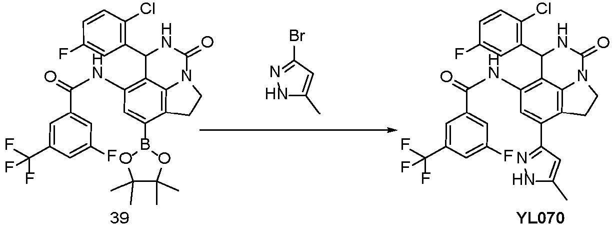

To a reaction flask were added compound 39 (50.00 mg, 78.89 µmol), 3-bromo-5-methyl-1H-pyrazole (25.40 mg, 157.78 µmol), potassium carbonate (32.71 mg, 236.67 µmol), 1,1'-bis(diphenylphosphino)ferrocene palladium(II) dichloride (11.54 mg, 15.78 µ mol), 1,4-dioxane (10 mL) and water (2 mL), and the mixture was heated to 100 °C and stirred for 1 hr under nitrogen protection, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/3 to 100/4) to give a white solid compound YL070 (12 mg, purity: 94 %, yield: 25.8 %). LC-MS (ESI): m/z 588.2 (M+H) ⁺; ¹H NMR (400 MHz, DMSO-*d₆*) *δ* 12.72 (s, 1H), 10.18 (s, 1H), 7.96 - 7.86 (m, 3H), 7.46 (s, 1H), 7.33 (dd, J = 8.8, 5.1 Hz, 1H), 7.18 - 7.07 (m, 2H), 6.88 (dd, J = 9.2, 3.2 Hz, 1H), 6.39 (s, 1H), 6.10 (d, J = 2.6 Hz, 1H), 4.09 - 3.97 (m, 2H), 3.45 (dd, J = 17.8, 9.4 Hz, 2H), 2.27 (s, 3H).

### Example 71: Synthetic route of compound YL071

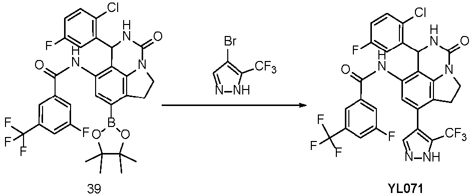

To a reaction vial were added compound 39 (250.00 mg, 394.44 µmol), 4-bromo-5-(trifluoromethyl)-1H-pyrazole (169.59 mg, 788.88 µmol), potassium carbonate (163.54 mg, 1.18 mmol), 1,1'-bis(diphenylphosphino)ferrocene palladium(II) dichloride (57.72 mg. 78.89 µmol), 1,4-dioxane (20 mL) and water (4 mL), and the mixture was heated to 110 °C and stirred for 3 h under nitrogen protection, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/5 to 100/6), and the resulting crude product was purified by prep-HPLC (Boston pHlex ODS, 21.2*250 mm, 10 um, water (0.05 % ammonium bicarbonate) / acetonitrile, flow rate 30 mL/min, column temperature 25 °C, wavelength 254 nm) to obtain the white solid compound YL071 (45 mg, purity: 96 %, yield: 17.7 %). LC-MS (ESI): m/z 642.2 (M+H) ⁺. ¹H NMR (400 MHz, DMSO-d6) δ 13.85 (s, 1H), 10.20 (s, 1H), 8.25 (s, 1H), 7.95 (d, J = 8.5 Hz, 1H), 7.88 (s, 2H), 7.54 (d, J = 2.7 Hz, 1H), 7.34 (dd, J = 8.9, 5.1 Hz, 1H), 7.11 (td, J = 8.3, 3.1 Hz, 1H), 6.88 (dd, J = 9.1, 3.0 Hz, 1H), 6.68 (s, 1H), 6.11 (d, J = 2.6 Hz, 1H), 3.99 (dtd, J = 28.2, 10.4, 6.6 Hz, 2H), 3.30 - 3.07 (m, 2H).

### Example 72: Synthetic route of compound YL072

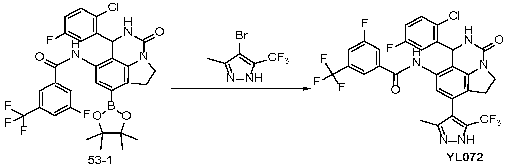

### Synthesis of compound YL072

To a reaction flask were added compound 39 (250.00 mg, 394.44 µmol), 4-bromo-3-methyl-5-(trifluoromethyl)-1H-pyrazole (180.65 mg, 788.88 µmol), potassium carbonate (163.54 mg, 1.18 mmol), 1,1'-bis(diphenylphosphino)ferrocene palladium(II) dichloride (57.72 mg, 78.89 µmol), 1,4-dioxane (20 mL) and water (4 mL), and the mixture was heated to 110 °C under nitrogen protection and stirred for 3 h, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/5 to 100/6), and the resulting crude product was purified by prep-HPLC (Boston pHlex ODS, 21.2*250 mm, 10 um, water (0.05 % ammonium bicarbonate) / acetonitrile, flow rate 30 mL/min, column temperature 25 °C, wavelength 254 nm) to obtain the white solid compound YL072 (16 mg, purity: 94 %, yield: 6.2 %). LC-MS (ESI): m/z 656.2 (M+H) ⁺. ¹H NMR (400 MHz, DMSO-*d₆*) δ 13.56 (s, 1H), 10.19 (s, 1H), 7.91 (d, J = 33.2 Hz, 3H), 7.51 (s, 1H), 7.36 (s, 1H), 7.12 (s, 1H), 6.87 (s, 1H), 6.55 (s, 1H), 6.11 (s, 1H), 4.07 - 3.90 (m, 2H), 2.93 (s, 2H), 2.16 (s, 3H).

### Example 73: Synthetic route of compound YL073

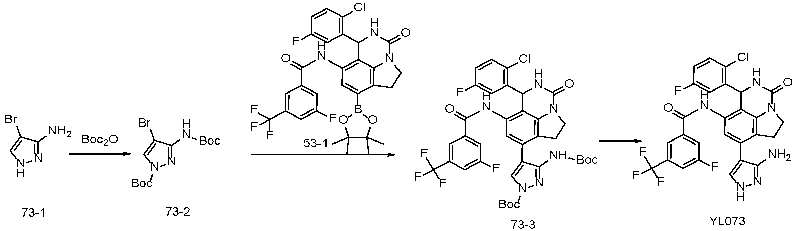

### Synthesis of compound 73-2

To a reaction flask were added 4-bromo-1H-pyrazol-3-amine (400 mg, 2.47 mmol), di-tert-butyl dicarbonate (2.16 g, 9.88 mmol), 4-dimethylaminopyridine (60.34 mg, 493.86 µmol), triethylamine (0.8 mL), and dichloromethane (6 mL) and the mixture was stirred at room temperature for 2 hours. The reaction mixture was cooled to room temperature and diluted with dichloromethane and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: petroleum ether/ethyl acetate 100/10 to 100/11) to give a white solid as compound 73-2 (4.6 g, purity: 88.5 %, yield: 46.5 %). m/z 364.0 (M+H) +

### Synthesis of compound 73-3

To a reaction vial were added 73-2 (200.00 mg, 315.55 µmol), compound 39 (228.60 mg, 631.11 µmol), potassium carbonate (130.83 mg, 946.66 µmol), 1,1'-bis(diphenylphosphino)ferrocene palladium(II) dichloride (46.18 mg, 63.11 µmol), 1. 4-dioxane (20 mL) and water (2 mL), and the mixture was heated to 110 °C and stirred for 3 h under nitrogen protection, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/8 to 100/10) to give a white solid compound 73-3 (75 mg, purity: 47 %, yield: 14 %). LC-MS (ESI): m/z 789.3 (M+H) ⁺.

### Synthesis of compound YL073

To a reaction vial were added compound 73-3 (200.00 mg, 315.55 µmol), dichloromethane (35 mL) and water (3.5 mL) and the mixture was stirred at room temperature for 1 h, then diluted with dichloromethane and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/8 to 100/10) to give a light brown solid as compound YL073 (8 mg, purity: 92 %, yield: 29.7 %). LC-MS (ESI): m/z 589.1 (M+H) ⁺. ¹H NMR (400 MHz, DMSO-*d₆*) δ 11.81 (s, 1H), 10.12 (s, 1H), 7.95 (d, J = 8.5 Hz, 1H), 7.93 - 7.85 (m, 2H), 7.60 (s, 1H), 7.47 (d, J = 2.8 Hz, 1H), 7.33 (dd, J = 8.7, 5.1 Hz, 1H), 7.09 (td, J = 8.4, 3.1 Hz, 1H), 6.91 - 6.85 (m, 2H), 6.11 (d, J = 3.2 Hz, 1H), 4.70 (s, 2H), 4.05 - 3.91 (m, 2H), 3.31 - 3.15 (m, 2H).

### Example 74: Synthetic route of compound YL074

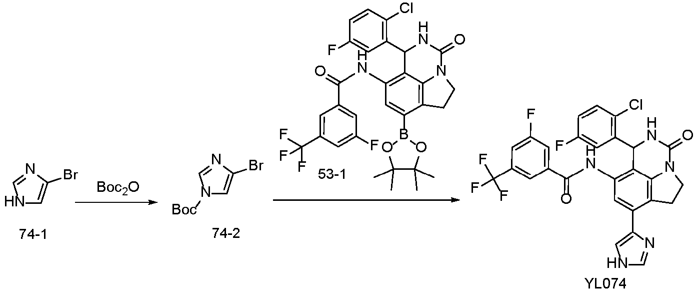

### Synthesis of compound 74-2

To a reaction flask were added 4-bromo-1H-imidazole (500.00 mg, 3.40 mmol), di-tert-butyl dicarbonate (3.71 g, 17.01 mmol), 4-dimethylaminopyridine (83.12 mg, 680.39 µmol), triethylamine (1 mL) and dichloromethane (10 mL) and the mixture was stirred at room temperature for 2 hours. The reaction mixture was cooled to room temperature and diluted with dichloromethane and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: petroleum ether/ethyl acetate 100/5 to 100/6) to give a white solid as compound 74-2, 700 mg, purity: 97.6 %, yield: 81.2 %). LC-MS (ESI): m/z 249.1 (M+H) ⁺.

### Synthesis of compound YL074

To a reaction vial were added compound 74-2 (200.00 mg, 315.55 µmol), compound 39 (155.94 mg, 631.11 µmol), potassium carbonate (130.83 mg, 946.66 µmol), 1,1'-bis(diphenylphosphino)ferrocene palladium(II) dichloride (46.18 mg, 63.11 µmol), 1,4-dioxane (15 mL) and water (1.5 mL), and the mixture was heated to 110 °C and stirred for 2 hours under nitrogen protection, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/8 to 100/9) to give a white solid compound YL074 (50 mg, purity: 97.5 %, yield: 22.9 %). LC-MS (ESI): m/z 574.2 (M+H) ⁺. ¹H NMR (400 MHz, DMSO-*d₆*) δ 12.33 (s, 1H), 10.20 (s, 1H), 7.99 - 7.85 (m, 3H), 7.77 (s, 1H), 7.47 (d, J = 14.6 Hz, 2H), 7.37 - 7.29 (m, 2H), 7.09 (td, J = 8.4, 3.2 Hz, 1H), 6.86 (dd, J = 9.1, 3.1 Hz, 1H), 6.08 (d, J = 2.6 Hz, 1H), 4.13 - 3.96 (m, 2H), 3.49 - 3.37 (m, 2H).

### Example 75: Synthetic route of compound YL075

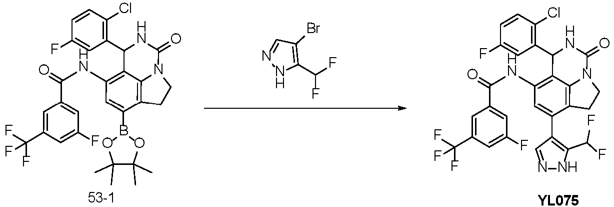

### Synthesis of compound YL075

To a reaction flask were added compound 39 (93.00 mg, 146.73 µmol, purity: 57.9 %), 4-bromo-5-(difluoromethyl)-1H-pyrazole (57.81 mg, 293.46 µmol), potassium carbonate (60.84 mg, 440.20 µmo), 1,1'-bis(diphenylphosphino)ferrocene palladium(II) dichloride (21.47 mg, 29.35 µmol), 1,4-dioxane (10 mL) and water (1 mL), and the mixture was heated to 110 °C and stirred for 3 h under nitrogen protection, then diluted with ethyl acetate and water. The organic phase was separated and the aqueous phase was extracted with ethyl acetate. The organic phases were combined, washed with brine, dried over anhydrous Na₂SO₄, filtered, concentrated at reduced pressure and purified by automated chromatography (Biotage) (mobile phase: dichloromethane/methanol 100/7 to 100/8), and the resulting crude product was prepared and purified (Boston pHlex ODS, 21.2*250 mm, 10 um, water (0.05 % ammonium bicarbonate) / acetonitrile, flow rate 30 mL/min, column temperature 25 °C, wavelength 254 nm) to obtain a white solid compound YL075 (6 mg, purity: 94 %, yield: 10.6 %). LC-MS (ESI): m/z 624.1 (M+H) ⁺; ¹H NMR (400 MHz, DMSO-d₆) δ 13.58 (s, 1H), 10.20 (s, 1H), 8.15 (s, 1H), 8.00 - 7.80 (m, 3H), 7.53 (d, J = 2.7 Hz, 1H), 7.33 (dd, J = 8.9, 5.1 Hz, 1H), 7.27 - 7.03 (m, 2H), 6.89 (dd, J = 9.3, 3.3 Hz, 1H), 6.80 (s, 1H), 6.11 (s, 1H), 4.07 - 3.96 (m, 2H), 3.19 (dd, J = 10.3, 5.9 Hz, 2H).

### Example 76 Synthetic route of compound YL076

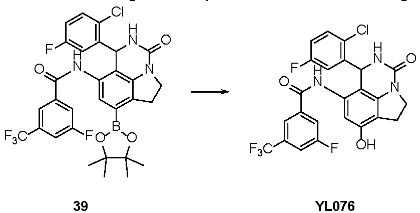

### Synthesis of compound YL076

Compound 39 (123 mg, 0.194 mmol), acetone (4 mL), water (4 mL), and potassium peroxymonosulfate (131.23 mg, 0.213 mmol) were added to a reaction vial and the mixture was stirred at room temperature for 2 h. The reaction was completed shown by LCMS. The reaction mixture was concentrated and the residue was added dichloromethane and the organic phase was concentrated and purified by column chromatography (eluent: dichloromethane: methanol = 40:1 to 20:1) to give YL076 (41 mg, 0.078 mmol, 40.33%). LC-MS (ESI): m/z 524.0 (M+H)⁺; ¹H NMR (400 MHz, DMSO-d6) δ 10.00 (s, 1H), 9.76 (s, 1H), 7.92 (dd, J = 8.6, 2.1 Hz, 1H), 7.83 (d, J = 11.1 Hz, 2H), 7.49-7.22 (m, 2H), 7.07 (ddd, J= 8.8, 7.9, 3.1 Hz, 1H), 6.79 (dd, J = 9.3, 3.1 Hz, 1H), 6.23 (s, 1H), 5.99 (dd, J = 2.6, 1.2 Hz, 1H), 4.04-3.79 (m, 2H), 3.07 (dd, J = 9.8, 7.4 Hz, 2H).

### Example 77: Synthetic route of compound YL077

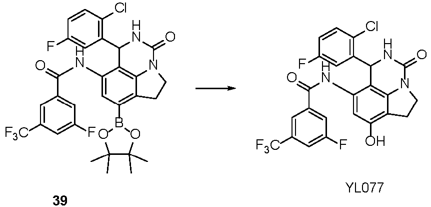

### Synthesis of compound YL077

To a three-necked flask (25 mL) were added 39 (123 mg, 0.194 mmol), acetone (4 mL), water (4 mL), and potassium peroxymonosulfate (131.23 mg, 0.213 mmol) sequentially. The reaction mixture was stirred at room temperature for 2h. When LCMS showed the reaction was complete, the reaction mixture was concentrated and the residue was added dichloromethane, the organic phase was concentrated and purified by column chromatography (eluent: dichloromethane: methanol = 40:1-20:1) to give YL077 (41 mg, 0.078 mmol, 40.33%). LC-MS (ESI): m/z 524.0 (M+H)⁺; ¹H NMR (400 MHz, DMSO-d6) δ 10.00 (s, 1H), 9.76 (s, 1H), 7.92 (dd, *J* = 8.6, 2.1 Hz, 1H), 7.83 (d, *J* = 11.1 Hz, 2H), 7.49-7.22 (m, 2H), 7.07 (ddd, *J*= 8.8, 7.9, 3.1 Hz, 1H), 6.79 (dd, *J* = 9.3, 3.1 Hz, 1H), 6.23 (s, 1H), 5.99 (dd, *J* = 2.6, 1.2 Hz, 1H), 4.04-3.79 (m, 2H), 3.07 (dd, *J* = 9.8, 7.4 Hz, 2H).

### Example 78: Synthetic route of compound YL078

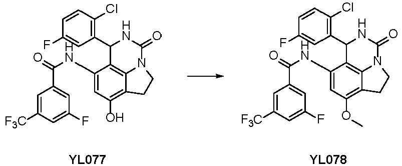

### Synthesis of compound YL078

Compound YL077 (28 mg, 0.053 mmol), potassium carbonate (8.86 mg, 0.064 mmol), *N*,*N*-dimethylformamide (2 mL), and iodomethane (0.005 mL, 0.059 mmol) were added sequentially to a three-necked flask (25 mL). The reaction mixture was stirred at room temperature for 1 h. LCMS showed part of compound YL077 reacted. Water and ethyl acetate were added to the reaction mixture and the organic phases were concentrated by reversed-phase column chromatography (eluent: 10 nM NH₄HCO₃: methanol = 40:60 to 20:80) to give YL078 (12 mg, 0.022 mmol) in 41.74% yield. LC-MS (ESI): m/z 538.2 (M+H)⁺; ¹H NMR (400 MHz, DMSO-d6) δ 10.14 (s, 1H), 8.02-7.79 (m, 3H), 7.40 (d, J=2.6 Hz, 1H), 7.31 (dd, J=8.8, 5.1 Hz, 1H), 7.08 (td, J=8.4, 3.1 Hz, 1H), 6.91-6.76 (m, 1H), 6.42 (s, 1H), 6.06-5.95 (m, 1H), 4.06-3.90 (m, 2H), 3.77 (s, 3H), 3.15-3.05 (m, 2H).

### Example 79: Synthetic route of compound YL079-P1 and YL079-P2

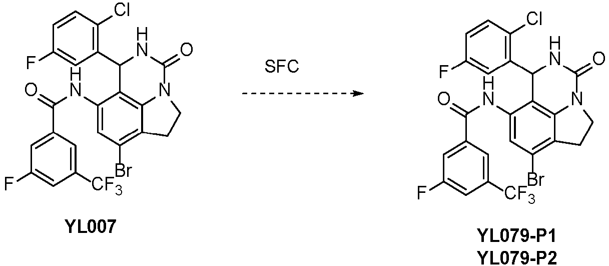

YL007 (89.5 mg) was separated by chiral resolution with the following conditions: instrument: SFC-150 (Waters); column: OJ-H 4.6* 100 mm 5um; mobile phase: CO₂/MeOH [0.2% NH3 (7 M in MeOH)]; flow rate: 3.0 mL/min; back pressure: 2000 psi; wavelength: 214 nm; Column temperature: 40 °C. YL079-P1 (retention time 0.707 min, 33.0 mg) and YL079-P2 (retention time 1.449 min, 29.6 mg) were obtained, LC-MS (ESI): m/z 588.0 (M+H)⁺;

Data for YL079-P2 : LC-MS (ESI): m/z 588.0 (M+H)⁺; ¹H NMR (400 MHz, DMSO-d₆) δ 10.16 (s, 1H), 7.95 (dt, J = 8.6, 1.9 Hz, 1H), 7.89 - 7.77 (m, 2H), 7.54 (d, J = 2.6 Hz, 1H), 7.32 (dd, J = 8.8, 5.2 Hz, 1H), 7.09 (ddd, J = 8.8, 8.0, 3.1 Hz, 1H), 6.95 (s, 1H), 6.90 (dd, J = 9.2, 3.1 Hz, 1H), 6.14 - 5.97 (m, 1H), 4.16 - 3.87 (m, 2H), 3.17 (ddd, J = 9.6, 6.9, 4.6 Hz, 2H).

### Example 80: Synthetic route of compound YL080

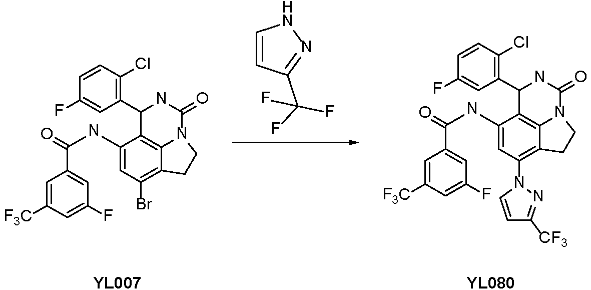

### Synthesis of compound YL080

To a three-necked flask (25 mL), 2-di-tert-butylphosphonium-3,4,5,6-tetramethyl-2',4',6'-triisopropylbiphenyl (8.19 mg, 0.017 mmol), tris(dibenzylideneacetone)dipalladium (7.80 mg, 0.009 mmol), 1,4-dioxane (2.5 mL), toluene (0.5 mL) were added sequentially. The reaction mixture was stirred at 110 °C for 30 min under nitrogen protection, then cooled to room temperature and was added compound YL007 (50 mg, 0.085 mmol), 3-(trifluoromethyl) pyrazole (34.79 mg, 0.256 mmol), potassium phosphate (54.26 mg, 0.256 mmol). The reaction was continued at 110 °C for 3 h under nitrogen protection. The LCMS showed the reaction was successful and the reaction mixture was concentrated to give YL080 (28.8 mg, 0.045 mmol, 52.65%) in reverse phase preparation (basic method). LC-MS (ESI): m/z 642.2 (M+H)⁺; ¹H NMR (400 MHz, DMSO-d6) δ 8.74 (s, 1H), 8.20 (dd, J = 10.4, 2.5 Hz, 1H), 8.09 (dd, J = 8.7, 2.4 Hz, 1H), 7.83 (d, J = 6.2 Hz, 1H), 7.13 (d, J = 2.6 Hz, 1H),7.03 (dd, J = 8.8, 5.2 Hz, 2H), 6.90 (td, J = 8.4, 3.2 Hz, 1H), 6.60 (dd, J = 9.4, 3.1 Hz, 1H), 6.01 (s, 1H), 5.78-5.65 (m, 1H), 5.23 (s, 2H), 3.95 (pd, J = 11.0, 10.4, 6.0 Hz, 2H), 2.97 (t, J = 8.5 Hz, 2H).

### Example 81: Synthetic route of compound YL081

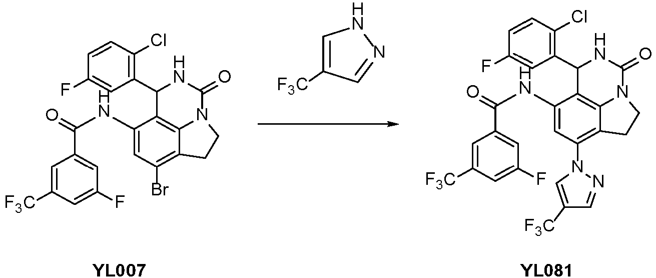

### Synthesis of compound YL081

To a three-necked flask (25 mL), 2-di-tert-butylphosphonium-3,4,5,6-tetramethyl-2',4',6'-triisopropylbiphenyl (8.19 mg, 0.017 mmol), tris(dibenzylideneacetone)dipalladium (7.80 mg, 0.009 mmol), 1,4-dioxane (2.5 mL), toluene (0.5 mL) were added sequentially. The reaction mixture was stirred at 110 °C for 30 min under nitrogen protection, then it was cooled to room temperature and was added YL007 (50 mg, 0.085 mmol), 4-(trifluoromethyl)-1H-pyrazole (34.79 mg, 0.256 mmol), potassium phosphate (54.26 mg, 0.256 mmol). The reaction was continued under nitrogen protection at 110 °C for 3 h. The LCMS the reaction was successful and the reaction mixture was concentrated and the residue was purified by prep-HPLC (basic method) to give YL081 (28.7 mg, 0.045 mmol, 52.47%). LC-MS (ESI): m/z 642.2 (M+H)⁺; ¹H NMR (400 MHz, DMSO-d6) δ 10.28 (s, 1H), 9.01 (t, J = 1.0 Hz, 1H), 8.26 (s, 1H), 8.06-7.78 (m, 3H), 7.63 (d, J = 2.6 Hz, 1H), 7.35 (dd, J = 8.9,5.1 Hz, 1H), 7.23 (s, 1H), 7.11 (ddd, J = 8.9, 7.9, 3.1 Hz, 1H), 6.94 (dd, J = 9.2, 3.1 Hz, 1H), 6.15 (dd, J = 2.7, 1.0 Hz, 1H), 4.04 (dtd, J = 30.4, 10.4, 6.7Hz, 2H), 3.66-3.35 (m, 2H).

### Example 82: Synthetic route of compound YL082

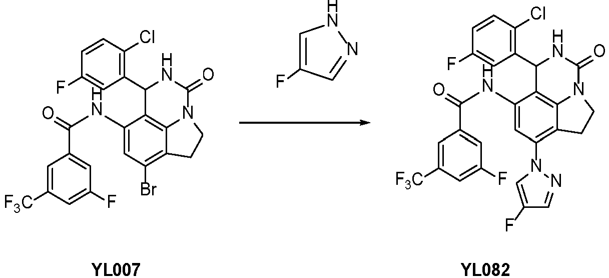

### Synthesis of compound YL082

To a three-necked flask (25 mL) were added 2-di-tert-butylphosphonium-3,4,5,6-tetramethyl-2',4',6'-triisopropylbiphenyl (8.19 mg, 0.017 mmol), tris(dibenzylideneacetone)dipalladium (7.80 mg, 0.009 mmol), 1,4-dioxane (2.5 mL) and toluene (0.5 mL) sequentially. The reaction mixture was stirred at 110 °C for 30 min under nitrogen protection, then it was cooled to room temperature and was added YL007 (50 mg, 0.085 mmol), 4-fluoro-1H-pyrazole (22.00 mg, 0.256 mmol), and potassium phosphate (54.26 mg, 0.256 mmol). The reaction continued at 110 °C for 3 h under nitrogen protection. The LCMS showed the reaction was successful and the reaction mixture was concentrated and the residue was purified by prep-HPLC (basic method) to give YL082 (22.8 mg, 0.039 mmol, 45.20%). LC-MS (ESI): m/z 592.2 (M+H)⁺; ¹H NMR (400 MHz, DMSO-d6) δ 10.26 (s, 1H), 8.54 (d, J = 4.4 Hz, 1H), 8.06-7.73 (m, 4H), 7.59 (d, J = 2.6 Hz, 1H), 7.34 (dd, J = 8.9, 5.2 Hz, 1H), 7.21-6.99 (m, 2H), 6.92 (dd, J = 9.2, 3.1 Hz, 1H), 6.12 (dd, J = 2.6, 1.0 Hz, 1H), 4.03 (dtd, J = 28.1, 10.4, 6.7 Hz, 2H), 3.61-3.38 (m, 2H).

### Example 83: Synthetic route of compound YL083

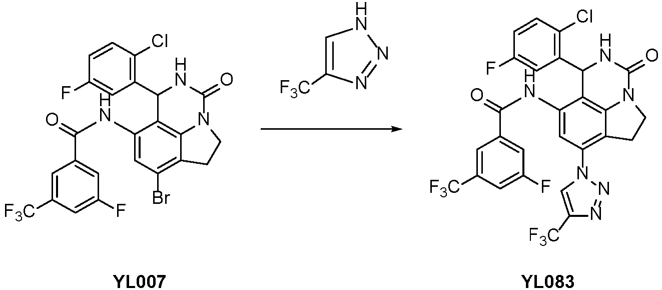

### Synthesis of compound YL083

To a three-necked flask (25 mL) were added 2-di-tert-butylphosphonium-3,4,5,6-tetramethyl-2',4',6'-triisopropylbiphenyl (8.19 mg, 0.017 mmol), tris(dibenzylideneacetone)dipalladium (7.80 mg, 0.009 mmol), 1,4-dioxane (2.5 mL), toluene (0.5 mL) sequentially. The reaction mixture was stirred at 110 °C for 30 min under nitrogen protection, then cooled to room temperature and was added YL007 (50 mg, 0.085 mmol), 5-(trifluoromethyl)-1H-1,2,3-triazole (35.04 mg, 0.256 mmol) and potassium phosphate (54.26 mg, 0.256 mmol). The reaction continued at 110 °C for 3 h under nitrogen protection. The LCMS showed the reaction was successful and the reaction mixture was concentrated and prepared in reverse phase (basic method) to give YL083 (5.8 mg, 0.009 mmol, 10.59%). LC-MS (ESI): m/z 643.2 (M+H)⁺.

### Example 84: Synthetic route of compound YL084

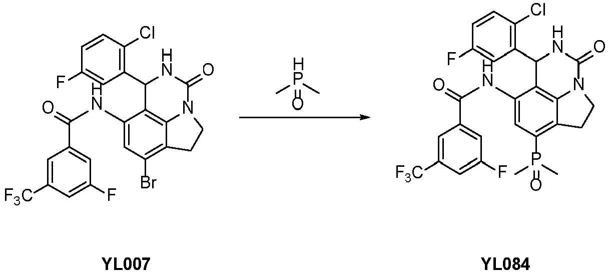

### Synthesis of compound YL084

To a three-necked flask (25 mL) were added YL007 (50 mg, 0.085 mmol), dimethylphosphine oxide (13.30 mg, 0.170 mmol), potassium phosphate (54.26 mg, 0.256 mmol), 4,5-bis(diphenylphosphino)-9,9-dimethylxanthene (9.86 mg, 0.017 mmol), tris(dibenzylidene acetone)dipalladium (7.80 mg, 0.009 mmol) and dioxane (3 mL) sequentially. The reaction mixture was stirred at 100 °C for 12 h under nitrogen protection. The LCMS showed the reaction was successful. The reaction was added ethyl acetate and water and the organic phase was concentrated and purified by column chromatography (eluent: dichloromethane: methanol = 40:1 -20:1) to give YL084 (20 mg, 0.034 mmol, 40.20%). LC-MS (ESI): m/z 584.2 (M+H)⁺; ¹HNMR (400 MHz, DMSO-d6) δ 10.25 (s, 1H), 7.95 (d, J = 8.4 Hz, 1H), 7.92-7.83 (m, 2H), 7.56 (d, J = 2.6 Hz, 1H), 7.34 (dd, J = 8.9, 5.2 Hz, 1H), 7.11(ddd, J = 8.8, 7.9, 3.1 Hz, 1H), 7.03 (d, J = 11.7 Hz, 1H), 6.89 (dd, J = 9.1, 3.1 Hz, 1H), 6.11 (d, J = 2.5 Hz, 1H), 4.14-3.84 (m, 2H), 3.43 (t, J = 8.6 Hz,2H), 1.68 (dd, J = 13.4, 2.8 Hz, 6H).

### Example 85: Synthetic route of compound YL085

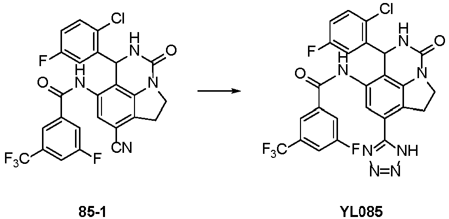

### Synthesis of compound YL085

To a three-necked flask (25 mL) were added 85-1 (50 mg, 0.094 mmol), sodium azide (61.00 mg, 0.938 mmol), ammonium chloride (50.19 mg, 0.938 mmol), and *N,N-*dimethylformamide (4 mL) sequentially. The reaction mixture was stirred at 70 °C for 18 h under nitrogen protection. The LCMS showed the reaction was successful. The reaction mixture was added ethyl acetate and water and the organic phase was concentrated by column chromatography (eluent: dichloromethane: methanol = 10:1-5:1) to give YL085 (9.3 mg, 0.016 mmol, 17.21%). LC-MS (ESI): m/z 574.0 (M+H)⁻; ¹HNMR (400 MHz, DMSO-d6) δ 10.23 (s, 1H), 8.07-7.68 (m, 3H), 7.41 (d, J=2.5 Hz, 2H), 7.33 (dd, J=8.9, 5.1 Hz, 1H), 7.09 (ddd, J=8.9, 7.9, 3.1 Hz,1H), 6.87 (dd, J = 9.3, 3.1 Hz, 1H), 6.11 (dd, J = 2.6, 1.0 Hz, 1H), 4.03 (dtd, J = 22.9, 10.3, 7.7 Hz, 2H), 3.60-3.48(m, 2H).

### Example 86: Synthetic route of compound YL086

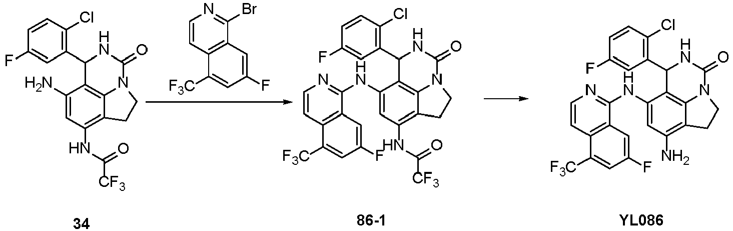

### Synthesis of compound 86-1

To a three-necked flask (25 mL) was sequentially added 1-bromo-7-fluoro-5-trifluoromethylisoquinoline (20.57 mg, 0.070 mmol), 34 (30 mg, 0.070 mmol), cesium carbonate (45.59 mg, 0.140 mmol), 4,5-bis(diphenylphosphino)-9,9-dimethylxanthene (8.10 mg, 0.014 mmol), tris(dibenzylideneacetone)dipalladium (6.41 mg, 0.007 mmol) and 1,4-dioxane (4 mL). The reaction mixture was stirred under nitrogen protection at 110 °C for 3 h. LCMS showed the reaction was successful. Water and ethyl acetate were added to the reaction mixture and the organic phase was concentrated and purified by column chromatography (eluent: dichloromethane: methanol = 20:1-10:1) to give 86-1 (17.1 mg, 0.027 mmol, 38.07%). LC-MS (ESI): m/z 642.1 (M+H)⁺; ¹H NMR (400 MHz, DMSO-d6) δ 11.23 (s, 1H), 9.00 (s, 1H), 8.18 (ddd, J = 28.6, 9.5, 2.5 Hz, 2H), 7.87 (d, J = 6.2 Hz, 1H), 7.41 (d, J = 2.5 Hz, 1H), 7.19-7.01 (m, 2H), 6.94 (td, J = 8.5, 3.1 Hz, 1H), 6.75 (s, 1H), 6.63 (dd, J = 9.2, 3.1 Hz, 1H), 5.92 (dd, J = 2.5, 1.2 Hz, 1H), 4.18-3.87 (m, 2H), 3.16 (tt, J = 9.6, 4.6 Hz, 2H).

### Synthesis of compound YL086

To a one-necked flask (25 mL) were added 86-1 (40 mg, 0.062 mmol), lithium hydroxide (7.84 mg, 0.187 mmol), methanol (6 mL) and water (2 mL) sequentially. The reaction mixture was stirred at 70 °C for 3 h. LCMS showed that the reaction was complete. Dichloromethane and water were added to the reaction mixture, and the organic phase was concentrated and purified by column chromatography (eluent: dichloromethane: methanol = 20:1 -15:1) to give YL086 (19.6 mg, 0.036 mmol, 57.62%). 20:1 -15:1) gave YL086 (19.6 mg, 0.036 mmol, 57.62%). LC-MS (ESI): m/z 546.0 (M+H)⁺; ¹H NMR (400 MHz, DMSO-d6) δ 8.74 (s, 1H), 8.20 (dd, J = 10.4, 2.5 Hz, 1H), 8.09 (dd, J = 8.7, 2.4 Hz, 1H), 7.83 (d, J = 6.2 Hz, 1H), 7.13 (d, J = 2.6 Hz, 1H),7.03 (dd, J = 8.8, 5.2 Hz, 2H), 6.90 (td, J = 8.4, 3.2 Hz, 1H), 6.60 (dd, J = 9.4, 3.1 Hz, 1H), 6.01 (s, 1H), 5.78-5.65 (m, 1H), 5.23 (s, 2H), 3.95 (pd, J = 11.0, 10.4,6.0 Hz, 2H), 2.97 (t, J = 8.5 Hz, 2H).

### Example 87: Synthetic route of compound YL087

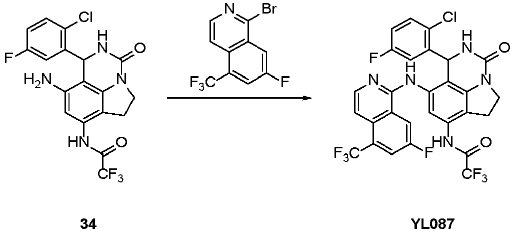

### Synthesis of compound YL087

To a three-necked flask (25 mL) were added 1-bromo-7-fluoro-5-trifluoromethylisoquinoline (20.57 mg, 0.070 mmol), compound 34 (30 mg, 0.070 mmol), cesium carbonate (45.59 mg, 0.140 mmol), 4,5-bis(diphenylphosphino)-9,9-dimethylxanthene (8.10 mg, 0.014 mmol), tris(dibenzylideneacetone)dipalladium (6.41 mg, 0.007 mmol) and 1,4-dioxane (4 mL) sequentially. The reaction mixture was stirred under nitrogen protection at 110 °C for 3 h. The LCMS showed the reaction was successful. Water and ethyl acetate were added to the reaction mixture and the organic phases were concentrated and purified by column chromatography (eluent: dichloromethane: methanol = 20:1-10:1) to give YL087 (17.1 mg, 0.027 mmol, 38.07%). LC-MS (ESI): m/z 642.1 (M+H)⁺; ¹H NMR (400 MHz, DMSO-d6) δ 11.23 (s, 1H), 9.00 (s, 1H), 8.18 (ddd, J = 28.6, 9.5, 2.5 Hz, 2H), 7.87 (d, J = 6.2 Hz, 1H), 7.41 (d, J = 2.5 Hz, 1H), 7.19-7.01 (m, 2H), 6.94 (td, J = 8.5, 3.1 Hz, 1H), 6.75 (s, 1H), 6.63 (dd, J = 9.2, 3.1 Hz, 1H), 5.92 (dd, J = 2.5, 1.2 Hz, 1H), 4.18-3.87 (m, 2H), 3.16 (tt, J = 9.6, 4.6 Hz, 2H).

### Example 88: Synthetic route of compound YL088

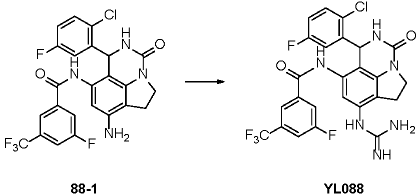

### Synthesis of compound YL088

To a one-necked flask (25 mL) were added 88-1 (50 mg, 0.096 mmol), cyanamide (8.04 mg, 0.191 mmol), 1,4-dioxane (2 mL) and hydrochloric acid (1,4-dioxane) (0.5 mL) in order. The above mixture was stirred at 80 C for 3 h. LCMS showed the reaction was successful. Saturated sodium carbonate solution and ethyl acetate were added to the reaction flask. The organic phases were concentrated and purified by column chromatography (eluent: dichloromethane: methanol = 20:1-10:1) to give YL088 (22.9 mg, 0.041 mmol, 42.39%). LC-MS (ESI): m/z 565.2 (M+H)⁺; ¹H NMR (400 MHz, DMSO-d6) δ 9.99 (s, 1H), 7.91 (d, J = 8.1 Hz, 1H), 7.83 (d, J = 11.7 Hz, 2H), 7.39-7.19 (m, 2H), 7.07 (td, J = 8.4, 3.1 Hz, 1H), 6.86 (dd, J= 9.3, 3.1 Hz, 1H), 6.19 (s, 1H), 6.03 (t, J = 1.9 Hz, 1H), 5.36 (s, 4H), 3.92 (pd, J = 10.5, 7.6 Hz, 2H), 2.96 (td, J = 7.9, 6.7, 2.4 Hz, 2H).

### Example 89: Synthetic route of compound YL089

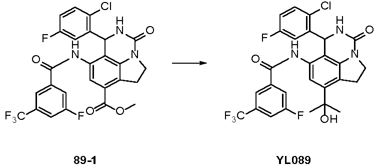

### Synthesis of compound YL089

To a three-necked flask (25 mL) were added 89-1 (80 mg, 0.141 mmol) and tetrahydrofuran (5 mL). The above mixture was added methylmagnesium bromide (0.423 mL, 1 mol/L) under nitrogen protection at -78°C, then stirred at -78 °C for 30 min. LCMS showed the reaction was successful. Saturated ammonium chloride and ethyl acetate were added to the reaction mixture, and the organic phases were concentrated and the residue was purified by column chromatography (eluent: dichloromethane: methanol = 20:1-10:1) to give YL089 (9.8 mg, 0.017 mmol, 12.25%). LC-MS (ESI): m/z 566.2 (M+H)⁺; ¹H NMR (400 MHz, DMSO-d6) δ 10.13 (s, 1H), 8.11-7.72 (m, 3H), 7.44 (d, J = 2.7 Hz, 1H), 7.32 (dd, J = 8.9, 5.2 Hz, 1H), 7.23 -7.02 (m, 1H), 6.90-6.66(m, 2H), 6.05 (dd, J = 2.6, 1.2 Hz, 1H), 5.06 (s, 1H), 4.11-3.80 (m, 2H), 3.39 (t, J = 8.7 Hz, 2H), 1.44 (d, J = 9.4 Hz, 6H).

### Example 90: Synthetic route of compound YL090

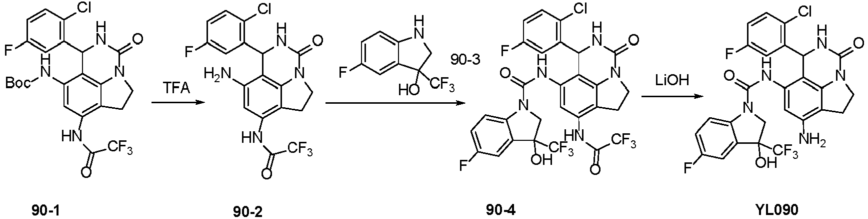

### Synthesis of compound 90-2

Compound 90-1 (280 mg, 0.53 mmol), dichloromethane (8 mL) and trifluoroacetic acid (2 mL) were added to a reaction flask. The mixture was stirred under nitrogen protection at room temperature for 1 hour. The reaction mixture was concentrated at reduced pressure and the residue was diluted by adding dichloromethane and saturated aqueous sodium bicarbonate solution. The organic phase was separated and the aqueous phase was extracted with dichloromethane. The organic phases were combined, washed with brine, concentrated at reduced pressure, and the resulting crude product was purified by automatied column (Biotage) (mobile phase: petroleum ether/ethyl acetate 55/45 to 45/55) to give the light yellow solid target compound 90-2 (180 mg). LC-MS (ESI): m/z 429.1 (M+H) ⁺.

### Synthesis of compound 90-4

Compound 90-2 (45 mg, 0.11 mmol), dichloromethane (3 mL), dioxane (0.4 mL) and *N*,*N*-diisopropylethylamine (492 mg, 4.1 mmol) were added to a reaction vial. A solution of triphosgene (12mg) in dichloromethane (1 mL) was added dropwise to the above mixture at 0°C. After addition, the reaction mixture continued stirring At 0°C for 30 min, then was added compound 90-3 (31mg) dropwise. After addition, the resulting mixture continued stirring in an ice-cold water bath for 40 min. Dichloromethane and saturated aqueous sodium bicarbonate solution were added to dilute the reaction mixture. The organic phase was separated and the aqueous phase was extracted with dichloromethane. The organic phases were combined, washed with brine, concentrated at reduced pressure, and the resulting crude product was purified by automated chromatography (Biotage) (mobile phase: methanol/dichloromethane 32/68 to 40/60) to give a light yellow solid target compound 90-4 (72 mg). LC-MS (ESI): m/z 674.1 (M-H)⁻.

### Synthesis of compound YL90

To a reaction vial were added 90-4 (52 mg, 0.08 mmol), lithium hydroxide (13 mg, 0.54 mmol), methanol (3 mL) and water (1 mL). The mixture was stirred under nitrogen protection at 60°C for 4 hours. The mixture was diluted by adding dichloromethane and saturated brine. The organic phase was separated and the aqueous phase was extracted with dichloromethane. The organic phases were combined, washed with brine, concentrated at reduced pressure, and the resulting crude product was purified by automated chromatography (Biotage) (mobile phase: methanol/dichloromethane 6/94 to 9/91) to obtain the crude product. The product was purified by automated chromatography (Biotage) (mobile phase: acetonitrile/10 mM aqueous ammonium bicarbonate 30/70 to 37/63) to give a light yellow solid as target compound YL090 (18 mg). YL090: LC-MS (ESI): m/z 440.1 (M+H) ⁺; ¹H NMR (400 MHz, DMSO-d6) δ 8.18 (d, J = 12.4 Hz, 1H), 7.89 (dd, J = 9.0, 4.7 Hz, 0.47H), 7.83 - 7.75 (m, 0.50H), 7.41 (d, J = 6.9 Hz, 1H), 7.32 - 7.18 (m, 4H), 7.08 (dtd, J = 11.7, 8.4, 3.1 Hz, 1H), 6.80 (dt, J = 9.3, 3.5 Hz, 1H), 6.03 - 5.93 (m, 2H), 5.23 (d, J = 3.3 Hz, 2H), 4.31 (d, J = 12.2 Hz, 0.64H), 3.99 - 3.89 (m, 2H), 3.83 (t, J = 11.9 Hz, 1.31H), 2.95 (t, J = 8.5 Hz, 2H).

### Example 91: Synthetic route of compound YL091

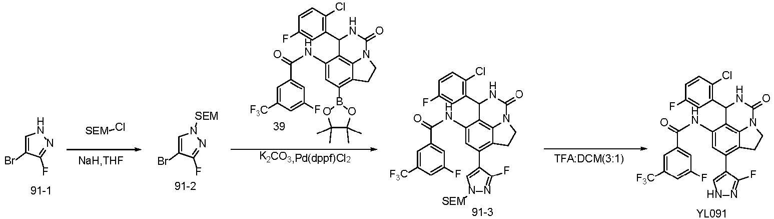

### synthesis of compound 91-2

Compound 91-1 (100 mg, 0.606 mmol) and THF (1.5 mL) were placed in a reaction flask (8 mL). After degassed and purged with nitrogen, the flask was placed in an ice-cold water bath, the mixture was added NaH (21.82 mg, 0.909 mmol), then restored to room temperature and stirred for 30 min, was added 2-(trimethylsilyl)ethoxymethyl chloride (151.59 mg, 0.909 mmol) , stirred for 1 h at room temperature. Two drops of saturated ammonium chloride were added to quench the reaciton, the reaction mixture was extracted with ethyl acetate and purified by column chromatography (petroleum ether: ethyl acetate = 3:1) to give a colorless liquid as compound 91-2 (105 mg, 0.356 mmol, 58.67%). LC-MS (ESI): m/z 295.02(M+H)⁺.

### Synthesis of compound 91-3

Compound 91-2 (42.4 mg, 0.144 mmol), compound 39 (100.13 mg, 0.158 mmol), potassium carbonate (39.70 mg, 0.287 mmol) and Pd(dppf)Cl₂ (10.51 mg, 0.014 mmol) were placed in a reaction vial (8 mL), then were added 1,4-dioxane (1 mL) and water (0.20 mL). After degassed and purged with nitrogen, the reaction mixture was stirred at 100 °C for one and half an hour, then mixed with silica and purified by column chromatography (ethyl acetate/petroleum ether = 1:3) to obtain the yellow solid compound 91-3 (60 mg, 0.079 mmol, 54.96%), LC-MS (ESI): m/z 722.17(M+H) ⁺.

### Synthesis of compound YL091

Compound 91-3 (50 mg, 0.069 mmol) was added to a reaction vial followed by DCM (6.00 mL) and TFA (2 mL) and the reaction mixture was stirred for one hour at room temperature, monitored. The reaction mixture was purified by prep-HPLC to give YL091 (4.5 mg, 0.008 mmol, 10.98%). LC-MS (ESI): m/z 592.09(M+H)⁺; ¹HNMR (400 MHz, DMSO-*d*₆) δ 12.73 (s, 1H),10.21 (s, 1H), 8.05 (d, *J* = 2.1 Hz, 1H), 7.95 (d, *J* = 8.4 Hz, 1H), 7.87 (d, *J =* 10.3 Hz, 2H), 7.51 (d, *J* = 2.6 Hz, 1H), 7.33 (dd, *J* = 8.7, 5.1 Hz, 1H), 7.10 (td, *J* = 8.6, 3.1 Hz, 1H), 6.88 (d, *J* = 9.1 Hz, 2H), 6.09 (s, 1H), 4.12 - 3.96 (m, 2H), 3.28 (s, 2H).

### Example 92: Synthetic route of compound YL092-P1 and YL092-P2

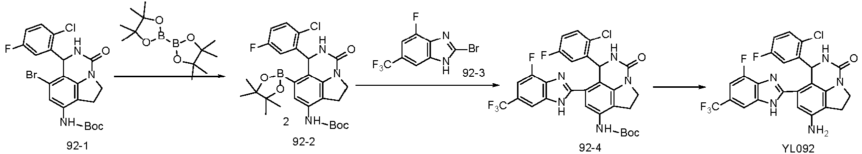

### Synthesis of compound 92-2

Compound 92-1 (100 mg, 0.201 mmol), biboronic acid pinacol ester (178.92 mg, 0.705 mmol), potassium acetate (78.91 mg, 0.805 mmol) and Pd(dppf)Cl₂ (29.46 mg, 0.040 mmol) were added to a reaction flask followed by dioxane (2 mL). After degassed and purged with nitrogen, the reaction mixture was stirred at 100°C for 2 hour, monitored and the solvent was removed and purified on a silica column (ethyl acetate:petroleum ether 1:2) to give a yellow oily solid YL92-2 (100 mg, 0.184 mmol, 91.34%). LC-MS (ESI): m/z 544.21(M+H) ⁺.

### Synthesis of compound 92-4

Compound 92-2 (50 mg, 0.092 mmol), compound 92-3 (26.02 mg, 0.092 mmol), potassium carbonate (31.77 mg, 0.230 mmol) and Pd(dppf)Cl₂ (6.73 mg, 0.009 mmol) were added to a reaction flask. After added dioxane, the mixture was degassed and purged with nitrogen, then stirred at 100 °C overnight. Monitored, the reaction was removed solvent and purified on a silica column (methanol: dichloromethane = 1:20) to give the compound 92-4 (17 mg, 0.027 mmol, 29.82%). LC-MS (ESI): m/z 620.14(M+H)⁺.

### Synthesis of compound YL092

Compound 92-4 (15 mg, 0.024 mmol) was dissolved in DCM (1 mL), then was added trifluoroacetic acid (0.2 mL, 2.612 mmol) and the reaction mixture was stirred at room temperature for 2 h, monitored. The reaction mixture was neutralized with sodium bicarbonate, extracted with ethyl acetate and purified on a silica column to give compound YL092 (3.7 mg, 0.007 mmol, 27.94%). LC-MS (ESI): m/z 520.09(M+H) ⁺.

### Synthesis of compounds YL092-P1 and YL092-P2

YL092-4 (200 mg, 0.323 mmol) was dissolved in DCM (6 mL), then TFA (2 mL, 26.118 mmol) was added and the reaction was monitored at room temperature for 2 h. The reaction was alkalized with sodium bicarbonate, extracted with ethyl acetate and purified by column chromatography without separation, then purified by reversed column to give YL092 (77 mg, 0.141 mmol, 43.62%) LC-MS (ESI): m/z 520.09(M+H)⁺, separated by SFC chiral resolution conditions: instrument: SFC-150 (Waters); column: OD 20*250 mm, 10um (Daicel); Column temperature: 35 °C; mobile phase: CO₂/MeOH (0.2% NH₃ (7M in MeOH) = 65/35; flow rate: 100 mL/min; back pressure: 100 bar; wavelength: 214 nm, and compounds YL092-P1 (retention time 3.245 min) and YL092-P2 (retention time 3.456 min) were obtained.

YL092-P2: ¹H NMR (400 MHz, DMSO-*d*₆) δ 13.35 (s, 1H), 7.58 (s, 1H), 7.50 - 7.17 (m, 3H), 6.98 (td, *J* = 8.4, 3.1 Hz, 1H), 6.79 (d, *J* = 36.1i Hz, 2H), 6.53 (dd, *J* = 9.5, 3.1 Hz, 1H), 5.53 (s, 2H), 4.09 - 3.87 (m, 2H), 3.16 - 2.97 (m, 2H).

### Example 93: Synthetic route of compound YL093

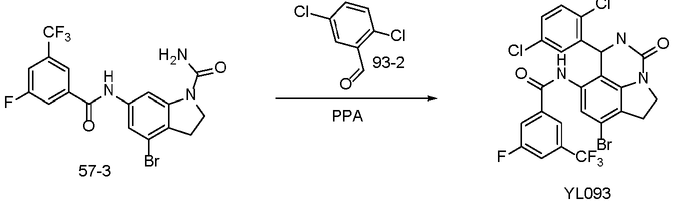

### Synthesis of compound YL093

Polyphosphoric acid (20 mL) in a two-necked flask was warmed to 105 °C and stirred steadily, then was added compound 57-3 (100 mg, 0.224 mmol) followed by compound 93-2 (78.40 mg, 0.448 mmol) and the mixture was stirred for 30 min, then was adjusted to basic with saturated aqueous sodium bicarbonate solution, extracted with dichloromethane and purified by column chromatography (dichloromethane: methanol = 20: 1) to give YL093 (70 mg, 0.116 mmol, 51.78%). LC-MS (ESI): m/z 601.96(M+H)⁺.

¹H NMR (400 MHz, DMSO-*d*₆) δ 10.17 (s, 1H), 7.96 (dd, *J* = 8.5, 2.2 Hz, 1H), 7.88 - 7.80 (m, 2H), 7.49 (d, *J* = 2.4 Hz, 1H), 7.33 (d, *J* = 8.6 Hz, 1H), 7.28 (d, *J* = 2.6 Hz, 1H), 7.06 (d, *J =* 2.5 Hz, 1H), 6.92 (s, 1H), 6.03 (d, *J =* 2.3 Hz, 1H), 4.16 - 3.88 (m, 2H), 3.26 - 3.04 (m,2H).

### Example 94: Synthetic route of compound YL094

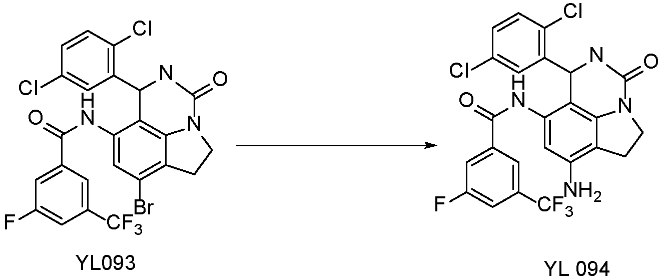

### Synthesis of compound YL094

Compound YL093 (77 mg, 0.128 mmol), L-proline (5.87 mg, 0.051 mmol), CuI (4.86 mg, 0.026 mmol), and potassium carbonate (52.93 mg, 0.383 mmol) were added to a reaction vessel, followed by 1 mL of DSMO. After degassed and purged with nitrogen, the mixture was added ammonia (25%) (11.19 mg, 0.319 mmol), and stirred at 100 °C for 18 h. The aqueous phase was extracted with dichloromethane and the solvent was removed and the residue was purified by prep-HPLC to give compound YL094 (25 mg, 0.046 mmol, 36.31%). LC-MS (ESI): m/z 539.06(M+H)⁺;

¹H NMR (500 MHz, DMSO-*d*₆) δ 9.93 (s, 1H), 7.92 (d, *J* = 8.4 Hz, 1H), 7.88 - 7.78 (m, 2H), 7.28 (d, *J* = 8.5 Hz, 1H), 7.23 (dd, *J* = 16.7, 2.7 Hz, 2H), 6.96 (d, *J* = 2.5 Hz, 1H), 6.00 (s, 1H), 5.89 (d, *J* = 2.5 Hz, 1H), 5.31 (s, 2H), 4.03 - 3.85 (m, 2H), 2.95 (td, *J* = 8.2, 2.6 Hz, 2H).

### Example 95: Synthetic route of compound YL095

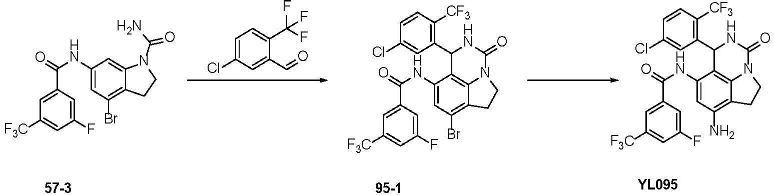

### Synthesis of compound 95-1

Compound 57-3 (0.4 g, 0.896 mmol) in polyphosphoric acid (50 g) was added 5-chloro-2-trifluoromethylbenzaldehyde (0.37 g, 1.793 mmol) and the mixture was stirred at 105 °C for 2 hours. The reaction mixture was dropped into ice-cold saturated sodium hydroxide solution and diluted with dichloromethane and water. The organic phases were separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 57-4 (260 mg, 45.5%). ESI:(m/z) = 636.1 [M+H]⁺.

### Synthesis of compound YL095

Compound 95-1 (260 mg, 0.408 mmol), potassium carbonate (170 mg, 1.225 mmol), L-proline (19 mg, 0.163 mmol), and cuprous iodide (15.5 mg, 0.082 mmol) were placed in a reaction vial and were added dimethyl sulfoxide (3 mL), then was added with ammonia (0.52 g 4.083 mmol, 25%) via a syringe under nitrogen atmosphere, and stirred at 100 °C overnight. Diluted with dichloromethane and water, the organic phase was removed, and the residue was purified by column chromatography (mobile phase: dichloromethane/methanol 1/0 to 20/1) to give compound YL095 (96 mg, 41%). LCMS (ESI): m/z 573.0 (M+H) ⁺;¹H NMR (400 MHz, DMSO-d6) δ 9.90 (s, 1H), 7.90 (d, J = 8.0 Hz, 1H), 7.84 - 7.74 (m, 2H), 7.56 (d, J = 8.6 Hz, 1H), 7.52 - 7.45 (m, 1H), 7.09 (d, J = 2.1 Hz, 1H), 6.99 (d, J = 2.7 Hz, 1H), 6.05 (s, 1H), 5.85 (d, J = 2.7 Hz, 1H), 5.40 (s, 2H), 4.05 - 3.88 (m, 2H), 3.00 (td, J = 7.8, 6.8, 2.9 Hz, 2H).

### Example 96: Synthetic route of compound YL096

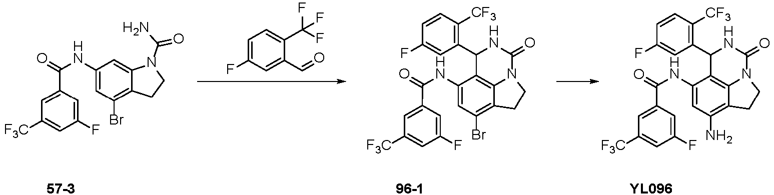

### Synthesis of compound 96-1

Compound 57-3 (0.4 g, 0.896 mmol) was dissolved in polyphosphoric acid (50 g), then was added 5-fluoro-2-(trifluoromethyl)benzaldehyde (0.34 g, 1.792 mmol) and stirred at 105 °C for 2 h. The reaction mixture was dropped into ice-cold saturated sodium hydroxide solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 96-1 (450 mg, 80.9%). ESI:(m/z) = 620.1 [M+H]⁺.

### Synthesis of compound YL096

Compound 96-1 (260 mg, 0.725 mmol), potassium carbonate (300 mg, 2.176 mmol), L-proline (33 mg, 0.29 mmol), and cuprous iodide (27.6 mg, 0.145 mmol) were placed in a reaction vial and were added dimethyl sulfoxide (5 mL), then was added with ammonia (0.9 g 7.255 mmol, 25%) via a syringe under nitrogen atmosphere, and stirred at 100 °C overnight, Diluted with dichloromethane and water, removed the organic phase, and purified by column chromatography (mobile phase: dichloromethane/methanol 1/0 to 20/1) to obtain compound YL096 (176 mg, 43.6%). LCMS (ESI): m/z 557.1 (M+H) ⁺;¹H NMR (400 MHz, DMSO-d6) δ 9.90 (s, 1H), 7.90 (d, J = 8.3 Hz, 1H), 7.84 - 7.75 (m, 2H), 7.62 (dd, J = 8.9, 5.4 Hz, 1H), 7.27 (td, J = 8.3, 2.6 Hz, 1H), 6.96 (d, J = 2.8 Hz, 1H), 6.87 (dd, J = 9.9, 2.6 Hz, 1H), 6.06 (s, 1H), 5.86 (s, 1H), 5.39 (s, 2H), 3.96 (dtd, J = 28.6, 10.3, 7.2 Hz, 2H), 3.00 (td, J = 7.7, 6.7, 3.3 Hz, 2H).

### Example 97: Synthetic route of compound YL097

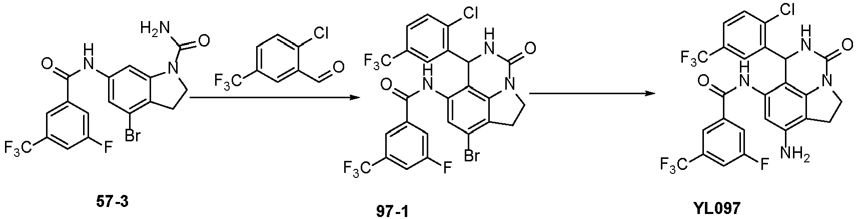

### Synthesis of compound 97-1

A solution of compound 57-3 (0.4 g, 0.896 mmol) in polyphosphoric acid (50 g) was added 2-chloro-5-trifluoromethylbenzaldehyde (0.37 g, 1.792 mmol) and the reaction mixture was stirred at 105 °C for 2 hours. The reaction mixture was dropped into ice-cold saturated sodium hydroxide solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 97-1 (420 mg, 73.6%). ESI:(m/z) = 636.1 [M+H]⁺.

### Synthesis of compound YL097

Compound 97-1 (420 mg, 0.66 mmol), potassium carbonate (273 mg, 1.98 mmol), L-proline (30 mg, 0.264 mmol), and cuprous iodide (25 mg, 0.132 mmol) were placed in a reaction vial and were added dimethyl sulfoxide (5 mL), followed by ammonia (0.8 g, 6.596 mmol, 25%) via a syringe under nitrogen atmosphere, raised to 100 °C, stirred overnight. The aqueous phase was extracted with dichloromethane, removed the organic phase, and the residue was purified by column chromatography (mobile phase: dichloromethane/methanol 1/0 to 20/1) to give compound YL097 (130 mg, 34.4%). LCMS (ESI): m/z 573.1 (M+H) ⁺; ¹H NMR (400 MHz, DMSO-d6) δ 9.89 (s, 1H), 7.91 (d, J = 8.5 Hz, 1H), 7.83 - 7.76 (m, 2H), 7.51 (d, J = 1.3 Hz, 2H), 7.24 (s, 1H), 7.14 (d, J = 2.4 Hz, 1H), 5.98 (d, J = 1.9 Hz, 2H), 5.29 (s, 2H), 3.95 (t, J = 8.4 Hz, 2H), 2.94 (dd, J = 9.7, 7.3 Hz, 2H).

### Example 98: Synthetic route of compound YL098

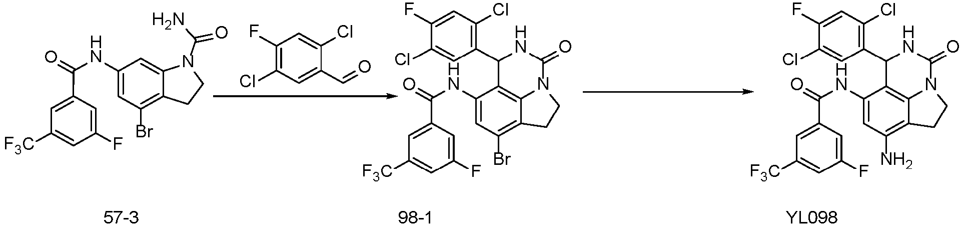

### Synthesis of compound 98-1

Compound 57-3 (0.4 g, 0.896 mmol) in polyphosphoric acid (50 g) was added 2-chloro-5-trifluoromethylbenzaldehyde (0.35 g, 1.792 mmol), and the reaction mixture was stirred at 105 °C for 1 hour. The reaction mixture was dropped into ice-cold saturated sodium hydroxide solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 98-1 (420 mg, 75.5%). ESI:(m/z) = 620.1 [M+H]⁺.

### Synthesis of compound YL098

Compound 98-1 (420 mg, 0.67 mmol), potassium carbonate (280 mg, 2.03 mmol), L-proline (31 mg, 0.27 mmol), and cuprous iodide (25 mg, 0.135 mmol) were placed in a reaction vial and added dimethyl sulfoxide (5 mL). The above mixture was added ammonia (0.85 g, 6.76 mmol, 25%) via a syringe under nitrogen atmosphere, then stirred at 100 °C overnight. The aqueous phase was extracted with dichloromethane and the organic phase was removed. The residue was purified by column chromatography (mobile phase: dichloromethane/methanol 1/0 to 20/1) to give compound YL098 (85 mg, 22.6%). LCMS (ESI): m/z 557.1 (M+H)⁺; ¹H NMR (400 MHz, DMSO-d6) δ 9.89 (s, 1H), 7.92 (d, J = 8.3 Hz, 1H), 7.86 - 7.79 (m, 2H), 7.49 (d, J = 9.1 Hz, 1H), 7.16 (d, J = 2.4 Hz, 1H), 7.07 (d, J = 7.9 Hz, 1H), 5.99 (s, 1H), 5.87 (d, J = 2.4 Hz, 1H), 5.30 (s, 2H), 4.00 - 3.88 (m, 2H), 2.96 (d, J = 9.5 Hz, 2H).

### Example 99: Synthetic route of compound YL099

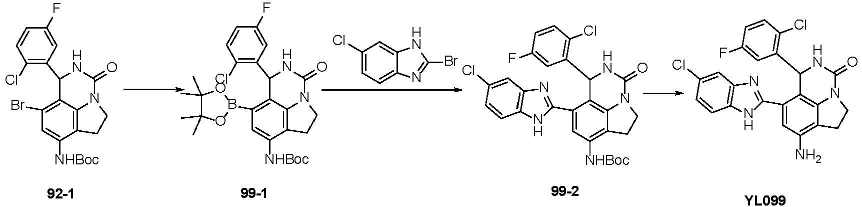

### Synthesis of compound 99-1

92-1 (220 mg, 0.443 mmol) in dioxane (5 mL) was added potassium acetate (175 mg, 1.771 mmol), biboronic acid pinacol ester (395 mg, 1.55 mmol) and Pd(dppf)Cl₂ (65 mg, 0.089 mmol), then stirred at 100 °C for 2 h, and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 99-1 (90 mg, 37.5 %). ESI:(m/z) = 544.3 [M+H]⁺.

### Synthesis of compound 99-2

99-1 (90 mg, 0.166 mmol) dissolved in dioxane (1 mL) and water (0.2 mL) was added potassium carbonate (92 mg, 0.663 mmol), 2-bromo-6-chloro-1H-benzimidazole (42 mg, 0.182 mmol) and Pd(dppf)Cl₂ (24 mg, 0.033 mmol), then stirred at 100 °C overnight. The mixture was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 99-2 (40 mg, 42.5 %). ESI:(m/z) = 568.1 [M+H]⁺.

### Synthesis of compound YL099

99-2 (40 mg, 0.07 mmol) in dichloromethane (3 mL) was added trifluoroacetic acid (1 mL) and stirred at room temperature for 1 h. The reaction mixture was adjusted to basic with saturated sodium bicarbonate solution. The aqueous phase was extracted with dichloromethane. The organic phases were combined, washed with water, brine, dried over anhydrous Na₂SO₄, filtered and the filtrate was evaporated to obtain compound YL099 (9 mg, 27 %). ESI:(m/z) = 468.1 [M+H]⁺ ; ¹H NMR (400 MHz, DMSO-d6) δ 12.69 (s, 1H), 7.53 - 7.27 (m, 4H), 7.15 (dd, J = 8.6, 2.0 Hz, 1H), 6.98 (td, J = 8.4, 3.1 Hz, 1H), 6.86 (dd, J = 3.6, 1.4 Hz, 1H), 6.71 (s, 1H), 6.51 (dd, J = 9.5, 3.1 Hz, 1H), 5.46 (s, 2H), 4.00 - 3.89 (m, 2H), 3.10 - 2.99 (m, 2H).

### Example 100: Synthetic route of compound YL100

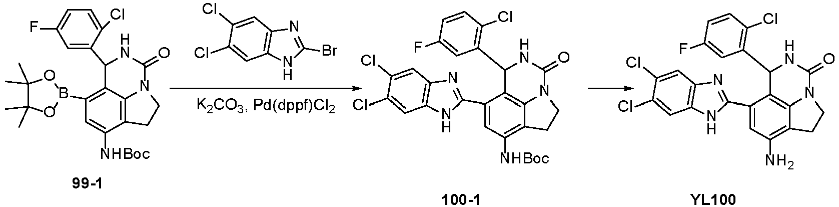

### Synthesis of compound 100-1

99-1 (50 mg, 0.092 mmol) in 1,4-dioxane (1 mL) and water (0.2 mL) was added potassium carbonate (51 mg, 0.368 mmol), 2-bromo-5,6-chloro-1H-benzimidazole (27 mg, 0.101 mmol) and Pd(dppf)Cl₂ (13.5 mg, 0.018 mmol), the mixture was stirred at 100 °C for 3 h, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 100-1 (30 mg, 54 %). ESI:(m/z) = 602.1 [M+H]⁺.

### Synthesis of compound YL100

100-1 (30 mg, 0.05 mmol) in dichloromethane (3 mL) was added trifluoroacetic acid (1 mL), then the mixture was stirred at room temperature for 1 h. The reaction mixture was adjusted to basic with saturated sodium bicarbonate solution, and the aqueous phase was extracted with dichloromethane. The organic phases were combined, washed with water, brine, dried over anhydrous Na₂SO₄, filtered and the filtrate was evaporated to obtain compound YL100 (12 mg, 48 %). ESI:(m/z) = 502.1 [M+H]⁺; ¹H NMR (400 MHz, DMSO-d6) δ 12.89 (s, 1H), 7.71 (s, 1H), 7.60 (s, 1H), 7.40 - 7.34 (m, 2H), 6.99 (ddd, J = 8.9, 8.0, 3.1 Hz, 1H), 6.81 (dd, J = 3.6, 1.4 Hz, 1H), 6.69 (s, 1H), 6.53 (dd, J = 9.5, 3.2 Hz, 1H), 5.49 (s, 2H), 3.96 (td, J = 9.1, 4.1 Hz, 2H), 3.05 (t, J = 8.6 Hz, 2H).

### Example 101: Synthetic route of compound YL101-P1 and YL101-P2

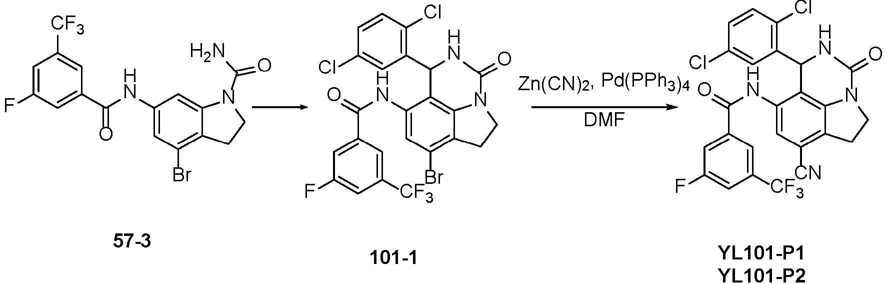

### Synthesis of compound 101-1

57-3 (4 g, 8.965 mmol) was dissolved in polyphosphoric acid (50 g), the mixture was added 2,5-dichlorobenzaldehyde (6.06 g, 22.413 mmol), and the reaction mixture was stirred at 105 °C for 2 hours. The reaction mixture was dropped into ice-cold saturated sodium hydroxide solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) to give compound 101-1 (2.5 g, 46.21 %). ESI:(m/z) = 603.9 [M+H]⁺.

### Synthesis of compounds YL101-P1 and YL101-P2

101-1 (2.5 g, 4.268 mmol) in DMF (15 mL) was added zinc cyanide (1 g, 8.536 mmol) and Pd(PPh₃)₄ (0.74 g, 0.64 mmol) and the resulting mixture was stirred at 100 °C overnight, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated and the residue was purified by column chromatography (mobile phase: dichloromethane/methanol 1/0 to 20/1) and SFC chiral resolution to give compounds YL101-P1 (0.7 g), YL101-P2 (0.9 g). ESI:(m/z) = 549.1 [M+H]⁺ ;
YL101-P1: ¹H NMR (400 MHz, DMSO-d6) δ 10.24 (s, 1H), 7.96 (dt, J = 8.5, 2.0 Hz, 1H), 7.89 - 7.76 (m, 2H), 7.56 (d, J = 2.3 Hz, 1H), 7.34 (d, J = 8.6 Hz, 1H), 7.28 (dd, J = 8.6, 2.5 Hz, 1H), 7.14 (s, 1H), 7.09 (d, J = 2.5 Hz, 1H), 6.12 (d, J = 2.3 Hz, 1H), 4.07 (td, J = 8.4, 5.8 Hz, 2H), 3.42 - 3.35 (m, 2H).
YL101-P2: ¹H NMR (400 MHz, DMSO-d6) δ 10.25 (s, 1H), 7.96 (dt, J = 8.5, 1.9 Hz, 1H), 7.88 - 7.79 (m, 2H), 7.56 (d, J = 2.3 Hz, 1H), 7.34 (d, J = 8.6 Hz, 1H), 7.28 (dd, J = 8.6, 2.5 Hz, 1H), 7.14 (s, 1H), 7.09 (d, J = 2.5 Hz, 1H), 6.12 (d, J = 2.2 Hz, 1H), 4.13 - 4.00 (m, 2H), 3.45 - 3.35 (m, 2H).

### Example 102: Synthetic route of compound YL102 (102-P1 & 102-P2)

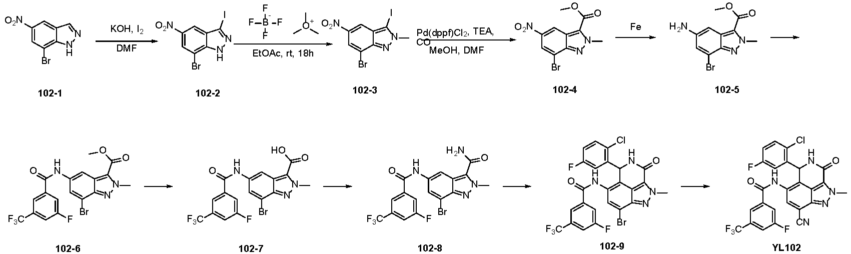

### Synthesis of compound 102-2

A solution of 7-Bromo-5-nitro-1H-indazole (Compound 102-1, 22 g, 90.898 mmol) dissolved in DMF (250 mL) was added potassium hydroxide (20.4 g, 363.591 mmol), then after degassed and purged with N₂, and was added iodine (46.14 g, 181.8 mmol), and the reaction mixture was stirred at 70 °C for 4 hours. The reaction mixture was washed twice with 5% aqueous sodium thiosulfate, then the aqueous phase was adjusted to weak acidic with aqueous citric acid, and extracted with ethyl acetate. The organic phases were combined, washed with water, brine, dried over anhydrous Na₂SO₄, filtered, and the filtrate was evaporated, the residue was pulped 3 times with dichloromethane/methanol=10/1, the solids were collected, and the filtrate was then purified by column chromatography (mobile phases: dichloromethane/methanol 1/0 to 20/1) to obtain compound 102-2 (27 g, 80.74%).

### Synthesis of compound 102-3

A solution of 102-2 (15 g, 40.769 mmol) in ethyl acetate (650 mL) was added trimethoxonium tetrafluoroborate (9.05 g, 61.153 mmol). After degassed and purged with N₂ and the reaction mixture was stirred at room temperature overnight. The reaction mixture precipitated as a solid, filtered, and the filtrate was evaporated to dryness and the resulting residue was pulped with dichloromethane/methanol to obtain crude compound 102-3 (11.7 g, 75.13%). ESI:(m/z) = 382.0 [M+H]⁺ ;

### Synthesis of compound 102-4

A mixture of 102-3 (11.7 g, 30.631 mmol) in DMF (100 mL) and methanol (100 mL) was added triethylamine (13 mL, 91.894 mmol) and Pd(dppf)Cl₂ (2.24 g, 3.063 mmol), then was degassed and purged with CO gas, and the reaction mixture was stirred at 70 °C for 8 hours. The methanol was removed, the residue was diluted with water and extracted with ethyl acetate. The organic phases were combined, washed with water, brine, dried over anhydrous Na₂SO₄, filtered, and the filtrate was evaporated, and the residue was purified by column chromatography (mobile phase: petroleum ether/dichloromethane ~40%/60%) to give compound 102-4 (1.6 g, 16.63%). ESI:(m/z) = 314.0 [M+H]⁺;

### Synthesis of compound 102-5

A mixture of 102-4 (1.6 g, 5.094 mmol) in ethanol (20 mL) and water (4 mL), was added solid ammonium chloride (0.82 g, 15.282 mmol) and iron powder (1.42 g, 25.47 mmol), and the reaction mixture was stirred at 70 °C for 4 hours. The reaction mixture was filtered with celite, washed with dichloromethane and the organic phase was concentrated to dryness to give compound 102-5 (1.4 g, 96.5%). ESI:(m/z) = 284.0 [M+H]⁺;

### Synthesis of compound 102-6

A solution of 102-5 (1.4 g, 4.928 mmol) in dichloromethane (30 mL) was added triethylamine (2.055 mL, 14.783 mmol). After degassed and purged with N₂, cooled down to 0°C, the above mixture was added 3-fluoro-5-(trifluoromethyl)benzoyl chloride (1.67 g, 7.392 mmol), and the reaction mixture was stirred for 1 h at room temperature. The reaction mixture was precipitated as a solid, filtered, the filtrate was concentrated to dryness and pulped with methylene chloride, and the resulting filtrate was purified by column chromatography (mobile phases: petroleum ether/ethyl acetate 1/0 to 3/1) to give compound 102-6 (2.12 g, 90.6%). ESI:(m/z) = 475.9 [M+H]⁺.

### Synthesis of compound 102-7

A mixture of 102-6 (2.1 g, 4.428 mmol) in tetrahydrofuran (120 mL) and water (50 mL) was added lithium hydroxide (0.56 g, 13.285 mmol), and the reaction mixture was stirred at 50 °C for 2 hours. Removed tetrahydrofuran, and the residue was added dilute hydrochloric acid (1 mol/L) to adjust pH=3-4, solid precipitated, extracted with ethyl acetate, concentrated to dryness to get the crude compound 102-7 (2.18 g, crude). ESI:(m/z) = 458.0 [M-H]⁺;

### Synthesis of compound 102-8

A mixture of 102-7 (2.18 g, 4.737 mmol) in DMF (20 mL) was added ammonium chloride solid (0.51 g, 9.475 mmol), HATU (2.7 g, 7.106 mmol) and DIEA (1.84 g, 14.212 mmol), and the resulting mixture was stirred for 2 h at room temperature. The reaction mixture was diluted with ethyl acetate and water. The organic phase was separated and the aqueous phase was extracted with ethyl acetate. The organic phases were combined, washed with water, brine, dried over anhydrous Na₂SO₄, filtered and the filtrate was evaporated to give the crude compound 102-8 (2.1 g, 96.3%). ESI:(m/z) = 458.9 [M+H]⁺.

### Synthesis of compound 102-9

A mixture of 102-8 (1.9 g, 4.138 mmol) in Eaton's reagent (20 mL) was added 2-chloro-5-fluorobenzaldehyde (0.79 g, 4.965 mmol), and the reaction mixture was stirred at 80 °C for 2 hours. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na₂SO₄, filtered, and the filtrate was evaporated, and the residue was purified by column chromatography (mobile phase: petroleum ether/ethyl acetate ~3/1) to give compound 102-9 (600 mg, 24.2%). ESI:(m/z) = 598.9 [M+H]⁺.

### Synthesis of compound YL102-P1 and YL102-P2

A mixture of 102-9 (200 mg, 0.333 mmol) in DMF (2 mL) was added zinc hydride (78 mg, 0.667 mmol) and Pd(PPh₃)₄ (57.8 mg, 0.05 mmol), and stirred at 120 °C for 1 h in microwave. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na₂SO₄, filtered, and the filtrate was evaporated, and the residue was purified by column chromatography (mobile phase: petroleum ether/ethyl acetate ~3/1) and SFC resolution to obtain compound YL102-P1 (17 mg, 9.4%), compound YL102-P2 (15 mg, 8.25%).
compound YL102-P1: H NMR:¹H NMR (400 MHz, DMSO-d6) δ 10.47 (s, 1H), 8.78 (d, J = 2.2 Hz, 1H), 7.98 (d, J = 8.4 Hz, 1H), 7.87 (s, 1H), 7.81 (d, J = 9.0 Hz, 1H), 7.77 (s, 1H), 7.32 (dd, J = 8.8, 5.1 Hz, 1H), 7.12 - 7.03 (m, 1H), 7.01 (dd, J = 9.1, 3.1 Hz, 1H), 6.45 (s, 1H), 4.50 (s, 3H).
compound YL102-P2: HNMR:¹H NMR (400 MHz, DMSO-d6) δ 10.48 (s, 1H), 8.78 (d, J = 2.2 Hz, 1H), 7.98 (d, J = 8.4 Hz, 1H), 7.87 (s, 1H), 7.81 (d, J = 9.4 Hz, 1H), 7.77 (s, 1H), 7.32 (dd, J = 8.8, 5.1 Hz, 1H), 7.07 (td, J = 8.4, 3.1 Hz, 1H), 7.04 - 6.98 (m, 1H), 6.46 (s, 1H), 4.50 (s, 3H).

### Synthetic route of compound YL103

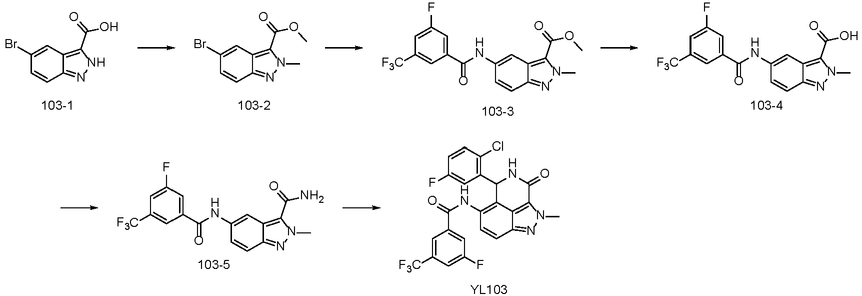

### Synthesis of compound 103-2

To a 250 mL three-necked flask were sequentially added compound 103-1 (9640 mg, 39.993 mmol), potassium carbonate (16581.10 mg, 119.979 mmol) and N,N-dimethylformamide (80 mL). Cooled to 0°C, the resulting mixture was added iodomethane (12.983 mL, 159.973 mmol), then was warmed to 50°C and stirred at this temperature overnight. LCMS showed that the reaction was complete, and water and ethyl acetate were added to the above mixture, and the organic phase was concentrated and the residue was purified by column chromatography (eluent: petroleum ether: ethyl acetate = 20:1-10:1) to give 103-2 (3500 mg, 13.006 mmol, 32.52%). LC-MS (ESI): m/z 269.0 (M+H) ⁺.

### Synthesis of compound 103-3

To a 100 mL three-necked flask were sequentially added 103-2 (725 mg, 2.694 mmol), 3-fluoro-5-(trifluoromethyl)benzamide (837.06 mg, 4.041 mmol), cesium carbonate (1755.63 mg, 5.388 mmol), 4,5-bisdiphenylphosphino-9,9-dimethylxanthene (311.79 mg , 0.539 mmol), tris(dibenzylideneacetone)dipalladium (246.71 mg, 0.269 mmol) and 1,4-dioxane (20 mL). The reaction mixture was stirred under nitrogen protection at 80°C for 5 h. LCMS showed that the reaction was successful, and at the end of the reaction, the reaction mixture was filtered, the filter cake was washed with a large amount of ethyl acetate, and the organic phase was concentrated and purified by column chromatography (eluent: petroleum ether: ethyl acetate=5:1-1:1) to give 103-3 (825 mg, 2.087 mmol, 77.46%). LC-MS (ESI): m/z 396.1 (M+H)⁺.

### Synthesis of compound 103-4

Methyl 5-(3-fluoro-5-trifluoromethyl)benzamido-2-methYL2H-indazole-3-carboxylate (Compound 103-3, 790 mg, 2 mmol), lithium hydroxide (144 mg, 6 mmol), tetrahydrofuran (10 mL) and water (5 mL) were added sequentially to a 50 mL three-necked flask. The reaction mixture was stirred for 2 hours at room temperature. After completion of the reaction, removed the excess tetrahydrofuran and added 1N hydrochloric acid to adjust the pH to 2. Then it was filtered and lyophilized to give 103-4 (530 mg, 1.390 mmol, 69.56%). LC-MS (ESI): m/z 382.1 (M+H)⁺.

### Synthesis of compound 103-5

To a 50 mL three-necked flask was added 103-4 (530 mg, 1.390 mmol), ammonium chloride (111.53 mg, 2.085 mmol), 1-(3-dimethylaminopropyl)-3-ethylcarbodiimide hydrochloride (399.70 mg, 2.085 mmol), 1-hydroxybenzotriazole (281.75 mg, 2.085 mmol), N,N-diisopropylethylamine(538.98 mg, 4.170 mmol) and N,N-dimethylformamide (10 mL) sequentially. The reaction mixture was stirred under nitrogen protection at room temperature for 6 h. Upon completion, the reaction was added a large amount of water, filtered, and the filter cake was lyophilized to give 103-5 (400 mg, 1.052 mmol, 75.67%). LC-MS (ESI): m/z 381.2 (M+H)⁺.

### Synthesis of compound YL103

To a 50 mL three-necked flask was sequentially added 103-5 (100 mg, 0.263 mmol), 2-chloro-5-fluorobenzaldehyde (62.54 mg, 0.394 mmol) and Eaton's reagent (5 mL). The reaction mixture was stirred under nitrogen protection at 80 °C for 2 h. LCMS showed that the reaction was complete, the reaction mixture was decanted into cold sodium bicarbonate solution, ethyl acetate was added, and the organic phase was concentrated in reversed-phase preparation to afford the compound YL103 (20 mg, 0.038 mmol, 14.60%). LC-MS (ESI): m/z 521.1 (M+H)⁺; 1H NMR (400 MHz, DMSO-d6) δ 10.41 (s, 1H), 8.45 (d, J = 2.2 Hz, 1H), 8.00 - 7.91 (m, 1H), 7.83 (dd, J = 9.2, 2.3 Hz, 1H), 7.76 (s, 1H), 7.66 (dd, J = 8.8, 1.0 Hz, 1H), 7.31 (dd, J = 8.9, 5.2 Hz, 1H), 7.18 (d, J = 8.8 Hz, 1H), 7.06 (td, J = 8.4, 3.1 Hz, 1H), 6.90 (dd, J = 9.3, 3.1 Hz, 1H), 6.41 (d, J = 2.0 Hz, 1H), 4.44 (s, 3H).

### Synthesis Route for compound YL104 (104-P1 and 104-P2)

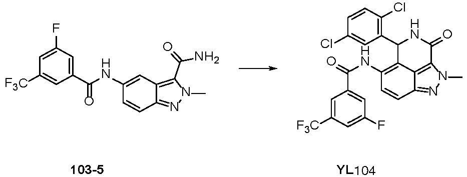

### Synthesis of compound YL104 (104-P1 and 104-P2)

To a 50 mL three-necked flask was sequentially added (130 mg, 0.342 mmol), 2,5-dichlorobenzaldehyde (89.74 mg, 0.513 mmol) and Eaton's reagent (5 mL), and the reaction mixture was stirred under nitrogen protection at 80°C for 2 h. LCMS showed that the reaction was complete, and ethyl acetate and aqueous sodium hydroxide were added to the reaction mixture, and the organic phase was concentrated and the residue was purified by prep-HPLC to yield YL104 (37 mg, 0.069 mmol, 20.14%), then separated by SFC resolution to obtain 104-P1 (10 mg) and 104-P2 (17 mg).

104-P1 (10 mg): LC-MS (ESI): m/z 537.0 (M+H)+;1H NMR (400 MHz, DMSO-d6) δ 10.41 (s, 1H), 8.42 (d, J = 2.2 Hz, 1H), 7.96 (dt, J = 8.5, 1.9 Hz, 1H), 7.82 (dt, J = 9.4, 1.9 Hz, 1H), 7.74 (s, 1H), 7.66 (dd,J = 8.8, 1.1 Hz, 1H), 7.32 (d, J = 8.5 Hz, 1H), 7.25 (dd, J = 8.5, 2.6 Hz, 1H), 7.16 (d, J = 8.9 Hz, 1H), 7.08 (d, J = 2.5 Hz, 1H), 6.34 (s, 1H), 4.44 (s, 3H).

104-P2 (17 mg): LC-MS (ESI): m/z 537.0 (M+H)+;1H NMR (400 MHz, DMSO-d6) δ 10.45 (s, 1H), 8.42 (d, J=2.2 Hz, 1H), 7.95 (d, J=8.4 Hz, 1H), 7.84 (d, J=9.1 Hz, 1H), 7.75 (s, 1H), 7.65 (dd,J = 8.8, 1.1 Hz, 1H), 7.32 (d, J = 8.5 Hz, 1H), 7.24 (dd, J = 8.6, 2.6 Hz, 1H), 7.17 (d, J = 8.9 Hz, 1H), 7.10 (d, J = 2.6 Hz, 1H), 6.35 (s, 1H), 4.44 (s, 3H).

Condition for SFC resolution : Instrument: SFC-150 (Waters); Column: AS 20*250mm, 10um (Daicel); Mobile phase: CO₂/MeOH[0.2%NH₃(7M in MeOH) ] = 45/55; Flow rate: 120 g/min; Back pressure: 100 bar; Detection wavelength: 214 nm; Cycle time: 13.8 min;

### Synthetic route of compound YL105

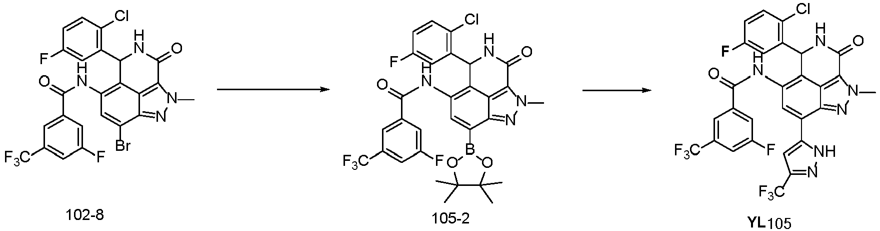

### Synthesis of compound 105-2

A mixture of 102-8 (300 mg, 0.5 mmol) in 1,4-dioxane (8 mL) was added pinacol bis(boronic acid) ester (445 mg, 1.751 mmol), potassium acetate (196 mg, 2.001 mmol) and Pd(dppf)Cl₂ (73.2 mg, 0.1 mmol). After degassed and purged with N2, the resulting mixture was stirred for 1 h at 100 °C. The reaction mixture was diluted with water and the aqueous phase was extracted with ethyl acetate. The organic phases were combined, washed with water, brine, dried over anhydrous Na₂SO₄, filtered and the filtrate was evaporated to dryness to give crude compound 105-2 (300 mg, crude).

### Synthesis of compound YL105

105-2 (300 mg, 0.531 mmol), 5-bromo-3(trifluoromethyl)pyrazole (114 mg, 0.531 mmol), potassium carbonate (220 mg, 1.594 mmol) and Pd(dppf)Cl₂ (38.8 mg, 0.053 mmol) in a reaction flask were added dioxane (2 mL) and water (0.4 mL). After degassed and purged with nitrogen, the reaction mixture was stirred at 105 °C for 1 h. Extracted with ethyl acetate and water, concentrated to dryness, and the residue was purified by prep-HPLC to give compound YL105 (12 mg, 3.4%).

### Synthetic route of compound YL106

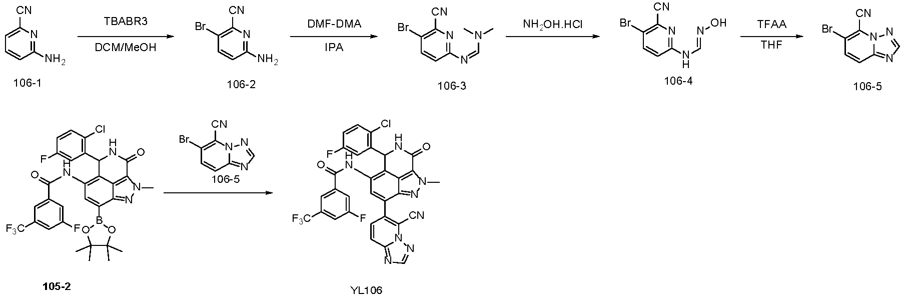

### Synthesis of compound 106-2

Tetrabutylammonium tribromide (69.82 g, 144.8 mmol) was added to a mixture of 2-Amino-6-cyanopyridine (compound 106-1, 15 g, 125.91 mmol) in dichloromethane (60 mL) and methanol (60 mL), and the reaction mixture was stirred at room temperature for 1 hour. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na₂SO₄, filtered, and the filtrate was evaporated, and the residue was purified by column chromatography (mobile phase: petroleum ether/ethyl acetate ~3/1) to give compound 106-2 (10.2 g, 40.11%).

### Synthesis of compound 106-4

A mixture of 106-2 (10.2 g, 51.51 mmol) in isopropanol (150 mL) was added N,N-dimethylformamide dimethyl acetal (7.98 g, 66.96 mmol), and the reaction mixture was stirred at 80 °C for 3 h to obtain crude product 106-3. The reaction mixture was cooled to 50 °C, added hydroxylamine hydrochloride (4.65 g, 66.96 mmol), and stirred at 50 °C overnight. The reaction mixture was directly filtered and the filter cake was washed with ethanol to obtain compound 106-4 (10 g, 80.52%). ESI:(m/z) = 241.0 [M+H]⁺.

### Synthesis of compound 106-5

Trifluoroacetic anhydride (8.71 g, 41.485 mmol) was added dropwise to a mixture of 106-4 (5 g, 20.743 mmol) in tetrahydrofuran (50 mL) at 0 °C, and the reaction mixture was stirred at 60 °C overnight. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na2SO4, filtered, and the filtrate was evaporated, and the residue was purified by column chromatography (mobile phase: petroleum ether/ethyl acetate ~2/1) to give compound 106-5 (1.1 g, 23.76%). ESI: (m/z) = 223.0 [M+H]⁺. ¹H NMR (400 MHz, DMSO-d6) δ 8.73 (s, 1H), 8.20 (d, J = 9.5 Hz, 1H), 8.06 (d, J = 9.5 Hz, 1H).

### Synthesis of Compound YL106 (106-P1 & 106-P2)

105-2 (200 mg, 0.309 mmol), 106-5 (70 mg, 0.308 mmol), potassium carbonate (128 mg, 0.923 mmol) and Pd(dppf)Cl₂ (22.6 mg, 0.031 mmol) in a reaction flask were added dioxane (2 mL) and water (0.4 mL). After degassed and purged with nitrogen, the reaction mixture was stirred at 100 °C for 1.5 hours, extracted with ethyl acetate and water, concentrated to dryness, and the residue was purified by SFC to give the compounds YL106-P1 (9 mg) and YL106-P2 (YL16572) (28 mg). LCMS (ESI): m/z 663.0(M+H)⁺;

Conditions for SFC resolution : instrument: SFC-150 (Waters);column: AS 20*250mm, 10um (Daicel) ;mobile phase: CO₂/MeOH[0.2%NH₃(7M in MeOH) ] = 45/55;flow rate: 120 g/min ;back pressure: 100 bar ;wavelength: 214 nm.

YL106-P1: 1H NMR (400 MHz, DMSO-d6) δ 10.56 (s, 1H), 8.81 (s, 1H), 8.67 (s, 1H), 8.39 (d, J = 9.3 Hz, 1H), 8.19 (d, J = 9.3 Hz, 1H), 7.97 (d, J = 8.7 Hz, 1H), 7.90 - 7.78 (m, 2H), 7.61 (s, 1H), 7.36 (dd, J = 8.9, 5.2 Hz, 1H), 7.12 (d, J = 8.2 Hz, 1H), 7.01 (d, J = 9.1 Hz, 1H), 6.48 (s, 1H), 4.48 (s, 3H).

YL106-P2: 1H NMR (400 MHz, DMSO-d6) δ 10.56 (s, 1H), 8.81 (s, 1H), 8.66 (s, 1H), 8.39 (d, J = 9.3 Hz, 1H), 8.19 (d, J = 9.3 Hz, 1H), 7.97 (d, J = 8.3 Hz, 1H), 7.85 (d, J = 9.1 Hz, 1H), 7.80 (s, 1H), 7.61 (s, 1H), 7.36 (dd, J = 8.9, 5.1 Hz, 1H), 7.14 - 7.07 (m, 1H), 7.03 - 6.97 (m, 1H), 6.48 (s, 1H), 4.48 (s, 3H).

### Snthetic route of compound YL107

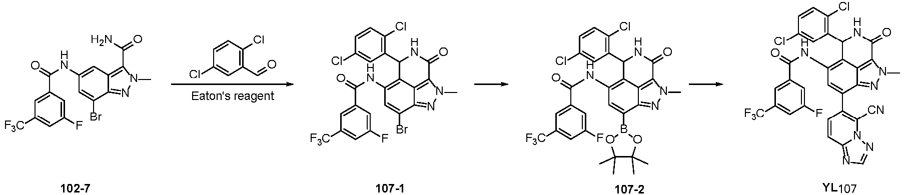

### Synthesis of compound 107-1

A mixture of 102-7 (1.5 g, 3.267 mmol) in Eaton's reagent (20 mL) was added 2,5-dichlorobenzene-1-carboxaldehyde (0.74 g, 4.247 mmol), and the reaction mixture was stirred at 80 °C for 2 hours. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na2SO4, filtered, and the filtrate was evaporated, and the residue was purified by column chromatography (mobile phase: petroleum ether/ethyl acetate ~3/1) to give compound 107-1 (450 mg, 22.4%). ESI:(m/z) = 617.0 [M+H]⁺.

### Synthesis of compound 107-2

A solution of 107-1 (100 mg, 0.162 mmol) in 1,4-dioxane (2 mL) was added pinacol bis(boronic acid) ester (62 mg, 0.243 mmol), potassium acetate (48 mg, 0.487 mmol) and Pd(dppf)Cl₂ (24 mg, 0.032 mmol), and the reaction mixture was stirred at 80°C for 2 hours. The reaction mixture went directly to the next step.

### Synthesis of compound YL107

YL107-2 (142 mg, 0.387 mmol), potassium carbonate (64 mg, 0.465 mmol) and Pd(dppf)Cl₂ (11 mg, 0.015 mmol) in a reaction flask were added dioxane (1 mL) and water (0.2 mL). After degassed and purged with nitrogen, the reaction mixture was stirred at 100 °C for 1 h. The reaction mixture was extracted with ethyl acetate and water. After concentrated to dryness, the residue was purified by prep-HPLC to give compound YL107 (38 mg). LCMS (ESI): m/z 679.0(M+H) ⁺;
Compound YL107: 1H NMR (400 MHz, DMSO-d6) δ 10.60 (s, 1H), 8.81 (s, 1H), 8.62 (s, 1H), 8.38 (d, J = 9.3 Hz, 1H), 8.19 (d, J = 9.3 Hz, 1H), 7.97 (d, J = 8.5 Hz, 1H), 7.84 (d, J = 9.5 Hz, 1H), 7.78 (s, 1H), 7.59 (s, 1H), 7.37 (d, J = 8.5 Hz, 1H), 7.29 (dd, J = 8.5, 2.5 Hz, 1H), 7.18 (d, J = 2.6 Hz, 1H), 6.42 (s, 1H), 4.48 (s, 3H).

### Synthetic route of compound YL108 (108-P1 &108-P2)

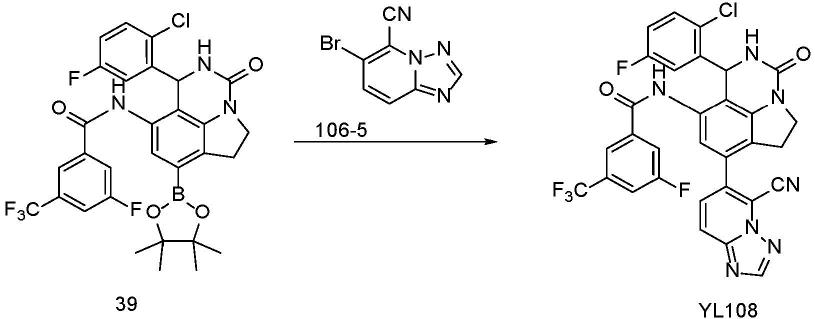

### Synthesis of Compound YL108 (108-P1 & 108-P2)

39 (200 mg, 0.341 mmol), 106-5 (76 mg, 0.341 mmol), potassium carbonate (141 mg, 1.023 mmol) and Pd(dppf)Cl₂ (25 mg, 0.034 mmol) in a reaction flask were added dioxane (2 mL) and water (0.4 mL). After degassed and purged with nitrogen, the reaction mixture was stirred at 100 °C for 1.5 hours, extracted with ethyl acetate and water, and the organic phase was concentrated to dryness and purified by SFC resolution to give compound YL108-P1 (13 mg) and compound YL108-P2 (63 mg). LCMS (ESI): m/z 650.0(M+H)⁺;
Conditions for SFC resolution: instrument: SFC-150 (Waters);column: AS 20*250mm, 10um (Daicel) ;mobile phase: CO₂/MeOH[0.2%NH₃(7M in MeOH) ] = 45/55;flow rate: 120 g/min ;back pressure: 100 bar; wavelength: 214 nm; cycle time: 13.8 min.

Compound YL108-P1: ¹H NMR (400 MHz, DMSO-d6) δ 10.34 (s, 1H), 8.78 (s, 1H), 8.32 (d, J = 9.3 Hz, 1H), 7.99 - 7.85 (m, 4H), 7.64 (d, J = 2.6 Hz, 1H), 7.37 (dd, J = 8.9, 5.2 Hz, 1H), 7.14 (td, J = 8.3, 3.0 Hz, 1H), 7.00 (s, 1H), 6.95 (dd, J = 9.2, 3.0 Hz, 1H), 6.19 (s, 1H), 4.13 - 3.95 (m, 2H), 3.23 (dd, J = 10.7, 7.0 Hz, 2H).

Compound YL108-P2: 1H NMR (400 MHz, DMSO-d6) δ 10.34 (s, 1H), 8.78 (s, 1H), 8.32 (d, J = 9.2 Hz, 1H), 8.00 - 7.85 (m, 4H), 7.64 (s, 1H), 7.37 (dd, J = 8.8, 5.2 Hz, 1H), 7.14 (td, J = 8.3, 3.1 Hz, 1H), 7.00 (s, 1H), 6.95 (dd, J = 9.2, 3.1 Hz, 1H), 6.18 (d, J = 2.5 Hz, 1H), 4.14 - 3.95 (m, 2H), 3.29 - 3.19 (m, 2H).

### Synthetic route of compound YL109 (109-P1 &109-P2)

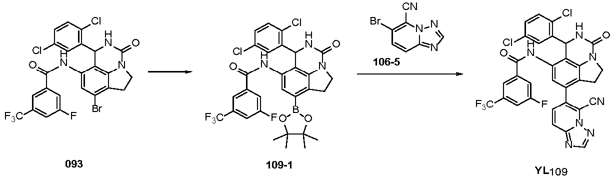

### Synthesis of compound YL109-1 (109-P1 & 109-P2)

A solution of 093 (100 mg, 0.162 mmol) in 1,4-dioxane (2 mL) was added pinacol bis(boronic acid) ester (62 mg, 0.243 mmol), potassium acetate (48 mg, 0.487 mmol) and Pd(dppf)Cl₂ (24 mg, 0.032 mmol), and the resulting mixture was stirred at 80 °C for 2 h. The obtained crude 109-1 was used directly in the next step.

109-1 (200 mg, 0.308 mmol), 106-5 (70 mg, 0.308 mmol), potassium carbonate (127 mg, 0.923 mmol) and Pd(dppf)Cl₂ (22.5 mg, 0.031 mmol) in a reaction flask were added dioxyhexacycline (2 mL) and water (0.4 mL). After degassed and purged with nitrogen, the reaction mixture was stirred at 100 °C for 1.5 hours, extracted with ethyl acetate and water, and the organic phase was concentrated to dryness and purified by SFC to give YL109-P1 (15 mg) and YL109-P2 (54 mg). LCMS (ESI): m/z 666.0(M+H) ⁺;
Conditions for SFC resolution: instrument: SFC-150 (Waters);column: AS 20*250mm, 10um (Daicel);mobile phase: CO₂/MeOH[0.2%NH₃(7M in MeOH) ] = 45/55;flow rate: 120 g/min ;back pressure: 100 bar; wavelength: 214 nm.

YL109-P1: ¹H NMR (400 MHz, DMSO-d6) δ 10.33 (s, 1H), 8.78 (s, 1H), 8.31 (d, J = 9.3 Hz, 1H), 7.99 - 7.84 (m, 4H), 7.56 (s, 1H), 7.38 (d, J = 8.6 Hz, 1H), 7.31 (dd, J = 8.5, 2.5 Hz, 1H), 7.11 (d, J = 2.6 Hz, 1H), 6.97 (s, 1H), 6.15 (d, J = 2.4 Hz, 1H), 4.10 - 3.94 (m, 2H), 3.28 - 3.19 (m, 2H).

YL109-P2: 1H NMR (400 MHz, DMSO-d6) δ 10.33 (s, 1H), 8.78 (s, 1H), 8.31 (d, J = 9.2 Hz, 1H), 8.00 - 7.83 (m, 4H), 7.56 (d, J = 2.4 Hz, 1H), 7.11 (d, J = 2.5 Hz, 1H), 6.97 (s, 1H), 6.15 (d, J = 2.4 Hz, 1H), 4.05 (td, J = 10.3, 7.1 Hz, 2H), 3.28 - 3.17 (m, 2H).

### Synthetic route of compound YL110

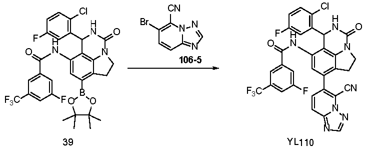

Compound 39 (250 mg, 0.426 mmol), 106-5 (90 mg, 0.426 mmol), potassium carbonate (177 mg, 1.278 mmol) and Pd(dppf)Cl₂ (31.2 mg, 0.043 mmol) in a reaction flask were added dioxane (2 mL) and water (0.4 mL). After degassed and purged with nitrogen, the reaction mixture was stirred for 1 hour, extracted with ethyl acetate and water, concentrated to dryness, and the residue was purified by prep-HPLC to give compound YL110 (52 mg). LCMS (ESI): m/z 639.3(M+H) ⁺;
YL110: 1H NMR (400 MHz, DMSO-d6) δ 10.23 (s, 1H), 8.57 (s, 1H), 7.94 (d, J = 8.5 Hz, 1H), 7.90 - 7.77 (m, 3H), 7.65 (d, J = 9.2 Hz, 1H), 7.55 (d, J = 2.6 Hz, 1H), 7.36 (dd, J = 8.8, 5.1 Hz, 1H), 7.12 (td, J = 8.4, 3.1 Hz, 1H), 6.96 (dd, J = 9.2, 3.1 Hz, 1H), 6.75 (s, 1H), 6.18 - 6.12 (m, 1H), 4.01 (h, J = 10.1 Hz, 2H), 3.07 (s, 2H), 2.67 (s, 3H).

### Synthetic route of compound YL111 (YL 111-P1 & YL 111-P2)

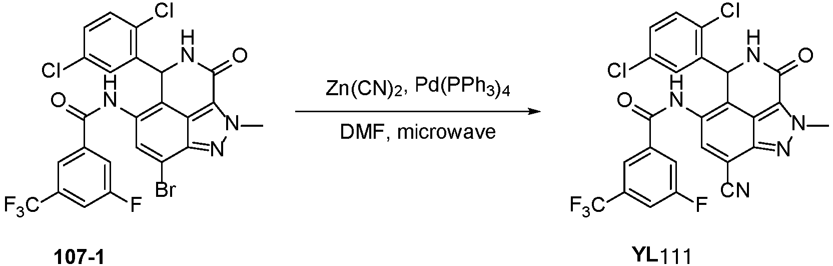

### Synthesis of compound YL111 (YL 111-P1 & YL 111-P2)

A mixture of 107-1 (200 mg, 0.333 mmol) in DMF (2 mL) was added zinc cyanide (76 mg, 0.667 mmol) and Pd(PPh₃)₄ (56.3 mg, 0.05 mmol), and the reaction mixture was stirred at 120 °C for 1 h in a microwave. The reaction mixture was diluted with ethyl acetate and water. The organic phase was separated and the aqueous phase was extracted with ethyl acetate. The organic phases were combined, washed with water, saturated aqueous sodium bicarbonate, dried over anhydrous Na₂SO₄, filtered, and the filtrate was evaporated, and the residue was purified by column chromatography (mobile phase: petroleum ether/ethyl acetate 3/1) and SFC resolution (acid method) to obtain the compound YL111-P1 (19 mg) and compound YL111-P2 (15.5 mg).

Conditions for SFC resolution: instrument: SFC-150 (Waters);column: AS 20*250mm, 10um (Daicel); mobile phase: CO₂/MeOH[0.2%NH₃(7M in MeOH) ] = 45/55; flow rate: 120 g/min; back pressure: 100 bar; wavelength: 214 nm.

YL111-P1: ¹H NMR (400 MHz, DMSO-d6) δ 10.48 (s, 1H), 8.72 (d, J = 2.2 Hz, 1H), 8.02 - 7.95 (m, 1H), 7.85 (s, 1H), 7.82 - 7.77 (m, 1H), 7.75 (s, 1H), 7.33 (d, J = 8.5 Hz, 1H), 7.26 (dd, J = 8.5, 2.5 Hz, 1H), 7.22 - 7.17 (m, 1H), 6.40 (s, 1H), 4.51 (s, 3H).

YL111-P2:¹H NMR (400 MHz, DMSO-d6) δ 10.48 (s, 1H), 8.72 (d, J = 2.2 Hz, 1H), 8.02 - 7.95 (m, 1H), 7.85 (s, 1H), 7.82 - 7.77 (m, 1H), 7.75 (s, 1H), 7.33 (d, J = 8.5 Hz, 1H), 7.26 (dd, J = 8.5, 2.5 Hz, 1H), 7.22 - 7.17 (m, 1H), 6.40 (s, 1H), 4.51 (s, 3H).

### Synthetic route of compound YL112

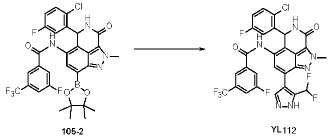

Under nitrogen protection, Pd(dppf)Cl₂ (45.24 mg, 0.062 mmol) was added to a mixture of 105-2 (280.0 mg, 0.309 mmol), 4-bromo-5-(difluoromethyl)-1H-pyrazole (60.90 mg, 0.309 mmol) and potassium carbonate (128.18 mg, 0.928 mmol) in a mixture of 1, 4-dioxane (10 mL) and water (2 mL), and the resulting mixture was heated at 90 °C for 1.5 hours. The reaction was quenched with water, extracted with ethyl acetate and the organic phase was filtered through celite, the filtrate was concentrated and purified by prep-HPLC (Base) to give an off-white product (30 mg, ~89%), which was purified again by preparative TLC (methanol/dichloromethane (v/v) = 1:20) to give the white product compound YL112 (10 mg, yield 5.1%). ESI: m/z 637.0 [M+H]⁺; ¹H NMR (400 MHz, DMSO-d₆) δ13.65 (s, 1H), 10.45 (s, 1H), 8.67 (s, 1H), 8.52 (d, J = 2.2 Hz, 1H), 7.96 (d, J = 8.4 Hz, 1H), 7.82 (d, J = 9.3 Hz, 1H), 7.73 (s, 1H), 7.39 (d, J = 5.1 Hz, 1H), 7.33 (dd, J = 8.9, 5.1 Hz, 1H), 7.08 (tt, J = 8.4, 4.1 Hz, 1H), 6.90 (dd, J = 9.2, 3.1 Hz, 1H), 6.38 (s, 1H), 4.49 (s, 3H).

### Synthetic route of compound YL113

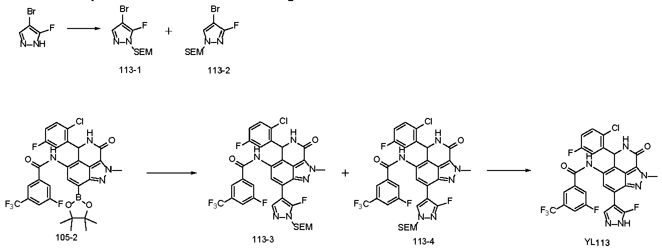

### Synthesis of compounds 113-1 & 113-2

NaH (18.19 mg, 0.455 mmol) was added to a solution of 4-bromo-3-fluoro-1H-pyrazole (50.0 mg, 0.303 mmol) in THF (3 mL) in an ice-water bath. After stirred for half an hour, the resulting mixture was added SEMCl (60.64 mg, 0.364 mmol), then warmed to room temperature and stirred for 1 hour. The reaction was quenched with water, extracted with ethyl acetate three times, partitioned and the organic phase was concentrated and purified by column chromatography (ethyl acetate/petroleum ether (v/v) = 0%~10%) to give a mixture of compounds 113-1 & 113-2 (30 mg, 33.6% yield).

### Synthesis of compounds 113-3 & 113-4

Pd(dppf)Cl₂ (18.10 mg, 0.025 mmol) was added to a mixture of 105-2 (80 mg, 0.124 mmol), compounds 113-3 & 113-4 (32.86 mg, 0.111 mmol) and potassium carbonate (51.28 mg, 0.371 mmol) in 1,4- dioxane (5 mL) and water (1 mL) under nitrogen protection, the resulting mixture was heated at 90 °C for 1.5 hours. The reaction mixture was diluted with water and ethyl acetate, partitioned to give the organic phase, which was concentrated and purified by column chromatography (methanol/dichloromethane (v/v) = 0~10%) to give compounds 113-3 & 113-4 (70 mg, 0.095 mmol, 76.98%). (ESI):(m/z) = 735.0 [M+H]⁺;

### Synthesis of compound YL113

TFA (1.2 mL, 15.671 mmol) was added dropwise to a solution of compounds 113-3 & 113-4 (30 mg, 0.041 mmol) in THF (4 mL) and the reaction was heated up to 80 °C for 8 hours. The reaction mixture was quenched to neutrality by addition of saturated sodium bicarbonate solution, extracted with dichloromethane, partitioned and the organic phase was concentrated and prepared and purified to give compound YL113 (6.5 mg). (ESI):(m/z) = 605.1 [M+H]⁺ ; ¹H NMR (400 MHz, DMSO-d₆)612.82 (s, 1H), 10.43 (s, 1H), 8.57 (d, J = 1.8 Hz, 1H), 8.51 (d, J = 2.2 Hz, 1H), 7.96 (d, J = 8.5 Hz, 1H), 7.82 (d, J = 9.0 Hz, 1H), 7.74 (s, 1H), 7.39 (s, 1H), 7.32 (dd, J = 8.8, 5.1 Hz, 1H), 7.07 (td, J = 8.4, 3.1 Hz, 1H), 6.89 (dd, J = 9.2, 3.1 Hz, 1H), 6.38 (s, 1H).

### Synthetic route of compound YL114 (114-P1 & 114-P2)

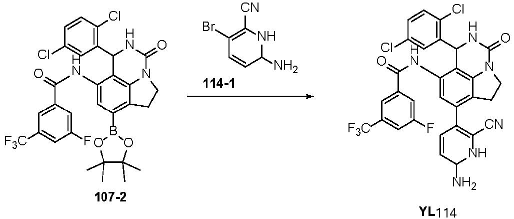

107-2 (230 mg, 0.354 mmol), 114-1 (70 mg, 0.354 mmol), potassium carbonate (147 mg, 1.061 mmol) and Pd(dppf)Cl₂ (26 mg, 0.035 mmol) in a reaction flask were added dioxyhexacycline (2 mL) and water (0.4 mL). The reaction mixture was stirred at 100 °C for 1 h. The reaction was extracted with ethyl acetate and water, concentrated to dryness, and the Compound YL114-P1 (49 mg), and the Compound YL114-P2 (50 mg) were obtained by purified by prep-HPLC and SFC resolution. LCMS (ESI): m/z 641.2(M+H) ⁺;
Conditions for SFC resolution: instrument: SFC-150 (Waters);column: AS 20*250mm, 10um (Daicel); mobile phase: CO₂/MeOH[0.2%NH₃(7M in MeOH) ] = 45/55; flow rate: 120 g/min; back pressure: 100 bar; wavelength: 214 nm, cycle time: 13.8min.

YL114-P1 : LCMS (ESI): m/z 641.2(M+H) ⁺; ¹H NMR (400 MHz, DMSO-d6) δ 10.24 (s, 1H), 8.01 - 7.81 (m, 3H), 7.62 (d, J = 8.7 Hz, 1H), 7.49 (d, J = 2.4 Hz, 1H), 7.35 (d, J = 8.6 Hz, 1H), 7.29 (dd, J = 8.6, 2.5 Hz, 1H), 7.07 (d, J = 2.5 Hz, 1H), 6.80 (d, J = 8.8 Hz, 1H), 6.75 (s, 2H), 6.12 (d, J = 2.4 Hz, 1H), 4.00 (dtd, J = 21.4, 10.4, 6.8 Hz, 2H), 3.26 - 3.05 (m, 2H).

YL114-P2 : LCMS (ESI): m/z 641.2(M+H) ⁺; ¹H NMR (400 MHz, DMSO-d6) δ 10.26 (s, 1H), 7.95 - 7.83 (m, 3H), 7.62 (d, J = 8.7 Hz, 1H), 7.47 (s, 1H), 7.35 (d, J = 8.6 Hz, 1H), 7.29 (dd, J = 8.6, 2.6 Hz, 1H), 7.06 (d, J = 2.5 Hz, 1H), 6.80 (d, J = 8.7 Hz, 1H), 6.75 (s, 2H), 6.12 (d, J = 2.5 Hz, 1H), 4.06 - 3.92 (m, 2H), 3.24 - 3.05 (m, 2H).

### Synthetic route of compound YL115

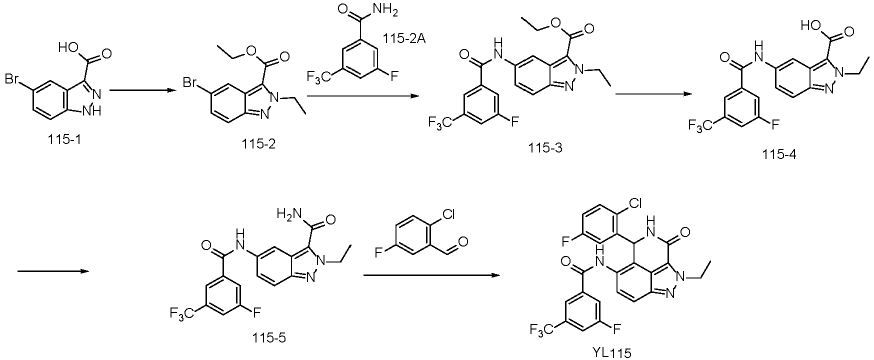

### Synthesis of compound 115-2

To a reaction vial were added 5-bromoindazole-3-carboxylic acid (115-1, 9 g, 37.338 mmol), potassium carbonate (15.48 g, 112.015 mmol), DMF (550 mL) and ethyl iodide (11.65 g, 74.676 mmol). The mixture was stirred at 80 °C overnight. Cooled to room temperature, removed most of the DMF by concentration at reduced pressure, the mixture was diluted with ethyl acetate and water. The organic phase was separated and the aqueous phase was extracted with ethyl acetate. The organic phases were combined, washed with brine, concentrated at reduced pressure, and the resulting crude product was purified by automated chromatography (Biotage) (mobile phases: petroleum ether/ethyl acetate 100/6 to 100/15) to afford the target compound 115-2 (4.9 g, Purity: 87.3 %, Yield: 38.56 %), ¹H NMR (400 MHz, DMSO-d6)δ 8.10 (dd, J = 1.9, 0.7 Hz, 1H), 7.77 (dd, J = 9.0, 0.7 Hz, 1H), 7.48 (dd, J = 9.1, 1.9 Hz, 1H), 4.84 (q, J = 7.2 Hz, 2H), 4.44 (q, J = 7.1 Hz, 2H), 1.47 (t, J = 7.2 Hz, 3H), 1.41 (t, J = 7.1 Hz, 3H), ESI:(m/z) =298.9 [M+H]⁺.

### Synthesis of compound 115-3

Cesium carbonate (2.83 g, 8.691 mmol), Xantphos (0.50 g, 0.869 mmol) and Pd₂(dba)₃ (0.40 g, 0.437 mmol) were added to a solution of 115-2 (2.65 g, 10.863 mmol) in dioxane (110 mL), and the reaction mixture was stirred for 3 h at 110 °C. It was diluted with ethyl acetate and water. The organic phase was separated and the aqueous phase was extracted with ethyl acetate. The organic phases were combined, washed with water, dried over anhydrous Na₂SO₄, filtered, and the filtrate was evaporated, and the residue was purified by column chromatography (mobile phases: ethyl acetate/petroleum ether 100/15 to 100/20) to give compound 115-3 (760 mg, Purity: 98.4%, Yield: 40.65 %). ESI: (m/z) =424.5 [M+H ]⁺.

### Synthesis of compound 115-4

A solution of 115-3 (750 mg, 1.772 mmol) was dissolved in tetrahydrofuran (120 mL) and water (40 mL) was added lithium hydroxide (743.32 mg, 17.715 mmol), and the resulting mixture was stirred overnight at room temperature, then was concentrated to dryness and the residue was diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 115-4 (470 mg, Purity: 87 %, Yield: 96 %). ESI:(m/z) =394.0 [M-H]⁻.

### Synthesis of compound 115-5

To a reaction vial were added 115-4 (450 mg, 1.138 mmol), ammonium chloride (182.67 mg, 3.415 mmol), HOBt (230.74 mg, 1.708 mmol), EDCI (327.33 mg, 1.708 mmol), DMF (45 mL) and DIEA (441.39 mg, 3.415 mmol). The reaction mixture was stirred at room temperature overnight. Upon completion, the reaction was quenched with water and diluted with ethyl acetate and water. The organic phase was separated and the aqueous phase was extracted with ethyl acetate. The organic phases were combined, washed with brine, concentrated at reduced pressure, and purified by column chromatography (mobile phase: petroleum ether/ethyl acetate 100/40 to 100/50) to obtain a white solid crude target compound 115-5 (310 mg, Purity: 96.8 %, Yield: 66.85%). ESI:(m/z) =395.1 [M+H]+ , ¹H NMR (400 MHz, DMSO-d6) δ 10.59 (s, 1H), 8.37 (d, J = 1.8 Hz, 1H), 8.21 (s, 1H), 8.16 (d, J = 9.1 Hz, 1H), 8.02 - 7.89 (m, 3H), 7.72 (m, 3H), 8.02 - 7.89 (m, 3H). 3H), 7.72 (d, J = 9.2 Hz, 1H), 7.63 (dd, J = 9.2, 1.9 Hz, 1H), 4.69 (q, J = 7.2 Hz, 2H), 1.46 (t, J = 7.2 Hz, 3H).

### Synthesis of compound YL115

A solution of 115-5 (305 mg, 0.773 mmol) in Eaton's reagent (40 mL) was added 2-chloro-5-fluorobenzaldehyde (245.28 mg, 1.547 mmol), and the reaction mixture was stirred at 80 °C for 2.5 hours. The reaction mixture was added dropwise to an ice-cold saturated sodium bicarbonate solution, and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated to dryness, and the resulting crude product was purified by prep-HPLC (Boston pHlex ODS, 21.2*250mm, 10um, water (0.05% ammonium bicarbonate)/acetonitrile, flow rate 30mL/min, column temperature 25°C, wavelength 254 nm) to give a white solid target compound. Compound YL115 (20 mg, Purity: 99.2 %, Yield: 4.80 %). ESI:(m/z) = 535.0 [M+H]⁺ , ¹H NMR (400 MHz, DMSO-d6) δ 10.36 (s, 1H), 8.44 (d, J = 2.2 Hz, 1H), 7.95 (dt, J = 8.6, 1.9 Hz, 1H), 7.81 (dt, J = 9.3, 1.9 Hz, 1H), 7.75 (s, 1H), 7.68 (dd, J = 8.8, 1.1 Hz, 1H), 7.31 (dd, J = 8.9, 5.1 Hz, 1H), 7.17 (d, J = 8.9 Hz, 1H), 7.06 (td, J = 8.4, 3.1 Hz, 1H), 6.87 (dd, J = 9.2, 3.1 Hz, 1H), 6.38 (s, 1H), 4.83 (qd, J = 13.2, 7.2 Hz, 2H), 1.55 (t, J = 7.2 Hz, 3H).

### Synthetic route of compound YL116

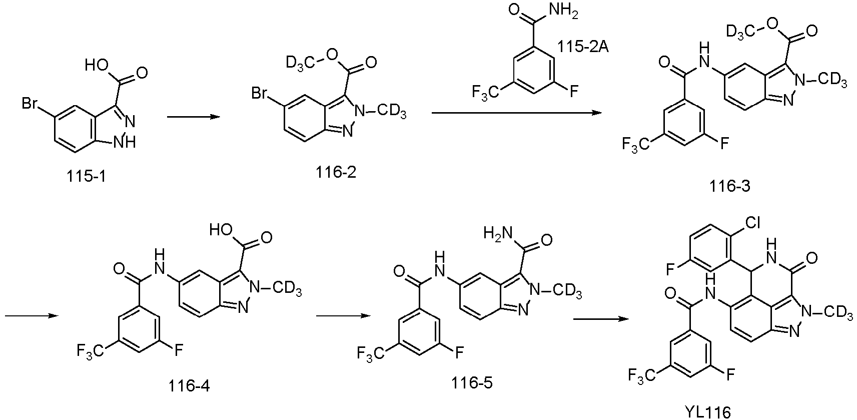

### Synthesis of compound 116-2

To a reaction vial were added 5-bromoindazole-3-carboxylic acid (115-1, 4 g, 16.595 mmol), potassium carbonate (6.88 g, 49.784 mmol), DMF (240 mL) and ethyl iodide (9.62 g, 66.379 mmol). The mixture was stirred at 80 °C overnight. Cooled to room temperature, removed most of the DMF by concentration at reduced pressure, the mixture was diluted with ethyl acetate and water. The organic phase was separated and the aqueous phase was extracted with ethyl acetate. The organic phases were combined, washed with brine, concentrated at reduced pressure, and the resulting crude product was purified by automated chromatography (Biotage) (mobile phases: petroleum ether/ethyl acetate 100/12 to 100/20) to give the product and by-products, and the less polar one was identified as the target compound 116-2 (2.1 g, Purity: 96 %, Yield: 44.16 %), ¹H NMR (400 MHz, DMSO-d6) δ 8.07 (dd, J = 2.0, 0.7 Hz, 1H), 7.73 (dd, J = 9.0, 0.7 Hz, 1H), 7.45 (dd, J = 9.1, 1.9 Hz, 1H). By-product (2.3 g, Purity: 97.3 %, Yield: 49.01 %), ¹H NMR (400 MHz, DMSO-d6) δ 8.13 (dd, J = 1.9, 0.7 Hz, 1H), 7.76 (dd, J = 8.9, 0.7 Hz, 1H), 7.60 (dd, J = 9.0, 1.9 Hz, 1H). ESI:(m/z) = 275.0/277.0 [M+H]⁺ .

### Synthesis of compound 116-3

115-2A (1.45 g, 6.979 mmol), cesium carbonate (3.79 g, 11.631 mmol), Xantphos (0.67 g, 1.163 mmol) and Pd₂(dba)₃ (0.53 g 0.582 mmol) were added to a solution of 116-2 (1.6 g, 5.815 mmol) in dioxane (230 mL). The mixture was stirred at 110 °C for 4 hours, then diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated to dryness, and the residue was purified by column chromatography (mobile phases: ethyl acetate/petroleum ether 100/14 to 100/17) to give compound 116-3 (1.5 g, Purity: 90.6 %, Yield: 34.94%), ¹H NMR (400 MHz, DMSO-d6) δ 10.65 (s, 1H), 8.52 (d, J = 1.9 Hz, 1H), 8.21 (s, 1H), 8.16 (dt, J = 9.5, 1.9 Hz, 1H), 7.98 (dt, J = 8.4, 1.9 Hz, 1H), 7.80 (dd, J = 8.4, 1.9 Hz, 1H), (0.6 g, Purity: 94.4 %, Yield: 24.27 %) 7.80 (dd, J = 9.2, 0.8 Hz, 1H), 7.71 (dd, J = 9.2, 2.0 Hz, 1H). ESI: (m/z) = 402.1[M+H]⁺.

### Synthesis of compound 116-4

Lithium hydroxide (0.73 g, 17.441 mmol) was added to a mixture of 116-3 (0.7 g, 1.744 mmol) in tetrahydrofuran (110 mL) and water (37 mL), and the reaction mixture was stirred overnight at room temperature. The reaction mixture was concentrated to dryness and the residue was diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give the crude compound 116-4 (650 mg, Purity: 91.2 %, Yield: 88.44 %).¹H NMR (400 MHz, DMSO-d6) δ 10.64 (s, 1H), 8.56 (d, J = 1.9 Hz, 1H), 8.21 (d, J = 1.9 Hz, 1H), 8.21 (d, J = 1.9 Hz, 1H). 1H), 8.21 (d, J = 17.0 Hz, 2H), 8.11 - 8.01 (m, 1H), 7.95 (d, J = 8.5 Hz, 1H), 7.67 - 7.57 (m, 2H), ESI:(m/z) =383.1 [M-H]⁻.

### Synthesis of compound 116-5

116-4 (640 mg, 1.665 mmol), ammonium chloride (267.24 mg, 4.996 mmol), HOBt (337.56 mg, 2.498 mmol), EDCI (478.88 mg, 2.498 mmol), DMF (80 mL), and DIEA (645.75 mg. 4.996 mmol) were combined in a reaction vial. The reaction mixture was stirred at room temperature overnight. Upon completion, the reaction was quenched with water and diluted with ethyl acetate and water. The organic phase was separated and the aqueous phase was extracted with ethyl acetate. The organic phases were combined, washed with brine, and concentrated at reduced pressure to give the target compound 116-5 (575 mg, Purity: 94.4 %, Yield: 85.03 %) as a crude off-white solid directly, ¹H NMR(400 MHz, DMSO-d6) δ 10.74 (s, 1H), 8.40 (s, 1H), 8.22 (d, J = 9.2 Hz, 2H), 8.06 (d, J = 27.6 Hz, 1H), 7.90 (d, J = 12.9 Hz, 2H), 7.69 (s, 2H). ESI: (m/z) =384.1 [M+H]⁺ .

### Synthesis of Compound YL116

A solution of 116-5 (570 mg, 1.487 mmol) in Eaton's reagent (42 mL) was added 2-chloro-5-fluorobenzaldehyde (471.56 mg, 2.974 mmol), and the reaction mixture was stirred at 80 °C for 2 hours. The reaction mixture was added dropwise to an ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phases: dichloromethane/methanol 100/1 to 100/2) to give compound 116 (66 mg, Purity: 92.7 %, Yield: 7.85 %).¹H NMR (400 MHz, DMSO-d6) δ 10.36 (s, 1H), 8.45 (d, J = 2.2 Hz, 1H), 7.95 (dt, J = 8.5, 2.0 Hz, 1H), 7.81 (dt, J = 9.3, 1.9 Hz, 1H), 7.75 (s, 1H), 7.66 (dd, J = 8.9, 1.1 Hz, 1H), 7.31 (dd, J = 8.8, 5.2 Hz, 1H), 7.17 (d, J = 8.9 Hz, 1H), 7.10 - 7.04 (m, 1H), 6.87 (dd, J = 9.2, 3.1 Hz, 1H), 6.38 (s, 1H), ESI:(m/z) = 524.0 [M+H]⁺.

### Synthetic route of compound YL117

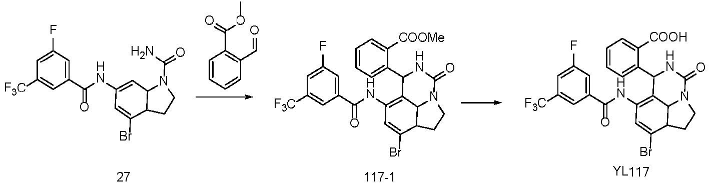

### Synthesis of compound 117-1

A solution of 27 (500 mg, 1.116 mmol) in Eaton's reagent (25 mL) was added methyl 2-formylbenzoate (366.25 mg, 2.231 mmol), and the reaction mixture was stirred at 80 °C for 2 hours. The reaction mixture was added dropwise to ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phases: dichloromethane/methanol 100/3 to 100/4) to give compound 117-1 (100 mg, Purity: 89.65 %, Yield: 13.52 %), ESI: (m/z) = 594.1 [M+H]⁺.

### Synthesis of compound YL117

Lithium hydroxide (63.75 mg, 1.519 mmol) was added to a mixture of compound YL117-1 (90 mg, 0.152 mmol) in tetrahydrofuran (50 mL) and water (17 mL), and the resulting mixture was stirred at room temperature for 2 days. The reaction mixture was concentrated to dryness and the residue was diluted with dichloromethane and water. The organic phase was separated and the aqueous phase was extracted with methylene chloride. The organic phases were combined, washed with water, brine, dried over anhydrous Na₂SO₄, filtered and the filtrate was evaporated to give Compound YL117 (45 mg, Purity: 92.17 %, Yield: 47.20 %).¹H NMR (400 MHz, DMSO-d6) δ 11.85 (s, 1H), 8.89 (s, 1H), 7.86 (d, J = 8.3 Hz, 1H), 7.81 (dd, J = 7.6, 1.5 Hz, 1H), 7.60 - 7.54 (m, 2H), 7.15 (td, J = 7.5, 1.3 Hz, 1H), 7.02 (td, J = 7.5, 1.6 Hz, 1H), 6.55 (s, 1H), 6.47 - 6.40 (m, 3H), 3.98 (ddd, J = 10.1, 7.8, 5.7 Hz, 2H), 3.10 (td, J = 8.1, 7.5, 3.5 Hz, 2H). ESI:(m/z) =577.9/579.9 [M+H]⁺ .

### Synthetic route of compound YL118

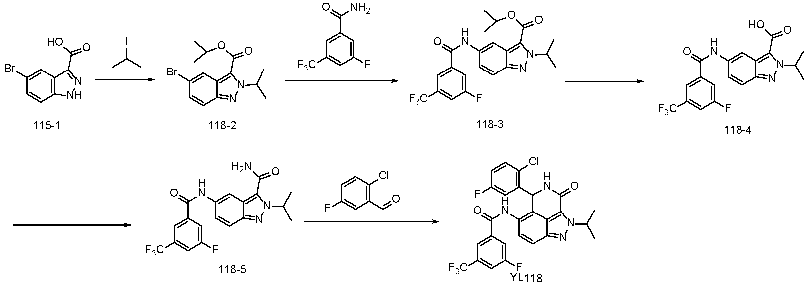

### Synthesis of compound 118-2

To a reaction vial were added 5-bromoindazole-3-carboxylic acid (115-1, 4 g, 16.595 mmol), potassium carbonate (6.88 g, 49.784 mmol), DMF (240 mL) and 2-iodopropane (11.28 g, 66.379 mmol). The mixture was stirred overnight at 60 °C for 24 h, then was reduced to room temperature. After removed most of the DMF by concentration at reduced pressure, it was diluted with ethyl acetate and water. The organic phase was separated and the aqueous phase was extracted with ethyl acetate. The organic phases were combined, washed with brine, concentrated at reduced pressure, and the resulting crude product was purified by automated chromatography (Biotage) (mobile phases: petroleum ether/ethyl acetate 100/12 to 100/20) to afford the target compound 118-2 (2.4 g, Purity:93.6 %, Yield:41.62 %), ¹H NMR (400 MHz, DMSO-d6) δ 8.10 (d, J = 1.8 Hz, 1H), 7.80 (d, J = 9.0 Hz, 1H), 7.47 (dd, J = 9.1, 1.9 Hz, 1H), 5.83 (p, J = 6.5 Hz, 1H), 5.25 (p, J = 6.3 Hz, 1H), 1.54 (d, J = 6.5 Hz, 6H), 1.42 (d, J = 6.2 Hz, 6H).

### Synthesis of compound 118-3

A solution of 118-2 (1.4 g, 4.305 mmol) in dioxane (175 mL) was added 115-2A (1.16 g, 5.596 mmol), cesium carbonate (2.81 g, 8.610 mmol), Xantphos (0.50 g, 0.869 mmol), Pd₂ (dba)₃ (0.39 g, 0.430 mmol), then the mixture was stirred at 110 °C overnight. The mixture was diluted with ethyl acetate and water, and the organic phase was separated, the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: ethyl acetate/petroleum ether 100/15 to 100/17) to give compound 118-3 (1.15 g, Purity: 98.8 %, Yield: 58.47 %).¹H NMR (400 MHz, DMSO-d6) δ 10.62 (s, 1H), 8.68 (d, J = 1.9 Hz, 1H), 8.20 - 8.12 (m, 2H), 7.96 (dt, J = 8.5, 1.9 Hz, 1H), 7.82 (d, J = 9.1 Hz, 1H), 7.62 (dd, J = 9.2, 2.0 Hz, 1H), 5.83 (hept, J = 6.5 Hz, 1H), 5.26 (hept, J = 6.2 Hz, 1H), 1.55 (d, J = 6.6 Hz, 6H), 1.44 (d, J = 6.2 Hz, 6H)., ESI:(m/z) =452.6 [M+H]⁺.

### Synthesis of compound 118-4

A solution of 118-3 (1.1 g, 2.437 mmol) in tetrahydrofuran (135 mL) and water (45 mL), was added lithium hydroxide (1.02 g, 24.368 mmol). The mixture was stirred at 50 °C overnight, then concentrated to dryness and the residue was diluted with dichloromethane and water. The organic phase was separated and the aqueous phase was extracted with dichloromethane. The organic phases were combined, washed with water, brine, dried over anhydrous Na₂SO₄, filtered and the filtrate was evaporated to give compound 118-4 (1 g, Purity: 91.6 %, Yield: 91.83 %) directly, ¹H NMR (400 MHz, DMSO-d6) δ 10.71 (s, 1H), 8.63 - 8.56 (m, 1H), 8.30 - 8.19 (m, 2H), 7.93 (dt, J = 8.5, 2.0 Hz, 1H), 7.60 (qd, J = 9.1, 1.4 Hz, 2H), 6.40 (p, J = 6.6 Hz, 1H), 1.48 (d, J = 6.6 Hz, 6H).. ESI:(m/z) =408.0 [M-H]⁻ .

### Synthesis of compound 118-5

To a reaction vial were added 118-4 (1 g, 2.443 mmol), ammonium chloride (0.39 g, 7.329 mmol), HOBt (0.50 g, 3.664 mmol), EDCI (0.70 g, 3.664 mmol), DMF (75 mL) and DIEA (0.95 g, 7.329 mmol). The reaction mixture was stirred at room temperature overnight. Upon completion, the reaction was quenched with water and diluted with ethyl acetate and water. The organic phase was separated and the aqueous phase was extracted with ethyl acetate. The organic phases were combined, washed with brine and concentrated at reduced pressure to give the target compound 118-5 (0.8 g, Purity: 93.4 %, Yield: 74.90 %) as a solid crude directly, ¹H NMR (400 MHz, DMSO-d6) δ 10.59 (s, 1H), 8.35 (d, J = 1.8 Hz, 1H), 8.22 (s, 1H), 8.17 (dt, J = 9.3, 2.0 Hz, 1H), 7.97 (dq, J = 7.3, 3.1, 2.6 Hz, 3H), 7.73 (d, J = 9.2 Hz, 1H), 7.63 (dd, J = 9.3, 1.9 Hz, 1H), 5.48 (hept, J = 6.5 Hz, 1H), 1.53 (d, J = 6.6 Hz, 6H). LC-MS (ESI): m/z 409.5 (M+H) ⁺.

### Synthesis of Compound YL118

A solution of 118-5 (555 mg, 1.359 mmol) in Eaton's reagent (30 mL) was added 2 - chloro-5-fluorobenzaldehyde (431.00 mg, 2.718 mmol), and the reaction mixture was stirred at 80 °C for 2 hours. The reaction mixture was added dropwise to an ice-cold saturated sodium bicarbonate solution and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phases: petroleum ether/ethyl acetate 100/35 to 100/40) to give the crude product (200 mg, Purity: 67.9 %). The resulting crude product was purified by prep-HPLC (Welch Xtimate C18, 21.2*250 mm, 10 um, water (10 mM ammonium bicarbonate)/acetonitrile, flow rate 30 mL/min, column temperature 25 °C, wavelength 254 nm), and finally lyophilized to obtain the solid compound YL118 (85 mg, Purity: 97.4 %, Yield: 11.10 %). ¹H NMR (400 MHz, DMSO-d6) δ 11.25 (s, 1H), 9.31 (d, J = 2.2 Hz, 1H), 8.84 (d, J = 8.2 Hz, 1H), 8.74 - 8.68 (m, 1H), 8.64 (s, 1H), 8.58 (d, J = 8.9 Hz, 1H), 8.20 (dd, J = 8.9, 5.1 Hz, 1H), 8.05 (d, J = 8.9 Hz, 1H), 7.96 (td, J = 8.4, 3.1 Hz, 1H), 7.76 (dd, J = 9.2, 3.1 Hz, 1H), 7.25 (s, 1H), 6.59 (p, J = 6.6 Hz, 1H), 4.22 (s, 6H). ESI:(m/z) = 549.2 [M+H]⁺.

### Synthetic route of compound YL119

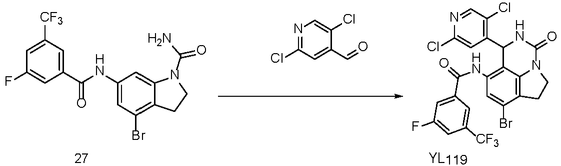

A solution of 27 (1.1 g, 2.465 mmol) in Eaton's reagent (30 mL) was added 2,5-dichloropyridine-4-carboxaldehyde (431.00 mg, 2.718 mmol), and the reaction mixture was stirred at 80 °C for 3 hours. The reaction mixture was added dropwise to an ice-cold saturated sodium bicarbonate solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: dichloromethane/methanol 100/4 to 100/6) to give Compound YL119 (670 mg, Purity: 69.7 %, Yield: 31.35 %), of which 400 mg was purified by prep-HPLC (Welch Xtimate C18, 21.2*250 mm, 10 um, water (10 mM ammonium bicarbonate)/acetonitrile, flow rate 30 mL/min, column temperature 25 °C, wavelength 254 nm), and finally lyophilized to obtain the solid Compound YL119 (145 mg, Purity: 92.8 %). ESI:(m/z) = 605.0 [M+H]⁺. ¹H NMR (400 MHz, DMSO-d6) δ 10.16 (s, 1H), 8.36 (s, 1H), 7.98 (d, J = 8.4 Hz, 1H), 7.90 (s, 1H), 7.85 (d, J = 9.4 Hz, 1H), 7.52 (d, J = 2.3 Hz, 1H), 7.19 (s, 1H), 6.94 (s, 1H), 6.01 (d, J = 2.2 Hz, 1H), 4.10 - 3.98 (m, 2H), 3.16 (ddd, J = 9.9, 7.0, 2.8 Hz, 2H).

### Synthetic route of compound YL120

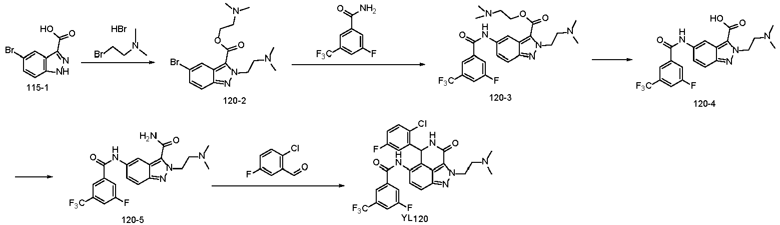

5-bromoindazole-3-carboxylic acid (115-1, 6.05 g, 25.100 mmol), cesium carbonate (32.71 g, 100.400 mmol), DMF (310 mL), and N, N-dimethylaminoethyl bromide hydrobromide (17.54 g, 75.299 mmol) were combined in a reaction vial. The mixture was stirred at room temperature overnight. After removal of most of the DMF by concentration at reduced pressure, the reaction mixture was diluted with ethyl acetate and water. The organic phase was separated and the aqueous phase was extracted with ethyl acetate. The organic phases were combined, washed with brine, concentrated at reduced pressure, and the resulting crude product was purified several times (mobile phase: dichloromethane/methanol 100/4 to 100/6) by an automated chromatography (Biotage) to give the target compound 120-2 (0.55 g, Purity:90.09 %, Yield:5.15 %). ¹H NMR (400 MHz, DMSO-d6) δ 8.29 (dd, J = 1.9, 0.7 Hz, 1H), 7.78 (dd, J = 9.0, 0.7 Hz, 1H), 7.48 (dd, J = 9.1, 1.9 Hz, 1H), 4.93 (t, J = 6.6 Hz, 2H), 4.46 (t, J = 5.6 Hz, 2H), 2.78 (t, J = 6.6 Hz, 2H), 2.68 (t, J = 5.5 Hz, 2H), 2.26 (s, 6H), 2.17 (s, 6H). ESI:(m/z) =383.2/ 385.2[M+H]⁺ , In addition, the NOESY results also confirmed the substitution of 2-indazole.

### Synthesis of compound 120-3

A solution of 120-2 (1015 mg, 2.648 mmol) in dioxane (130 mL) was added 115-2A (658.18 g, 3.178 mmol), cesium carbonate (1725.62 mg, 5.296 mmol), Xantphos (306.46 mg, 0.530 mmol) and Pd₂(dba)₃ (242.50 mg, 0.265 mmol), the reaction mixture was stirred at 110 °C for 3 h. Upon completion, the reaction was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: ethyl acetate/petroleum ether 100/10 to 100/20) to give compound 120-2 (285 mg, Purity: 93.9 %, Yield: 22.34 %).¹H NMR (400 MHz, DMSO-d6) δ 10.66 (s, 1H), 8.52 (d, J = 1.9 Hz, 1H), 8.24 - 8.13 (m, 2H), 7.98 (dt, J = 8.5, 2.0 Hz, 1H), 7.85 - 7.78 (m, 1H), 7.71 (dd, J = 9.2, 2.0 Hz, 1H), 4.94 (t, J = 6.7 Hz, 2H), 3.99 (s, 3H), 2.82 (t, J = 6.7 Hz, 2H), 2.22 (s, 6H), ESI:(m/z) =453.2 [M+H]⁺.

### Synthesis of compound 120-4

Lithium hydroxide (649.23 mg, 15.473 mmol) was added to a solution of 120-3 (700 mg, 1.547 mmol) in tetrahydrofuran (105 mL) and water (35 mL), and the reaction mixture was stirred for 3 hours at room temperature. Upon completion, the mixture was neutralized by adding hydrochloric acid slowly, then was concentrated to dryness and the residue was diluted with ethyl acetate and water. The organic phase was separated and the aqueous phase was extracted with ethyl acetate. The organic phases were combined, washed with brine and concentrated at reduced pressure to give the target compound 120-4 (340 mg, Purity: 72.7 %, Yield: 36.44 %).¹H NMR (400 MHz, DMSO-d6) δ 10.69 (s, 1H), 8.58 (d, J = 1.9 Hz, 1H), 8.24 (d, J = 8.7 Hz, 2H), 7.99 - 7.92 (m, 1H), 7.66 (dd, J = 9.2, 2.0 Hz, 1H), 7.59 (d, J = 9.2 Hz, 1H), 5.09 (t, J = 6.6 Hz, 2H), 4.18 (s, 1H), 3.00 (s, 2H), 2.34 (s, 6H). ESI:(m/z) = 437.2 [M-H]⁻.

### Synthesis of compound 120-5

120-4 (330 mg, 0.753 mmol), ammonium chloride (120.80 mg, 2.258 mmol), HATU (429.35 mg, 1.129 mmol), DMF (70 mL) and DIEA (291.89 mg, 2.258 mmol) were combined in a reaction vial. The reaction mixture was stirred at room temperature for 2 h. Upon completion, the reaction mixture was quenched with water and diluted with ethyl acetate and water. The organic phase was separated and the aqueous phase was extracted with ethyl acetate. The organic phases were combined, washed with brine and concentrated at reduced pressure to give the target compound 120-5 as a solid crude directly (380 mg, Purity: 88.7 %, Yield: 86.72 %). LC-MS (ESI): m/z 438.7 (M+H)⁺.

### Synthesis of Compound YL120

120-5 (350 mg, 0.800 mmol) was dissolved in Eaton's reagent (35 mL), 2-chloro-5-fluorobenzaldehyde (253.75 mg, 1.600 mmol) was added, and the reaction mixture was stirred at 80 °C for 3 hours. The reaction mixture was added dropwise to ice-cold saturated sodium bicarbonate solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: dichloromethane/methanol 100/14 to 100/16) to give Compound YL120 (120 mg, Purity: 42.4 %), and the resulting crude was prepared and purified (Welch Xtimate C18, 21.2*, 250 mm, 10 um, water). 250 mm, 10 um, water (10 mM ammonium bicarbonate)/acetonitrile, flow rate 30 mL/min, column temperature 25 °C, wavelength 254 nm), and finally lyophilized to obtain the solid target compound YL120 (14 mg, Purity: 92.9 %, Yield: 2.81 %). ESI:(m/z) =578.4 [M+H]⁺. ¹H NMR (400 MHz, DMSO-d6) δ 10.40 (s, 1H), 8.43 (d, J = 2.2 Hz, 1H), 7.95 (d, J = 8.5 Hz, 1H), 7.84 (d, J = 8.9 Hz, 1H), 7.76 (s, 1H), 7.67 (dd, J = 8.9, 1.1 Hz, 1H), 7.32 (dd, J = 8.8, 5.2 Hz, 1H), 7.18 (d, J = 8.9 Hz, 1H), 7.06 (td, J = 8.4, 3.1 Hz, 1H), 6.87 (dd, J = 9.2, 3.1 Hz, 1H), 6.42 - 6.35 (m, 1H), 4.97 (dt, J = 13.1, 6.5 Hz, 1H), 4.85 (dt, J = 13.0, 6.1 Hz, 1H), 2.89 (dq, J = 24.8, 6.3 Hz, 2H), 2.19 (s, 6H).

### Synthetic route of compound YL121

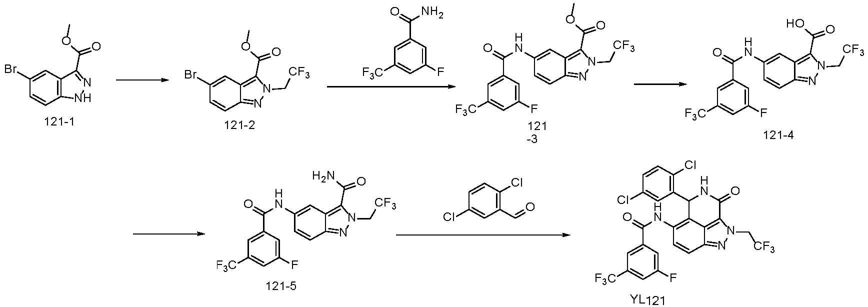

### Synthesis of compound 121-2

Methyl 5-bromo-1H-indazole-3-carboxylate (121-1, 3.10 g, 12.15 mmol), potassium carbonate (5.04 g, 36.46 mmol), DMF (110 mL) and 2,2,2-trifluoroethyl trifluoromethanesulfonate (5.64 g, 24.31 mmol) were combined in a reaction vial. The mixture was stirred at room temperature for 3 h and completed. After removal of most of the DMF by concentration at reduced pressure, the mixture was diluted with ethyl acetate and water. The organic phase was separated and the aqueous phase was extracted with ethyl acetate. The organic phases were combined, washed with brine, concentrated at reduced pressure, and the resulting crude product was purified by automated chromatography (Biotage) (mobile phase: petroleum ether/ethyl acetate 100/8 to 100/15) to obtain the target compound 121-2 (1.1 g, Purity:97.1 %, Yield:26.07%).¹H NMR (400 MHz, DMSO-d6) δ 8.24 - 8.18 (m, 1H), 7.87 (dd, J = 9.2, 0.7 Hz, 1H), 7.58 (dd, J = 9.2, 1.9 Hz, 1H), 5.86 (q, J = 8.7 Hz, 2H), 4.02 (s, 3H).

### Synthesis of compound 121-3

To a solution of 121-2 (1.1 g, 3.26 mmol) in dioxane (140 mL) was added 115-2A (811.07 mg, 3.92 mmol), cesium carbonate (2.13 g, 6.53 mmol), Xantphos (377.63 mg, 0.653 mmol), and Pd₂(dba)₃ (298.82 mg , 0.326 mmol), and the mixture was stirred at 110 °C for 2 h, then diluted with ethyl acetate and water. The organic phase was separated and the aqueous phase was extracted with ethyl acetate. The organic phases were combined, washed with water, brine, dried over anhydrous Na2SO4, filtered, and the filtrate was evaporated, and the residue was purified by column chromatography (mobile phase: methanol/dichloromethane 100/4 to 100/6) to afford compound 121-3 (1.2 g, Purity: 69.3 %, Yield: 55.01 %), ESI:(m/z) = 464.3[M+H]⁺.

### Synthesis of compound 121-4

A solution of 121-3 (1.2 g, 2.59 mmol) in tetrahydrofuran (165 mL) and water (55 mL) was added lithium hydroxide (620.24 mg, 25.90 mmol), and the reaction mixture was stirred at room temperature for 3 hours. Upon completion, the reaction was neutralized by adding diluted hydrochloric acid slowly, then the reaction mixture was concentrated to dryness and the residue was diluted with ethyl acetate and water. The organic phase was separated and the aqueous phase was extracted with ethyl acetate. The organic phases were combined, washed with brine and concentrated at reduced pressure to give the target compound 121-4 (800 mg, Purity: 77.6 %, Yield: 53.35 %). ¹H NMR (400 MHz, DMSO-d6) δ 10.64 (s, 1H), 8.64 (t, J = 1.5 Hz, 1H), 8.23 (s, 1H), 8.17 (dd, J = 9.2, 2.2 Hz, 1H), 8.01 - 7.93 (m, 1H), 7.67 (d, J = 1.4 Hz, 2H), 6.07 (q, J = 9.0 Hz, 2H), 4.03 (q, J = 7.1 Hz, 1H). LC-MS (ESI): m/z =448.0 [M-H]⁻.

### Synthesis of compound 121-5

121-4 (800 mg, 1.78 mmol), ammonium chloride (285.73 mg, 5.34 mmol), HATU (1015.58 mg, 2.67 mmol), DMF (70 mL) and DIEA (690.41 mg, 5.34 mmol) were added in a reaction vial. The reaction mixture was stirred at room temperature for 2 h. Upon completion, the reaction mixture was quenched with water and diluted with ethyl acetate and water. The organic phase was separated and the aqueous phase was extracted with ethyl acetate. The organic phases were combined, washed with brine and concentrated at reduced pressure to give a solid crude target compound 121-5 (800 mg, Purity: 88 %, Yield: 88.19 %) directly. LC-MS (ESI): m/z 449.2 (M+H) ⁺.

### Synthesis of Compound YL121

A solution of 121-5 (435 mg, 0.97 mmol) in Eaton's reagent (25 mL) was added 2-chloro-5-chlorobenzaldehyde ( 339.63 mg, 1.94 mmol), and the reaction mixture was stirred at 80 °C for 2 hours, then was added dropwise to ice-cold saturated sodium bicarbonate solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: dichloromethane/methanol 100/6 to 100/8), and the resulting crude product was purified by prep-HPLC (Welch Xtimate C18, 21.2*250mm, 10um, water (10mM ammonium bicarbonate)/acetonitrile, flow rate 30mL/min, column temperature 25°C, wavelength 254 nm), the prepared sample was purified again on a column (mobile phase: dichloromethane/methanol 100/3 to 100/5) and finally lyophilized to obtain a solid target compound YL121 (28 mg, Purity: 97.5 %, Yield: 4.65 %).¹H NMR (400 MHz, DMSO-d6) δ 10.51 (s, 1H), 8.63 (d, J = 2.2 Hz, 1H), 7.96 (d, J = 8.3 Hz, 1H), 7.84 (dd, J = 9.3, 2.3 Hz, 1H), 7.78 - 7.71 (m, 2H), 7.34 (d, J = 8.5 Hz, 1H), 7.26 (dd, J = 8.7, 2.9 Hz, 2H), 7.11 (d, J = 2.5 Hz, 1H), 6.36 (s, 1H), 5.75 (q, J = 8.7 Hz, 2H), ESI:(m/z) =605.1/607.1 [M+H]⁺.

### Synthetic route of compound YL122

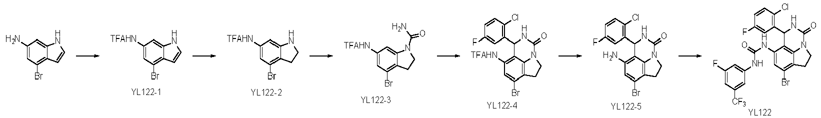

### Synthesis of compound YL122-1

4-Bromo-6-aminoindole (10 g, 47.380 mmol) was dissolved in dichloromethane (150 mL), to which was added triethylamine (19.757 mL, 142.140 mmol). After degassed and purged with N₂, the above mixture was added trifluoroacetic anhydride (19.90 g, 94.760 mmol) at 0 °C, and the reaction mixture was warmed to room temperature and stirred for 3 hours. The reaction mixture was diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated to give the compound YL122-1 (14.2 g, Purity 87.16%, Yield 82.15) directly. ESI:(m/z) = 308.7 [M+H]⁺.

### Synthesis of compound YL122-2

Sodium cyanoborohydride (5.81 g, 92.487 mmol) was added to a solution of YL122-1 (14.2 g, 46.244 mmol) dissolved in acetic acid (250 mL), and the reaction mixture was stirred for 16 h at room temperature. The reaction mixture was concentrated to dryness, and the residue was poured into ice-cold saturated aqueous sodium bicarbonate solution, and diluted with dichloromethane and water. The organic phase was separated and the aqueous phase was extracted with dichloromethane. The organic phases were combined, washed with water, brine, dried over anhydrous Na₂SO₄, filtered, and the filtrate was evaporated, and the residue was purified by column chromatography (mobile phases: petroleum ether/ethyl acetate 100/13 to 100/14) to give the compound YL122-2 (4.6 g, Purity 96 %, Yield 30.90 %). ESI:(m/z) = 310.6 [M+H]⁺.

### Synthesis of compound YL122-3

YL122-2 (4.5 g, 14.559 mmol) was dissolved in acetic acid (100 mL), and the mixture was cooled to 10 °C, then was added potassium cyanate (2.36 g, 29.118 mmol) and the reaction mixture was stirred at 10 °C for 0.5 hours. The reaction mixture was concentrated to dryness, and saturated sodium bicarbonate aqueous solution was added to adjust to basic, and filtered with water, the crude compound YL122-3 (5.15 g, Purity74.4 %, Yield 74.74 %) was obtained. ESI:(m/z) =353.8 [M+H]⁺ .

### Synthesis of compound YL122-4

2-chloro-5-fluorobenzaldehyde (4.59 g, 28.968 mmol) was added to a solution of YL122-3 (5.1 g, 14.484 mmol) in Eaton's reagent (45 mL), and the reaction mixture was stirred at 80 °C for 2 hours. Diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified by column chromatography (mobile phases: dichloromethane/methanol 100/3 to 100/5) to give the compound YL122-4 (6.8 g, Purity 70 %, Yield 66.71 %). ESI:(m/z) = 493.8 [M+H]⁺.

### Synthesis of compound YL122-5

Lithium hydroxide (10.14 g, 241.551 mmol) was added to a solution of YL122-4 (6.8 g, 9.662 mmol) in methanol (150 mL) and water (50 mL), and the mixture wasstirred at 80 °C for 7 hours. The reaction mixture was concentrated to dryness and the residue was diluted with dichloromethane and water. The organic phase was separated and the aqueous phase was extracted with dichloromethane. The organic phases were combined, washed with water, brine, dried over anhydrous Na2SO4, filtered and evaporated to give the crude compound YL122-5 (3.7 g, Purity 63.8 %, Yield 61.60 %). ESI:(m/z) = 397.8 [M+H]⁺ .

### Synthesis of compound YL122

YL122-5 (250 mg, 0.630 mmol), dichloromethane (20 mL), dioxane (15 mL) and N,N-diisopropylethylamine (1629.31 mg, 12.606 mmol) were combined in a reaction vial. Triphosgene (224.43 mg, dichloromethane solution) was added dropwise to the above mixture at 0°C. After addition, continued stirring At 0°C for 30 min. 3-Trifluorometh-5-fluoroaniline (338.69 mg, 5 mL of dichloromethane) was added dropwise to the above mixture. After addition, continued stirring in an ice-cold water bath for 2 hours. Diluted with dichloromethane and saturated aqueous sodium bicarbonate, the reaction mixture was separated the organic phase and extracted the aqueous phase with dichloromethane. The organic phases were combined, washed with brine, concentrated at reduced pressure, the resulting crude product was purified by automatic column (Biotage) (mobile phase: methanol/dichloromethane 100/4 to 100/5) to obtain the crude target compound as a light yellow solid, and the resulting crude product was purified by prep-HPLC (Boston pHlex ODS, 21.2*250mm, 10um, water (0.05% ammonium bicarbonate) / ethanol, water (0.05% ammonium bicarbonate) / acetonitrile), flow rate 30mL/min, column temperature 25°C, wavelength 254nm) to give a white solid as target compound YL122 (25 mg, Purity: 99.2 %, Yield: 6.54 %). LC-MS (ESI): m/z 602.9 (M+H)⁺. 1H NMR (400 MHz, DMSO-d6) δ 9.25 (s, 1H), 8.24 (s, 1H), 7.61 - 7.57 (m, 2H), 7.53 (dt, J = 11.3, 2.2 Hz, 1H), 7.42 (dd, J = 8.8, 5.2 Hz, 1H), 7.21 (d, J = 8.5 Hz, 1H), 7.12 (td, J = 8.4, 3.1 Hz, 1H), 6.97 (s, 1H), 6.90 (dd, J = 9.3, 3.1 Hz, 1H), 6.08 (d, J = 3.0 Hz, 1H), 4.04 - 3.96 (m, 2H), 3.18 - 3.10 (m, 2H).

### Synthetic route of compound YL123

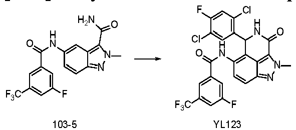

### Synthesis of compound YL123

2,5-dichloro-4-fluorobenzaldehyde (334.95 mg, 1.735 mmol) was added to a solution of 103-5 (330 mg, 0.868 mmol) in Eaton's reagent (20 mL), and the reaction mixture was stirred at 80 °C for 2 hours. The reaction mixture was added dropwise to an ice-cold saturated sodium bicarbonate solution and diluted with ethyl acetate and water. The organic phase was separated and the aqueous phase was extracted with ethyl acetate. The organic phases were combined, washed with water, brine, dried over anhydrous Na₂SO₄, filtered, and the filtrate was evaporated, and the residue was purified on a silica column (mobile phases: dichloromethane/methanol 100/3 to 100/5) to obtain the crude product (250 mg, Purity: 37.7 %), and the resulting crude product was purified by prep-HPLC (Welch Xtimate C18, 21.2*250mm, 10um, water (10mM ammonium bicarbonate)/acetonitrile, flow rate 30mL/min, column temperature 25°C, wavelength 254nm), and finally lyophilized to obtain target compound, YL123 (30 mg, Purity: 99.4 %, Yield: 6.19 %) a solid. ESI:(m/z) =555.0 [M+H]⁺.¹H NMR (400 MHz, DMSO-d6) δ 10.38 (s, 1H), 8.42 - 8.32 (m, 1H), 7.96 (d, J = 8.3 Hz, 1H), 7.84 (d, J = 9.1 Hz, 1H), 7.71 (s, 1H), 7.66 (dd, J = 8.8, 1.1 Hz, 1H), 7.56 (d, J = 9.0 Hz, 1H), 7.25 (d, J = 7.9 Hz, 1H), 7.16 (d, J = 8.9 Hz, 1H), 6.33 (s, 1H), 4.44 (s, 3H).

### Synthetic route of compound YL124

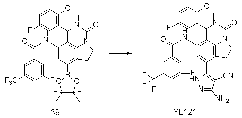

### Synthesis of compound YL124

39 (350 mg, 0.552 mmol), 5-amino-3-bromo-1H-pyrazole-4-carbonitrile (123.92 mg, 0.663 mmol), potassium carbonate (228.95 mg, 1.657 mmol), 1,1'-bis(diphenylphosphino)ferrocene palladium(II) dichloride (80.81 mg, 0.110 mmol), 1,4-dioxane (40 mL) and water (8 mL) were combined in a flask, and the mixture was stirred at 100 °C under nitrogen protection for 9 hours, then diluted with ethyl acetate and water. The organic phase was separated and the aqueous phase was extracted with ethyl acetate. The organic phases were combined, washed with brine, dried over anhydrous Na₂SO₄, filtered, and the filtrate was concentrated at reduced pressure, and purified by an automatic column (Biotage) (mobile phases: dichloromethane/methanol 100/8 to 100/9), and the resulting crude product was purified by prep-HPLC (Welch Xtimate C18, 21.2*250mm, 10um, water (10mM ammonium bicarbonate) / acetonitrile, flow rate 30mL), column temperature 25°C, wavelength 254 nm), and finally lyophilized to obtain the solid target compound YL124 (45 mg, Purity: 99.3 %, Yield: 13.18 %). ESI:(m/z) =614.1 [M+H]⁺. ¹H NMR (400 MHz, DMSO-d6) δ 12.19 (s, 1H), 10.27 (s, 1H), 7.96 (d, J = 8.8 Hz, 1H), 7.86 (d, J = 12.5 Hz, 2H), 7.51 (d, J = 2.5 Hz, 1H), 7.36 - 7.32 (m, 1H), 7.11 (dt, J = 11.5, 3.3 Hz, 2H), 6.89 (dd, J = 9.3, 3.1 Hz, 1H), 6.49 (s, 2H), 6.09 (d, J = 2.4 Hz, 1H), 4.05 - 3.95 (m, 2H), 3.49 - 3.36 (m, 2H).

### Synthetic route of compound YL125

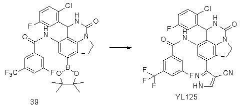

### Synthesis of compound YL125

To a reaction flask were added 39 (350 mg, 0.552 mmol), 3-bromo-1H-pyrazole-4-carbonitrile (113.97 mg, 0.663 mmol), potassium carbonate (228.95 mg, 1.657 mmol), 1,1'-bis(diphenylphosphino)ferrocene palladium(II) dichloride (80.81 mg, 0.110 mmol), 1,4-dioxane (35 mL) water (7 mL), and the mixture was stirred at 100 °C under nitrogen protection for 9 hours. The mixture was diluted with ethyl acetate and water. The organic phase was separated and the aqueous phase was extracted with ethyl acetate. The organic phases were combined, washed with brine, dried over anhydrous Na₂SO₄, filtered, and the filtrate was concentrated at reduced pressure, and purified by an automatic column (Biotage) (mobile phases: dichloromethane/methanol 100/5 to 100/6), and the resulting crude product was purified by prep-HPLC (Welch Xtimate C18, 21.2*250mm, 10um, water (10mM ammonium bicarbonate) / acetonitrile, flow rate 30mL, column temperature 25°C, wavelength 254 nm), and finally lyophilized to obtain the solid target compound YL125 (32 mg, Purity: 98.3 %, Yield: 9.51 %). ESI:(m/z) =599.1 [M+H]⁺.¹H NMR (400 MHz, DMSO-d6) δ 13.98 (s, 1H), 10.30 (s, 1H), 8.68 (s, 1H), 7.96 (d, J = 8.4 Hz, 1H), 7.87 (d, J = 10.4 Hz, 2H), 7.55 (d, J = 2.6 Hz, 1H), 7.34 (dd, J = 8.9, 5.1 Hz, 1H), 7.17 (s, 1H), 7.11 (td, J = 8.4, 3.0 Hz, 1H), 6.92 (dd, J = 9.2, 3.1 Hz, 1H), 6.13 (d, J = 2.5 Hz, 1H), 4.03 (dtd, J = 27.4, 10.4, 6.8 Hz, 2H), 3.52 - 3.38 (m, 2H).

### Synthetic route of compound YL126

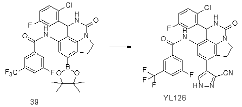

### Synthesis of compound YL126

To a reaction vial were added 39 (550 mg, 0.868 mmol), 3-bromo-5-methYL1H-pyrazole (223.87 mg, 1.302 mmol), potassium carbonate (359.78 mg, 2.603 mmol), 1,1'-bis(diphenylphosphino)ferrocene palladium(II) dichloride (126.99 mg, 0.174 mmol), 1,4-dioxane (40 mL) and water (8 mL), and the mixture was stirred at 100°C under nitrogen protection for 9 hours. Upon completion, the reaction mixture was added an appropriate amount of saturated aqueous ethylenediaminetetraacetic acid and stirred at room temperature for 1 h. The mixture was then diluted with ethyl acetate and water. The organic phase was separated and the aqueous phase was extracted with ethyl acetate. The organic phases were combined, washed with brine, dried over anhydrous Na₂SO₄, filtered, and the filtrate was concentrated at reduced pressure, and purified by an automatic column (Biotage) (mobile phases: dichloromethane/methanol 100/6 to 100/8), and the resulting crude product was purified by prep-HPLC (Welch Xtimate C18, 21.2*250mm, 10um, water (10mM ammonium bicarbonate) / acetonitrile, flow rate 30mL, column temperature 25°C, wavelength 254 nm), and finally lyophilized to obtain the solid target compound YL126 (95 mg, Purity: 97.5 %, Yield: 17.82 %). ESI:(m/z) =599.1 [M+H]⁺.¹H NMR (400 MHz, DMSO-d6) δ 10.28 (s, 1H), 8.38 (s, 1H), 7.99 - 7.92 (m, 1H), 7.93 - 7.84 (m, 2H), 7.56 (d, J = 2.6 Hz, 1H), 7.34 (dd, J = 8.9, 5.2 Hz, 1H), 7.11 (td, J = 8.4, 3.1 Hz, 1H), 7.00 - 6.87 (m, 2H), 6.12 (d, J = 2.5 Hz, 1H), 4.13 - 3.94 (m, 2H), 3.38 (td, J = 10.0, 5.1 Hz, 1H), 3.27 (ddd, J = 16.4, 10.4, 6.1 Hz, 1H).

### Synthetic route of compound YL127

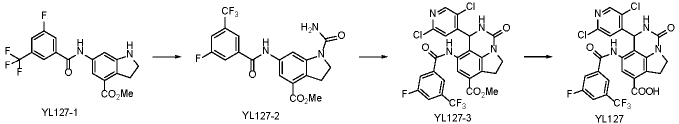

### Synthesis of compound YL127-2

Trimethylsilyl isocyanate (723.25 mg, 6.278 mmol) was added to a solution of YL127-1 (600 mg, 1.569 mmol) in isopropanol (120 mL) at room temperature, and the reaction mixture was stirred at room temperature for 24 h. Upon completion, the reaction was added excess ice-cold water, and a large amount of white solid was precipitated, and filtered, and the filter cake was firstly washed with brine, and then water, and finally evaporated the residual solvent to obtain the crude compound YL127-2 (560 mg, Purity: 71.7 %, Yield: 60.15 %) ESI:(m/z) = 426.1 [M+H]⁺.

### Synthesis of compound YL127-3

2,5-dichloro-4-pyridinecarboxaldehyde (463.44 mg, 2.633 mmol) was added to a solution of YL127-2 (560 mg, 1.317 mmol) in Eaton's reagent (30 mL), and the reaction mixture was stirred at 80 °Cfor 24 hours. The reaction mixture was added dropwise to ice-cold saturated sodium bicarbonate solution and diluted with ethyl acetate and water. The organic phase was separated and the aqueous phase was extracted with ethyl acetate. The organic phases were combined, washed with water, brine, dried over anhydrous Na2SO4, filtered, and the filtrate was evaporated, and the residue was purified on a column (mobile phases: dichloromethane/methanol 100/4 to 100/6) to give compound YL127-3 (240 mg, Purity: 76.5 %, Yield: 23.91 %). ESI:(m/z) = 583.1/585.1 [M+H]⁺.¹H NMR (400 MHz, DMSO-d6) δ 10.23 (s, 1H), 8.37 (s, 1H), 7.98 (d, J = 8.5 Hz, 2H), 7.92 (s, 1H), 7.53 (d, J = 2.2 Hz, 1H), 7.28 (s, 1H), 7.19 (s, 1H), 6.11 (d, J = 2.2 Hz, 1H), 4.03 (d, J = 7.1 Hz, 2H), 3.85 (s, 3H), 3.47 (dd, J = 9.9, 7.7 Hz, 2H).

### Synthesis of compound YL127

Lithium hydroxide (165.45 mg, 3.943 mmol) was added to a solution of YL127-3 (230 mg, 0.394 mmol) in tetrahydrofuran (48 mL) and water (16 mL), and the reaction mixture was stirred at room temperature for 3 hours. Upon completion, the reaction was added diluted hydrochloric acid to neutralize the reaction mixture, then the reaction mixture was concentrated to dryness and the residue was diluted with ethyl acetate and water. The organic phase was separated and the aqueous phase was extracted with ethyl acetate. The organic phases were combined, washed with brine, concentrated at reduced pressure, and purified by column chromatography (mobile phase: dichloromethane/methanol 100/8 to 100/10) to obtain the target compound YL127 (90 mg, Purity:92.6 %, Yield:37.13 %).¹H NMR (400 MHz, DMSO-d6) δ 10.34 (s, 1H), 8.35 (s, 1H), 7.95 (d, J = 7.0 Hz, 3H), 7.39 (d, J = 2.3 Hz, 1H), 7.21 (d, J = 25.8 Hz, 2H), 6.15 (s, 1H), 3.96 (q, J = 8.7 Hz, 2H), 3.47 (t, J = 9.1 Hz, 2H). ESI:(m/z) = 567.1/569.1 [M-H]⁻.

### Synthetic route of compound YL128

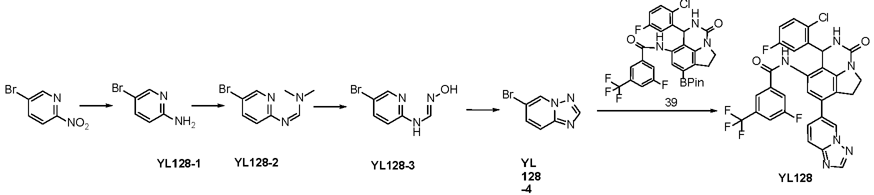

### Synthesis of compound YL128-1

Iron powder (13.75 g, 246.305 mmol) was added to a mixed solution of 5-bromo-2-nitropyridine (10.00 g, 49.261 mmol) and ammonium chloride (7.90 g, 147.783 mmol) in ethanol (100 mL) and water (20 mL) under nitrogen protection and the reaction mixture was stirred at 70 °C for 10 hours. The reaction mixture was diluted with dichloromethane, filtered through celite and the filtrate was concentrated to remove the organic solvent. The residue was then diluted with ethyl acetate and water, and the organic phase was separated, then dried over anhydrous sodium sulfate and filtered again. The filtrate was concentrated and purified by column chromatography (ethyl acetate/petroleum ether (v/v)=30%~90%) to afford YL128-1 (6.50 g, 76.29% yield). (ESI): m/z 173.0,175.0 [M+H] ⁺, tR=1.577min.

### Synthesis of compound YL128-3

DMF-DMA (N, N-dimethylformamide dimethyl acetal) (0.90 g, 7.514 mmol) was added to a solution of YL128-1 (1.00 g, 5.780 mmol) in isopropanol (2 mL) and the reaction mixture was stirred at 90 °C for 3 h. LCMS monitored the intermediate YL128-2 (ESI: m/z 227.8, 229.8 [M+H] ⁺, tR= 1.737min.). The reaction mixture was cooled to 50 °C, was added hydroxylamine hydrochloride (0.52 g, 7.514 mmol), and stirred at 50 °C for 16 hours. The reaction mixture was concentrated to give a crude product, which was purified by column chromatography (ethyl acetate/petroleum ether (v/v)=50%~90%) to give YL128-3 (1.00 g, 80.00% yield). (ESI): m/z 215.8, 217.8 [M+H]⁺, tR= 1.270min. ¹H NMR (400 MHz, DMSO-d6) δ10.19 (s, 1H), 9.56 (d, J = 9.9 Hz, 1H), 8.23 (dd, J= 2.5, 0.6 Hz, 1H), 7.80 (dd, J = 8.8, 2.5 Hz, 1H). 2.5 Hz, 1H), 7.77 (d, J = 9.9 Hz, 1H), 7.05 (dd, J = 8.9, 0.7 Hz, 1H).

### Synthesis of compound YL128-4

Trifluoroacetic anhydride (1.26 g, 6.017 mmol) was added dropwise to a solution of YL128-3 (1.00 g, 4.629 mmol) in tetrahydrofuran (20 mL), and the reaction mixture was stirred at 80 °C for 4 hours. The reaction mixture was quenched with saturated sodium bicarbonate solution to neutral, extracted with ethyl acetate and the organic phase was separated, dried over anhydrous sodium sulfate. Filtered, and the filtrate was concentrated and purified by gel column chromatography (ethyl acetate/petroleum ether (v/v)=0~50%) to give YL128-4 (700 mg, 76.09% yield). (ESI): m/z 197.8, 199.8[M+H]⁺, tR= 1.603min.

### Synthesis of compound YL128

Under nitrogen protection, Pd(dppf)Cl₂ (1,1'-bis(diphenylphosphino)ferrocene palladium(II) dichloride) (69 mg, 0.095 mmol) was added to a solution of YL128-4 (141 mg, 0.710 mmol),39 (300 mg, 0.473 mmol) and potassium carbonate (196 mg, 1.420 mmol) in 1,4 -dioxane (18 mL) and water (3 mL) in a mixed solution and the mixture was stirred at 90 °C for 2 hours. The reaction mixture was quenched with water and extracted with ethyl acetate to separate the organic phase, which was rapidly filtered through celite, and the filtrate was concentrated and purified by prep-HPLC (base) to give YL128 (70 mg, 23.66% yield). (ESI): m/z 625.0 [M+H]⁺, tR= 1.603min. ¹H NMR (400 MHz, DMSO-d₆)δ10.27 (s, 1H), 9.17 (s, 1H), 8.57 (s, 1H), 7.98 (s, 1H), 7.95 (d, J = 3.6 Hz, 2H), 7.92-7.83 (m, 2H), 7.62 (d, J = 2.7 Hz, 1H), 7.36 (dd, J = 8.9, 5.1 Hz, 1H), 7.12 (td, J = 8.4, 3.1 Hz, 1H), 7.03 (s, 1H), 6.95 (dd, J = 9.2, 3.1 Hz, 1H), 6.19 (d, J = 2.6 Hz, 1H), 4.06 (dd, J = 10.3, 5.7 Hz, 1H), 3.99 (q, J = 9.8 Hz, 1H), 3.51 (dt, J = 17.2, 9.0 Hz, 1H).

### Synthetic route of compound YL129

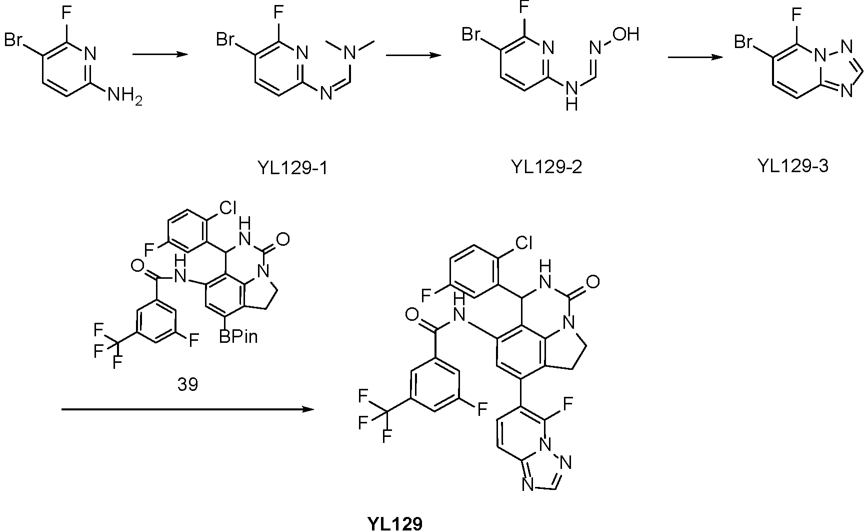

### Synthesis of compound YL129-2

DMF-DMA (N,N-dimethylformamide dimethyl acetal) (8.11 g, 68.063 mmol) was added to a solution of 5-bromo-6-fluoropyridin-2-amine (10.00 g, 52.356 mmol) in isopropanol (20 mL) and the reaction mixture was stirred at 90 °C for 3 h. TLC (ethyl acetate/petroleum ether (v/v) = 1:3) monitored intermediate state YL129-1, and the raw material disappeared. The reaction mixture was cooled to 50 °C, was added hydroxylamine hydrochloride (4.73 g, 68.063 mmol) and stirred at 50 °C for 16 hours. The reaction mixture was concentrated to obtain the crude product, which was purified by column chromatography (ethyl acetate/petroleum ether (v/v)=50%~90%) to obtain YL129-2 (11.00 g, 89.80% yield). (ESI): m/z 233.7, 235.8 [M+H]⁺; tR=1.891min. 1H NMR (400 MHz, DMSO-d6) δ 10.38 (s, 1H), 9.88 (d, J = 9.8 Hz, 1H), 8.01 (dd, J = 9.3, 8.5 Hz, 1H), 7.61 (d, J = 9.8 Hz , 1H), 6.94 (dd, J = 8.5, 1.2 Hz, 1H).

### Synthesis of compound YL129-3

YL129-2 (6.00 g, 25.638 mmol) was added to a solution of Eaton's Reagent (Phosphorus Pentoxide Methane Sulfonic Acid) (50 mL) and the reaction mixture was stirred at 80 °C for 2 hours. The reaction mixture was diluted with ethyl acetate and quenched to neutrality with saturated sodium bicarbonate solution in an ice-cold water bath. The organic phase was separated, concentrated and the residue was purified by column chromatography (ethyl acetate/petroleum ether (v/v)=0~30%) to give YL129-3 (3.00 g, 54.15% yield). (ESI): m/z 216.0, 218.0 [M+H]⁺; tR=1.605min. ¹HNMR (400 MHz, DMSO-d6) δ 8.64 (s, 1H), 7.99 (dd, J = 9.4, 7.2 Hz, 1H), 7.76 (d, J = 9.4 Hz, 1H).

### Synthesis of compound YL129

Pd(dppf)Cl₂ (69 mg, 0.095 mmol) was added to a solution of YL129-3 (23 mg, 0.118 mmol) 39 (300 mg, 0.473 mmol) and potassium carbonate (131 mg, 0.947 mmol) in a mixed solution of 1,4- dioxane (15 mL) and water (3 mL) under nitrogen protection. The reaction mixture was stirred at 90 °C for 2 hours. The reaction was quenched with water, extracted with ethyl acetate, the organic phase was separated, and was rapidly filtered through celite, and the filtrate was concentrated and purified by prep-HPLC (basic) to give YL129 (40 mg, 13.14% yield). (ESI): m/z 643.1 [M+H]⁺; tR=4.664min. ¹H NMR (400 MHz, DMSO-d₆) δ 10.28 (s, 1H), 8.68 (s, 1H), 7.96 (d, J = 8.4 Hz, 1H), 7.90 (d, J = 6.5 Hz, 2H), 7.89 - 7.84 (m, 2H), 7.61 (d, J = 2.6 Hz, 1H), 7.36 (dd, J = 8.9, 5.1 Hz, 1H), 7.13 (td, J = 8.4, 3.0 Hz, 1H), 7.01 - 6.95 (m, 1H), 6.94 (s, 1H), 6.24 - 6.12 (m, 1H), 4.11 - 4.04 (m, 1H), 4.04 - 3.95 (m, 1H), 3.25 (t, J = 8.8 Hz,2H).

### Synthetic route of compound YL130

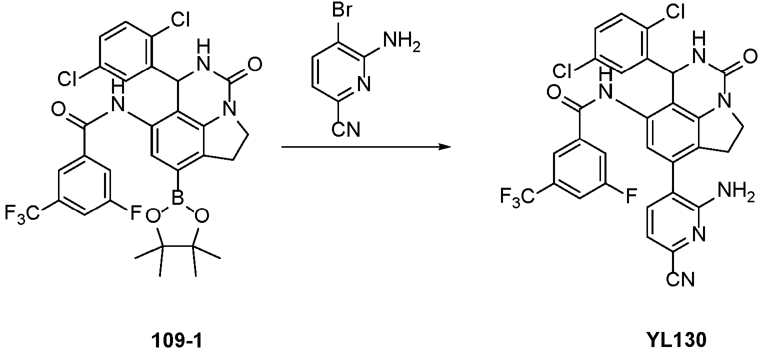

### Synthesis of compound YL130

Dioxyhexacyclohexane (2 mL) and water (0.4 mL) were added to a reaction flask charged with 109-1 (170 mg, 0.261 mmol), 6-amino-5-bromopicolinonitrile (52 mg, mmol), potassium carbonate (108 mg, 0.784 mmol) and Pd(dppf)Cl₂ (19 mg, 0.026 mmol). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, and saturated aqueous sodium bicarbonate, dried over anhydrous Na₂SO₄, filtered, and the filtrate was evaporated to dryness, and the residue was purified by prep-HPLC to obtain YL130 (53 mg). LCMS (ESI): m/z 641.4 (M+H) ⁺; ¹H NMR (400 MHz, DMSO-d6) δ 10.20 (s, 1H), 7.97 - 7.91 (m, 1H), 7.84 (d, J = 11.0 Hz, 2H), 7.50 (d, J = 7.4 Hz, 1H), 7.45 (d, J = 2.4 Hz, 1H), 7.33 (d, J = 8.6 Hz, 1H), 7.28 (dd, J = 8.6, 2.4 Hz, 1H), 7.21 - 7.17 (m, 2H), 6.65 (s, 1H), 6.43 (s, 2H), 6.15 (d, J = 2.4 Hz, 1H), 3.99 (td, J = 8.6, 4.1 Hz, 2H), 3.11 - 3.00 (m, 2H).

### Synthetic route of compound YL131

### Synthesis of compound YL131

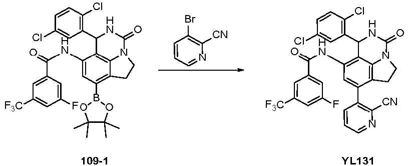

109-1 (185 mg, 0.285 mmol), 3-bromo-2-cyanopyridine (52 mg, 0.285 mmol), potassium carbonate (118 mg, 0.854 mmol), and Pd(dppf)Cl₂ (20.8 mg, 0.028 mmol) were added to a reaction flask charged with dioxane (2 mL) and water (0.4 mL). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated to dryness, and the residue was purified by prep-HPLC to obtain YL131 (61 mg). LCMS (ESI): m/z 626.4 (M+H) ⁺; ¹H NMR (400 MHz, DMSO-d6) δ 10.30 (s, 1H), 8.80 (dd, J = 4.7, 1.6 Hz, 1H), 8.16 (dd, J = 8.0, 1.6 Hz, 1H), 7.95 (d, J = 8.2 Hz, 1H), 7.92 - 7.82 (m, 3H), 7.54 (d, J = 2.4 Hz, 1H), 7.37 (d, J = 8.6 Hz, 1H), 7.30 (dd, J = 8.6, 2.5 Hz, 1H), 7.10 (d, J = 2.5 Hz, 1H), 6.85 (s, 1H), 6.14 (d, J = 2.4 Hz, 1H), 4.10 - 3.95 (m, 2H), 3.26 - 3.07 (m, 2H).

### Synthetic route of compound YL132

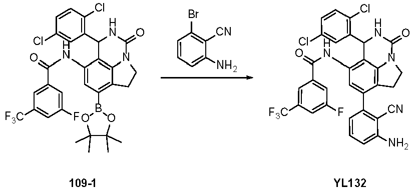

### Synthesis of compound YL132

109-1 (185 mg, 0.285 mmol), 2-amino-6-bromobenzonitrile (56 mg, 0.285 mmol), potassium carbonate (118 mg, 0.854 mmol), and Pd(dppf)Cl₂ (20.8 mg, 0.028 mmol) in a reaction flask were added dioxane (2 mL) and water (0.4 mL). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated to dryness, and the residue was purified by prep-HPLC to obtain YL132 (72 mg). LCMS (ESI): m/z 640.4 (M+H) ⁺; ¹H NMR (400 MHz, DMSO-d6) δ 10.30 (s, 1H), 8.80 (dd, J = 4.7, 1.6 Hz, 1H), 8.16 (dd, J = 8.0, 1.6 Hz, 1H), 7.95 (d, J = 8.2 Hz, 1H), 7.92 - 7.82 (m, 3H), 7.54 (d, J = 2.4 Hz, 1H), 7.37 (d, J = 8.6 Hz, 1H), 7.30 (dd, J = 8.6, 2.5 Hz, 1H), 7.10 (d, J = 2.5 Hz, 1H), 6.85 (s, 1H), 6.14 (d, J = 2.4 Hz, 1H), 4.11 - 3.95 (m, 2H), 3.27 - 3.07 (m, 2H).

### Synthetic route of compound YL133

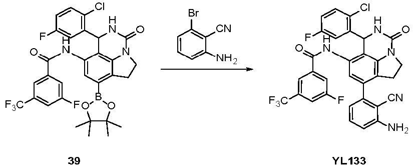

### Synthesis of compound YL133

39 (200 mg, 0.316 mmol), 2-amino-6-bromobenzonitrile (62 mg, 0.316 mmol), potassium carbonate (131 mg, 0.947 mmol), and Pd(dppf)Cl₂ (23 mg, 0.032 mmol) in a reaction flask were added dioxyhexacyclohexane (2 mL) and water (0.4 mL). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated to dryness, and the residue was purified by prep-HPLC to obtain YL133 (101 mg). LCMS (ESI): m/z 624.0 (M+H) ⁺; ¹H NMR (400 MHz, DMSO-d6) δ 10.24 (s, 1H), 7.99 - 7.82 (m, 3H), 7.57 (d, J = 2.7 Hz, 1H), 7.41 - 7.29 (m, 2H), 7.12 (td, J = 8.4, 3.1 Hz, 1H), 6.90 (dd, J = 9.2, 3.1 Hz, 1H), 6.83 (d, J = 8.4 Hz, 1H), 6.71 (s, 1H), 6.60 (d, J = 7.4 Hz, 1H), 6.16 (s, 3H), 4.08 - 3.92 (m, 2H), 3.23 - 3.03 (m, 2H).

### Synthetic route of compound YL134

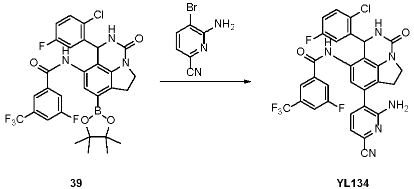

### Synthesis of compound YL134

39 (200 mg, 0.316 mmol), 6-amino-5-bromopicolinonitrile (62.5 mg, 0.316 mmol), potassium carbonate (131 mg, 0.947 mmol), and Pd(dppf)Cl₂ (23 mg, 0.032 mmol) in a reaction flask were added dioxyhexane (2 mL) and water (0.4 mL). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, saturated aqueous sodium bicarbonate solution, dried over anhydrous Na₂SO₄, filtered, and the filtrate was evaporated to dryness, and the residue was purified by prep-HPLC to obtain YL134 (87 mg). LCMS (ESI): m/z 625.0 (M+H) ⁺; ¹H NMR (400 MHz, DMSO-d6) δ 10.21 (s, 1H), 7.94 (d, J = 8.4 Hz, 1H), 7.84 (d, J = 13.4 Hz, 2H), 7.54 - 7.47 (m, 2H), 7.33 (dd, J = 8.8, 5.2 Hz, 1H), 7.19 (d, J = 7.4 Hz, 1H), 7.11 (td, J = 8.4, 3.1 Hz, 1H), 7.07 - 7.02 (m, 1H), 6.66 (s, 1H), 6.47 (s, 2H), 6.21 - 6.16 (m, 1H), 3.99 (td, J = 9.9, 7.0 Hz, 2H), 3.15 - 2.96 (m, 2H).

### Synthetic route of compound YL135

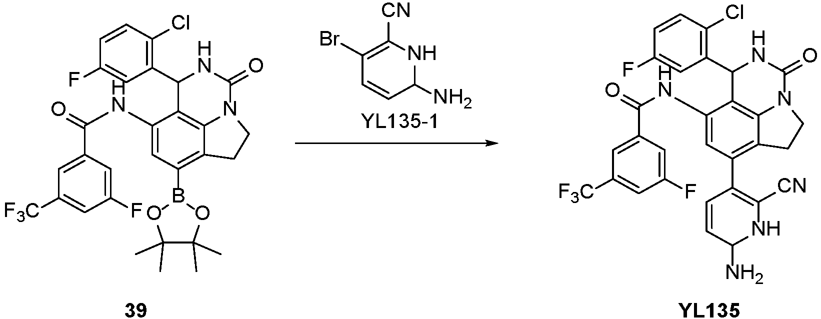

### Synthesis of compound YL135

39 (200 mg, 0.316 mmol), YL135-1 (63 mg, 0.316 mmol), potassium carbonate (131 mg, 0.947 mmol) and Pd(dppf)Cl₂ (23 mg, 0.032 mmol) in a reaction flask were added dioxyhexacyclohexane (2 mL) and water (0.4 mL). After degassed and purged with nitrogen, the reaction mixture was stirred at 100 °C for 1 hour, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered and evaporated to dryness, and the residue was purified by prep-HPLC to obtain YL135 (90 mg). LCMS (ESI): m/z 625.0 (M+H) ⁺;¹H NMR (400 MHz, DMSO-d6) δ 10.25 (s, 1H), 7.94 (d, J = 8.5 Hz, 1H), 7.88 (d, J = 17.9 Hz, 2H), 7.63 (d, J = 8.8 Hz, 1H), 7.57 (d, J = 2.7 Hz, 1H), 7.35 (dd, J = 8.8, 5.2 Hz, 1H), 7.11 (td, J = 8.4, 3.1 Hz, 1H), 6.90 (dd, J = 9.1, 3.1 Hz, 1H), 6.80 (d, J = 8.7 Hz, 1H), 6.75 (d, J = 2.9 Hz, 3H), 6.15 (d, J = 2.5 Hz, 1H), 4.09 - 3.92 (m, 2H), 3.25 - 3.07 (m, 2H).

### Synthetic route of compound YL136

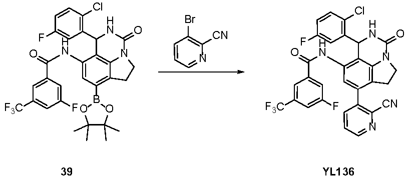

### Synthesis of compound YL136

39 (200 mg, 0.316 mmol), 3-bromo-2-cyanopyridine (58 mg, 0.316 mmol), potassium carbonate (131 mg, 0.947 mmol), and Pd(dppf)Cl₂ (23 mg, 0.032 mmol) in a reaction flask were added dioxyhexacyclohexane (2 mL) and water (0.4 mL). After degassed and purged with nitrogen, the reaction mixture was stirred at 100 °C for 1 h, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated to dryness, and the residue was purified by prep-HPLC to obtain YL136 (102 mg). LCMS (ESI): m/z 609.9 (M+H) ⁺;¹H NMR (400 MHz, DMSO-d6) δ 10.31 (s, 1H), 8.81 (dd, J = 4.7, 1.6 Hz, 1H), 8.16 (dd, J = 8.1, 1.6 Hz, 1H), 7.95 (d, J = 8.4 Hz, 1H), 7.92 - 7.82 (m, 3H), 7.63 (d, J = 2.6 Hz, 1H), 7.37 (dd, J = 8.9, 5.2 Hz, 1H), 7.13 (td, J = 8.4, 3.1 Hz, 1H), 6.93 (dd, J = 9.2, 3.1 Hz, 1H), 6.88 (s, 1H), 6.22 - 6.12 (m, 1H), 4.12 - 3.92 (m, 2H), 3.28 - 3.09 (m, 2H).

By SFC resolution, YL136-P1(YL16609)(36.4 mg) and YL136-P2(YL16610) (38.7 mg) were obtained.

### Synthetic route of compound YL137

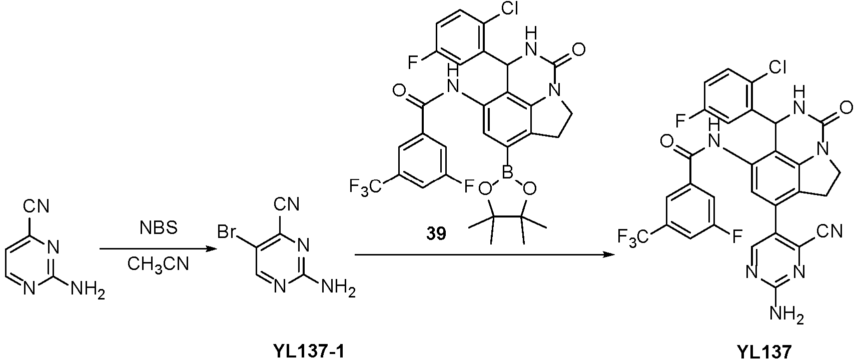

### Synthesis of compound YL137-1

N-bromosuccinimide (2.22 g, 12.49 mmol) was added to a solution of 2-Amino-4-cyanopyrimidine (1 g, 8.326 mmol) in acetonitrile (15 mL), and the reaction mixture was stirred at room temperature overnight. The reaction mixture was concentrated to dryness, the residue was pulped with ethyl acetate and filtered to give YL137-1 (1.5 g, 90.36%) as a white solid. LCMS (ESI): m/z 199.0 (M+H) ⁺; ¹H NMR (400 MHz, DMSO-d6) δ 8.65 (s, 1H), 7.52 (s, 2H).

### Synthesis of compound YL137

39 (200 mg, 0.316 mmol), YL137-1 (63 mg, 0.316 mmol), potassium carbonate (131 mg, 0.947 mmol) and Pd(dppf)Cl₂ (23 mg, 0.032 mmol) in a reaction flask were added dioxane (2 mL) and water (0.4 mL). After degassed and purged with nitrogen, the reaction mixture was stirred at 100 °C for 1 hour, then diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated, and the residue was purified by prep-HPLC to obtain YL137 (91 mg, 45.5%). LCMS (ESI): m/z 626.0 (M+H) ⁺;¹H NMR (400 MHz, DMSO-d6) δ 10.28 (s, 1H), 8.63 (s, 1H), 7.95 (d, J = 8.4 Hz, 1H), 7.93 - 7.85 (m, 2H), 7.59 (d, J = 2.6 Hz, 1H), 7.48 (s, 2H), 7.35 (dd, J = 8.9, 5.1 Hz, 1H), 7.12 (td, J = 8.4, 3.1 Hz, 1H), 6.90 (dd, J = 9.1, 3.1 Hz, 1H), 6.82 (s, 1H), 6.17 - 6.11 (m, 1H), 4.10 - 3.92 (m, 2H), 3.29 - 3.14 (m, 2H).

### Synthetic route of compound YL138

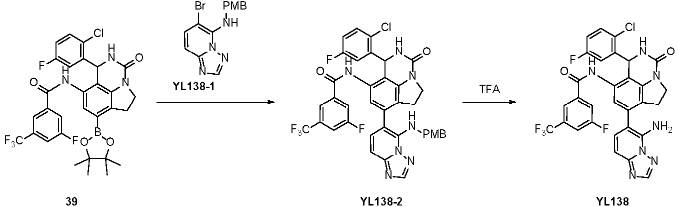

### Synthesis of compound YL138-2

39 (300 mg, 0.511 mmol), YL138-1 (170 mg, 0.511 mmol), potassium carbonate (212 mg, 1.534 mmol) and Pd(dppf)Cl₂ (37.4 mg, 0.051 mmol) in a reaction flask were added dioxyhexacyclohexane (5 mL) and water (1 mL). After degassed and purged with nitrogen, the reaction mixture was stirred at 100 °C for 2 h, then was diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, and the filtrate was evaporated, and the residue was purified by column chromatography (dichloromethane/methanol = 10:1 ) to give YL138-2 (350 mg, 89.74%). LCMS (ESI): m/z 760 (M+H) ⁺.

### Synthesis of compound YL138

YL138-2 (300 mg, 0.395 mmol) in a reaction vial was added trifluoroacetic acid (5 mL), and the reaction mixture was stirred at room temperature for 2 h. The reaction mixture was adjusted neutral with saturated aqueous sodium bicarbonate and the aqueous phase was extracted with ethyl acetate. The organic phases were combined, washed with water, washed with saturated aqueous sodium bicarbonate solution, dried over anhydrous Na₂SO₄, filtered, and the filtrate was evaporated, and the residue was purified by prep-HPLC to obtain YL138 (100 mg, 40%). LCMS (ESI): m/z 640.0 (M+H) ⁺; ¹H NMR (400 MHz, DMSO-d6) δ 10.22 (s, 1H), 8.46 (s, 1H), 7.94 (d, J = 8.4 Hz, 1H), 7.86 (d, J = 14.5 Hz, 2H), 7.52 (d, J = 2.7 Hz, 1H), 7.43 (d, J = 8.8 Hz, 1H), 7.34 (dd, J = 8.8, 5.2 Hz, 1H), 7.16 - 7.09 (m, 1H), 7.05 (d, J = 8.7 Hz, 2H), 6.91 (s, 2H), 6.74 (s, 1H), 6.20 (dd, J = 2.7, 1.3 Hz, 1H), 4.01 (td, J = 10.7, 10.2, 6.9 Hz, 2H), 3.18 - 2.98 (m, 2H).

### Synthetic route of compound YL139 and YL 140

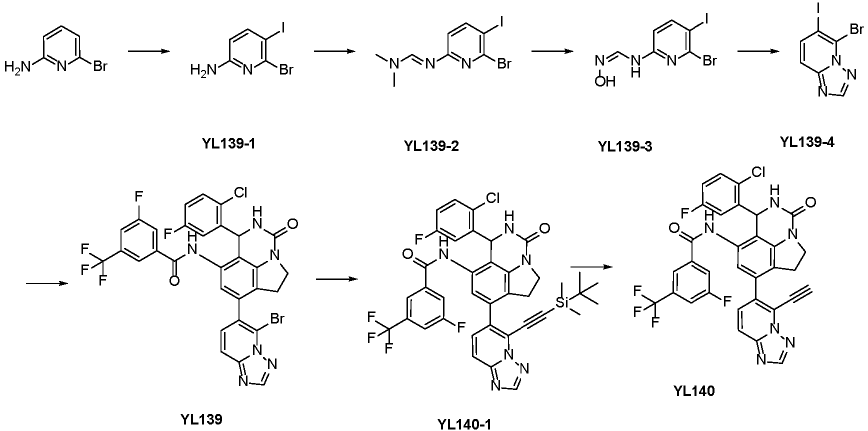

### Synthesis of compound YL139-1

N-iodosuccinimide (4949.49 mg, 21.999 mmol) was added to a 100 mL three-necked flask charged with 6-bromo-2-aminopyridine (3460 mg, 19.999 mmol) and N,N-dimethylformamide (20 mL) at room temperature. The reaction mixture was stirred under nitrogen protection at room temperature for 18 h. LCMS showed the reaction was successful, to which a large amount of water was added, filtered, the filter cake was washed with water, and the filter cake was dried to give YL139-1 (3280 mg, 10.973 mmol, 54.87%). LC-MS (ESI): m/z 300.7 (M+H)⁺;

### Synthesis of compound YL139-2

To a 100 mL three-necked flask was sequentially added YL139-1 (3280 mg, 10.973 mmol), isopropanol (35 mL) and N,N-dimethylformamide dimethyl acetal (1307.57 mg, 10.973 mmol). The reaction mixture was stirred under nitrogen protection at 80 °C for 3 h. LCMS showed the reaction was successful. The reaction mixture was used directly in the next step. LC-MS (ESI): m/z 355.6 (M+H)⁺;

### Synthesis of compound YL139-3

To a 100 mL three-necked flask were added YL139-2 (3884.40 mg, 10.973 mmol), hydroxylamine hydrochloride (1067.54 mg, 15.362 mmol) and isopropanol (35 mL) sequentially. The reaction mixture was stirred under nitrogen protection at 50 °C for 2 h. LCMS showed that the reaction was complete. Direct filtered the reaction mixture to give YL139-3 (3420 mg, 10.002 mmol, 91.15%).LC-MS (ESI): m/z 343.7 (M+H)⁺;

### Synthesis of compound YL139-4

To a 100 mL three-necked flask were sequentially added YL139-3 (3420 mg, 10.002 mmol) and tetrahydrofuran (35 mL). Cooled to 0°C, the above mixture was added trifluoroacetic anhydride (4201.46 mg, 20.004 mmol) slowly. The reaction mixture was stirred at 60°C for 18 h. LCMS showed that the reaction was complete, aqueous sodium bicarbonate and ethyl acetate were added to the reaction mixture, and the organic phase was concentrated by column chromatography (petroleum ether: ethyl acetate = 15:1-8:1) to give YL139-4 (1600 mg, 4.939 mmol, 49.38%). LC-MS (ESI): m/z 325.7 (M +H)⁺;

### Synthesis of compound YL139

To a 100 mL three-necked flask were sequentially added 39 (1141 mg, 1.800 mmol), YL139-4 (583.13 mg, 1.800 mmol), potassium carbonate (746.37 mg, 5.401 mmol), 1,1-bis(diphenylphosphino)diperylferrocene dichloropalladium (131.72 mg, 0.180 mmol), 1, 4-dioxohexacyclo(diphenylphosphine)dichloropalladium (131.72 mg, 0.180 mmol), 4-dioxane (30 mL) and water (6 mL). The reaction mixture was stirred under nitrogen protection at 75°C for 2 h. LCMS showed that the reaction was successful, and water and ethyl acetate were added to the reaction mixture, and the organic phase was concentrated and the residue was purified by column chromatography (petroleum ether: ethyl acetate = 2:1-1:1) to give YL139 (585 mg, 0.831 mmol, 46.17%). LC-MS (ESI): m/z 705.0 (M+H)⁺; ¹H NMR (400 MHz, DMSO-d6) δ 10.28 (s, 1H), 8.67 (s, 1H), 7.96 (t, J = 10.5 Hz, 2H), 7.87 (d, J = 13.1 Hz, 2H), 7.72 (d, J = 9.0 Hz, 1H), 7.59 (d, J =2.6 Hz, 1H), 7.37 (dd, J = 8.9, 5.1 Hz, 1H), 7.13 (td, J = 8.4, 3.1 Hz, 1H), 6.93 (dd, J = 9.2, 3.1 Hz, 1H), 6.79 (s, 1H), 6.17 (d, J = 2.5 Hz, 1H), 4.16-3.91 (m, 2H), 3.09 (tt, J = 16.5, 9.3 Hz, 2H).

### Synthesis of compound YL140-1

To a 10 mL microwave tube were sequentially added YL139 (135 mg, 0.192 mmol), (tert-butyldimethylsilyl)acetylene (53.82 mg, 0.384 mmol), cuprous iodide (73.06 mg, 0.384 mmol), potassium iodide (31.84 mg, 0.192 mmol), diisopropylamine (0.135 mL, 0.959 mmol), tetrakis(triphenylphosphine)palladium (44.33 mg, 0.038 mmol) and N,N-dimethylformamide (6 mL). The reaction mixture was stirred under nitrogen protection at 100 °C for 1 h. LCMS showed it was successful, water and ethyl acetate were added to the reaction mixture and the organic phase was concentrated and used directly in the next step. LC-MS (ESI): m/z 763.2 (M+H)⁺;

### Synthesis of compound YL140

To a 50 mL three-necked flask were added YL140-1 (146 mg, 0.191 mmol) and tetrahydrofuran (5 mL) sequentially. Under nitrogen protection, the above mixture was added tetrabutylammonium fluoride (0.287 mL, 0.287 mmol). The reaction mixture was stirred at room temperature for 1 h. LCMS the reaction was successful, water and ethyl acetate were added to the reaction mixture and the organic phase was concentrated and the residue was purified by prep-HPLC to give YL140 (21 mg, 0.032 mmol, 16.92%). LC-MS (ESI): m/z 649.1 (M+H)⁺;¹H NMR (400 MHz, DMSO-d₆) δ 10.27 (s, 1H), 8.64 (s, 1H), 8.02 (d, J = 9.2 Hz, 1H), 7.95 (d, J = 8.4 Hz, 1H), 7.88 (d, J = 14.4 Hz, 2H), 7.73 (d, J =9.1 Hz, 1H), 7.59 (d, J = 2.6 Hz, 1H), 7.36 (dd, J = 8.9, 5.1 Hz, 1H), 7.13 (td, J = 8.4, 3.1 Hz, 1H), 6.95-6.84 (m, 2H), 6.18 (d, J = 2.6 Hz, 1H), 5.20 (s,1H), 4.20-3.75 (m, 2H), 3.28-2.93 (m, 2H).

### Synthesis of compound YL141

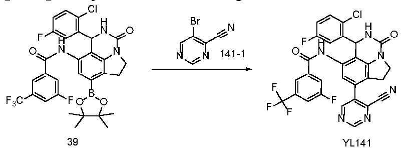

To a reaction vial was added compound 39 (300 mg, 0.473 mmol), 5-bromopyrimidine-4-carbonitrile (113.22 mg, 0.615 mmol), sodium carbonate (150.50 mg, 1.420 mmol), 1,1'-bis(diphenylphosphino)ferrocene palladium(II) dichloride (69.27 mg, 0.095 (69.27 mg, 0.095 mmol), tetrakis(triphenylphosphine)palladium (109.39 mg, 0.095 mmol), N,N-dimethylformamide (15 mL) and water (3 mL), and the mixture was heated at 130 °C with stirring under nitrogen protection for 1.5 hours. Upon completion, the mixture was concentrated directly under reduced pressure and purified by automatic column chromatography (Biotage) (mobile phase: dichloromethane/methanol 100/4 to 100/5), and the resulting crude product was purified (Welch Xtimate C18, 21.2*250 mm, 10 um, water (10 mM ammonium bicarbonate)/acetonitrile, flow rate of 30 mL/min, column temperature 25 °C, wavelength 254 nm), and finally lyophilized. 254 nm), and finally lyophilized to obtain the solid target compound YL141 (25 mg, Purity:94.3%, Yield:8.15 %).ESI:(m/z) =611.1 [M+H]⁺. ¹H NMR (400 MHz, DMSO-d₆) δ 10.35 (s, 1H), 9.45 (s, 1H), 9.30 (s, 1H), 7.99 - 7.87 (m, 3H), 7.65 (d, J = 2.6 Hz, 1H), 7.37 (dd, J = 8.9, 5.1 Hz, 1H), 7.13 (td, J = 8.5, 3.0 Hz, 1H), 7.03 - 6.90 (m, 2H), 6.21 - 6.13 (m, 1H), 4.03 (dt, J = 28.7, 8.6 Hz, 2H), 3.25 (dd, J = 18.0, 8.4 Hz, 2H).

### Synthesis of compound YL142 (YL142-P1& YL142-P2)

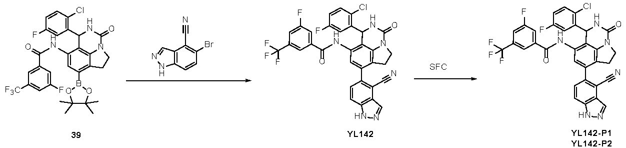

Compound 39 (400 mg, 0.631 mmol), 5-bromo-1H-indazole-4-carbonitrile (154.14 mg, 0.694 mmol), potassium carbonate (261.66 mg, 1.893 mmol), and Pd(dppf)Cl₂ (69.27 mg, 0.095 mmol) were added to a reaction flask containing 1 , 4 dioxane (42 mL) and water (7 mL), degassed and purged with nitrogen, the above mixture was stirred at 100 °C for 1.5 h, then was diluted with ethyl acetate and water. The organic phase was separated and the aqueous phase was extracted with ethyl acetate. The organic phases were combined, washed with brine, dried over anhydrous Na₂SO₄, filtered, and the filtrate was concentrated under reduced pressure and purified by an automatic column chromatography (Biotage) (mobile phases: dichloromethane/methanol 100/6 to 100/7), to obtain 80 mg of the crude product (purity: 91%), which was then purified by a reversed column (Welch Xtimate C18, 20 g, water ( 0.05 % ammonium bicarbonate)/acetonitrile, flow rate 20 mL/min, room temperature, wavelength 254 nm), and finally lyophilized to obtain the solid target compound YL142 (28 mg, Purity: 96.3 %, Yield:6.58 %). ESI:(m/z) =649.0 [M+H]⁺.¹H NMR (400 MHz, DMSO-d6) δ 13.70 (s, 1H), 10.30 (s, 1H), 8.33 (s, 1H), 8.05 - 7.99 (m, 1H), 7.99 - 7.85 (m, 3H), 7.66 - 7.55 (m, 2H), 7.37 (dd, J = 8.9, 5.1 Hz, 1H), 7.13 (td, J = 8.4, 3.1 Hz, 1H), 6.95 (dd, J = 9.1, 3.1 Hz, 1H), 6.85 (s, 1H), 6.19 (d, J = 2.6 Hz, 1H), 4.03 (dtd, J = 28.6, 10.4, 6.6 Hz, 2H), 3.27 - 3.08 (m, 2H).

Compound YL142 was treated by SFC resolution, condiction: SFC-150 (Waters); chromatographic column: AS 20*250mm, 10um (Daicel); column temperature: 35 °C; mobile phase: CO₂/MeOH [0.2%NH₃(7M in MeOH) ] = 70/30; flow rate: 100 g/min; back pressure: 100 bar; wavelength: 214 nm; to give YL142-P1 and YL142-P2;
YL142-P1 (retation time 2.58min) ESI:(m/z) =649.0 [M+H]⁺.¹H NMR (400 MHz, DMSO-d6) δ 13.70 (s, 1H), 10.30 (s, 1H), 8.33 (s, 1H), 8.05 - 7.99 (m, 1H), 7.99 - 7.85 (m, 3H), 7.66 - 7.55 (m, 2H), 7.37 (dd, J = 8.9, 5.1 Hz, 1H), 7.13 (td, J = 8.4, 3.1 Hz, 1H), 6.95 (dd, J = 9.1, 3.1 Hz, 1H), 6.85 (s, 1H), 6.19 (d, J = 2.6 Hz, 1H), 4.03 (dtd, J = 28.6, 10.4, 6.6 Hz, 2H), 3.27 - 3.08 (m, 2H).
YL142-P2 (retation time 2.91min) ESI:(m/z) =649.1 [M+H]⁺.¹H NMR (400 MHz, DMSO-d6) δ 13.70 (s, 1H), 10.30 (s, 1H), 8.33 (s, 1H), 8.09 - 7.92 (m, 1H), 7.99 - 7.85 (m, 3H), 7.69 - 7.52 (m, 2H), 7.34 (dd, J = 8.9, 5.1 Hz, 1H), 7.16 (td, J = 8.4, 3.1 Hz, 1H), 6.94 (dd, J = 9.1, 3.1 Hz, 1H), 6.83 (s, 1H), 6.31 (d, J = 2.6 Hz, 1H), 4.02 (dtd, J = 28.6, 10.4, 6.6 Hz, 2H), 3.25 - 3.03 (m, 2H).

### Syntheis of compound YL143

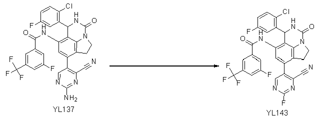

Compound YL137 (480 mg, 0.320 mmol) was dissolved in a solution of tetrafluoroborate in water (100 mL, 40 % ), placed in ethyl acetate dry-ice solution, lowered to a temperature of minus 50°C, and then was immediately added to a prepared aqueous sodium nitrite (7.5 ml, 0.5 M ), and the reaction was rapidly warmed to room temperature to continue stirring for 2.5 hours. The LCMS monitored that the raw material was no longer consumed and the product was no longer increased, then the reaction mixture was lowered to below 0°C, and was added saturated sodium hydroxide slowly to adjust pH close to neutral, followed by dilution with ethyl acetate and water. The organic phase was separated and the aqueous phase was extracted with ethyl acetate. The organic phases were combined, washed with brine, dried over anhydrous Na₂SO₄, filtered, and the filtrate was concentrated under reduced pressure, and purified by an automatic column chromatography (Biotage) (mobile phases: dichloromethane/methanol 100/4 to 100/6), to obtain 230 mg of the crude product, 10 mg of which were weighed and put into the reaction, and the remaining 220 mg of which were all purified by prep-HPLC (Welch Xtimate C18, 21.2*250 mm, 10.0 mm, 10.0 mm, 10.0 mm, 10.0 mm). 21.2*250 mm, 10 um, water (10 mM ammonium bicarbonate)/acetonitrile, flow rate 30 mL/min, column temperature 25 °C, wavelength 254 nm), but due to the alkaline environment, the sample state is unstable, the purity of the total collection of the purified solution was seriously decreased, so the recovery of the sample solution to purify (Welch Xtimate C18, 21.2*250 mm, 10 um, water (0.05 mM ammonium bicarbonate) / acetonitrile, flow rate of 30 mL/min, column temperature 25 °C, wavelength 254 nm). 10 um, water (0.05% TFA)/acetonitrile, flow rate 30 mL/min, column temperature 25 °C, wavelength 254 nm), and finally lyophilized to obtain the solid target compound YL143 (2.2 mg, Purity: 97.5 %, Yield: 0.44 %). ESI:(m/z) =629.2 [M+H]⁺.

### Synthetic route of compound YL144

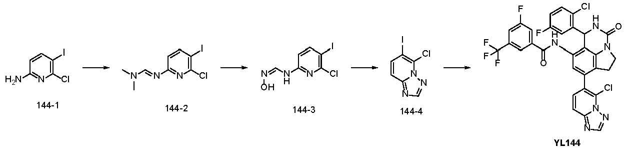

### Synthesis of compound 144-2

To a 100 mL three-necked flask was sequentially added 6-chloro-5-iodo-2-aminopyridine (144-1, 2545 mg, 10.002 mmol), isopropanol (25 mL) and N,N-dimethylformamide dimethyl acetal (1549.32 mg, 13.002 mmol). The reaction mixture was stirred under nitrogen protection at 80 °C for 3 h. LCMS showed it was a successful reaction. The reaction mixture was used directly in the next step of the reaction. LC-MS (ESI): m/z 312.0 (M+H)⁺;

### Synthesis of compound 144-3

To a 100 mL three-necked flask was sequentially added 144-2 (3095 mg, 9.999 mmol), hydroxylamine hydrochloride (972.73 mg, 13.998 mmol), and isopropanol (25 mL). The reaction mixture was stirred under nitrogen protection at 50°C for 4 h. LCMS showed that the reaction was complete. The reaction mixture was filtered directly to give 144-3 (2300 mg, 7.732 mmol, 77.33%).LC-MS (ESI): m/z 297.9 (M+H)⁺;

### Synthesis of Compound 144-4

To a 100 mL three-necked flask was sequentially added 144-3 (1300 mg, 4.370 mmol), tetrahydrofuran (20 mL). Cooled to 0°C, the above mixture was added trifluoroacetic anhydride (1835.68 mg, 8.740 mmol) slowly, then heated at 60°C for 18 h. LCMS showed that the reaction was complete. Aqueous sodium bicarbonate and ethyl acetate were added to the reaction mixture, and the organic phase was concentrated and the residue was purified by column chromatography (petroleum ether: ethyl acetate = 15:1-8:1) to give 144-4 (590 mg, 2.111 mmol, 48.31%). LC-MS (ESI): m/z 279.9 (M+H)⁺;

### Synthesis of the compound YL144

To a 100 mL three-necked flask was added sequentially 144-4 (317 mg, 0.500 mmol), compound 39 (139.78 mg, 0.500 mmol), potassium carbonate (207.36 mg, 1.500 mmol), 1,1-bis(diphenylphosphine)diperazinium iron palladium dichloride (36.60 mg. 0.050 mmol), 1,4-dioxane (15 mL) and water (3 mL). The reaction mixture was stirred under nitrogen protection at 75°C for 2 h. LCMS showed it was a successful reaction, to which water and ethyl acetate were added and the organic phase was concentrated and the residue was purified by column chromatography (petroleum ether: ethyl acetate = 1:1-1:2) to give YL144 (124 mg, 0.188 mmol, 37.60%).LC-MS (ESI): m/z 659.1 (M+H)⁺;¹H NMR (400 MHz, DMSO-d₆) δ 10.28 (s, 1H), 8.69 (s, 1H), 7.97 (dd, J = 14.0, 8.7 Hz, 2H), 7.88(d, J = 14.4 Hz, 2H), 7.79 (d, J = 9.1 Hz, 1H), 7.59(d, J = 2.6Hz, 1H), 7.37 (dd, J = 8.9, 5.2 Hz, 1H), 7.13 (td, J = 8.4, 3.1 Hz, 1H), 6.95 (dd, J = 9.2, 3.1 Hz, 1H), 6.84 (s, 1H), 6.23-6.08 (m, 1H), 4.17-3.89 (m, 2H), 3.20-2.90 (m, 2H).

### Synthetic route of compound YL145

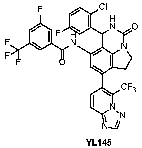

Referring to the synthetic route of YL144, substitute Cl for trifluoromethyl to obtain compound YL145. YL145 (119 mg, 0.172 mmol, 34.34%).LC-MS (ESI): m/z 693.1 (M+H)⁺;¹H NMR (400 MHz, DMSO-d₆) δ10.27 (d, J = 20.9 Hz, 1H), 8.77 (s, 1H), 8.26 (d, J = 9.2 Hz, 1H), 7.94 (d, J = 8.4 Hz, 1H), 7.85 (d, J = 13.9 Hz, 2H), 7.70 (t, J = 9.7 Hz, 1H), 7.57 (dd, J = 13.6, 2.6 Hz, 1H), 7.37 (ddd, J = 26.5, 8.8, 5.1 Hz, 1H), 7.13 (dtd, J = 16.7, 8.4, 3.1 Hz, 1H), 6.89 (ddd, J = 41.7, 9.2, 3.1 Hz, 1H), 6.75 (d, J = 16.5 Hz, 1H), 6.24-6.02 (m, 1H), 4.02 (ddd, J = 19.6, 10.3, 7.0 Hz, 2H), 3.19-2.82 (m, 2H).

### Synthetic route of compound YL146

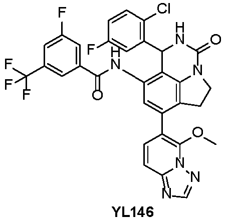

Referring to the synthetic route of YL144, substituted Cl for methoxy. Compound YL146: LC-MS (ESI): m/z 655.1 (M+H)⁺;¹H NMR (400 MHz, DMSO-d₆) δ10.24 (s, 1H), 8.58 (s, 1H), 8.02-7.83 (m, 3H), 7.71 (d, J=2.1 Hz, 2H), 7.56 (d, J=2.6 Hz, 1H), 7.36(dd, J=8.8, 5.1Hz, 1H), 7.12 (td, J = 8.4, 3.1 Hz, 1H), 6.94(dd, J =9.2, 3.1 Hz, 1H), 6.83(s, 1H), 6.16(d, J = 2.4 Hz, 1H), 4.24-3.71(m, 5H), 3.16 (t, J = 8.5 Hz, 2H).

### Synthetic route of compound YL147

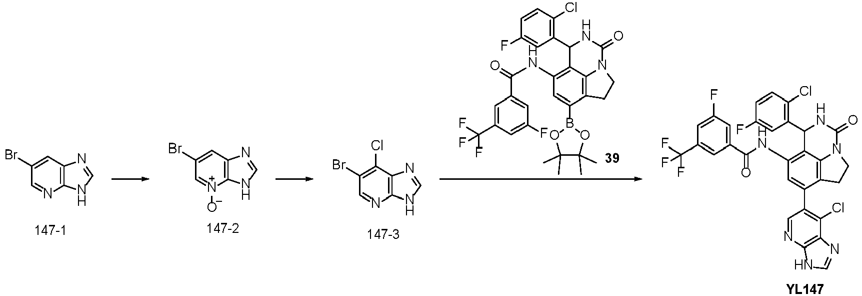

### Synthesis of compound 147-2

6-Bromo-3H-imidazo[5,4-b]pyridine (1980 mg, 9.999 mmol), acetic acid (20 mL), m-chloroperoxybenzoic acid (1725.43 mg, 9.999 mmol) were added sequentially to a 100 mL three-necked flask. The reaction mixture was stirred under nitrogen protection at room temperature for 18 h. LCMS showed that the reaction was successful, the reaction mixture was concentrated to dryness, to which 50 mL of ethyl acetate was added, the resulting mixture was stirred at 80 °C for 1 h. Filtered, the filter cake was washed with ethyl acetate, dried to give 147-2 (1980 mg, 9.251 mmol, 92.52%). IC-MS (ESI): m/z 215.9 (M+H)⁺;

### Synthesis of compound 147-3

To a 25 mL microwave tube was sequentially added 147-2 (5000 mg, 23.362 mmol), phosphorus trichloride (15 mL). The microwave reaction mixture was stirred under nitrogen protection at 80 °C for 30 min. LCMS showed that the reaction was complete, and the reaction mixture was decanted into ice-cold water, then the mixture was added with 2N sodium hydroxide solution to adjust pH to about 6, was added ethyl acetate, and the organic phase was concentrated and the residue was purified by column chromatography (dichloromethane: methanol = 30:1-20:1) to give 147-3 (310 mg, 1.334 mmol, 5.71%). LC-MS (ESI): m/z 233.8 (M+H)⁺;

### Synthesis of compound 147

Compound 39 (170 mg, 0.268 mmol), 147-3 (62.35 mg, 0.268 mmol), cesium carbonate (262.17 mg, 0.805 mmol), 1,1-bis(diphenylphosphino)diperylferrocene dichloropalladium (19.63 mg, 0.027 mmol) were added sequentially to a 25 mL microwave tube containing N,N-dimethylformamide (5 mL) and water (1 mL). The reaction mixture was stirred under nitrogen protection under microwave at 120 °C for 30 min. LCMS showed that the reaction was successful. Water and ethyl acetate were added to the reaction mixture and the organic phase was concentrated and the residue was purified by column chromatography (dichloromethane:methanol=20:1-10:1) to give YL147 (36 mg, 0.055 mmol, 20.35%). LC-MS (ESI): m/z 659.1 (M+H)⁺;¹H NMR (400 MHz, DMSO-d₆) δ13.50 (s, 1H), 10.26 (s, 1H), 8.60 (s, 1H), 8.35 (s, 1H), 8.00-7.80 (m, 3H), 7.58 (d, J = 2.7 Hz, 1H), 7.37 (dd, J = 8.8, 5.1 Hz, 1H), 7.14 (td, J = 8.4, 3.0 Hz, 1H), 6.94 (dd, J = 9.2, 3.1 Hz, 1H), 6.76 (s, 1H), 6.23-6.01 (m, 1H), 4.15-3.79 (m, 2H), 3.09 (q, J = 8.5 Hz, 2H).

### Synthetic route of compound YL148

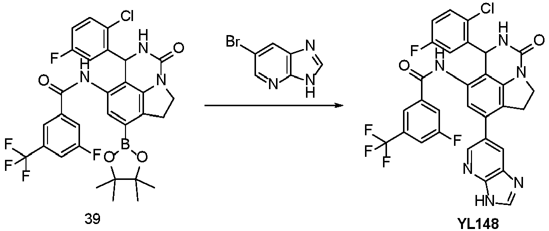

### Synthesis of compound YL148

To a 25 mL microwave tube was sequentially added compound 39 (317 mg, 0.500 mmol), 6-bromo-3H-imidazo[5,4-b]pyridine (99.04 mg, 0.500 mmol), cesium carbonate (488.88 mg, 1.500 mmol), 1,1-bis(diphenylphosphino)diperyleneferrocene palladium dichloride (36.60 mg, 0.050 mmol), N,N-dimethylformamide (10 mL) and water (2 mL). The reaction mixture was stirred under nitrogen protection under microwave at 120 °C for 30 min. LCMS showed it was a successful reaction. Water and ethyl acetate were added to the reaction mixture, and the organic phase was concentrated and the residue was purified by column chromatography (dichloromethane:methanol=20:1-10:1) to afford YL148 (36 mg, 0.058 mmol, 11.52%).LC-MS (ESI): m/z 625.2 (M+H)⁺;¹H NMR (400 MHz, DMSO-d₆) δ13.05 (s, 1H), 10.25 (s, 1H), 8.51 (s, 2H), 8.22 (s, 1H), 8.02-7.84 (m, 3H), 7.59 (d, J = 2.7 Hz, 1H), 7.35 (dd, J=8.9, 5.2 Hz, 1H), 7.18-7.03 (m, 1H), 6.96(d, J = 8.2 Hz, 2H), 6.18 (dd, J = 2.7, 1.1 Hz, 1H), 4.13-3.91 (m, 2H), 3.53-3.35 (m, 2H).

### compound YL149

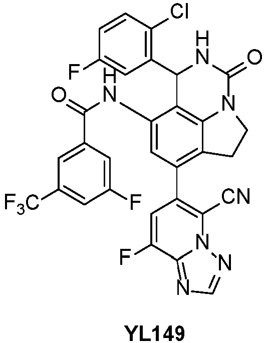

Referring to the synthetic route of compoundYL144 to obtain YL149. LCMS (ESI): m/z 668.0 (M+H) ⁺; ¹H NMR (400 MHz, DMSO-d6) δ 10.36 (s, 1H), 8.86 (s, 1H), 8.08 (d, J = 10.4 Hz, 1H), 7.96 (d, J = 8.0 Hz, 1H), 7.94 - 7.86 (m, 2H), 7.66 (d, J = 2.6 Hz, 1H), 7.38 (dd, J = 8.9, 5.1 Hz, 1H), 7.14 (td, J = 8.4, 3.0 Hz, 1H), 6.99 (s, 1H), 6.94 (dd, J = 9.1, 3.1 Hz, 1H), 6.19 (d, J = 2.5 Hz, 1H), 4.15 - 3.95 (m, 2H), 3.30 - 3.16 (m, 2H).

### Synthetic route of compound YL150

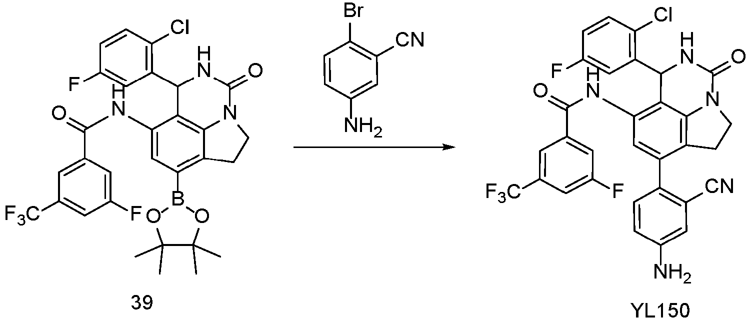

39 (200 mg, 0.316 mmol), 5-amino-2-bromobenzonitrile (62 mg, 0.316 mmol), potassium carbonate (131 mg, 0.947 mmol), and Pd(dppf)Cl₂ (23 mg, 0.032 mmol) were added to a reaction vial containing dioxane (2 mL) and water (0.4 mL). After degassed and purged with nitrogen, the reaction mixture was stirred at 100 °C for 1 h. The reaction mixture was diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered and evaporated to dryness, and the residue was purified by prep-HPLC to afford YL150 (120 mg). LCMS (ESI): m/z 624.0 (M+H) ⁺; H NMR: ¹H NMR (400 MHz, DMSO-d6) δ 10.23 (s, 1H), 7.97 - 7.93 (m, 1H), 7.92 - 7.85 (m, 2H), 7.56 (d, J = 2.7 Hz, 1H), 7.35 (dd, J = 8.9, 5.2 Hz, 1H), 7.26 (d, J = 8.4 Hz, 1H), 7.11 (ddd, J = 8.8, 7.9, 3.1 Hz, 1H), 6.97 (d, J = 2.4 Hz, 1H), 6.94 - 6.86 (m, 2H), 6.70 (s, 1H), 6.14 (dd, J=2.6, 1.1 Hz, 1H), 5.78 (s, 2H), 3.99 (dtd, J=31.6, 10.4, 6.7Hz, 2H), 3.22 (ddd, J= 17.3, 10.3, 7.5 Hz, 1H), 3.09 (ddd, J= 16.3, 10.3, 5.8 Hz, 1H).

### Synthetic route of compound YL151

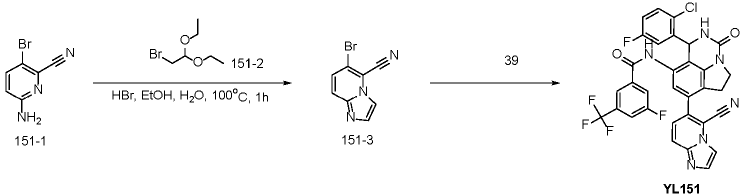

### Synthesis of compounds 151-3

A mixture of 40% hydrobromic acid solution (2 mL) and 2-bromo-1,1-diethoxyethane (1.49 g, 7.575 mmol) was added to a solution of 151-1 (1.00 g, 5.050 mmol) in ethanol (25 mL) and water (15 mL), then the resulting mixture was heated at 100 °C for 1 hour. The reaction mixture was concentrated to remove the organic phase, quenched to neutrality by addition of saturated sodium bicarbonate solution, and extracted with ethyl acetate to separate the organic phase. The organic phase was concentrated and purified by column chromatography (ethyl acetate/petroleum ether (v/v)=0~30%) to afford the target compound 151-3 (1.00 g, 89.29% yield).(ESI): m/z 245.0[M(⁷⁹Br)+Na]⁺, 247.0[M(⁸¹Br)+Na]⁺; ¹H NMR (400 MHz, DMSO-d₆)δ8.19 (t, J = 1.0 Hz, 1H), 7.95 (dd, J = 9.5, 0.7 Hz, 1H), 7.85 (d, J = 1.3 Hz, 1H), 7.60 (d, J = 9.5 Hz, 1H).

### Synthesis of compound YL 151

Under nitrogen protection, Pd(dppf)Cl₂ (1,1 '-bis(diphenylphosphino)ferrocene palladium(II) dichloride) (40 mg, 0.054 mmol) was added to a mixture of 39 (133 mg, 0.598 mmol), 151-3 (300 mg, 0.544 mmol) and potassium carbonate (150 mg, 1.088 mmol) in amixed solution of 1,4-dioxane (8 mL) and water (1.6 mL) , then heated at 90 °C for 2 hours. The reaction mixture was quenched with water and extracted with ethyl acetate to separate the organic phase. The organic phase was concentrated under reduced pressure and purified by silica gel column chromatography (ethyl acetate/petroleum ether (v/v) = 30% to 100%) to give crude purified product (100 mg). It was then purified by prep-HPLC (base) to give YL151 (40 mg, 11.33% yield). (ESI)m/z 649.1 [M+H]⁺; ¹H NMR (400 MHz, DMSO-d₆)δ10.32 (s, 1H), 8.21 (s, 1H), 8.07 (d, J = 9.3 Hz, 1H), 7.96 (d, J = 8.5 Hz, 1H), 7.93-7.86 (m, 3H), 7.63 (d, J = 2.6 Hz, 1H), 7.49 (d, J = 9.3 Hz, 1H), 7.37 (dd, J = 8.8, 5.1 Hz, 1H), 7.14 (td, J = 8.4, 3.0 Hz, 1H), 6.96 (s, 1H), 6.96-6.90 (m, 1H), 6.18 (d, J = 2.5 Hz, 1H), 4.17-3.92 (m, 2H), 3.30-3.15 (m, 2H).

### Synthetic route of compound YL152

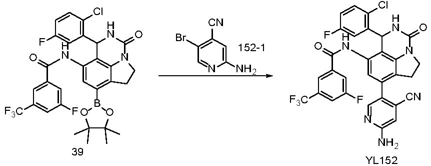

### Synthesis of compound YL152

Compound 39 (200 mg, 0.316 mmol), 2-amino-5-bromoisonicotinonitrile (63 mg, 0.316 mmol), potassium carbonate (131 mg, 0.947 mmol), and Pd(dppf)Cl₂ (23 mg, 0.032 mmol) were added to a reaction vial containing dioxane (2 mL) and water (0.4 mL). After degassed and purged with nitrogen, the reaction mixture was stirred at 100 °C for 1 h, then was diluted with ethyl acetate and water. The organic phase was separated and the aqueous phase was extracted with ethyl acetate. The organic phases were combined, washed with water, washed with saturated aqueous sodium bicarbonate, dried over anhydrous Na₂SO₄, filtered, evaporated, and purified by column chromatography(dichloromethane/methanol ~20/1) to give YL152 (120 mg). LCMS (ESI): m/z 625.0 (M+H) ⁺;
YL152: ¹H NMR (400 MHz, DMSO-d6) δ 10.25 (s, 1H), 8.18 (s, 1H), 7.94 (d, J = 8.6 Hz, 1H), 7.92 - 7.85 (m, 2H), 7.58 (d, J = 2.6 Hz, 1H), 7.35 (dd, J = 8.9, 5.1 Hz, 1H), 7.12 (td, J = 8.4, 3.1 Hz, 1H), 6.91 (dd, J = 9.2, 3.1 Hz, 1H), 6.86 (s, 1H), 6.74 (s, 1H), 6.71 (s, 2H), 6.15 (d, J = 2.6 Hz, 1H), 4.00 (dtd, J = 28.8, 10.3, 6.6 Hz, 2H), 3.29 - 3.06 (m, 2H).

### Synthetic route of compound YL153

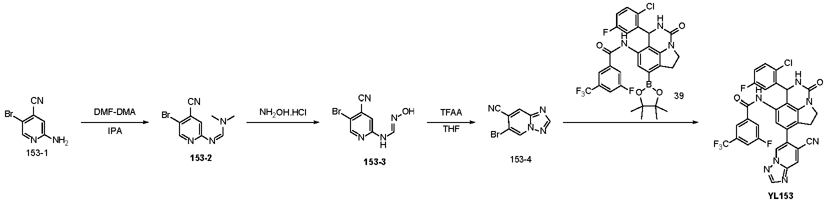

### Synthesis of compound 153-3

2-Amino-5-bromoisonicotinonitrile (500 mg, 2.525 mmol) was dissolved in isopropanol (10 mL), N,N-dimethylformamide dimethyl acetal (905 mg, 7.575 mmol) was added, and the reaction mixture was stirred at 80 °C for 3 hours. The reaction mixture was cooled down to 50 °C, was added hydroxylamine hydrochloride (350 mg, 5.05 mmol), and the reaction mixture was stirred at 50 °C for 3 hours. The reaction mixture was concentrated to dryness, the residue was added ethanol and pulped, concentrated until a solid precipitated, filtered to obtain a solid, and the filtrate was repeated the above procedure to obtain compound 153-3 (300 mg, 49.18%).ESI:(m/z) = 241.0 [M+H]⁺ .¹H NMR (400 MHz, DMSO-d6) δ 10.46 (s, 1H), 9.94 (d, J = 9.8 Hz, 1H), 8.53(s, 1H), 7.76(d, J = 9.8 Hz, 1H), 7.49 (d, J = 0.6 Hz, 1H).

### Synthesis of the compound 153-4

A solution of 153-2 (260 mg, 2.251 mmol) in tetrahydrofuran (10 mL) was cooled down to 0 °C to obtain the crude 153-3, to which trifluoroacetic anhydride (453 mg, 2.157 mmol) was added dropwise, and the reaction mixture was stirred at 60 °C overnight. The reaction mixture was diluted with ethyl acetate and water, and the aqueous phase was extracted with ethyl acetate. The organic phases were combined, washed with water, washed with saturated aqueous sodium bicarbonate, dried over anhydrous Na₂SO₄, filtered, evaporated, and the residue was purified by column chromatography (mobile phase: petroleum ether/ethyl acetate ~2/1) to give compound 153-3 (100 mg, 41.67%).ESI:(m/z) = 224.0 [M+H]⁺. YL153-4 HNMR: ¹H NMR (400 MHz, DMSO-d6) δ 9.72 (d, J = 0.8 Hz, 1H), 8.84 (d, J = 0.8 Hz, 1H), 8.77 (s, 1H).

### Synthesis of compound 153

39 (200 mg, 0.316 mmol), 153-4 (70 mg, 0.316 mmol), potassium carbonate (130 mg, 0.947 mmol) and Pd(dppf)Cl₂ (23 mg, 0.032 mmol) were added to a reaction flask containing dioxane (5 mL) and water (1 mL). After degassed and purged with nitrogen, the reaction mixture was stirred at 100 °C for 1 hr, then was added ethyl acetate and water to extract. The organic phase was concentrated to dryness, and purified by column chromatography (mobile phase: dichloromethane/methanol ~20/1) to give 153 (120 mg). LCMS (ESI): m/z 650.0(M+H) ⁺;

YL153 NMR: ¹H NMR (400 MHz, DMSO-d6) δ 10.34 (s, 1H), 9.38 (s, 1H), 8.84 (s, 1H), 8.81 (s, 1H), 7.96 (d, J = 8.7 Hz, 1H), 7.93 - 7.85 (m, 2H), 7.64 (d, J = 2.7 Hz, 1H), 7.38 (dd, J = 8.8, 5.1 Hz, 1H), 7.14 (td, J = 8.4, 3.1 Hz, 1H), 6.96 - 6.86 (m, 2H), 6.19 (d, J = 2.5 Hz, 1H), 4.04 (dtd, J = 33.0, 10.4, 6.6 Hz, 2H), 3.30 - 3.14 (m, 2H).

### Effect Example IC₅₀ of PI3Kα and PI3KαH1047R enzyme activity inhibition evaluation assay

The ADP-Glo Kinase Assay Kit was used for the experiments. The following buffer solutions were prepared: 50 mM HEPES, pH 7.5, 3 mM MgCl₂, 1 mM EGTA, 100 mM NaCl, 0.03% CHAPS, 2 mM DTT. The tested compound samples were dissolved in DMSO and added to the screening system at a certain starting concentration of, for example, 10 µM, diluted 3-fold, while setting a DMSO control and an unkinase control. The optimal concentrations of PI3Kα, PI3Kα H1047R, substrate (PIP2) and ATP were prepared with buffers. The enzyme reaction system included: buffer, ATP 25µM, kinase substrate (PIP2, 50µg/mL), kinase PI3Kα (0.15µg/mL), PI3Kδ H1047R (0.05µg/mL), etc. The reaction system was reacted at room temperature for 1 hour, then terminated by adding a terminal reagent (ADP-Glo reagent, 5µL), and the ADP content in the system was detected using a detection reagent (Kinase Detection Reagent, 10µL). Signal data was collected using the Envision instrument. Calculated the % inhibition according to the following formula: % inhibition = (DMSO control signal value - sample signal value)/ (DMSO control signal value - unkinase control signal value). Inhibition curves were plotted using the formula Y=Bottom + (Top-Bottom)/(1+(IC₅₀/X)^HillSlope) to obtain IC₅₀ values and the results are shown in Table 1.

+ represents IC₅₀ <= 250nM ++ represents 250 nM < IC₅₀ < = 10µM +++ represents IC₅₀ > 10 µM

**Table 1**

| Compound No. | PI3KαH1047RIC₅₀ | PI3Kα IC₅₀ | Compound No. | PI3KαH1047RIC₅₀ | PI3Kα IC₅₀ |
|---|---|---|---|---|---|
| YL001 | + | ++ | YL002 | + | ++ |
| YL003 | ++ | +++ | YL004 | ++ | +++ |
| YL005 | ++ | +++ | YL006 | + | ++ |
| YL007 | + | ++ | YL008 | + | ++ |
| YL009 | + | ++ | YL010 | + | ++ |
| YL011 | + | ++ | YL012 | + | ++ |
| YL013 | + | ++ | YL014 | + | ++ |
| YL015 | + | ++ | YL016 | + | ++ |
| YL017 | + | ++ | YL018 | + | ++ |
| YL019 | + | ++ | YL020 | ++ | +++ |
| YL021 | + | ++ | YL022 | + | ++ |
| YL023 | + | ++ | YL024 | + | ++ |
| YL025 | + | ++ | YL026 | + | ++ |
| YL027 | + | ++ | YL028 | + | ++ |
| YL029 | + | ++ | YL030 | + | +++ |
| YL031 | + | +++ | YL032 | + | ++ |
| YL035 | + | +++ | YL036 | + | ++ |
| YL037 | ++ | +++ | YL038 | ++ | +++ |
| YL039 | ++ | +++ | YL040 | ++ | +++ |
| YL041 | ++ | ++ | YL042 | + | ++ |
| YL043 | + | ++ | YL044 | + | ++ |
| YL045 | ++ | +++ | YL046 | ++ | +++ |
| YL047-P1 | ++ | +++ | YL047-P2 | + | ++ |
| YL048 | + | ++ | YL049 | ++ | +++ |
| YL050 | + | ++ | YL051 | +++ | ++++ |
| YL052 | ++ | +++ | YL053 | + | ++ |
| YL053-P1 | ++ | +++ | YL053-P2 | + | ++ |
| YL054 | + | ++ | YL055 | + | ++ |
| YL056 | ++ | +++ | YL057 | ++ | +++ |
| YL058 | ++ | +++ | YL059 | + | ++ |
| YL060 | ++ | +++ | YL061 | ++ | +++ |
| YL062 | ++ | +++ | YL063 | + | ++ |
| YL064 | ++ | +++ | YL065 | + | ++ |
| YL065-P1 | ++ | +++ | YL065-P2 | + | ++ |
| YL066 | ++ | +++ | YL067 | ++ | +++ |
| YL068 | + | ++ | YL069 | ++ | +++ |
| YL070 | ++ | +++ | YL071 | + | ++ |
| YL072 | + | ++ | YL073 | + | ++ |
| YL074 | + | ++ | YL075 | ++ | +++ |
| YL076 | + | ++ | YL077 | + | ++ |
| YL078 | ++ | +++ | YL079-P1 | ++ | +++ |
| YL079-P2 | + | ++ | YL080 | ++ | +++ |
| YL081 | ++ | +++ | YL082 | ++ | +++ |
| YL083 | ++ | +++ | YL084 | ++ | +++ |
| YL085 | ++ | +++ | YL086 | + | ++ |
| YL087 | ++ | +++ | YL088 | + | ++ |
| YL089 | ++ | +++ | YL090 | ++ | +++ |
| YL091 | + | ++ | YL092 | + | ++ |
| YL093 | + | ++ | YL094 | + | ++ |
| YL095 | + | ++ | YL096 | + | ++ |
| YL097 | + | ++ | YL098 | + | ++ |
| YL010-P1 | +++ | +++ | YL010-P2 | + | ++ |
| YL099 | ++ | ++ | YL100 | ++ | ++ |
| YL101-P1 | +++ | +++ | YL101-P2 | + | +++ |
| YL102-P1 | +++ | ++++ | YL102-P2 | + | +++ |
| YL103 | + | +++ | YL104 | + | +++ |
| YL105 | ++ | +++ | YL106 | + | +++ |
| YL107 | + | +++ | YL110 | + | +++ |
| YL108-P1 | +++ | ++++ | YL108-P2 | + | +++ |
| YL109-P1 | +++ | ++++ | YL109-P2 | + | +++ |
| YL111-P1 | +++ | ++++ | YL111-P2 | + | +++ |
| YL112 | ++ | +++ | YL113 | ++ | +++ |
| YL114-P1 | +++ | ++++ | YL114-P2 | + | +++ |
| YL115 | + | +++ | YL116 | + | +++ |
| YL117 | ++++ | ++++ | YL118 | + | ++ |
| YL119 | ++++ | ++++ | YL120 | ++++ | ++++ |
| YL121 | + | +++ | YL122 | + | +++ |
| YL123 | +++ | ++++ | YL124 | + | +++ |
| YL125 | +++ | ++++ | YL126 | + | +++ |
| YL127 | +++ | ++++ | YL128 | + | +++ |
| YL129 | ++ | +++ | YL130 | ++ | +++ |
| YL131 | +++ | ++++ | YL132 | + | +++ |
| YL133 | + | +++ | YL134 | + | +++ |
| YL135 | + | +++ | YL136 | + | ++ |
| YL137 | + | +++ | YL138 | +++ | +++ |
| YL139 | + | +++ | YL140 | + | +++ |
| YL141 | + | +++ | YL142 | + | +++ |
| YL143 | + | +++ | YL144 | + | ++ |
| YL145 | + | +++ | YL146 | + | +++ |
| YL147 | + | +++ | YL148 | + | +++ |
| YL149 | + | +++ | YL150 | + | ++ |
| YL151 | + | +++ | YL152 | + | +++ |
| YL153 | + | +++ | | | |

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof: 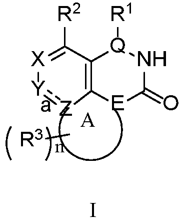 wherein
Z is N or C;
E is N, C or CH;
X is CH, CR^{X} or N;
Y is -C(O), CR^{Y} or N;
Q is CH or N;
a is a single bond or a double bond;
when a is a single bond, Y is -C(O)- or N;
when a is a double bond, Y is CR^{Y} or N;
R¹ is-L¹-R^{1A}; L¹ is a bond, R^{1A} is C₆₋₂₀ aryl , C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
R² is -L²-R^{2A}; L² is a bond,-NH-,-NH-C(O)-or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3}, 5- to 6- membered heteroaryl containing 1-4 heteroatoms independently selected from O, S and N substituted with one or more R^{2C4} or C₈₋₁₀ benzocycloalkenyl substituted with one or more R^{2C5}; R^{2C1}, R^{2C2}, R^{2C3}, R^{2C4} and R^{2C5} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
R³ is -L³-R^{3A}; L³ is a bond, R^{3A} is halogen, C₁₋₆ alkyl, =O or C₁₋₆ alkyl substituted with one or more R^{3C} , or, two optional R³ together with the atom they are attached form C₃₋₈ cycloalkane or C₆₋₂₀ aromatic ring;
R^{3C} is independently deuterium, halogen or-NR^{3C1}R^{3C2};
R^{3C1} and R^{3C2}are independently hydrogen or C₁₋₆ alkyl
R^{Y} and R^{X} are independently -L^{Y}-R^{YA}; L^{Y} is a bond,-NH-or -NH-C(O)-; R^{YA} is hydrogen, halogen, cyano, -N₃, hydroxyl,-NH₂, C₂₋₆ alkynyl. 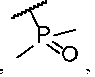 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N , 4- to 10-membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{YA4} , -COR ^{YA5}, 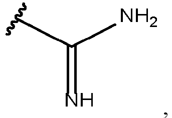 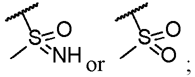
R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
Ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
n is 0, 1, 2, 3 or 4.

2. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein the nitrogen-containing heterocyclic compound represented by formula I having the structure as represented by formula II: 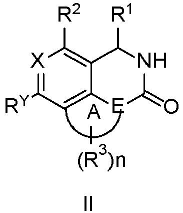 wherein E is N, CH or C;
X is N or CH;
R¹ is-L¹-R^{1A}; L¹ is a bond, R^{1A} is C₆₋₂₀ aryl, C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
R² is -L²-R^{2A}; L² is a bond,-NH-,-NH-C(O)- or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3}, 5- to 6- membered heteroaryl containing 1-4 heteroatoms independently selected from O, S and N substituted with one or more R^{2C4} or C₈₋₁₀ benzocycloalkenyl substituted with one or more R^{2C5}; R^{2C1}, R^{2C2}, R^{2C3}, R^{2C4} and R^{2C5} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
R³ is -L³-R^{3A}; L³ is a bond, R^{3A} is halogen, C₁₋₆ alkyl, =O or C₁₋₆ alkyl substituted with one or more R^{3C} , or, two optional R³ together with the atom they are attached form C₃₋₈ cycloalkane or C₆₋₂₀ aromatic ring;
R^{3C} is independently deuterium, halogen or -NR^{3C1}R^{3C2};
R^{3C1} and R^{3C2} are independently hydrogen or C₁₋₆ alkyl
R^{Y} is -L^{Y}-R^{YA}; L^{Y} is a bond,-NH-or -NH-C(O)-; R^{YA} is hydrogen, halogen, cyano, -N₃, hydroxyl, -NH₂, -C₂₋₆ alkynyl, 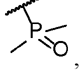 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} -COR ^{YA5}, 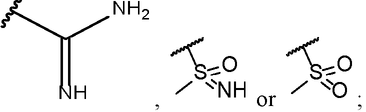
R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
n is 0, 1 or 2.

3. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein
when the definition of R¹, R^{1C}, R^{1D}, R^{2C1}, R^{2C2}, R^{2C3}, R^{2C4}, R^{2C5}, R^{2C11}, R^{3A}, R^{3C}, R^{YA}, R^{YA1}, R^{YA2}, R^{YA3}, R^{YA4} R^{YA5} and R^{YA11} refers to halogen, the halogen is fluorine, chlorine, bromine or iodine;
and/or, when the definition of R^{2C1}, R^{2C2}, R^{2C3}, R^{2C4}, R^{2C5}, R^{YA}, R^{YA1}, R^{YA2}, R^{YA3}, R^{YA4} and R^{YA5} refers to C₃₋₈ cycloalkyl, the C₃₋₈ cycloalkyl is cyclohexyl, -cyclopentyl, cyclobutyl or cyclopropyl;
and/or, when the definition of R^{1C}, R^{1D}, R^{2C1}, R^{2C2}, R^{2C3}, R^{2C4}, R^{2C5}, R^{3A}, R^{3C1}, R^{3C2}, R^{YA}, R^{YA1}, R^{YA2}, R^{YA3} R^{YA4} and R^{YA5} refers to C₁₋₆ alkyl or -OC₁₋₆ alkyl, the C₁₋₆ alkyl is C₁₋₄ alkyl, for example, methyl, ethyl, n-propyl, isopropyl, n-butyl, sec-butyl, isobutyl or tert-butyl;
and/or, when the definition of R^{1A}, R^{2A}, R^{YA1}, R^{YA2}, R^{YA3}, R^{YA4} andR^{YA5} refers to C₆₋₂₀ aryl, the C₆₋₂₀ aryl is phenyl or naphthyl;
and/or, when R^{2A} and R^{YA} are independently "5- to 6- membered heteroaryl containing 1-4 heteroatoms independently selected from O, S and N", the"5- to 6- membered heteroaryl containing 1-4 heteroatoms independently selected from O, S and N" is"5- to 6- membered heteroaryl containing 1-4 heteroatoms independently selected from O and N";
and/or, when R^{2A} and R^{YA} are independently 8- to 10- membered bicyclic heteroaryl containing 1-3 heteroatoms independently selected from O, S and N, the "8- to 10- membered bicyclic heteroaryl containing 1-3 heteroatoms independently selected from O, S and N" is [1,2,4]-Triazolo[1,5-a]pyridinyl, indazolyl, indolyl, isoquinolinyl, Benzothienyl or benzimidazolyl;
and/or, X is N or CH;
and/or, Z is C;
and/or, E is N or C;
and/or, when R^{1A} is "5- to 12-membered heteroaryl containing 1 to 4 heteroatoms independently selected from O, S and N" substituted with one or more R^{1D} , the R^{1A} is "5- to 6-membered heteroaryl containing 1 to 2 heteroatoms independently are N" substituted with one or more R^{1D};
and/or, when R^{2A} is 8- to 10- membered bicyclic heteroaryl containing 1-3 heteroatoms independently selected from O, S and N, or "8- to 10- membered bicyclic heteroaryl containing 1-3 heteroatoms independently selected from O, S and N" substituted with one or more R^{2C1}, the "8- to 10- membered bicyclic heteroaryl containing 1-3 heteroatoms independently selected from O, S and N" is "9- to 10- membered bicyclic heteroaryl containing 1-2 heteroatoms being N";
and/or, when R^{2A} is C₈₋₁₀ benzoheterocycloalkenyl containing 1-3 heteroatoms independently selected from O, S and N, the "C₈₋₁₀ benzoheterocycloalkenyl containing 1-3 heteroatoms independently selected from O, S and N" is 8- to 10- membered benzoheterocycloalkenyl containing 1-2 N;
and/or, when R^{2A} is "5- to 12-membered heteroaryl containing 1 to 4 heteroatoms independently selected from O, S and N" substituted with one or more R^{2C3} , the "5- to 12-membered heteroaryl containing 1 to 4 heteroatoms independently selected from O, S and N" is"5- to 6-membered heteroaryl containing 1 to 2 heteroatoms independently are N";
and/or, when R^{2A} is C₈₋₁₀ benzocycloalkenyl, the C₈₋₁₀ benzocycloalkenyl is C₉₋₁₀ benzocycloalkenyl;
and/or, when L^{Y} is -NH-C(O)-, the end C is connected with R^{YA};
when R^{YA} is "4- to 10- membered cyclic olefin containing 1-3 heteroatoms independently selected from O, S and N" or "4- to 10- membered cyclic olefin containing 1-3 heteroatoms independently selected from O, S and N" substituted with R^{YA2} , the "4- to 10- membered cyclic olefin containing 1-3 heteroatoms independently selected from O, S and N" is "4- to 6-membered cyclic olefin containing 1-3 heteroatoms independently selected from O and N";
and/or, when R^{YA} is "5- to 6-membered heteroaryl containing 1 to 4 heteroatoms independently selected from O, S and N" or "5- to 12-membered heteroaryl containing 1 to 4 heteroatoms independently selected from O, S and N" substituted with one or more R^{YA3}, the "5- to 6-membered heteroaryl containing 1 to 4 heteroatoms independently selected from O, S and N" is "5- to 6-membered heteroaryl containing 1 to 4 heteroatoms independently selected from O and N";
and/or, when R^{YA} is "8- to 10- membered bicyclic heteroaryl containing 1-3 heteroatoms independently selected from O, S and N", or "8- to 10- membered bicyclic heteroaryl containing 1-3 heteroatoms independently selected from O, S and N" substituted with one or more R^{YA4}, the "8- to 10- membered bicyclic heteroaryl containing 1-3 heteroatoms independently selected from O, S and N" is "9- to 10- membered bicyclic heteroaryl containing 1-3 heteroatoms N";
and/or, when R^{YA1}, R^{YA2}, R^{YA3}, R^{YA4} and R^{YA5}are independently C₁₋₆ alkyl substituted with one or more R^{YA11} and R^{YA11} is halogen, the C₁₋₆ alkyl substituted with one or more R^{YA11} is C₁₋₂ alkyl substituted with one or more halogen.

4. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein
a is a double bond, Y is CR^{Y}, E is N, C or CH;
and/or, R¹ is 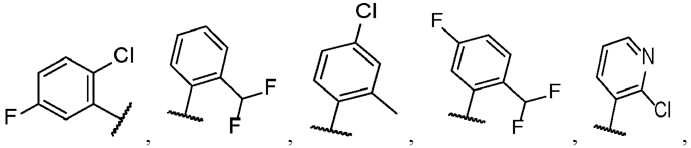 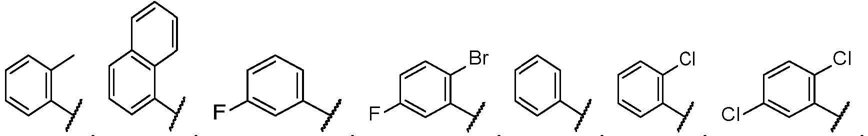 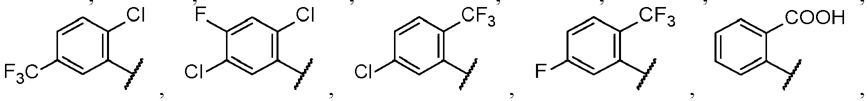 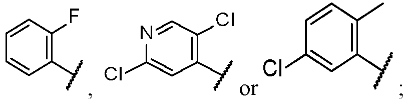
and/or, R² is 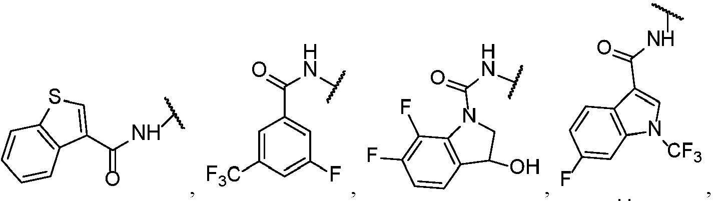 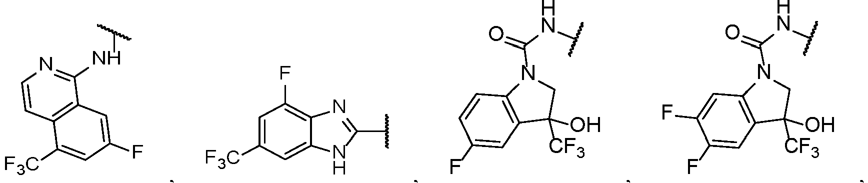 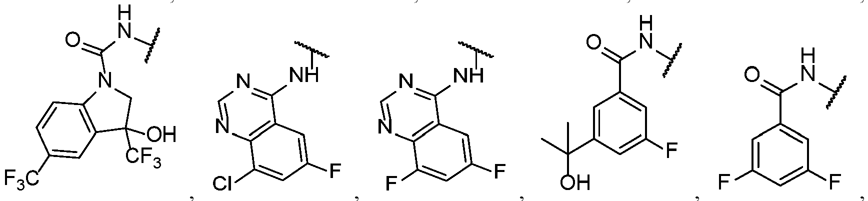 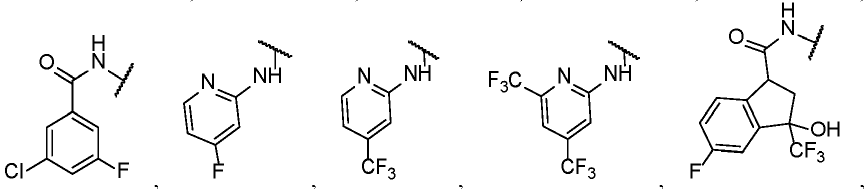 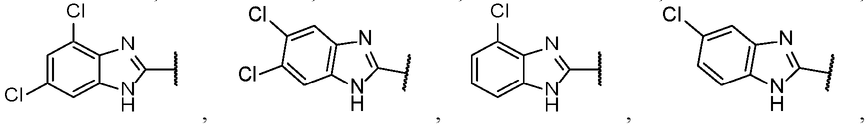 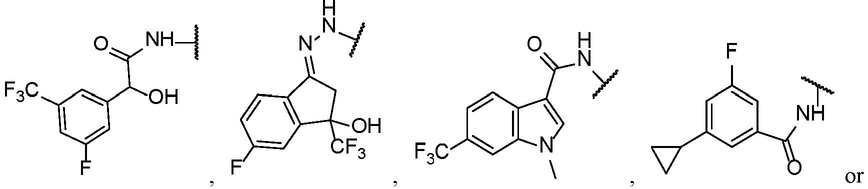 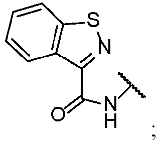
and/or, R^{Y} is hydrogen, -CN, -N₃, -F, Br, -OH, -NH₂, -B(OH)₂, -COOH, -CONH₂, - COOMe, -CH₃, -OCH₃, 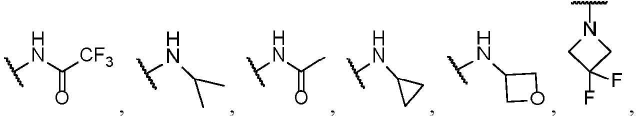 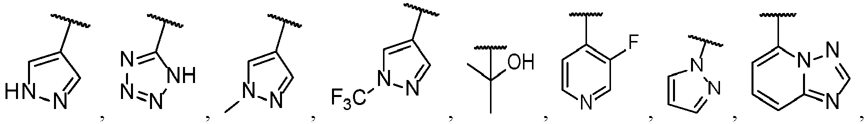 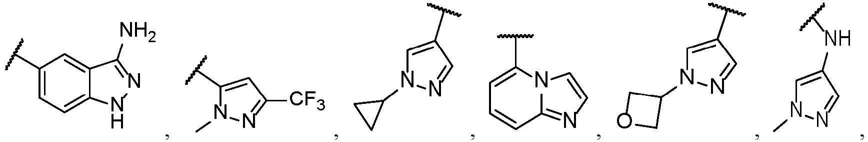 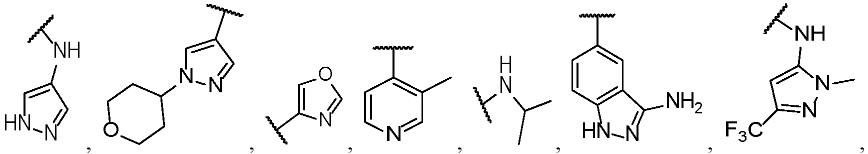 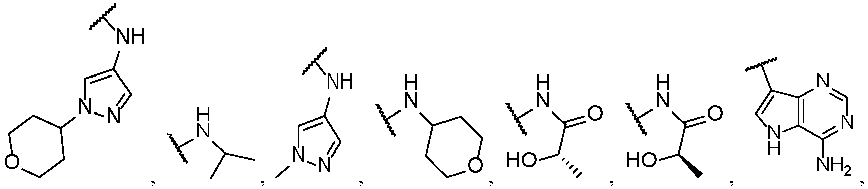 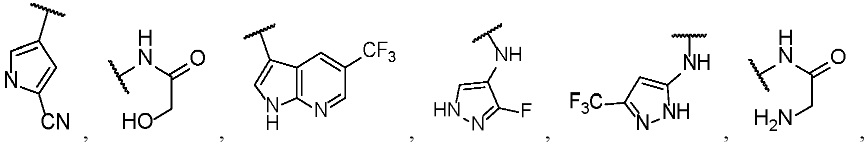 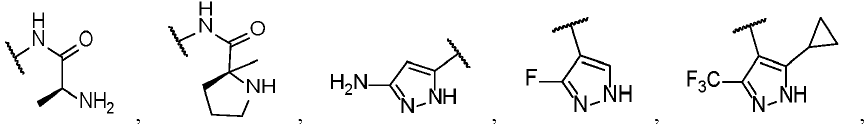 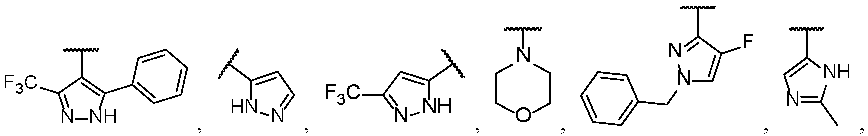 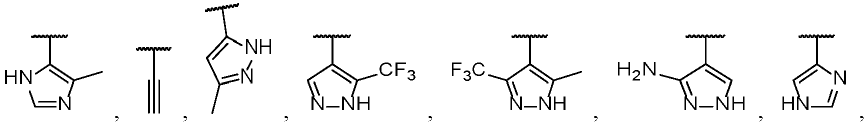 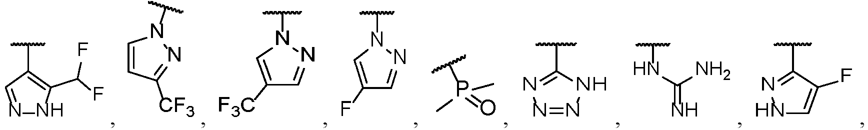 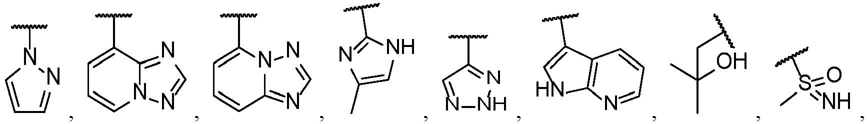 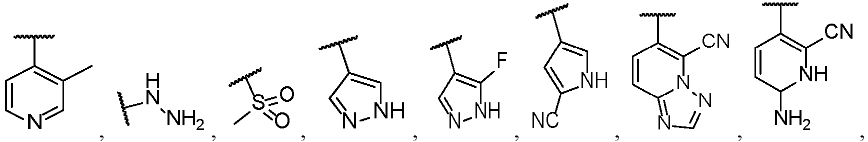 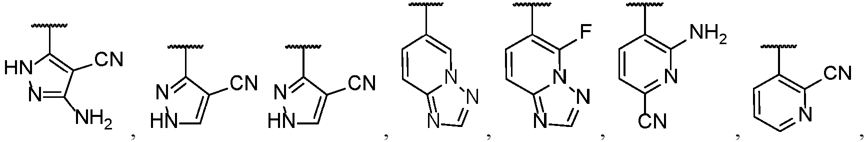 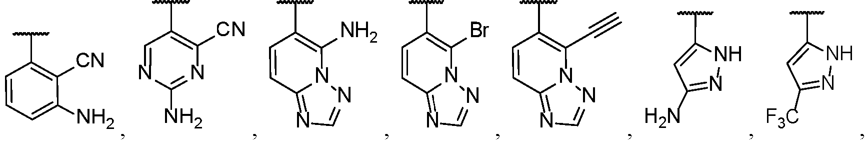 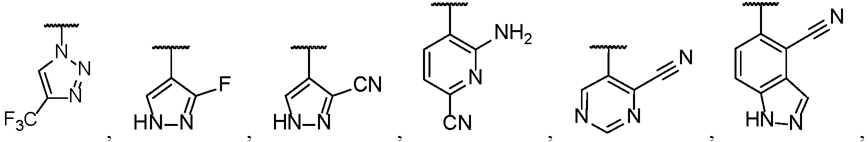 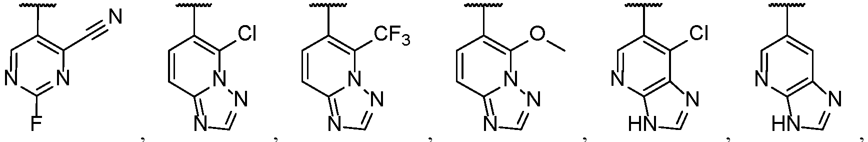 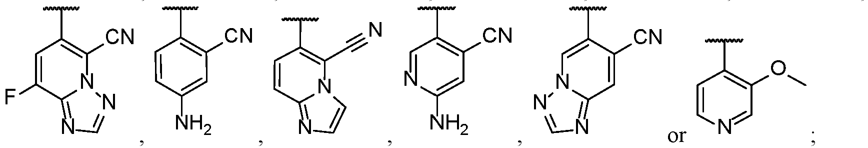
and/or, R³ is F, -Me, -Et, -CD₃, 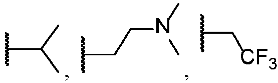 or =O, two optional R³ together with the atom they are attached form Cyclopropane or benzene ring;
and/or, n is 0, 1 or 2;
and/or, Q is CH;
and/or, 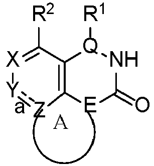 is

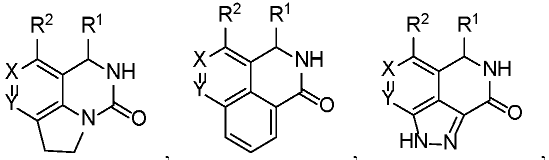

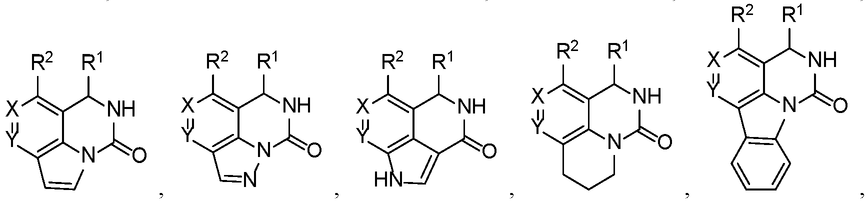

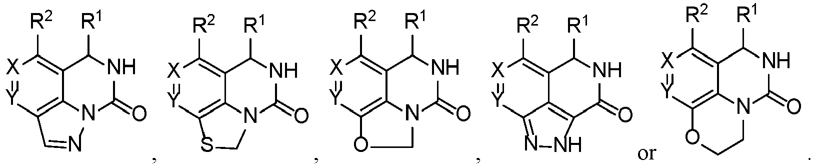

5. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein 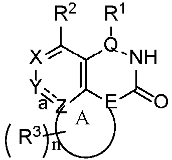 is

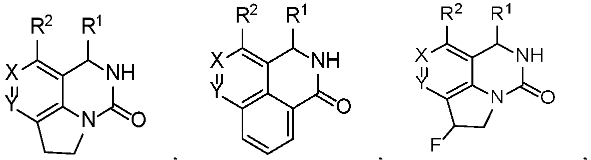

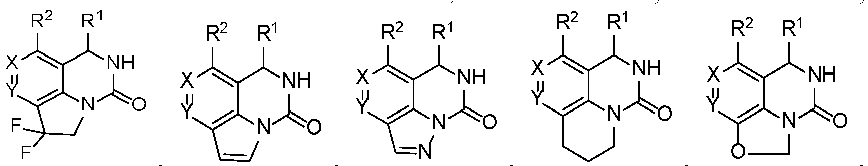

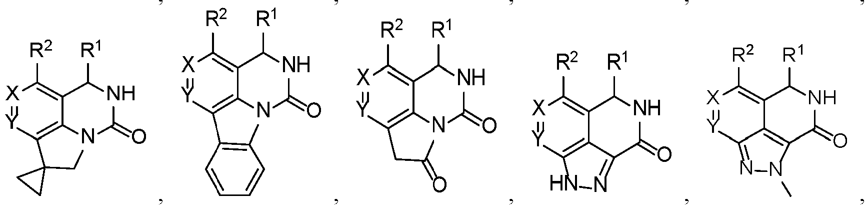

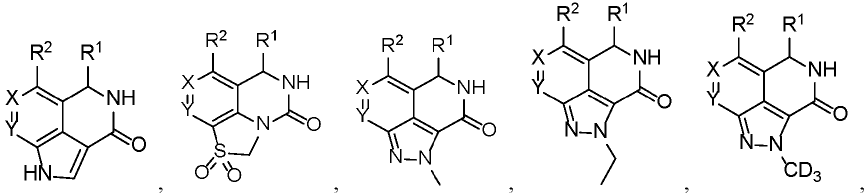

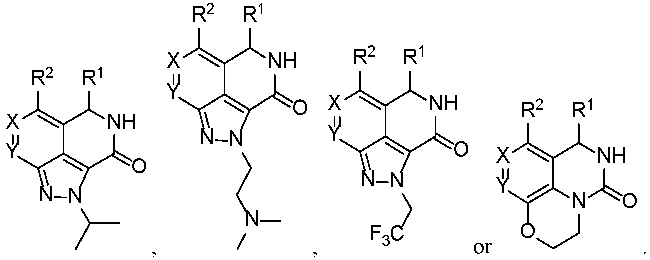

6. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 2, wherein the nitrogen-containing heterocyclic compound represented by formula II is defined as solution 1, solution 2 or solution 3:
solution 1:
E is N or C;
X is CH;
R¹ is-L¹-R^{1A}; L¹ is a bond, R^{1A} is C₆₋₂₀ aryl, C₆₋₂₀ aryl substituted with one or more R^{1C} or "5- to 12-membered heteroaryl containing 1 to 3 heteroatoms independently selected from O, S and N" substituted with one or more R^{1D}; R^{1C} and R^{1D} are independently halogen, C₁₋₆ alkyl, -COOH or C₁₋₆ alkyl substituted with halogen;
R² is -L²-R^{2A}; L² is a bond, -NH- or -NH-C(O)-, R^{2A} is 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{2C1}, C₆₋₂₀ aryl substituted with one or more R^{2C2} or C₈₋₁₀ benzoheterocycloalkenyl containing 1-3 heteroatoms independently selected from O, S and N substituted with one or more R^{2C3}; R^{2C1}, R^{2C2} and R^{2C3} are independently halogen, hydroxyl or C₁₋₆ alkyl substituted with one or more R^{2C11}; R^{2C11} is independently halogen;
R³ is -L³-R^{3A}; L³ is a bond, R^{3A} is C₁₋₆ alkyl or C₁₋₆ alkyl substituted with one or more R^{3C};
R^{3C} is independently deuterium, halogen or -NR^{3C1}R^{3C2};
R^{3C1} and R^{3C2} are independently hydrogen or C₁₋₆ alkyl;
R^{Y} is -L^{Y}-R^{YA}; L^{Y} is a bond, -NH- or -NH-C(O)-; R^{YA} is hydrogen, halogen, cyano, -N₃, hydroxyl, -NH₂, -C₂₋₆ alkynyl, 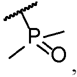 -B(OH)₂, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3}, 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} ,-COR ^{YA5} or 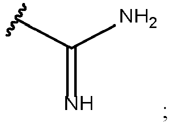
R^{YA1}, R^{YA2}, R^{YA3}, R^{YA4} and R^{YA5} are independently halogen, hydroxyl, cyano, -NH₂, C₁₋₆ alkyl, -OC₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
ring A is C₆₋₁₀ aromatic ring, 4- to 10- membered cyclic olefin containing 1-3 heteroatoms independently selected from O, S and N or 5- to 12- membered heteroaromatic ring containing 1-3 heteroatoms independently selected from O, S and N;
n is 0, 1 or 2;
solution 2:
E is N, CH or C;
X is N or CH;
R' is -L¹-R^{1A}; L¹ is a bond, R^{1A} is C₆₋₂₀ aryl or C₆₋₂₀ aryl substituted with one or more R^{1C}; R^{1C} is independently halogen, C₁₋₆ alkyl or C₁₋₆ alkyl substituted with halogen;
R² is -L²-R^{2A}; L² is a bond,-NH-,-NH-C(O)- or -NH-C(O)CHOH-, R^{2A} is 8- to 10-membered bicyclic heteroaryl containing 1-3 heteroatoms independently selected from O, S and N, 8- to 10- membered bicyclic heteroaryl containing 1-3 heteroatoms independently selected from O, S and N substituted with one or more R^{2C1}, C₆₋₂₀ aryl substituted with one or more R^{2C2} , C₈₋₁₀ benzoheterocycloalkenyl containing 1-3 heteroatoms independently selected from O, S and N substituted with one or more R^{2C3} or 5- to 6- membered heteroaryl containing 1-4 heteroatoms independently selected from O, S and N substituted with one or more R^{2C4}; R^{2C1}, R^{2C2}, R^{2C3} and R^{2C4} are independently halogen, hydroxyl, C₁₋₆ alkyl, C₃₋₈ cycloalkyl or C₁₋₆ alkyl substituted with one or more R^{2C11}; R^{2C11} is independently halogen or hydroxyl;
R³ is -L³-R^{3A}; L³ is a bond, R^{3A} is halogen or =O, or, two optional R³ together with the atom they are attached form C₃₋₈ cycloalkane or C₆₋₂₀ aromatic ring;
R^{Y} is -L^{Y}-R^{YA}; L^{Y} is a bond,-NH-or -NH-C(O)-; R^{YA} is hydrogen, halogen, cyano, -N₃, hydroxyl, -NH₂, 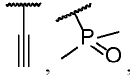 , -B(OH)₂, -COOH, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} , -COR ^{YA5}, 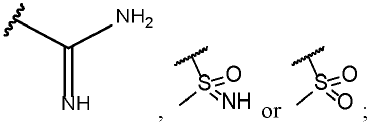
R^{YA1}, R^{YA2}, R^{YA3}, R^{YA4} and R^{YA5} are independently halogen, hydroxyl, cyano, -NH₂, C₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
ring A is C₆₋₁₀ aromatic ring, 4- to 10- membered cyclic olefin containing 1-3 heteroatoms independently selected from O, S and N, 5- to 12- membered heteroaromatic ring containing 1-3 heteroatoms independently selected from O, S and N or C₈₋₁₀ benzoheterocyclic olefin;
n is 0, 1 or 2;
solution 3:
E is N or C;
X is CH;
R¹ is -L¹-R^{1A}; L¹ is a bond, R^{1A} is C₆₋₂₀ aryl or C₆₋₂₀ aryl substituted with one or more R^{1C}; R^{1C} is independently halogen or C₁₋₆;
R² is -L²-R^{2A}; L² is a bond, -NH- or -NH-C(O)-, R^{2A} is 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{2C1}, C₆₋₂₀ aryl substituted with one or more R^{2C2} or C₈₋₁₀ benzoheterocycloalkenyl containing 1-3 heteroatoms independently selected from O, S and N substituted with one or more R^{2C3}; R^{2C1}, R^{2C2} and R^{2C3} are independently halogen, hydroxyl or C₁₋₆ alkyl substituted with one or more R^{2C11}; R^{2C11} is independently halogen;
n is 0;
R^{Y} is -L^{Y}-R^{YA}; L^{Y} is a bond,-NH-or -NH-C(O)-; R^{YA} is hydrogen, halogen, cyano, -N₃, hydroxyl, 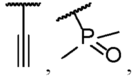 -B(OH)₂, -COOH, C₁₋₆ alkyl, C₁₋₆ alkyl substituted with one or more R^{YA1}, -OC₁₋₆ alkyl, C₃₋₈ cycloalkyl, 4- to 10- membered heterocycloalkyl containing 1-4 heteroatoms independently selected from O, S and N, 4- to 10- membered heterocycloalkyl containing 1-4 heteroatoms independently selected from O, S and N substituted with one or more R^{YA2}, 5- to 6- membered heteroaryl containing 1-4 heteroatoms independently selected from O, S and N , 5- to 6- membered heteroaryl containing 1-4 heteroatoms independently selected from O, S and N substituted with one or more R^{YA3} , 8- to 10- membered bicyclic heteroaryl containing 1-3 heteroatoms independently selected from O, S and N, 8- to 10-membered bicyclic heteroaryl containing 1-3 heteroatoms independently selected from O, S and N substituted with one or more R^{YA4} , -COR ^{YA5} or 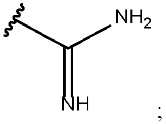
R^{YA1}, R^{YA2} , R^{YA3}, R^{YA4} and R^{YA5} are independently halogen, hydroxyl, cyano, -NH₂, C₁₋₆ alkyl, 4- to 10- membered heterocycloalkyl containing 1-4 heteroatoms independently selected from O, S and N, C₃₋₈ cycloalkyl, C₆₋₂₀ aryl or C₁₋₆ alkyl substituted with one or more R^{YA11}; R^{YA11} is independently halogen or phenyl;
ring A is C₆₋₁₀ aromatic ring, 4- to 10- membered cyclic olefin containing 1-3 heteroatoms independently selected from O, S and N or 5- to 12- membered heteroaromatic ring containing 1-3 heteroatoms independently selected from O, S and N.

7. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein the nitrogen-containing heterocyclic compound represented by formula I is any one of the following compounds:

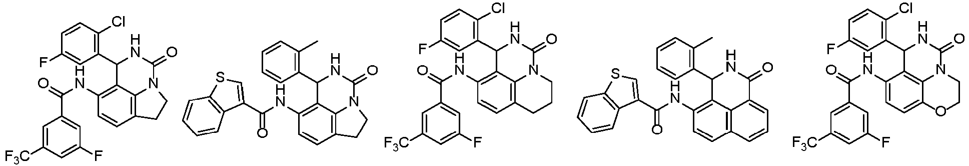

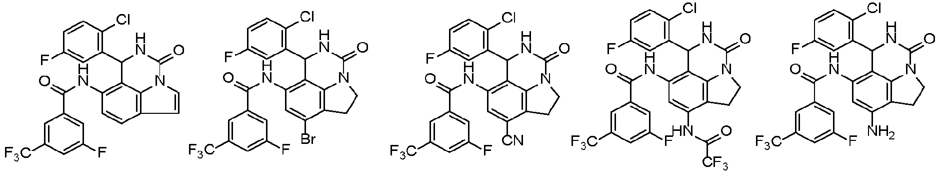

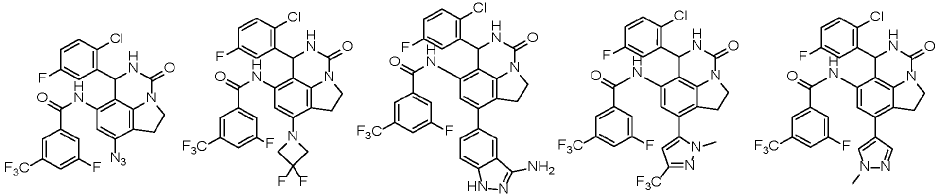

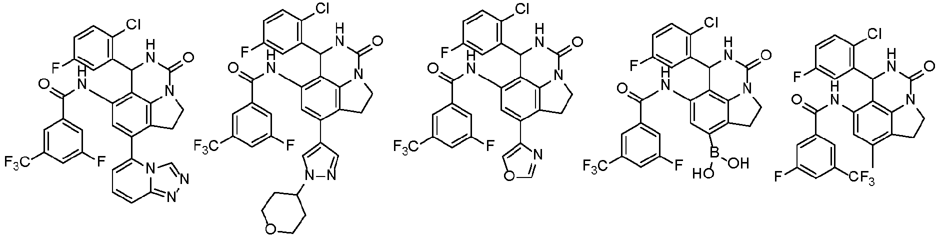

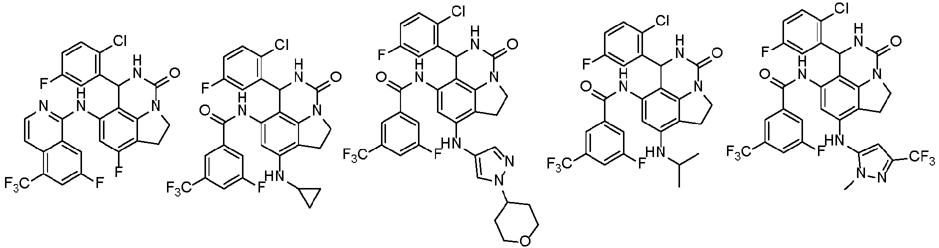

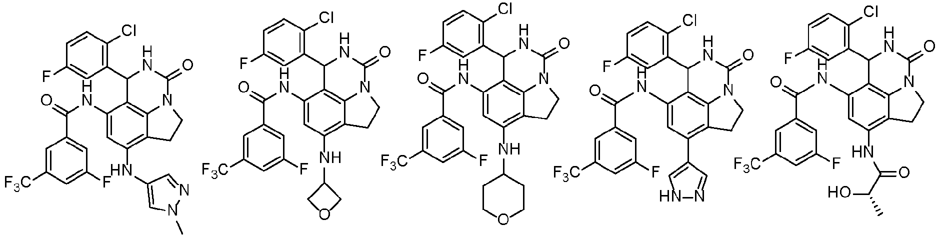

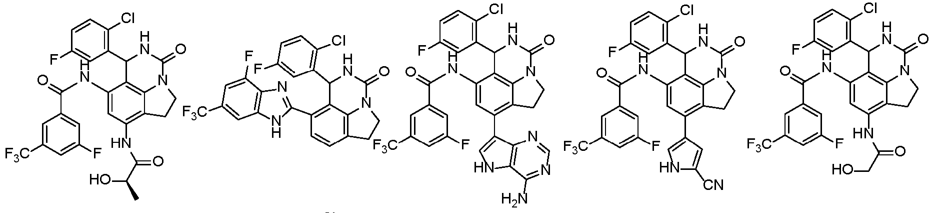

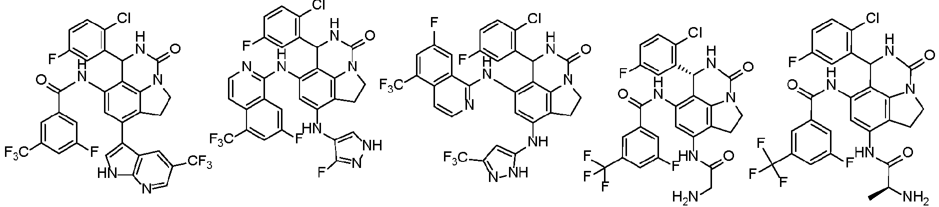

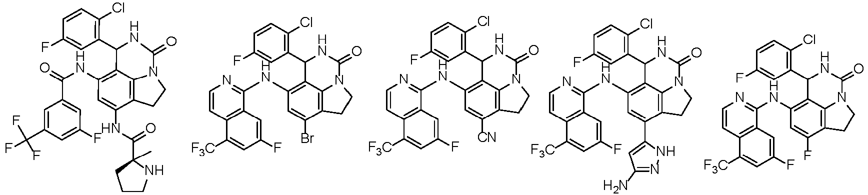

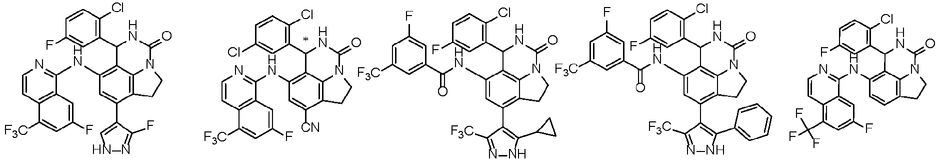

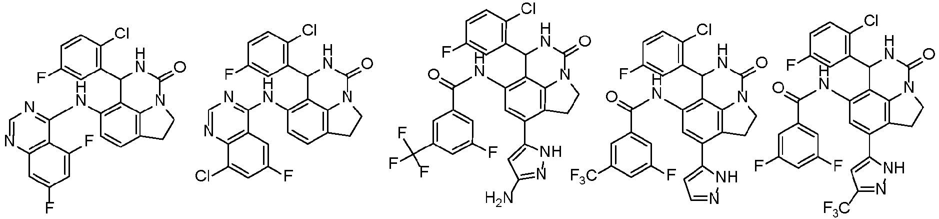

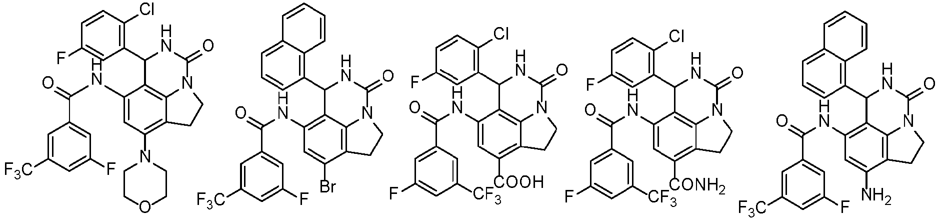

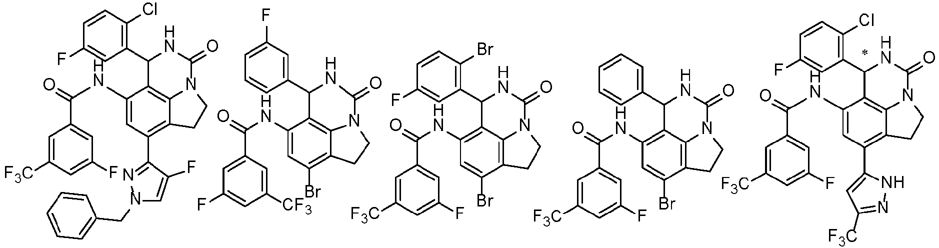

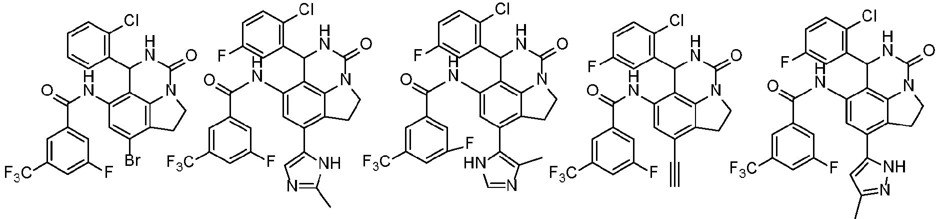

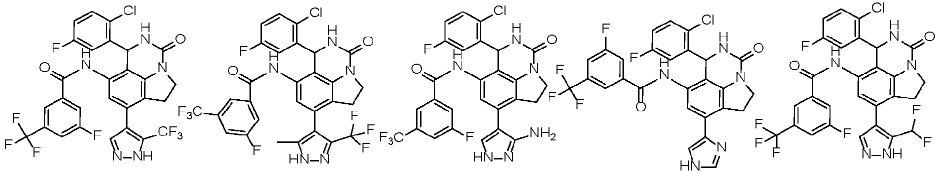

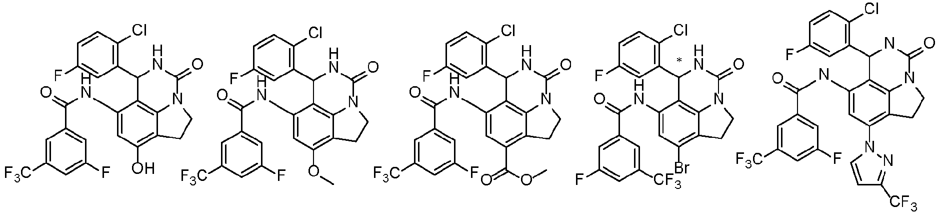

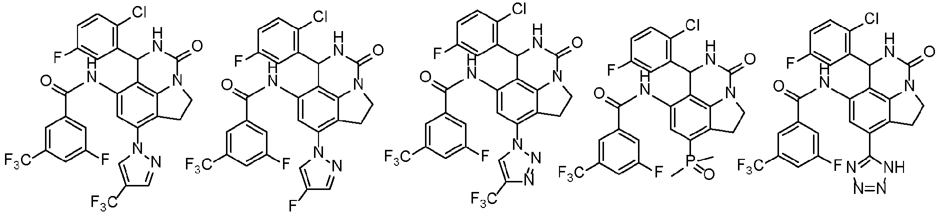

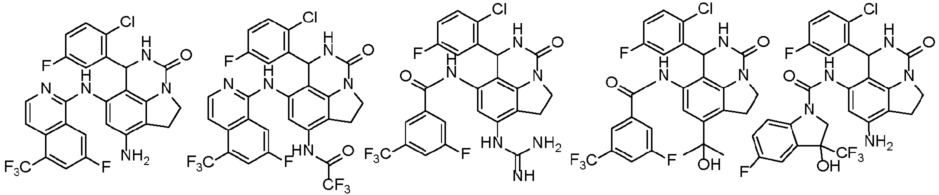

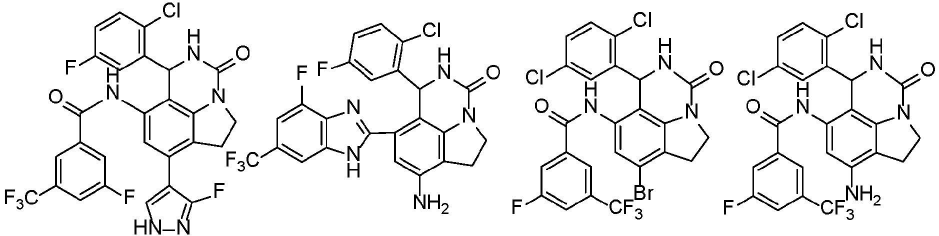

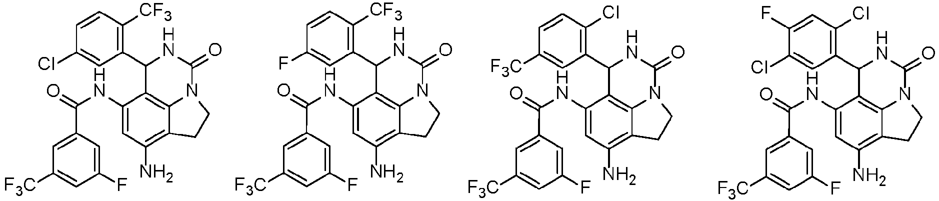

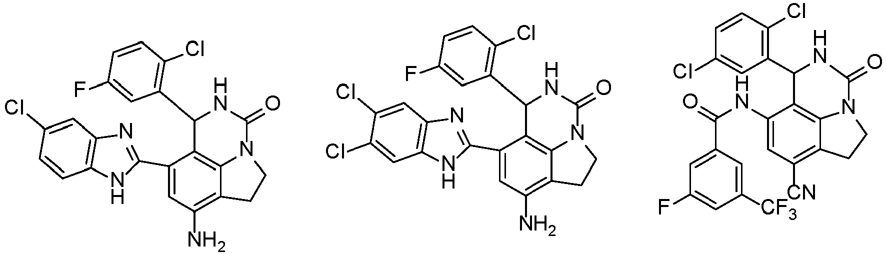

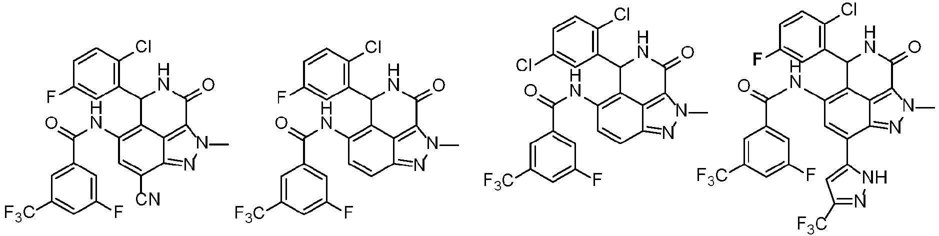

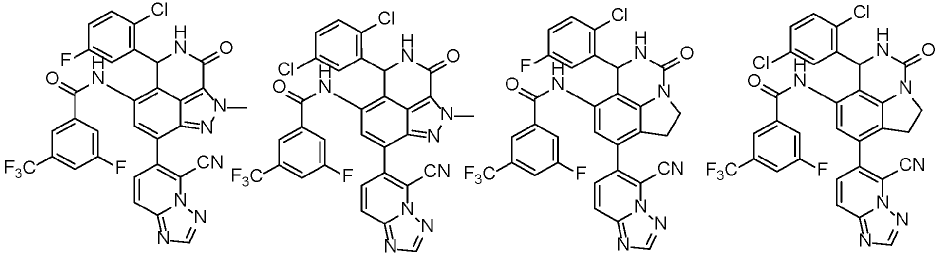

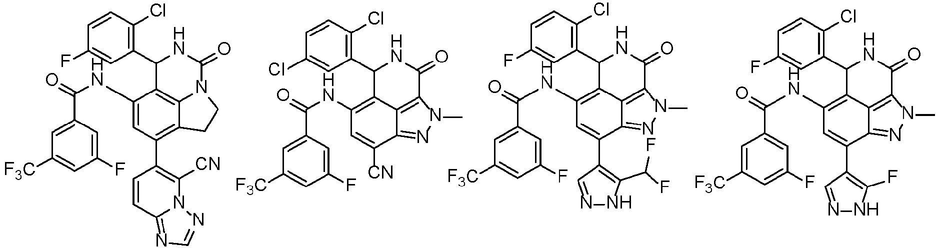

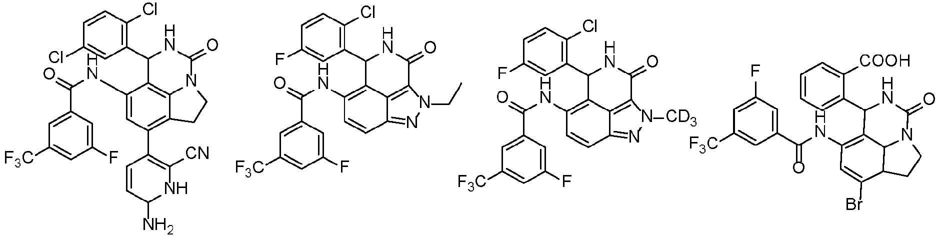

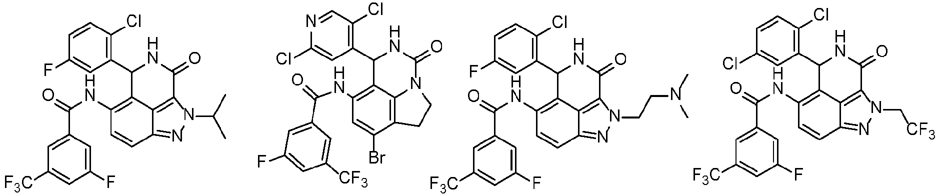

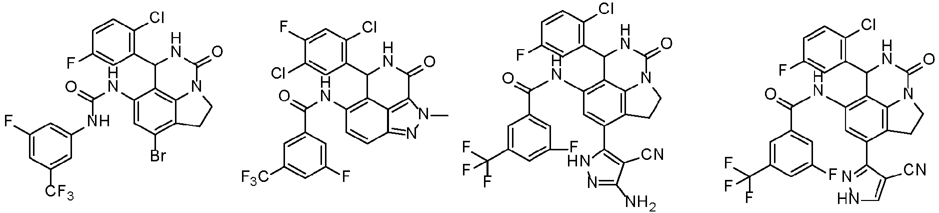

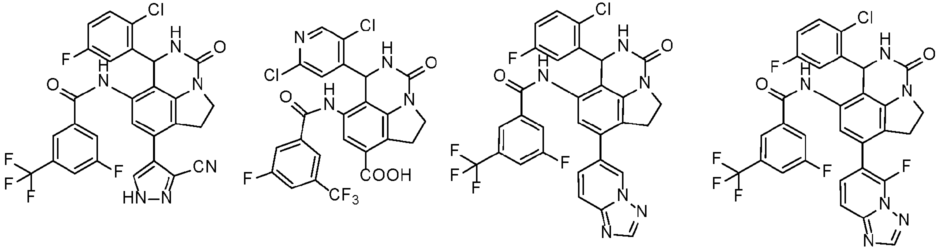

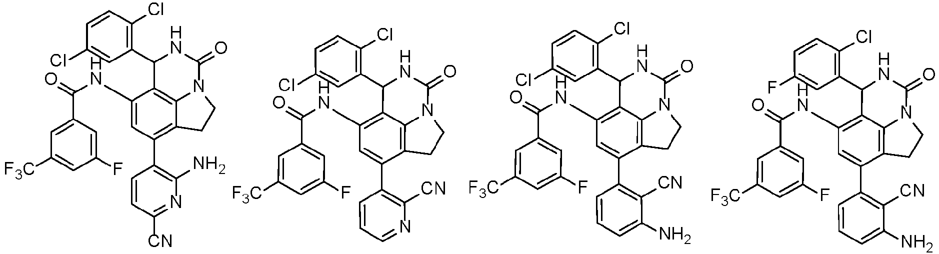

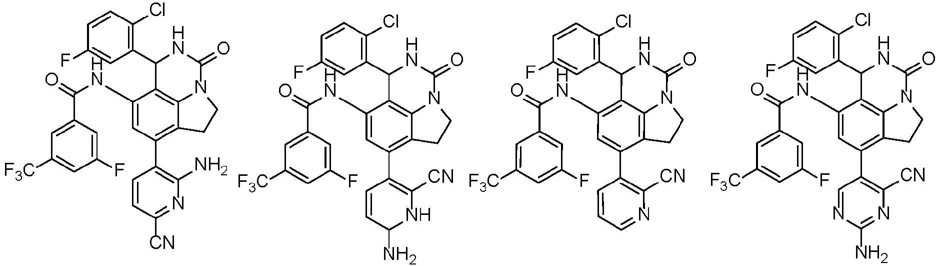

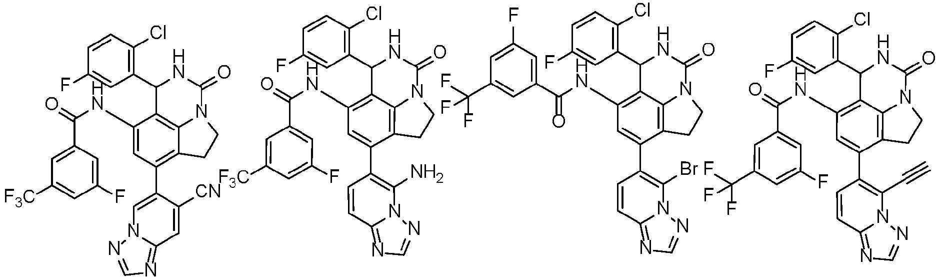

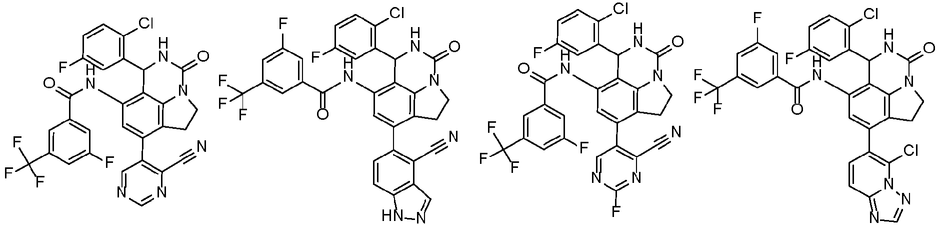

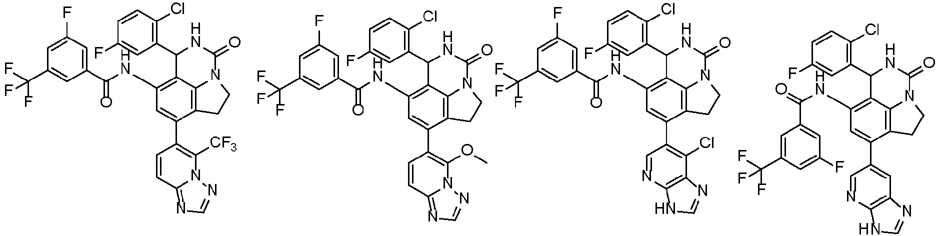

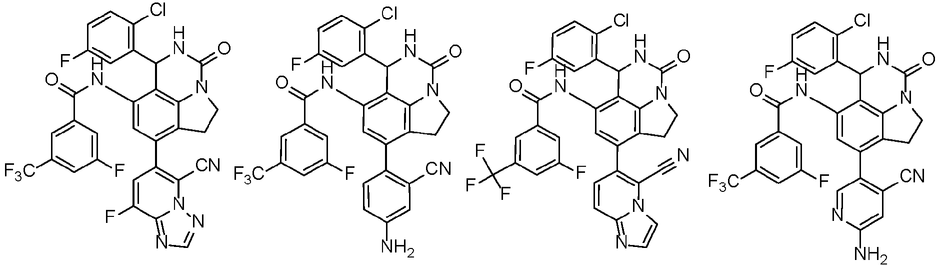

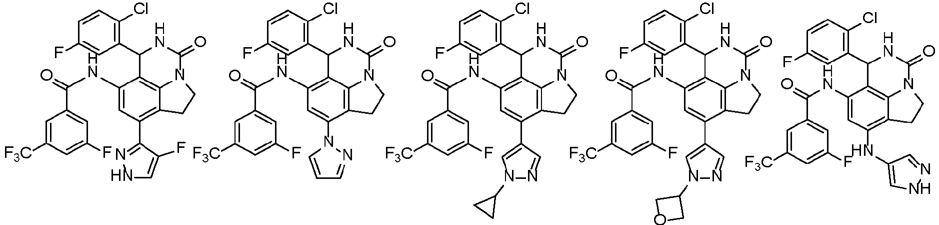

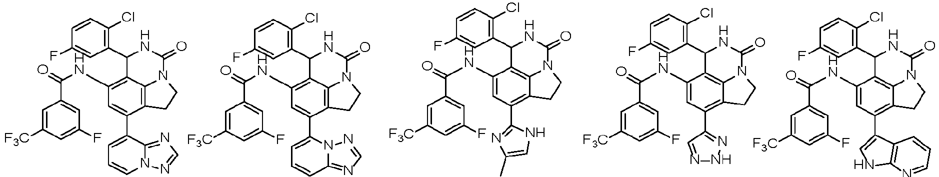

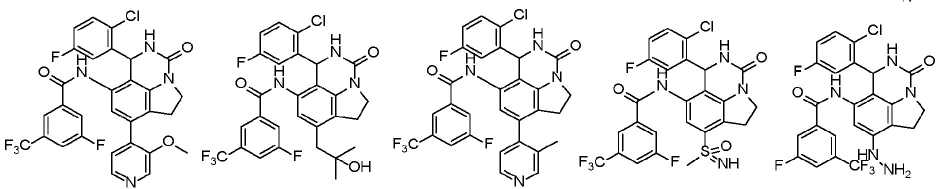

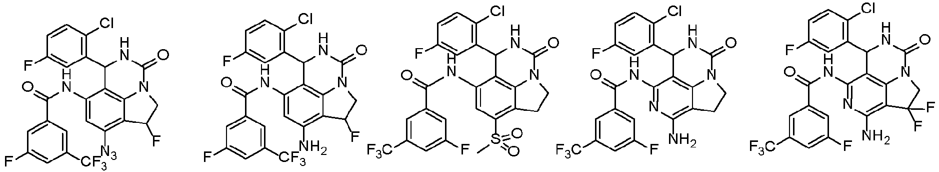

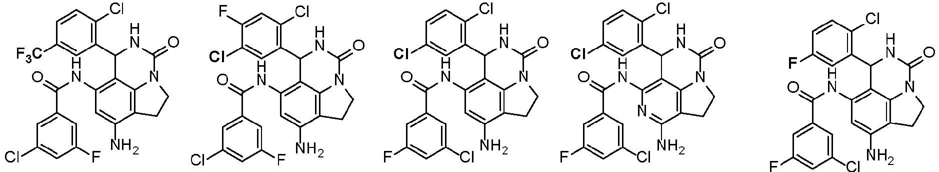

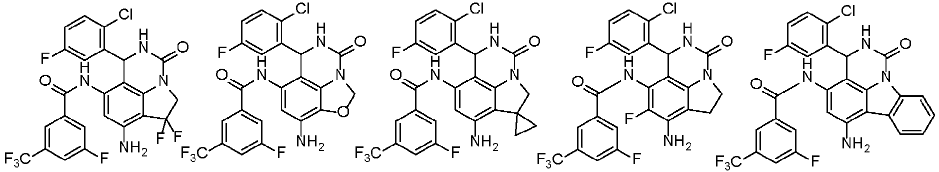

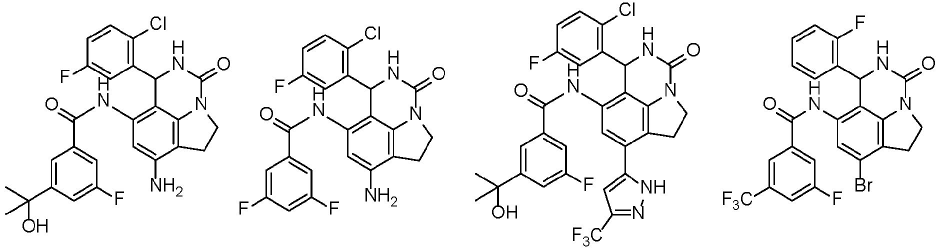

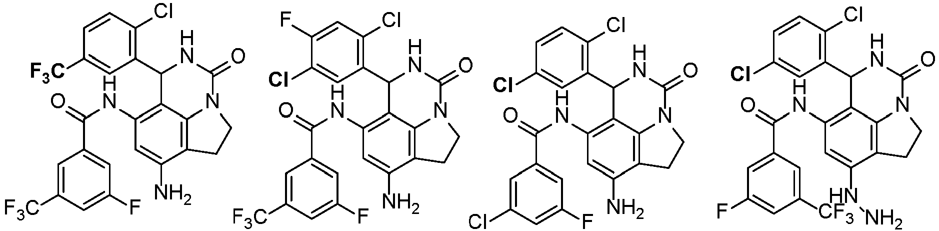

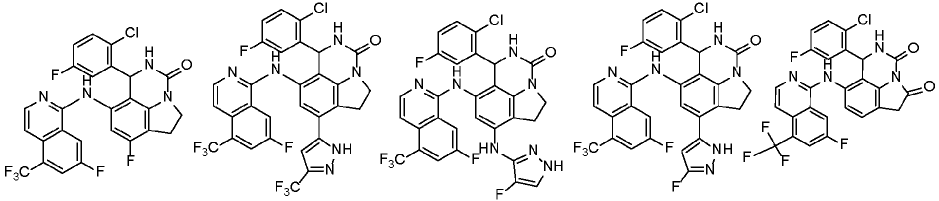

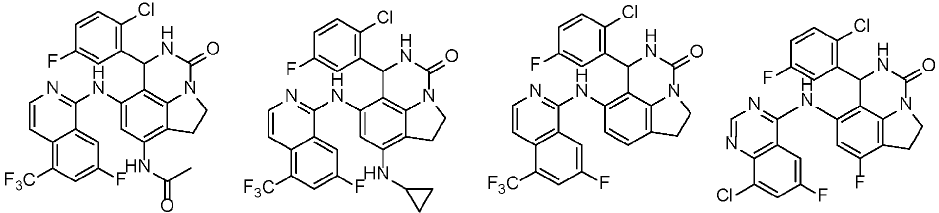

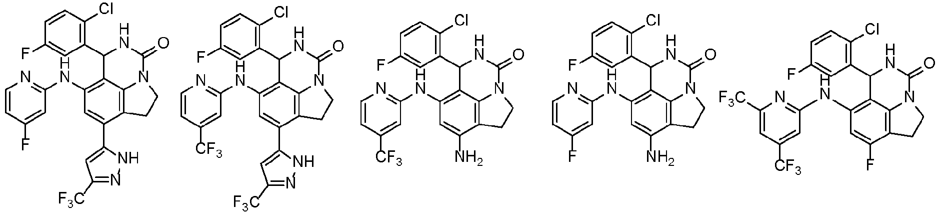

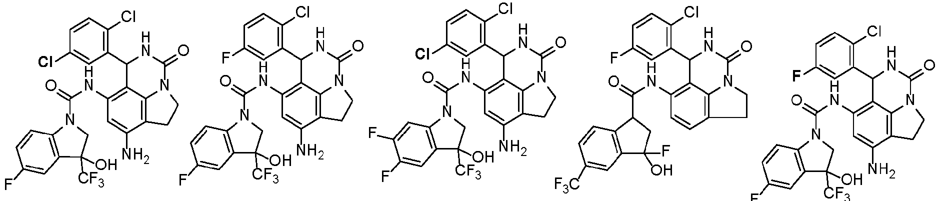

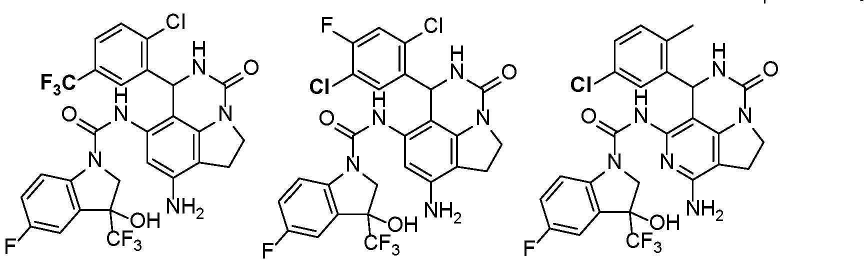

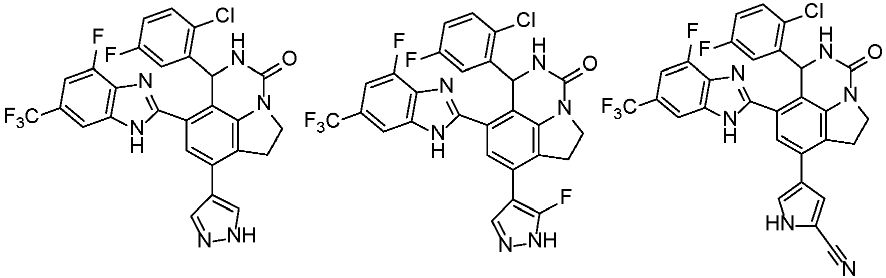

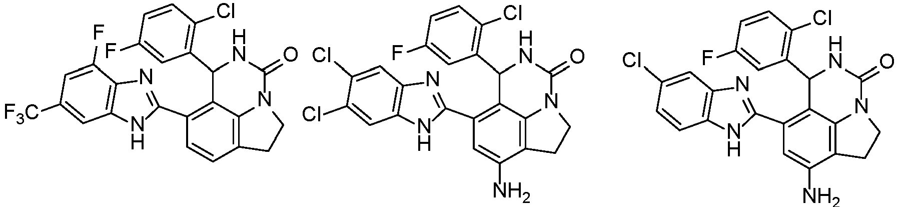

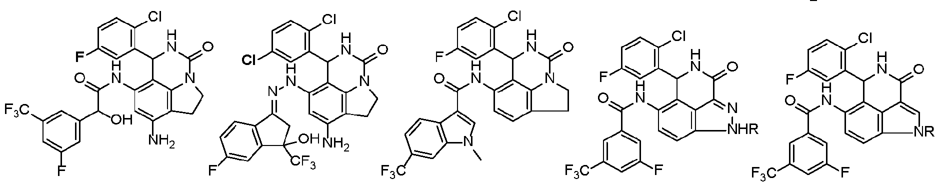

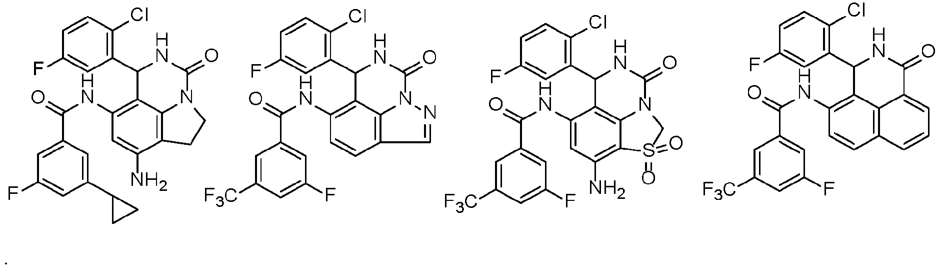

8. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein the nitrogen-containing heterocyclic compound represented by formula I is any one of the following compounds:
compound 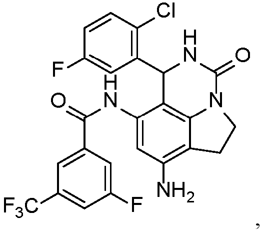 which has a retention time of 5.153 min under the following conditions: equipments: SFC-150 (Waters);chromatographic column: AS 20*250mm, 10um (Daicel); column temperature: 35 °C; mobile phase: CO2/MeOH[0.2%NH3(7M in MeOH) ] = 70/30; flow rate: 100 g/min; back pressure: 100 bar; wavelength: 214 nm;
compound 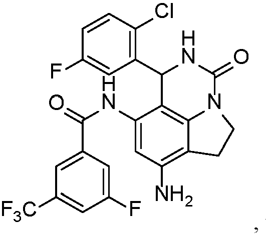 which has a retention time of 5.581min under the following conditions: equipments: SFC-150 (Waters);chromatographic column:AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar; wavelength: 214 nm;
compound 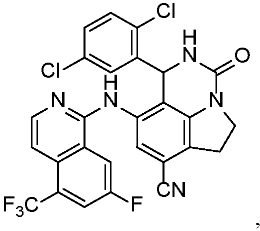 which has a retention time of 4.512 min under the following conditions: equipments: SFC-150 (Waters);chromatographic column: AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar ; wavelength: 214 nm;
compound 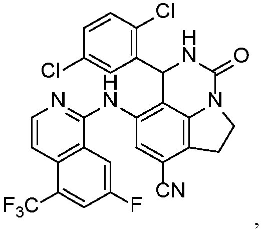 which has a retention time of 5.408min under the following conditions: equipments: SFC-150 (Waters);chromatographic column: AS 20*250mm,10um (Daicel); column temperature: 35 °C; mobile phase: CO₂/MeOH[0.2%NH₃(7M in MeOH) ] = 70/30; flow rate: 100 g/min ; back pressure: 100 bar; wavelength: 214 nm;
compound 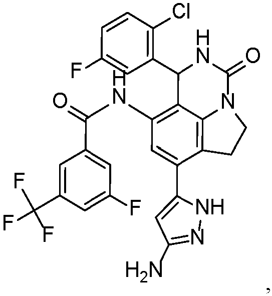 which has a retention time of 4.561min under the following conditions: equipments: SFC-150 (Waters);chromatographic column: AS 20*250mm, 10um (Daicel); column temperature:35 °C; mobile phase: CO₂/MeOH[0.2%NH₃(7M in MeOH) ] = 60/40; flow rate: 100 g/min; back pressure: 100 bar; wavelength: 214 nm;
compound 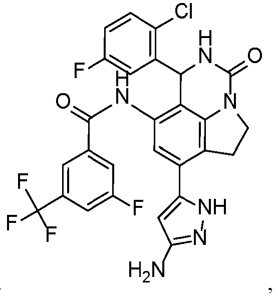 which has a retention time of 5.012min under the following conditions: equipments: SFC-150 (Waters);chromatographic column:AS 20*250mm,10um (Daicel); column temperature:35 °C; mobile phase: CO₂/MeOH[0.2%NH₃(7M in MeOH) ] = 60/40; flow rate: 100 g/min ; back pressure: 100 bar; wavelength: 214 nm;
compound 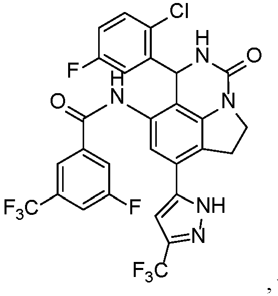 which has a retention time of 4.561min under the following conditions: equipments: SFC-150 (Waters);chromatographic column: AS 20*250mm,10um (Daicel); column temperature:35 °C; mobile phase: CO₂/MeOH[0.2%NH₃(7M in MeOH) ] = 70/30; flow rate: 100 g/min ; back pressure: 100 bar; wavelength: 214 nm;
compound 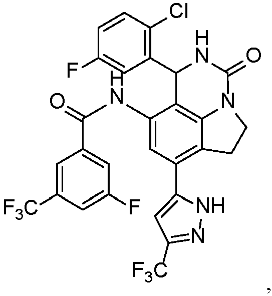 which has a retention time of 5.561min under the following conditions: equipments: SFC-150 (Waters);chromatographic column:AS 20*250mm,10um (Daicel); column temperature:35 °C; mobile phase: CO₂/MeOH[0.2%NH₃(7M in MeOH) ] = 70/30; flow rate: 100 g/min ; back pressure: 100 bar; wavelength: 214 nm;
compound 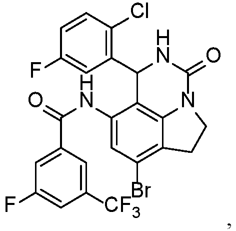 which has a retention time of 0.707min under the following conditions: equipments: SFC-150 (Waters);chromatographic column:OJ-H 4.6*100mm 5um; column temperature: 40 °C; mobile phase:CO₂/MeOH[0.2%NH₃(7M in MeOH) ]; flow rate: 3.0mL/min ; back pressure: 2000psi; wavelength: 214 nm;
compound 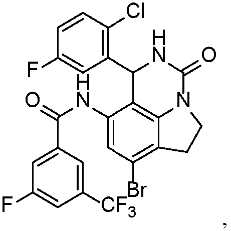 which has a retention time of 1.449min under the following conditions: equipments: SFC-150 (Waters);chromatographic column:OJ-H 4.6*100mm 5um; column temperature: 40 °C; mobile phase: CO₂/MeOH[0.2%NH₃(7M in MeOH) ]; flow rate: 3.0mL/min ; back pressure: 2000psi; wavelength: 214 nm;
compound 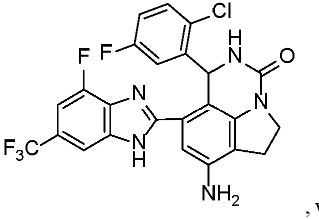 which has a retention time of 3.245min under the following conditions: equipments: SFC-150 (Waters);chromatographic column: OD 20*250mm, 10um (Daicel); column temperature: 35°C; mobile phase: CO₂/MeOH[0.2%NH₃(7M in MeOH) ] = 65/35; flow rate: 100.0mL/min; back pressure: 100bar; wavelength: 214 nm;
compound 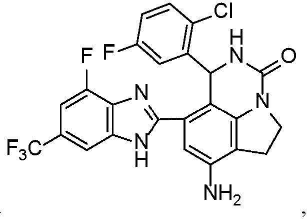 which has a retention time of 3.456min under the following conditions: equipments: SFC-150 (Waters);chromatographic column: OD 20*250mm,10um (Daicel); column temperature:35 °C; mobile phase: CO₂/MeOH[0.2%NH₃(7M in MeOH) ] = 65/35; flow rate: 100.0mL/min; back pressure: 100bar; wavelength: 214 nm;
compound 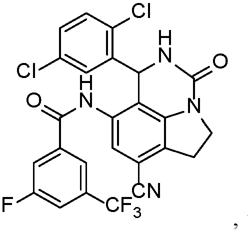 which has a retention time of 0.56 min under the following conditions: equipments: SFC-150 (Waters);chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 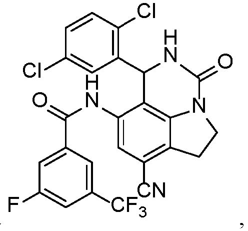 which has a retention time of 1.21min under the following conditions: equipments: SFC-150 (Waters);chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 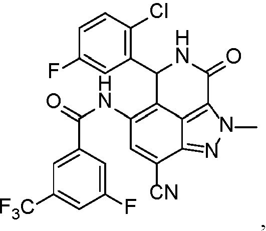 which has a retention time of 0.38min under the following conditions: equipments: SFC-150 (Waters);chromatographic column: AS 20*250mm, 10um (Daicel); column temperature: 35 °C; mobile phase: CO₂/MeOH[0.2%NH₃(7M in MeOH) ] = 45/55; flow rate: 120 g/min; back pressure:100bar; wavelength: 214 nm;
compound 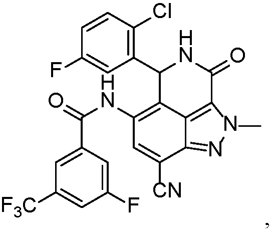 which has a retention time of 0.8min under the following conditions: equipments: SFC-150 (Waters);chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 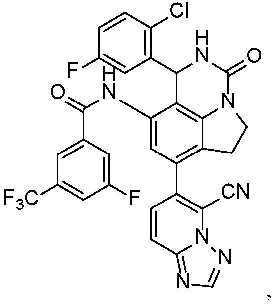 which has a retention time of 1.74min under the following conditions: equipments: SFC-150 (Waters);chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 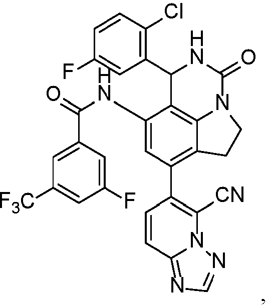 which has a retention time of 3.45min under the following conditions: equipments: SFC-150 (Waters);chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 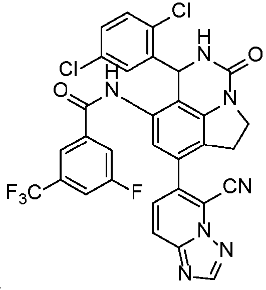 , which has a retention time of 1.08min under the following conditions: equipments: SFC-150 (Waters);chromatographic column: AS 20*250mm,10um (Daicel); column temperature: 35°C; mobile phase: CO₂/MeOH[0.2%NH₃(7M in MeOH) ] = 45/55; flow rate: 120 g/min; back pressure:100bar; wavelength: 214 nm;
compound 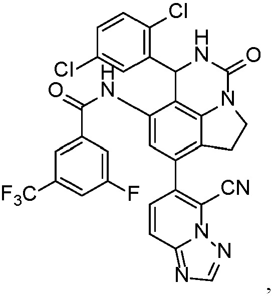 which has a retention time of 2.32min under the following conditions: equipments:SFC-150(Waters);chromatographic column: AS 20*250mm, 10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 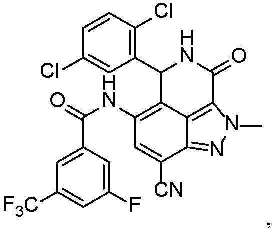 which has a retention time of 0.38min under the following conditions: equipments:SFC-150 (Waters);chromatographic column: AS 20*250mm,10um(Daicel); column temperature:35°C; mobile phase: CO₂/MeOH[0.2%NH₃(7M in MeOH) ] = 45/55; flow rate: 120 g/min; back pressure: 100 bar; wavelength: 214 nm;
compound 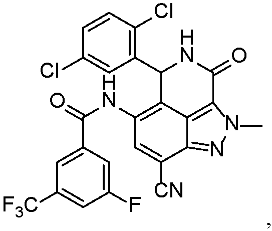 which has a retention time of 0.84min under the following conditions: equipments: SFC-150 (Waters);chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 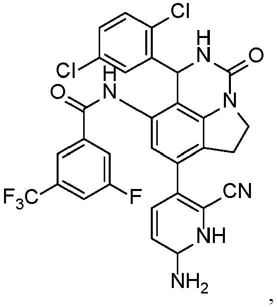 which has a retention time of 2.32min under the following conditions: equipments: SFC-150 (Waters); chromatographic column: AS 20*250mm,10um (Daicel); column temperature:35°C; mobile phase: CO₂/MeOH[0.2%NH₃(7M in MeOH) ] = 45/55; flow rate: 120 g/min; back pressure:100bar; wavelength: 214 nm;
compound 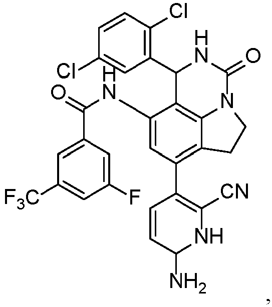 which has a retention time of 3.38min under the following conditions: equipments:SFC-150(Waters);chromatographic column: AS 20*250mm,10um (Daicel); column temperature: 35°C; mobile phase: CO₂/MeOH[0.2%NH₃(7M in MeOH) ] = 45/55; flow rate: 120 g/min; back pressure:100bar; wavelength: 214 nm;
compound 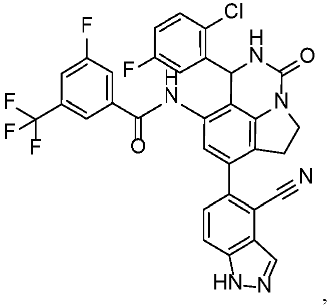 which has a retention time of 2.58min under the following conditions: equipments:SFC-150(Waters); chromatographic column: AS 20*250mm,10um (Daicel); column temperature: 35°C; mobile phase: CO₂/MeOH[0.2%NH₃(7M in MeOH) ] = 45/55; flow rate: 100 g/min; back pressure:100bar; wavelength: 214 nm;
compound 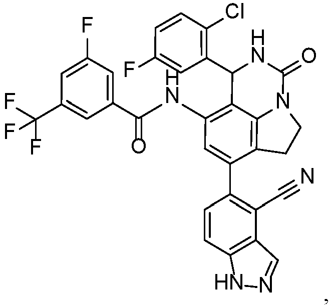 which has a retention time of 2.91min under the following conditions: equipments:SFC-150(Waters); chromatographic column: AS 20*250mm,10um (Daicel); column temperature: 35°C; mobile phase: CO₂/MeOH[0.2%NH₃(7M in MeOH) ] = 45/55; flow rate: 100 g/min; back pressure: 100bar; wavelength: 214 nm.

9. A nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to claim 1, wherein the nitrogen-containing heterocyclic compound represented by formula I is any one of the following compounds:

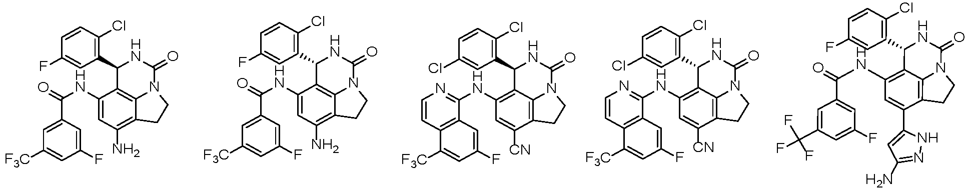

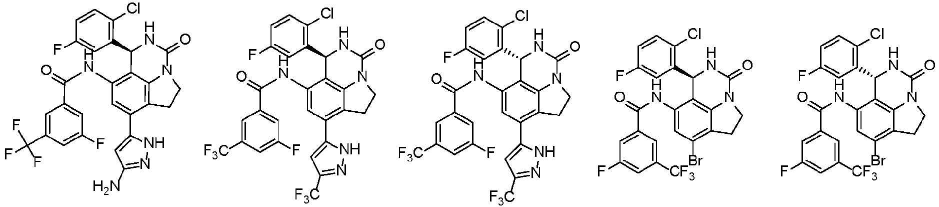

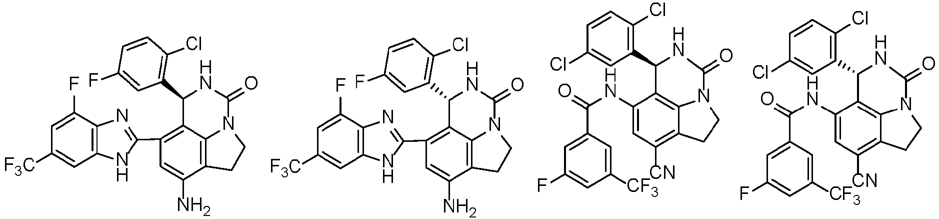

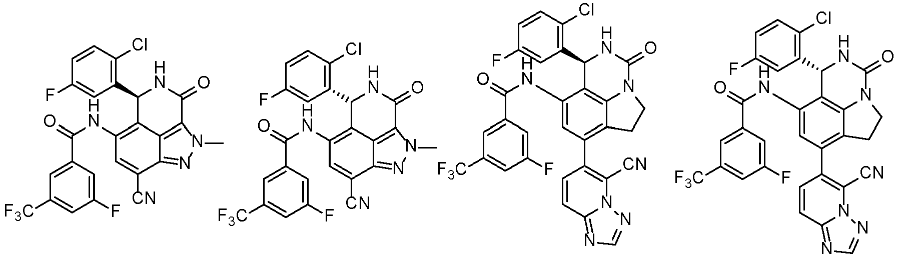

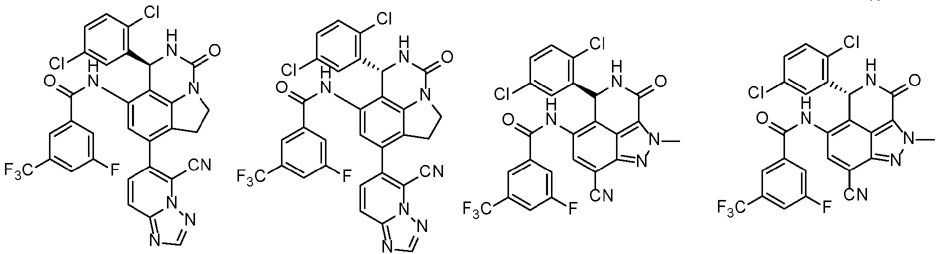

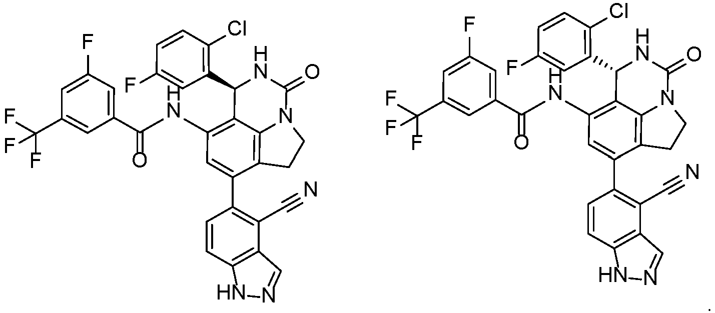

10. A pharmaceutical composition, comprising substance A and a pharmaceutical adjuvant; the substance A is the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to any one of claims 1-9.

11. Use of a substance A in preparation of a PI3K inhibitor, wherein the substance A is the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to any one of claims 1-9.

12. Use of a substance A in preparation of a medicament for treating or preventing an PI3K-mediated disease,
wherein the substance A is the nitrogen-containing heterocyclic compound represented by formula I, a pharmaceutically acceptable salt thereof, a solvate thereof, a solvate of pharmaceutically acceptable salt thereof, a crystal thereof, a stereoisomer thereof, a tautomer thereof or an isotopically labeled compound thereof according to any one of claims 1-9.
